# EUROPEAN PATENT APPLICATION

(11) **EP 3 178 312 A1**
(43) Date of publication of application: **14.06.2017**
(21) Application number: 16202167.9
(22) Date of filing: 01.09.2010
(51) Int. Cl.: A01H 5/00, C12Q 1/04, C12N 15/82, C12N 15/52

(54) **HERBICIDE-TOLERANT PLANTS**

(30) Priority: 01.09.2009 US 238906 P; 16.07.2010 US 365298 P
(62) Divisional of application: 10814446.0
(71) Applicant: BASF Agrochemical Products, B.V., 6835 EA Arnhem (NL)
(72) Inventor: MANKIN, Scots, L., Raleigh, NC 27612 (US); SCHOFL, Ulrich, Apex, NC 27523 (US); HONG, Haiping, Cary, NC 27519 (US); WENCK, Allan, R., Durham, NC 27705 (US); NEUTEBOOM, Leon, Youngsville, NC 27596 (US); WHITT, Sherry, R., Raleigh, NC 27614 (US); CARLSON, Dale, R., Apex, NC 27502 (US)
(74) Representative: Reitstötter Kinzebach

(57) **Abstract**

The present invention provides herbicide-tolerant plants. The present invention also provides methods for controlling the growth of weeds by applying an herbicide to which herbicide-tolerant plants of the invention are tolerant. Plants of the invention may express an acetyl-Coenzyme A carboxylase enzyme that is tolerant to the action of acetyl-Coenzyme A carboxylase enzyme inhibitors.

SEQUENCE LISTING

<110> BASF AGROCHEMICAL PRODUCTS, B.V.

<120> HERBICIDE-TOLERANT PLANTS

<130> M/53106-EP-DIV1

<140>
<141>

<150> EP108144446.0
<151> 2010-09-01

<150> PCT/US2010/047571
<151> 2010-09-01

<150> US61/365,298
<151> 2010-07-16

<150> US61/238,906
<151> 2009-09-01

<160> 26

<170> PatentIn version 3.5

<210> 1
<211> 2320
<212> PRT
<213> Alopecurus myosuroides

<400> 1
Met Gly Ser Thr His Leu Pro Ile Val Gly Phe Asn Ala Ser Thr Thr
1               5                   10                  15

Pro Ser Leu Ser Thr Leu Arg Gln Ile Asn Ser Ala Ala Ala Ala Phe
            20                  25                  30

Gln Ser Ser Ser Pro Ser Arg Ser Ser Lys Lys Lys Ser Arg Arg Val
        35                  40                  45

Lys Ser Ile Arg Asp Asp Gly Asp Gly Ser Val Pro Asp Pro Ala Gly
    50                  55                  60

His Gly Gln Ser Ile Arg Gln Gly Leu Ala Gly Ile Ile Asp Leu Pro
65                  70                  75                  80

Lys Glu Gly Ala Ser Ala Pro Asp Val Asp Ile Ser His Gly Ser Glu
                85                  90                  95

Asp His Lys Ala Ser Tyr Gln Met Asn Gly Ile Leu Asn Glu Ser His
            100                 105                 110

Asn Gly Arg His Ala Ser Leu Ser Lys Val Tyr Glu Phe Cys Thr Glu
        115                 120                 125

Leu Gly Gly Lys Thr Pro Ile His Ser Val Leu Val Ala Asn Asn Gly
    130                 135                 140

Met Ala Ala Ala Lys Phe Met Arg Ser Val Arg Thr Trp Ala Asn Asp
145                 150                 155                 160

Thr Phe Gly Ser Glu Lys Ala Ile Gln Leu Ile Ala Met Ala Thr Pro
                165                 170                 175

Glu Asp Met Arg Ile Asn Ala Glu His Ile Arg Ile Ala Asp Gln Phe
            180                 185                 190

Val Glu Val Pro Gly Gly Thr Asn Asn Asn Asn Tyr Ala Asn Val Gln
        195                 200                 205

Leu Ile Val Glu Ile Ala Glu Arg Thr Gly Val Ser Ala Val Trp Pro
    210                 215                 220

Gly Trp Gly His Ala Ser Glu Asn Pro Glu Leu Pro Asp Ala Leu Thr
225                 230                 235                 240

Ala Lys Gly Ile Val Phe Leu Gly Pro Pro Ala Ser Ser Met Asn Ala
                245                 250                 255

Leu Gly Asp Lys Val Gly Ser Ala Leu Ile Ala Gln Ala Ala Gly Val
            260                 265                 270

Pro Thr Leu Ala Trp Ser Gly Ser His Val Glu Ile Pro Leu Glu Leu
        275                 280                 285

Cys Leu Asp Ser Ile Pro Glu Glu Met Tyr Arg Lys Ala Cys Val Thr
    290                 295                 300

Thr Ala Asp Glu Ala Val Ala Ser Cys Gln Met Ile Gly Tyr Pro Ala
305                 310                 315                 320

Met Ile Lys Ala Ser Trp Gly Gly Gly Gly Lys Gly Ile Arg Lys Val
                325                 330                 335

Asn Asn Asp Asp Glu Val Lys Ala Leu Phe Lys Gln Val Gln Gly Glu
            340                 345                 350

Val Pro Gly Ser Pro Ile Phe Ile Met Arg Leu Ala Ser Gln Ser Arg
        355                 360                 365

His Leu Glu Val Gln Leu Leu Cys Asp Glu Tyr Gly Asn Val Ala Ala

370                 375                 380

Leu His Ser Arg Asp Cys Ser Val Gln Arg Arg His Gln Lys Ile Ile
385                 390                 395                 400

Glu Glu Gly Pro Val Thr Val Ala Pro Arg Glu Thr Val Lys Glu Leu
                405                 410                 415

Glu Gln Ala Ala Arg Arg Leu Ala Lys Ala Val Gly Tyr Val Gly Ala
            420                 425                 430

Ala Thr Val Glu Tyr Leu Tyr Ser Met Glu Thr Gly Glu Tyr Tyr Phe
        435                 440                 445

Leu Glu Leu Asn Pro Arg Leu Gln Val Glu His Pro Val Thr Glu Ser
    450                 455                 460

Ile Ala Glu Val Asn Leu Pro Ala Ala Gln Val Ala Val Gly Met Gly
465                 470                 475                 480

Ile Pro Leu Trp Gln Ile Pro Glu Ile Arg Arg Phe Tyr Gly Met Asp
                485                 490                 495

Asn Gly Gly Gly Tyr Asp Ile Trp Arg Lys Thr Ala Ala Leu Ala Thr
            500                 505                 510

Pro Phe Asn Phe Asp Glu Val Asp Ser Gln Trp Pro Lys Gly His Cys
        515                 520                 525

Val Ala Val Arg Ile Thr Ser Glu Asn Pro Asp Asp Gly Phe Lys Pro
    530                 535                 540

Thr Gly Gly Lys Val Lys Glu Ile Ser Phe Lys Ser Lys Pro Asn Val
545                 550                 555                 560

Trp Gly Tyr Phe Ser Val Lys Ser Gly Gly Gly Ile His Glu Phe Ala
                565                 570                 575

Asp Ser Gln Phe Gly His Val Phe Ala Tyr Gly Glu Thr Arg Ser Ala
            580                 585                 590

Ala Ile Thr Ser Met Ser Leu Ala Leu Lys Glu Ile Gln Ile Arg Gly
        595                 600                 605

Glu Ile His Thr Asn Val Asp Tyr Thr Val Asp Leu Leu Asn Ala Pro
    610                 615                 620

Asp Phe Arg Glu Asn Thr Ile His Thr Gly Trp Leu Asp Thr Arg Ile
625                 630                 635                 640

Ala Met Arg Val Gln Ala Glu Arg Pro Pro Trp Tyr Ile Ser Val Val
                645                 650                 655

Gly Gly Ala Leu Tyr Lys Thr Ile Thr Thr Asn Ala Glu Thr Val Ser
            660                 665                 670

Glu Tyr Val Ser Tyr Leu Ile Lys Gly Gln Ile Pro Pro Lys His Ile
        675                 680                 685

Ser Leu Val His Ser Thr Ile Ser Leu Asn Ile Glu Glu Ser Lys Tyr
    690                 695                 700

Thr Ile Glu Ile Val Arg Ser Gly Gln Gly Ser Tyr Arg Leu Arg Leu
705                 710                 715                 720

Asn Gly Ser Leu Ile Glu Ala Asn Val Gln Thr Leu Cys Asp Gly Gly
                725                 730                 735

Leu Leu Met Gln Leu Asp Gly Asn Ser His Val Ile Tyr Ala Glu Glu
            740                 745                 750

Glu Ala Gly Gly Thr Arg Leu Leu Ile Asp Gly Lys Thr Cys Leu Leu
        755                 760                 765

Gln Asn Asp His Asp Pro Ser Arg Leu Leu Ala Glu Thr Pro Cys Lys
    770                 775                 780

Leu Leu Arg Phe Leu Ile Ala Asp Gly Ala His Val Asp Ala Asp Val
785                 790                 795                 800

Pro Tyr Ala Glu Val Glu Val Met Lys Met Cys Met Pro Leu Leu Ser
                805                 810                 815

Pro Ala Ala Gly Val Ile Asn Val Leu Leu Ser Glu Gly Gln Ala Met
            820                 825                 830

Gln Ala Gly Asp Leu Ile Ala Arg Leu Asp Leu Asp Asp Pro Ser Ala
        835                 840                 845

Val Lys Arg Ala Glu Pro Phe Glu Gly Ser Phe Pro Glu Met Ser Leu
    850                 855                 860

Pro Ile Ala Ala Ser Gly Gln Val His Lys Arg Cys Ala Ala Ser Leu
865                 870                 875                 880

Asn Ala Ala Arg Met Val Leu Ala Gly Tyr Asp His Ala Ala Asn Lys
                885                 890                 895

Val Val Gln Asp Leu Val Trp Cys Leu Asp Thr Pro Ala Leu Pro Phe
            900                 905                 910

Leu Gln Trp Glu Glu Leu Met Ser Val Leu Ala Thr Arg Leu Pro Arg
        915                 920                 925

Arg Leu Lys Ser Glu Leu Glu Gly Lys Tyr Asn Glu Tyr Lys Leu Asn
    930                 935                 940

Val Asp His Val Lys Ile Lys Asp Phe Pro Thr Glu Met Leu Arg Glu
945                 950                 955                 960

Thr Ile Glu Glu Asn Leu Ala Cys Val Ser Glu Lys Glu Met Val Thr
                965                 970                 975

Ile Glu Arg Leu Val Asp Pro Leu Met Ser Leu Leu Lys Ser Tyr Glu
            980                 985                 990

Gly Gly Arg Glu Ser His Ala His Phe Ile Val Lys Ser Leu Phe Glu
        995                 1000                1005

Glu Tyr Leu Ser Val Glu Glu Leu Phe Ser Asp Gly Ile Gln Ser
    1010                1015                1020

Asp Val Ile Glu Arg Leu Arg Leu Gln Tyr Ser Lys Asp Leu Gln
    1025                1030                1035

Lys Val Val Asp Ile Val Leu Ser His Gln Gly Val Arg Asn Lys
    1040                1045                1050

Thr Lys Leu Ile Leu Ala Leu Met Glu Lys Leu Val Tyr Pro Asn
    1055                1060                1065

Pro Ala Ala Tyr Arg Asp Gln Leu Ile Arg Phe Ser Ser Leu Asn
    1070                1075                1080

His Lys Arg Tyr Tyr Lys Leu Ala Leu Lys Ala Ser Glu Leu Leu
    1085                1090                1095

Glu Gln Thr Lys Leu Ser Glu Leu Arg Thr Ser Ile Ala Arg Asn
    1100                1105                1110

Leu Ser Ala Leu Asp Met Phe Thr Glu Glu Lys Ala Asp Phe Ser
    1115                1120                1125

Leu Gln Asp Arg Lys Leu Ala Ile Asn Glu Ser Met Gly Asp Leu
    1130                1135                1140

Val Thr Ala Pro Leu Pro Val Glu Asp Ala Leu Val Ser Leu Phe
    1145                1150                1155

Asp Cys Thr Asp Gln Thr Leu Gln Gln Arg Val Ile Gln Thr Tyr
    1160                1165                1170

Ile Ser Arg Leu Tyr Gln Pro Gln Leu Val Lys Asp Ser Ile Gln
    1175                1180                1185

Leu Lys Tyr Gln Asp Ser Gly Val Ile Ala Leu Trp Glu Phe Thr
    1190                1195                1200

Glu Gly Asn His Glu Lys Arg Leu Gly Ala Met Val Ile Leu Lys
    1205                1210                1215

Ser Leu Glu Ser Val Ser Thr Ala Ile Gly Ala Ala Leu Lys Asp
    1220                1225                1230

Ala Ser His Tyr Ala Ser Ser Ala Gly Asn Thr Val His Ile Ala
    1235                1240                1245

Leu Leu Asp Ala Asp Thr Gln Leu Asn Thr Thr Glu Asp Ser Gly
    1250                1255                1260

Asp Asn Asp Gln Ala Gln Asp Lys Met Asp Lys Leu Ser Phe Val
    1265                1270                1275

Leu Lys Gln Asp Val Val Met Ala Asp Leu Arg Ala Ala Asp Val
    1280                1285                1290

Lys Val Val Ser Cys Ile Val Gln Arg Asp Gly Ala Ile Met Pro
    1295                1300                1305

Met Arg Arg Thr Phe Leu Leu Ser Glu Glu Lys Leu Cys Tyr Glu
    1310                1315                1320

Glu Glu Pro Ile Leu Arg His Val Glu Pro Pro Leu Ser Ala Leu
    1325                1330                1335

Leu Glu Leu Asp Lys Leu Lys Val Lys Gly Tyr Asn Glu Met Lys
    1340                1345                1350

Tyr Thr Pro Ser Arg Asp Arg Gln Trp His Ile Tyr Thr Leu Arg

1355                1360                1365

Asn Thr Glu Asn Pro Lys Met Leu His Arg Val Phe Phe Arg Thr
    1370                1375                1380

Leu Val Arg Gln Pro Ser Ala Gly Asn Arg Phe Thr Ser Asp His
    1385                1390                1395

Ile Thr Asp Val Glu Val Gly His Ala Glu Glu Pro Leu Ser Phe
    1400                1405                1410

Thr Ser Ser Ser Ile Leu Lys Ser Leu Lys Ile Ala Lys Glu Glu
    1415                1420                1425

Leu Glu Leu His Ala Ile Arg Thr Gly His Ser His Met Tyr Leu
    1430                1435                1440

Cys Ile Leu Lys Glu Gln Lys Leu Leu Asp Leu Val Pro Val Ser
    1445                1450                1455

Gly Asn Thr Val Val Asp Val Gly Gln Asp Glu Ala Thr Ala Cys
    1460                1465                1470

Ser Leu Leu Lys Glu Met Ala Leu Lys Ile His Glu Leu Val Gly
    1475                1480                1485

Ala Arg Met His His Leu Ser Val Cys Gln Trp Glu Val Lys Leu
    1490                1495                1500

Lys Leu Val Ser Asp Gly Pro Ala Ser Gly Ser Trp Arg Val Val
    1505                1510                1515

Thr Thr Asn Val Thr Gly His Thr Cys Thr Val Asp Ile Tyr Arg
    1520                1525                1530

Glu Val Glu Asp Thr Glu Ser Gln Lys Leu Val Tyr His Ser Thr
    1535                1540                1545

Ala Leu Ser Ser Gly Pro Leu His Gly Val Ala Leu Asn Thr Ser
    1550                1555                1560

Tyr Gln Pro Leu Ser Val Ile Asp Leu Lys Arg Cys Ser Ala Arg
    1565                1570                1575

Asn Asn Lys Thr Thr Tyr Cys Tyr Asp Phe Pro Leu Thr Phe Glu
    1580                1585                1590

Ala Ala Val Gln Lys Ser Trp Ser Asn Ile Ser Ser Glu Asn Asn
    1595                1600                1605

Gln Cys Tyr Val Lys Ala Thr Glu Leu Val Phe Ala Glu Lys Asn
    1610                1615                1620

Gly Ser Trp Gly Thr Pro Ile Ile Pro Met Gln Arg Ala Ala Gly
    1625                1630                1635

Leu Asn Asp Ile Gly Met Val Ala Trp Ile Leu Asp Met Ser Thr
    1640                1645                1650

Pro Glu Phe Pro Ser Gly Arg Gln Ile Ile Val Ile Ala Asn Asp
    1655                1660                1665

Ile Thr Phe Arg Ala Gly Ser Phe Gly Pro Arg Glu Asp Ala Phe
    1670                1675                1680

Phe Glu Ala Val Thr Asn Leu Ala Cys Glu Lys Lys Leu Pro Leu
    1685                1690                1695

Ile Tyr Leu Ala Ala Asn Ser Gly Ala Arg Ile Gly Ile Ala Asp
    1700                1705                1710

Glu Val Lys Ser Cys Phe Arg Val Gly Trp Thr Asp Asp Ser Ser
    1715                1720                1725

Pro Glu Arg Gly Phe Arg Tyr Ile Tyr Met Thr Asp Glu Asp His
    1730                1735                1740

Asp Arg Ile Gly Ser Ser Val Ile Ala His Lys Met Gln Leu Asp
    1745                1750                1755

Ser Gly Glu Ile Arg Trp Val Ile Asp Ser Val Val Gly Lys Glu
    1760                1765                1770

Asp Gly Leu Gly Val Glu Asn Ile His Gly Ser Ala Ala Ile Ala
    1775                1780                1785

Ser Ala Tyr Ser Arg Ala Tyr Glu Glu Thr Phe Thr Leu Thr Phe
    1790                1795                1800

Val Thr Gly Arg Thr Val Gly Ile Gly Ala Tyr Leu Ala Arg Leu
    1805                1810                1815

Gly Ile Arg Cys Ile Gln Arg Ile Asp Gln Pro Ile Ile Leu Thr
    1820                1825                1830

Gly Phe Ser Ala Leu Asn Lys Leu Leu Gly Arg Glu Val Tyr Ser
    1835                1840                1845

Ser His Met Gln Leu Gly Gly Pro Lys Ile Met Ala Thr Asn Gly
    1850                1855                1860

Val Val His Leu Thr Val Pro Asp Asp Leu Glu Gly Val Ser Asn
    1865                1870                1875

Ile Leu Arg Trp Leu Ser Tyr Val Pro Ala Asn Ile Gly Gly Pro
    1880                1885                1890

Leu Pro Ile Thr Lys Ser Leu Asp Pro Ile Asp Arg Pro Val Ala
    1895                1900                1905

Tyr Ile Pro Glu Asn Thr Cys Asp Pro Arg Ala Ala Ile Ser Gly
    1910                1915                1920

Ile Asp Asp Ser Gln Gly Lys Trp Leu Gly Gly Met Phe Asp Lys
    1925                1930                1935

Asp Ser Phe Val Glu Thr Phe Glu Gly Trp Ala Lys Thr Val Val
    1940                1945                1950

Thr Gly Arg Ala Lys Leu Gly Gly Ile Pro Val Gly Val Ile Ala
    1955                1960                1965

Val Glu Thr Gln Thr Met Met Gln Leu Val Pro Ala Asp Pro Gly
    1970                1975                1980

Gln Pro Asp Ser His Glu Arg Ser Val Pro Arg Ala Gly Gln Val
    1985                1990                1995

Trp Phe Pro Asp Ser Ala Thr Lys Thr Ala Gln Ala Met Leu Asp
    2000                2005                2010

Phe Asn Arg Glu Gly Leu Pro Leu Phe Ile Leu Ala Asn Trp Arg
    2015                2020                2025

Gly Phe Ser Gly Gly Gln Arg Asp Leu Phe Glu Gly Ile Leu Gln
    2030                2035                2040

Ala Gly Ser Thr Ile Val Glu Asn Leu Arg Thr Tyr Asn Gln Pro
    2045                2050                2055

Ala Phe Val Tyr Ile Pro Lys Ala Ala Glu Leu Arg Gly Gly Ala
    2060                2065                2070

Trp Val Val Ile Asp Ser Lys Ile Asn Pro Asp Arg Ile Glu Cys
    2075                2080                2085

Tyr Ala Glu Arg Thr Ala Lys Gly Asn Val Leu Glu Pro Gln Gly
    2090                2095                2100

Leu Ile Glu Ile Lys Phe Arg Ser Glu Glu Leu Lys Glu Cys Met
    2105                2110                2115

Gly Arg Leu Asp Pro Glu Leu Ile Asp Leu Lys Ala Arg Leu Gln
    2120                2125                2130

Gly Ala Asn Gly Ser Leu Ser Asp Gly Glu Ser Leu Gln Lys Ser
    2135                2140                2145

Ile Glu Ala Arg Lys Lys Gln Leu Leu Pro Leu Tyr Thr Gln Ile
    2150                2155                2160

Ala Val Arg Phe Ala Glu Leu His Asp Thr Ser Leu Arg Met Ala
    2165                2170                2175

Ala Lys Gly Val Ile Arg Lys Val Val Asp Trp Glu Asp Ser Arg
    2180                2185                2190

Ser Phe Phe Tyr Lys Arg Leu Arg Arg Arg Leu Ser Glu Asp Val
    2195                2200                2205

Leu Ala Lys Glu Ile Arg Gly Val Ile Gly Glu Lys Phe Pro His
    2210                2215                2220

Lys Ser Ala Ile Glu Leu Ile Lys Lys Trp Tyr Leu Ala Ser Glu
    2225                2230                2235

Ala Ala Ala Ala Gly Ser Thr Asp Trp Asp Asp Asp Asp Ala Phe
    2240                2245                2250

Val Ala Trp Arg Glu Asn Pro Glu Asn Tyr Lys Glu Tyr Ile Lys
    2255                2260                2265

Glu Leu Arg Ala Gln Arg Val Ser Arg Leu Leu Ser Asp Val Ala
    2270                2275                2280

Gly Ser Ser Ser Asp Leu Gln Ala Leu Pro Gln Gly Leu Ser Met
    2285                2290                2295

Leu Leu Asp Lys Met Asp Pro Ser Lys Arg Ala Gln Phe Ile Glu

2300                    2305                    2310

Glu Val  Met Lys Val Leu Lys
    2315                2320

<210> 2
<211> 2327
<212> PRT
<213> Oryza sativa

<400> 2
Met Thr Ser Thr His Val Ala Thr Leu Gly Val Gly Ala Gln Ala Pro
1               5                   10                  15

Pro Arg His Gln Lys Lys Ser Ala Gly Thr Ala Phe Val Ser Ser Gly
            20                  25                  30

Ser Ser Arg Pro Ser Tyr Arg Lys Asn Gly Gln Arg Thr Arg Ser Leu
        35                  40                  45

Arg Glu Glu Ser Asn Gly Gly Val Ser Asp Ser Lys Lys Leu Asn His
    50                  55                  60

Ser Ile Arg Gln Gly Leu Ala Gly Ile Ile Asp Leu Pro Asn Asp Ala
65                  70                  75                  80

Ala Ser Glu Val Asp Ile Ser His Gly Ser Glu Asp Pro Arg Gly Pro
                85                  90                  95

Thr Val Pro Gly Ser Tyr Gln Met Asn Gly Ile Ile Asn Glu Thr His
            100                 105                 110

Asn Gly Arg His Ala Ser Val Ser Lys Val Val Glu Phe Cys Thr Ala
        115                 120                 125

Leu Gly Gly Lys Thr Pro Ile His Ser Val Leu Val Ala Asn Asn Gly
    130                 135                 140

Met Ala Ala Ala Lys Phe Met Arg Ser Val Arg Thr Trp Ala Asn Asp
145                 150                 155                 160

Thr Phe Gly Ser Glu Lys Ala Ile Gln Leu Ile Ala Met Ala Thr Pro
                165                 170                 175

Glu Asp Leu Arg Ile Asn Ala Glu His Ile Arg Ile Ala Asp Gln Phe
            180                 185                 190

Val Glu Val Pro Gly Gly Thr Asn Asn Asn Asn Tyr Ala Asn Val Gln
        195                 200                 205

Leu Ile Val Glu Ile Ala Glu Arg Thr Gly Val Ser Ala Val Trp Pro
    210                 215                 220

Gly Trp Gly His Ala Ser Glu Asn Pro Glu Leu Pro Asp Ala Leu Thr
225                 230                 235                 240

Ala Lys Gly Ile Val Phe Leu Gly Pro Pro Ala Ser Ser Met His Ala
                245                 250                 255

Leu Gly Asp Lys Val Gly Ser Ala Leu Ile Ala Gln Ala Ala Gly Val
            260                 265                 270

Pro Thr Leu Ala Trp Ser Gly Ser His Val Glu Val Pro Leu Glu Cys
        275                 280                 285

Cys Leu Asp Ser Ile Pro Asp Glu Met Tyr Arg Lys Ala Cys Val Thr
    290                 295                 300

Thr Thr Glu Glu Ala Val Ala Ser Cys Gln Val Val Gly Tyr Pro Ala
305                 310                 315                 320

Met Ile Lys Ala Ser Trp Gly Gly Gly Gly Lys Gly Ile Arg Lys Val
                325                 330                 335

His Asn Asp Asp Glu Val Arg Thr Leu Phe Lys Gln Val Gln Gly Glu
            340                 345                 350

Val Pro Gly Ser Pro Ile Phe Ile Met Arg Leu Ala Ala Gln Ser Arg
        355                 360                 365

His Leu Glu Val Gln Leu Leu Cys Asp Gln Tyr Gly Asn Val Ala Ala
    370                 375                 380

Leu His Ser Arg Asp Cys Ser Val Gln Arg Arg His Gln Lys Ile Ile
385                 390                 395                 400

Glu Glu Gly Pro Val Thr Val Ala Pro Arg Glu Thr Val Lys Glu Leu
                405                 410                 415

Glu Gln Ala Ala Arg Arg Leu Ala Lys Ala Val Gly Tyr Val Gly Ala
            420                 425                 430

Ala Thr Val Glu Tyr Leu Tyr Ser Met Glu Thr Gly Glu Tyr Tyr Phe
        435                 440                 445

Leu Glu Leu Asn Pro Arg Leu Gln Val Glu His Pro Val Thr Glu Trp

450                 455                 460

Ile Ala Glu Val Asn Leu Pro Ala Ala Gln Val Ala Val Gly Met Gly
465                 470                 475                 480

Ile Pro Leu Trp Gln Ile Pro Glu Ile Arg Arg Phe Tyr Gly Met Asn
                485                 490                 495

His Gly Gly Gly Tyr Asp Leu Trp Arg Lys Thr Ala Ala Leu Ala Thr
            500                 505                 510

Pro Phe Asn Phe Asp Glu Val Asp Ser Lys Trp Pro Lys Gly His Cys
        515                 520                 525

Val Ala Val Arg Ile Thr Ser Glu Asp Pro Asp Asp Gly Phe Lys Pro
    530                 535                 540

Thr Gly Gly Lys Val Lys Glu Ile Ser Phe Lys Ser Lys Pro Asn Val
545                 550                 555                 560

Trp Ala Tyr Phe Ser Val Lys Ser Gly Gly Gly Ile His Glu Phe Ala
                565                 570                 575

Asp Ser Gln Phe Gly His Val Phe Ala Tyr Gly Thr Thr Arg Ser Ala
            580                 585                 590

Ala Ile Thr Thr Met Ala Leu Ala Leu Lys Glu Val Gln Ile Arg Gly
        595                 600                 605

Glu Ile His Ser Asn Val Asp Tyr Thr Val Asp Leu Leu Asn Ala Ser
    610                 615                 620

Asp Phe Arg Glu Asn Lys Ile His Thr Gly Trp Leu Asp Thr Arg Ile
625                 630                 635                 640

Ala Met Arg Val Gln Ala Glu Arg Pro Pro Trp Tyr Ile Ser Val Val
                645                 650                 655

Gly Gly Ala Leu Tyr Lys Thr Val Thr Ala Asn Thr Ala Thr Val Ser
            660                 665                 670

Asp Tyr Val Gly Tyr Leu Thr Lys Gly Gln Ile Pro Pro Lys His Ile
        675                 680                 685

Ser Leu Val Tyr Thr Thr Val Ala Leu Asn Ile Asp Gly Lys Lys Tyr
    690                 695                 700

Thr Ile Asp Thr Val Arg Ser Gly His Gly Ser Tyr Arg Leu Arg Met
705                 710                 715                 720

Asn Gly Ser Thr Val Asp Ala Asn Val Gln Ile Leu Cys Asp Gly Gly
                725                 730                 735

Leu Leu Met Gln Leu Asp Gly Asn Ser His Val Ile Tyr Ala Glu Glu
            740                 745                 750

Glu Ala Ser Gly Thr Arg Leu Leu Ile Asp Gly Lys Thr Cys Met Leu
        755                 760                 765

Gln Asn Asp His Asp Pro Ser Lys Leu Leu Ala Glu Thr Pro Cys Lys
    770                 775                 780

Leu Leu Arg Phe Leu Val Ala Asp Gly Ala His Val Asp Ala Asp Val
785                 790                 795                 800

Pro Tyr Ala Glu Val Glu Val Met Lys Met Cys Met Pro Leu Leu Ser
                805                 810                 815

Pro Ala Ser Gly Val Ile His Val Val Met Ser Glu Gly Gln Ala Met
            820                 825                 830

Gln Ala Gly Asp Leu Ile Ala Arg Leu Asp Leu Asp Asp Pro Ser Ala
        835                 840                 845

Val Lys Arg Ala Glu Pro Phe Glu Asp Thr Phe Pro Gln Met Gly Leu
    850                 855                 860

Pro Ile Ala Ala Ser Gly Gln Val His Lys Leu Cys Ala Ala Ser Leu
865                 870                 875                 880

Asn Ala Cys Arg Met Ile Leu Ala Gly Tyr Glu His Asp Ile Asp Lys
                885                 890                 895

Val Val Pro Glu Leu Val Tyr Cys Leu Asp Thr Pro Glu Leu Pro Phe
            900                 905                 910

Leu Gln Trp Glu Glu Leu Met Ser Val Leu Ala Thr Arg Leu Pro Arg
        915                 920                 925

Asn Leu Lys Ser Glu Leu Glu Gly Lys Tyr Glu Glu Tyr Lys Val Lys
    930                 935                 940

Phe Asp Ser Gly Ile Ile Asn Asp Phe Pro Ala Asn Met Leu Arg Val
945                 950                 955                 960

Ile Ile Glu Glu Asn Leu Ala Cys Gly Ser Glu Lys Glu Lys Ala Thr
                965                 970                 975

Asn Glu Arg Leu Val Glu Pro Leu Met Ser Leu Leu Lys Ser Tyr Glu
            980                 985                 990

Gly Gly Arg Glu Ser His Ala His Phe Val Val Lys Ser Leu Phe Glu
        995                 1000                1005

Glu Tyr Leu Tyr Val Glu Glu Leu Phe Ser Asp Gly Ile Gln Ser
    1010                1015                1020

Asp Val Ile Glu Arg Leu Arg Leu Gln His Ser Lys Asp Leu Gln
    1025                1030                1035

Lys Val Val Asp Ile Val Leu Ser His Gln Ser Val Arg Asn Lys
    1040                1045                1050

Thr Lys Leu Ile Leu Lys Leu Met Glu Ser Leu Val Tyr Pro Asn
    1055                1060                1065

Pro Ala Ala Tyr Arg Asp Gln Leu Ile Arg Phe Ser Ser Leu Asn
    1070                1075                1080

His Lys Ala Tyr Tyr Lys Leu Ala Leu Lys Ala Ser Glu Leu Leu
    1085                1090                1095

Glu Gln Thr Lys Leu Ser Glu Leu Arg Ala Arg Ile Ala Arg Ser
    1100                1105                1110

Leu Ser Glu Leu Glu Met Phe Thr Glu Glu Ser Lys Gly Leu Ser
    1115                1120                1125

Met His Lys Arg Glu Ile Ala Ile Lys Glu Ser Met Glu Asp Leu
    1130                1135                1140

Val Thr Ala Pro Leu Pro Val Glu Asp Ala Leu Ile Ser Leu Phe
    1145                1150                1155

Asp Cys Ser Asp Thr Thr Val Gln Gln Arg Val Ile Glu Thr Tyr
    1160                1165                1170

Ile Ala Arg Leu Tyr Gln Pro His Leu Val Lys Asp Ser Ile Lys
    1175                1180                1185

Met Lys Trp Ile Glu Ser Gly Val Ile Ala Leu Trp Glu Phe Pro
    1190                1195                1200

Glu Gly His Phe Asp Ala Arg Asn Gly Gly Ala Val Leu Gly Asp
    1205                1210                1215

Lys Arg Trp Gly Ala Met Val Ile Val Lys Ser Leu Glu Ser Leu
    1220                1225                1230

Ser Met Ala Ile Arg Phe Ala Leu Lys Glu Thr Ser His Tyr Thr
    1235                1240                1245

Ser Ser Glu Gly Asn Met Met His Ile Ala Leu Leu Gly Ala Asp
    1250                1255                1260

Asn Lys Met His Ile Ile Gln Glu Ser Gly Asp Asp Ala Asp Arg
    1265                1270                1275

Ile Ala Lys Leu Pro Leu Ile Leu Lys Asp Asn Val Thr Asp Leu
    1280                1285                1290

His Ala Ser Gly Val Lys Thr Ile Ser Phe Ile Val Gln Arg Asp
    1295                1300                1305

Glu Ala Arg Met Thr Met Arg Arg Thr Phe Leu Trp Ser Asp Glu
    1310                1315                1320

Lys Leu Ser Tyr Glu Glu Glu Pro Ile Leu Arg His Val Glu Pro
    1325                1330                1335

Pro Leu Ser Ala Leu Leu Glu Leu Asp Lys Leu Lys Val Lys Gly
    1340                1345                1350

Tyr Asn Glu Met Lys Tyr Thr Pro Ser Arg Asp Arg Gln Trp His
    1355                1360                1365

Ile Tyr Thr Leu Arg Asn Thr Glu Asn Pro Lys Met Leu His Arg
    1370                1375                1380

Val Phe Phe Arg Thr Leu Val Arg Gln Pro Ser Val Ser Asn Lys
    1385                1390                1395

Phe Ser Ser Gly Gln Ile Gly Asp Met Glu Val Gly Ser Ala Glu
    1400                1405                1410

Glu Pro Leu Ser Phe Thr Ser Thr Ser Ile Leu Arg Ser Leu Met
    1415                1420                1425

Thr Ala Ile Glu Glu Leu Glu Leu His Ala Ile Arg Thr Gly His

1430                1435                1440

Ser His Met Tyr Leu His Val Leu Lys Glu Gln Lys Leu Leu Asp
    1445                1450                1455

Leu Val Pro Val Ser Gly Asn Thr Val Leu Asp Val Gly Gln Asp
    1460                1465                1470

Glu Ala Thr Ala Tyr Ser Leu Leu Lys Glu Met Ala Met Lys Ile
    1475                1480                1485

His Glu Leu Val Gly Ala Arg Met His His Leu Ser Val Cys Gln
    1490                1495                1500

Trp Glu Val Lys Leu Lys Leu Asp Cys Asp Gly Pro Ala Ser Gly
    1505                1510                1515

Thr Trp Arg Ile Val Thr Thr Asn Val Thr Ser His Thr Cys Thr
    1520                1525                1530

Val Asp Ile Tyr Arg Glu Met Glu Asp Lys Glu Ser Arg Lys Leu
    1535                1540                1545

Val Tyr His Pro Ala Thr Pro Ala Ala Gly Pro Leu His Gly Val
    1550                1555                1560

Ala Leu Asn Asn Pro Tyr Gln Pro Leu Ser Val Ile Asp Leu Lys
    1565                1570                1575

Arg Cys Ser Ala Arg Asn Asn Arg Thr Thr Tyr Cys Tyr Asp Phe
    1580                1585                1590

Pro Leu Ala Phe Glu Thr Ala Val Arg Lys Ser Trp Ser Ser Ser
    1595                1600                1605

Thr Ser Gly Ala Ser Lys Gly Val Glu Asn Ala Gln Cys Tyr Val
    1610                1615                1620

Lys Ala Thr Glu Leu Val Phe Ala Asp Lys His Gly Ser Trp Gly
    1625                1630                1635

Thr Pro Leu Val Gln Met Asp Arg Pro Ala Gly Leu Asn Asp Ile
    1640                1645                1650

Gly Met Val Ala Trp Thr Leu Lys Met Ser Thr Pro Glu Phe Pro
    1655                1660                1665

Ser Gly Arg Glu Ile Ile Val Val Ala Asn Asp Ile Thr Phe Arg
    1670                1675                1680

Ala Gly Ser Phe Gly Pro Arg Glu Asp Ala Phe Phe Glu Ala Val
    1685                1690                1695

Thr Asn Leu Ala Cys Glu Lys Lys Leu Pro Leu Ile Tyr Leu Ala
    1700                1705                1710

Ala Asn Ser Gly Ala Arg Ile Gly Ile Ala Asp Glu Val Lys Ser
    1715                1720                1725

Cys Phe Arg Val Gly Trp Ser Asp Asp Gly Ser Pro Glu Arg Gly
    1730                1735                1740

Phe Gln Tyr Ile Tyr Leu Ser Glu Glu Asp Tyr Ala Arg Ile Gly
    1745                1750                1755

Thr Ser Val Ile Ala His Lys Met Gln Leu Asp Ser Gly Glu Ile
    1760                1765                1770

Arg Trp Val Ile Asp Ser Val Val Gly Lys Glu Asp Gly Leu Gly
    1775                1780                1785

Val Glu Asn Ile His Gly Ser Ala Ala Ile Ala Ser Ala Tyr Ser
    1790                1795                1800

Arg Ala Tyr Lys Glu Thr Phe Thr Leu Thr Phe Val Thr Gly Arg
    1805                1810                1815

Thr Val Gly Ile Gly Ala Tyr Leu Ala Arg Leu Gly Ile Arg Cys
    1820                1825                1830

Ile Gln Arg Leu Asp Gln Pro Ile Ile Leu Thr Gly Tyr Ser Ala
    1835                1840                1845

Leu Asn Lys Leu Leu Gly Arg Glu Val Tyr Ser Ser His Met Gln
    1850                1855                1860

Leu Gly Gly Pro Lys Ile Met Ala Thr Asn Gly Val Val His Leu
    1865                1870                1875

Thr Val Ser Asp Asp Leu Glu Gly Val Ser Asn Ile Leu Arg Trp
    1880                1885                1890

Leu Ser Tyr Val Pro Ala Tyr Ile Gly Gly Pro Leu Pro Val Thr
    1895                1900                1905

Thr Pro Leu Asp Pro Pro Asp Arg Pro Val Ala Tyr Ile Pro Glu
    1910                1915                1920

Asn Ser Cys Asp Pro Arg Ala Ala Ile Arg Gly Val Asp Asp Ser
    1925                1930                1935

Gln Gly Lys Trp Leu Gly Gly Met Phe Asp Lys Asp Ser Phe Val
    1940                1945                1950

Glu Thr Phe Glu Gly Trp Ala Lys Thr Val Val Thr Gly Arg Ala
    1955                1960                1965

Lys Leu Gly Gly Ile Pro Val Gly Val Ile Ala Val Glu Thr Gln
    1970                1975                1980

Thr Met Met Gln Thr Ile Pro Ala Asp Pro Gly Gln Leu Asp Ser
    1985                1990                1995

Arg Glu Gln Ser Val Pro Arg Ala Gly Gln Val Trp Phe Pro Asp
    2000                2005                2010

Ser Ala Thr Lys Thr Ala Gln Ala Leu Leu Asp Phe Asn Arg Glu
    2015                2020                2025

Gly Leu Pro Leu Phe Ile Leu Ala Asn Trp Arg Gly Phe Ser Gly
    2030                2035                2040

Gly Gln Arg Asp Leu Phe Glu Gly Ile Leu Gln Ala Gly Ser Thr
    2045                2050                2055

Ile Val Glu Asn Leu Arg Thr Tyr Asn Gln Pro Ala Phe Val Tyr
    2060                2065                2070

Ile Pro Met Ala Ala Glu Leu Arg Gly Gly Ala Trp Val Val Val
    2075                2080                2085

Asp Ser Lys Ile Asn Pro Asp Arg Ile Glu Cys Tyr Ala Glu Arg
    2090                2095                2100

Thr Ala Lys Gly Asn Val Leu Glu Pro Gln Gly Leu Ile Glu Ile
    2105                2110                2115

Lys Phe Arg Ser Glu Glu Leu Gln Asp Cys Met Ser Arg Leu Asp
    2120                2125                2130

Pro Thr Leu Ile Asp Leu Lys Ala Lys Leu Glu Val Ala Asn Lys
    2135                2140                2145

Asn Gly Ser Ala Asp Thr Lys Ser Leu Gln Glu Asn Ile Glu Ala
    2150                2155                2160

Arg Thr Lys Gln Leu Met Pro Leu Tyr Thr Gln Ile Ala Ile Arg
    2165                2170                2175

Phe Ala Glu Leu His Asp Thr Ser Leu Arg Met Ala Ala Lys Gly
    2180                2185                2190

Val Ile Lys Lys Val Val Asp Trp Glu Glu Ser Arg Ser Phe Phe
    2195                2200                2205

Tyr Lys Arg Leu Arg Arg Arg Ile Ser Glu Asp Val Leu Ala Lys
    2210                2215                2220

Glu Ile Arg Ala Val Ala Gly Glu Gln Phe Ser His Gln Pro Ala
    2225                2230                2235

Ile Glu Leu Ile Lys Lys Trp Tyr Ser Ala Ser His Ala Ala Glu
    2240                2245                2250

Trp Asp Asp Asp Asp Ala Phe Val Ala Trp Met Asp Asn Pro Glu
    2255                2260                2265

Asn Tyr Lys Asp Tyr Ile Gln Tyr Leu Lys Ala Gln Arg Val Ser
    2270                2275                2280

Gln Ser Leu Ser Ser Leu Ser Asp Ser Ser Ser Asp Leu Gln Ala
    2285                2290                2295

Leu Pro Gln Gly Leu Ser Met Leu Leu Asp Lys Met Asp Pro Ser
    2300                2305                2310

Arg Arg Ala Gln Leu Val Glu Glu Ile Arg Lys Val Leu Gly
    2315                2320                2325

<210> 3
<211> 2327
<212> PRT
<213> Oryza sativa

<400> 3
Met Thr Ser Thr His Val Ala Thr Leu Gly Val Gly Ala Gln Ala Pro
1               5                   10                  15

Pro Arg His Gln Lys Lys Ser Ala Gly Thr Ala Phe Val Ser Ser Gly
            20                  25                  30

Ser Ser Arg Pro Ser Tyr Arg Lys Asn Gly Gln Arg Thr Arg Ser Leu
        35                  40                  45

Arg Glu Glu Ser Asn Gly Gly Val Ser Asp Ser Lys Lys Leu Asn His
    50                  55                  60

Ser Ile Arg Gln Gly Leu Ala Gly Ile Ile Asp Leu Pro Asn Asp Ala
65                  70                  75                  80

Ala Ser Glu Val Asp Ile Ser His Gly Ser Glu Asp Pro Arg Gly Pro
                85                  90                  95

Thr Val Pro Gly Ser Tyr Gln Met Asn Gly Ile Ile Asn Glu Thr His
            100                 105                 110

Asn Gly Arg His Ala Ser Val Ser Lys Val Val Glu Phe Cys Thr Ala
        115                 120                 125

Leu Gly Gly Lys Thr Pro Ile His Ser Val Leu Val Ala Asn Asn Gly
    130                 135                 140

Met Ala Ala Ala Lys Phe Met Arg Ser Val Arg Thr Trp Ala Asn Asp
145                 150                 155                 160

Thr Phe Gly Ser Glu Lys Ala Ile Gln Leu Ile Ala Met Ala Thr Pro
                165                 170                 175

Glu Asp Leu Arg Ile Asn Ala Glu His Ile Arg Ile Ala Asp Gln Phe
            180                 185                 190

Val Glu Val Pro Gly Gly Thr Asn Asn Asn Asn Tyr Ala Asn Val Gln
        195                 200                 205

Leu Ile Val Glu Ile Ala Glu Arg Thr Gly Val Ser Ala Val Trp Pro
    210                 215                 220

Gly Trp Gly His Ala Ser Glu Asn Pro Glu Leu Pro Asp Ala Leu Thr
225                 230                 235                 240

Ala Lys Gly Ile Val Phe Leu Gly Pro Pro Ala Ser Ser Met His Ala
                245                 250                 255

Leu Gly Asp Lys Val Gly Ser Ala Leu Ile Ala Gln Ala Ala Gly Val
            260                 265                 270

Pro Thr Leu Ala Trp Ser Gly Ser His Val Glu Val Pro Leu Glu Cys
        275                 280                 285

Cys Leu Asp Ser Ile Pro Asp Glu Met Tyr Arg Lys Ala Cys Val Thr
    290                 295                 300

Thr Thr Glu Glu Ala Val Ala Ser Cys Gln Val Val Gly Tyr Pro Ala
305                 310                 315                 320

Met Ile Lys Ala Ser Trp Gly Gly Gly Gly Lys Gly Ile Arg Lys Val
                325                 330                 335

His Asn Asp Asp Glu Val Arg Thr Leu Phe Lys Gln Val Gln Gly Glu
            340                 345                 350

Val Pro Gly Ser Pro Ile Phe Ile Met Arg Leu Ala Ala Gln Ser Arg
        355                 360                 365

His Leu Glu Val Gln Leu Leu Cys Asp Gln Tyr Gly Asn Val Ala Ala
    370                 375                 380

Leu His Ser Arg Asp Cys Ser Val Gln Arg Arg His Gln Lys Ile Ile
385                 390                 395                 400

Glu Glu Gly Pro Val Thr Val Ala Pro Arg Glu Thr Val Lys Glu Leu
                405                 410                 415

Glu Gln Ala Ala Arg Arg Leu Ala Lys Ala Val Gly Tyr Val Gly Ala
            420                 425                 430

Ala Thr Val Glu Tyr Leu Tyr Ser Met Glu Thr Gly Glu Tyr Tyr Phe
        435                 440                 445

Leu Glu Leu Asn Pro Arg Leu Gln Val Glu His Pro Val Thr Glu Trp
    450                 455                 460

Ile Ala Glu Val Asn Leu Pro Ala Ala Gln Val Ala Val Gly Met Gly
465                 470                 475                 480

Ile Pro Leu Trp Gln Ile Pro Glu Ile Arg Arg Phe Tyr Gly Met Asn
                485                 490                 495

His Gly Gly Gly Tyr Asp Leu Trp Arg Lys Thr Ala Ala Leu Ala Thr
            500                 505                 510

Pro Phe Asn Phe Asp Glu Val Asp Ser Lys Trp Pro Lys Gly His Cys
        515                 520                 525

Val Ala Val Arg Ile Thr Ser Glu Asp Pro Asp Asp Gly Phe Lys Pro

530                 535                 540

Thr Gly Gly Lys Val Lys Glu Ile Ser Phe Lys Ser Lys Pro Asn Val
545                 550                 555                 560

Trp Ala Tyr Phe Ser Val Lys Ser Gly Gly Gly Ile His Glu Phe Ala
                565                 570                 575

Asp Ser Gln Phe Gly His Val Phe Ala Tyr Gly Thr Thr Arg Ser Ala
            580                 585                 590

Ala Ile Thr Thr Met Ala Leu Ala Leu Lys Glu Val Gln Ile Arg Gly
        595                 600                 605

Glu Ile His Ser Asn Val Asp Tyr Thr Val Asp Leu Leu Asn Ala Ser
    610                 615                 620

Asp Phe Arg Glu Asn Lys Ile His Thr Gly Trp Leu Asp Thr Arg Ile
625                 630                 635                 640

Ala Met Arg Val Gln Ala Glu Arg Pro Pro Trp Tyr Ile Ser Val Val
                645                 650                 655

Gly Gly Ala Leu Tyr Lys Thr Val Thr Ala Asn Thr Ala Thr Val Ser
            660                 665                 670

Asp Tyr Val Gly Tyr Leu Thr Lys Gly Gln Ile Pro Pro Lys His Ile
        675                 680                 685

Ser Leu Val Tyr Thr Thr Val Ala Leu Asn Ile Asp Gly Lys Lys Tyr
    690                 695                 700

Thr Ile Asp Thr Val Arg Ser Gly His Gly Ser Tyr Arg Leu Arg Met
705                 710                 715                 720

Asn Gly Ser Thr Val Asp Ala Asn Val Gln Ile Leu Cys Asp Gly Gly
                725                 730                 735

Leu Leu Met Gln Leu Asp Gly Asn Ser His Val Ile Tyr Ala Glu Glu
            740                 745                 750

Glu Ala Ser Gly Thr Arg Leu Leu Ile Asp Gly Lys Thr Cys Met Leu
        755                 760                 765

Gln Asn Asp His Asp Pro Ser Lys Leu Leu Ala Glu Thr Pro Cys Lys
    770                 775                 780

Leu Leu Arg Phe Leu Val Ala Asp Gly Ala His Val Asp Ala Asp Val
785                 790                 795                 800

Pro Tyr Ala Glu Val Glu Val Met Lys Met Cys Met Pro Leu Leu Ser
                805                 810                 815

Pro Ala Ser Gly Val Ile His Val Val Met Ser Glu Gly Gln Ala Met
            820                 825                 830

Gln Ala Gly Asp Leu Ile Ala Arg Leu Asp Leu Asp Asp Pro Ser Ala
        835                 840                 845

Val Lys Arg Ala Glu Pro Phe Glu Asp Thr Phe Pro Gln Met Gly Leu
    850                 855                 860

Pro Ile Ala Ala Ser Gly Gln Val His Lys Leu Cys Ala Ala Ser Leu
865                 870                 875                 880

Asn Ala Cys Arg Met Ile Leu Ala Gly Tyr Glu His Asp Ile Asp Lys
                885                 890                 895

Val Val Pro Glu Leu Val Tyr Cys Leu Asp Thr Pro Glu Leu Pro Phe
            900                 905                 910

Leu Gln Trp Glu Glu Leu Met Ser Val Leu Ala Thr Arg Leu Pro Arg
        915                 920                 925

Asn Leu Lys Ser Glu Leu Glu Gly Lys Tyr Glu Glu Tyr Lys Val Lys
    930                 935                 940

Phe Asp Ser Gly Ile Ile Asn Asp Phe Pro Ala Asn Met Leu Arg Val
945                 950                 955                 960

Ile Ile Glu Glu Asn Leu Ala Cys Gly Ser Glu Lys Glu Lys Ala Thr
                965                 970                 975

Asn Glu Arg Leu Val Glu Pro Leu Met Ser Leu Leu Lys Ser Tyr Glu
            980                 985                 990

Gly Gly Arg Glu Ser His Ala His  Phe Val Val Lys Ser  Leu Phe Glu
        995                 1000                1005

Glu Tyr  Leu Tyr Val Glu Glu  Leu Phe Ser Asp Gly  Ile Gln Ser
    1010                1015                1020

Asp Val  Ile Glu Arg Leu Arg  Leu Gln His Ser Lys  Asp Leu Gln
    1025                1030                1035

Lys Val Val Asp Ile Val Leu Ser His Gln Ser Val Arg Asn Lys
    1040                1045                1050

Thr Lys Leu Ile Leu Lys Leu Met Glu Ser Leu Val Tyr Pro Asn
    1055                1060                1065

Pro Ala Ala Tyr Arg Asp Gln Leu Ile Arg Phe Ser Ser Leu Asn
    1070                1075                1080

His Lys Ala Tyr Tyr Lys Leu Ala Leu Lys Ala Ser Glu Leu Leu
    1085                1090                1095

Glu Gln Thr Lys Leu Ser Glu Leu Arg Ala Arg Ile Ala Arg Ser
    1100                1105                1110

Leu Ser Glu Leu Glu Met Phe Thr Glu Glu Ser Lys Gly Leu Ser
    1115                1120                1125

Met His Lys Arg Glu Ile Ala Ile Lys Glu Ser Met Glu Asp Leu
    1130                1135                1140

Val Thr Ala Pro Leu Pro Val Glu Asp Ala Leu Ile Ser Leu Phe
    1145                1150                1155

Asp Cys Ser Asp Thr Thr Val Gln Gln Arg Val Ile Glu Thr Tyr
    1160                1165                1170

Ile Ala Arg Leu Tyr Gln Pro His Leu Val Lys Asp Ser Ile Lys
    1175                1180                1185

Met Lys Trp Ile Glu Ser Gly Val Ile Ala Leu Trp Glu Phe Pro
    1190                1195                1200

Glu Gly His Phe Asp Ala Arg Asn Gly Gly Ala Val Leu Gly Asp
    1205                1210                1215

Lys Arg Trp Gly Ala Met Val Ile Val Lys Ser Leu Glu Ser Leu
    1220                1225                1230

Ser Met Ala Ile Arg Phe Ala Leu Lys Glu Thr Ser His Tyr Thr
    1235                1240                1245

Ser Ser Glu Gly Asn Met Met His Ile Ala Leu Leu Gly Ala Asp
    1250                1255                1260

Asn Lys Met His Ile Ile Gln Glu Ser Gly Asp Asp Ala Asp Arg
    1265                1270                1275

Ile Ala Lys Leu Pro Leu Ile Leu Lys Asp Asn Val Thr Asp Leu
    1280                1285                1290

His Ala Ser Gly Val Lys Thr Ile Ser Phe Ile Val Gln Arg Asp
    1295                1300                1305

Glu Ala Arg Met Thr Met Arg Arg Thr Phe Leu Trp Ser Asp Glu
    1310                1315                1320

Lys Leu Ser Tyr Glu Glu Glu Pro Ile Leu Arg His Val Glu Pro
    1325                1330                1335

Pro Leu Ser Ala Leu Leu Glu Leu Asp Lys Leu Lys Val Lys Gly
    1340                1345                1350

Tyr Asn Glu Met Lys Tyr Thr Pro Ser Arg Asp Arg Gln Trp His
    1355                1360                1365

Ile Tyr Thr Leu Arg Asn Thr Glu Asn Pro Lys Met Leu His Arg
    1370                1375                1380

Val Phe Phe Arg Thr Leu Val Arg Gln Pro Ser Val Ser Asn Lys
    1385                1390                1395

Phe Ser Ser Gly Gln Ile Gly Asp Met Glu Val Gly Ser Ala Glu
    1400                1405                1410

Glu Pro Leu Ser Phe Thr Ser Thr Ser Ile Leu Arg Ser Leu Met
    1415                1420                1425

Thr Ala Ile Glu Glu Leu Glu Leu His Ala Ile Arg Thr Gly His
    1430                1435                1440

Ser His Met Tyr Leu His Val Leu Lys Glu Gln Lys Leu Leu Asp
    1445                1450                1455

Leu Val Pro Val Ser Gly Asn Thr Val Leu Asp Val Gly Gln Asp
    1460                1465                1470

Glu Ala Thr Ala Tyr Ser Leu Leu Lys Glu Met Ala Met Lys Ile
    1475                1480                1485

His Glu Leu Val Gly Ala Arg Met His His Leu Ser Val Cys Gln
    1490                1495                1500

Trp Glu Val Lys Leu Lys Leu Asp Cys Asp Gly Pro Ala Ser Gly

1505                1510                1515

Thr Trp Arg Ile Val Thr Thr Asn Val Thr Ser His Thr Cys Thr
    1520                1525                1530

Val Asp Ile Tyr Arg Glu Met Glu Asp Lys Glu Ser Arg Lys Leu
    1535                1540                1545

Val Tyr His Pro Ala Thr Pro Ala Ala Gly Pro Leu His Gly Val
    1550                1555                1560

Ala Leu Asn Asn Pro Tyr Gln Pro Leu Ser Val Ile Asp Leu Lys
    1565                1570                1575

Arg Cys Ser Ala Arg Asn Asn Arg Thr Thr Tyr Cys Tyr Asp Phe
    1580                1585                1590

Pro Leu Ala Phe Glu Thr Ala Val Arg Lys Ser Trp Ser Ser Ser
    1595                1600                1605

Thr Ser Gly Ala Ser Lys Gly Val Glu Asn Ala Gln Cys Tyr Val
    1610                1615                1620

Lys Ala Thr Glu Leu Val Phe Ala Asp Lys His Gly Ser Trp Gly
    1625                1630                1635

Thr Pro Leu Val Gln Met Asp Arg Pro Ala Gly Leu Asn Asp Ile
    1640                1645                1650

Gly Met Val Ala Trp Thr Leu Lys Met Ser Thr Pro Glu Phe Pro
    1655                1660                1665

Ser Gly Arg Glu Ile Ile Val Val Ala Asn Asp Ile Thr Phe Arg
    1670                1675                1680

Ala Gly Ser Phe Gly Pro Arg Glu Asp Ala Phe Phe Glu Ala Val
    1685                1690                1695

Thr Asn Leu Ala Cys Glu Lys Lys Leu Pro Leu Ile Tyr Leu Ala
    1700                1705                1710

Ala Asn Ser Gly Ala Arg Ile Gly Ile Ala Asp Glu Val Lys Ser
    1715                1720                1725

Cys Phe Arg Val Gly Trp Ser Asp Asp Gly Ser Pro Glu Arg Gly
    1730                1735                1740

Phe Gln Tyr Ile Tyr Leu Ser Glu Glu Asp Tyr Ala Arg Ile Gly
    1745                1750                1755

Thr Ser Val Ile Ala His Lys Met Gln Leu Asp Ser Gly Glu Ile
    1760                1765                1770

Arg Trp Val Ile Asp Ser Val Val Gly Lys Glu Asp Gly Leu Gly
    1775                1780                1785

Val Glu Asn Ile His Gly Ser Ala Ala Ile Ala Ser Ala Tyr Ser
    1790                1795                1800

Arg Ala Tyr Lys Glu Thr Phe Thr Leu Thr Phe Val Thr Gly Arg
    1805                1810                1815

Thr Val Gly Ile Gly Ala Tyr Leu Ala Arg Leu Gly Ile Arg Cys
    1820                1825                1830

Ile Gln Arg Leu Asp Gln Pro Ile Ile Leu Thr Gly Tyr Ser Ala
    1835                1840                1845

Leu Asn Lys Leu Leu Gly Arg Glu Val Tyr Ser Ser His Met Gln
    1850                1855                1860

Leu Gly Gly Pro Lys Ile Met Ala Thr Asn Gly Val Val His Leu
    1865                1870                1875

Thr Val Ser Asp Asp Leu Glu Gly Val Ser Asn Ile Leu Arg Trp
    1880                1885                1890

Leu Ser Tyr Val Pro Ala Tyr Ile Gly Gly Pro Leu Pro Val Thr
    1895                1900                1905

Thr Pro Leu Asp Pro Pro Asp Arg Pro Val Ala Tyr Ile Pro Glu
    1910                1915                1920

Asn Ser Cys Asp Pro Arg Ala Ala Ile Arg Gly Val Asp Asp Ser
    1925                1930                1935

Gln Gly Lys Trp Leu Gly Gly Met Phe Asp Lys Asp Ser Phe Val
    1940                1945                1950

Glu Thr Phe Glu Gly Trp Ala Lys Thr Val Val Thr Gly Arg Ala
    1955                1960                1965

Lys Leu Gly Gly Ile Pro Val Gly Val Ile Ala Val Glu Thr Gln
    1970                1975                1980

Thr Met Met Gln Thr Ile Pro Ala Asp Pro Gly Gln Leu Asp Ser
    1985                1990                1995

Arg Glu Gln Ser Val Pro Arg Ala Gly Gln Val Trp Phe Pro Asp
    2000                2005                2010

Ser Ala Thr Lys Thr Ala Gln Ala Leu Leu Asp Phe Asn Arg Glu
    2015                2020                2025

Gly Leu Pro Leu Phe Ile Leu Ala Asn Trp Arg Gly Phe Ser Gly
    2030                2035                2040

Gly Gln Arg Asp Leu Phe Glu Gly Ile Leu Gln Ala Gly Ser Thr
    2045                2050                2055

Ile Val Glu Asn Leu Arg Thr Tyr Asn Gln Pro Ala Phe Val Tyr
    2060                2065                2070

Ile Pro Met Ala Ala Glu Leu Arg Gly Gly Ala Trp Val Val Val
    2075                2080                2085

Asp Ser Lys Ile Asn Pro Asp Arg Ile Glu Cys Tyr Ala Glu Arg
    2090                2095                2100

Thr Ala Lys Gly Asn Val Leu Glu Pro Gln Gly Leu Ile Glu Ile
    2105                2110                2115

Lys Phe Arg Ser Glu Glu Leu Gln Asp Cys Met Ser Arg Leu Asp
    2120                2125                2130

Pro Thr Leu Ile Asp Leu Lys Ala Lys Leu Glu Val Ala Asn Lys
    2135                2140                2145

Asn Gly Ser Ala Asp Thr Lys Ser Leu Gln Glu Asn Ile Glu Ala
    2150                2155                2160

Arg Thr Lys Gln Leu Met Pro Leu Tyr Thr Gln Ile Ala Ile Arg
    2165                2170                2175

Phe Ala Glu Leu His Asp Thr Ser Leu Arg Met Ala Ala Lys Gly
    2180                2185                2190

Val Ile Lys Lys Val Val Asp Trp Glu Glu Ser Arg Ser Phe Phe
    2195                2200                2205

Tyr Lys Arg Leu Arg Arg Arg Ile Ser Glu Asp Val Leu Ala Lys
    2210                2215                2220

Glu Ile Arg Ala Val Ala Gly Glu Gln Phe Ser His Gln Pro Ala
    2225                2230                2235

Ile Glu Leu Ile Lys Lys Trp Tyr Ser Ala Ser His Ala Ala Glu
    2240                2245                2250

Trp Asp Asp Asp Asp Ala Phe Val Ala Trp Met Asp Asn Pro Glu
    2255                2260                2265

Asn Tyr Lys Asp Tyr Ile Gln Tyr Leu Lys Ala Gln Arg Val Ser
    2270                2275                2280

Gln Ser Leu Ser Ser Leu Ser Asp Ser Ser Ser Asp Leu Gln Ala
    2285                2290                2295

Leu Pro Gln Gly Leu Ser Met Leu Leu Asp Lys Met Asp Pro Ser
    2300                2305                2310

Arg Arg Ala Gln Leu Val Glu Glu Ile Arg Lys Val Leu Gly
    2315                2320                2325

<210> 4
<211> 6963
<212> DNA
<213> Alopecurus myosuroides

<400> 4
atgggatcca cacatctgcc cattgtcggg tttaatgcat ccacaacacc atcgctatcc      60

actcttcgcc agataaactc agctgctgct gcattccaat cttcgtcccc ttcaaggtca     120

tccaagaaga aaagccgacg tgttaagtca ataagggatg atggcgatgg aagcgtgcca     180

gaccctgcag gccatggcca gtctattcgc caaggtctcg ctggcatcat cgacctccca     240

aaggagggcg catcagctcc agatgtggac atttcacatg ggtctgaaga ccacaaggcc     300

tcctaccaaa tgaatgggat actgaatgaa tcacataacg ggaggcacgc ctctctgtct     360

aaagtttatg aattttgcac ggaattgggt ggaaaaacac caattcacag tgtattagtc     420

gccaacaatg gaatggcagc agctaagttc atgcggagtg tccggacatg ggctaatgat     480

acatttgggt cagagaaggc gattcagttg atagctatgg caactccgga agacatgaga     540

ataaatgcag agcacattag aattgctgat cagtttgttg aagtacctgg tggaacaaac     600

aataacaact atgcaaatgt ccaactcata gtggagatag cagagagaac tggtgtctcc     660

gccgtttggc ctggttgggg ccatgcatct gagaatcctg aacttccaga tgcactaact     720

gcaaaaggaa ttgtttttct tgggccacca gcatcatcaa tgaacgcact aggcgacaag     780

gttggttcag ctctcattgc tcaagcagca ggggttccca ctcttgcttg gagtggatca     840

catgtggaaa ttccattaga actttgtttg gactcgatac ctgaggagat gtataggaaa 900

gcctgtgtta caaccgctga tgaagcagtt gcaagttgtc agatgattgg ttaccctgcc 960

atgatcaagg catcctgggg tggtggtggt aaaggattta gaaaggttaa taatgatgac 1020

gaggtgaaag cactgtttaa gcaagtacag ggtgaagttc ctggctcccc gatatttatc 1080

atgagacttg catctcagag tcgtcatctt gaagtccagc tgctttgtga tgaatatggc 1140

aatgtagcag cacttcacag tcgtgattgc agtgtgcaac gacgacacca aaagattatc 1200

gaggaaggac cagttactgt tgctcctcgt gaaacagtga aagagctaga gcaagcagca 1260

aggaggcttg ctaaggccgt gggttacgtc ggtgctgcta ctgttgaata tctctacagc 1320

atggagactg gtgaatacta ttttctggag cttaatccac ggttgcaggt tgagcaccca 1380

gtcaccgagt cgatagctga agtaaatttg cctgcagccc aagttgcagt tgggatgggt 1440

ataccccttt ggcagattcc agagatcaga cgtttctacg gaatggacaa tggaggaggc 1500

tatgatattt ggaggaaaac agcagctctc gctactccat tcaactttga tgaagtagat 1560

tctcaatggc cgaagggtca ttgtgtggca gttaggataa ccagtgagaa tccagatgat 1620

ggattcaagc ctactggtgg aaaagtaaag gagataagtt ttaaaagtaa gccaaatgtc 1680

tggggatatt tctcagttaa gtctggtgga ggcattcatg aatttgcgga ttctcagttt 1740

ggacacgttt ttgcctatgg agagactaga tcagcagcaa taaccagcat gtctcttgca 1800

ctaaaagaga ttcaaattcg tggagaaatt catacaaacg ttgattacac ggttgatctc 1860

ttgaatgccc cagacttcag agaaaacacg atccataccg gttggctgga taccagaata 1920

gctatgcgtg ttcaagctga gaggcctccc tggtatattt cagtggttgg aggagctcta 1980

tataaaacaa taaccaccaa tgcggagacc gtttctgaat atgttagcta tctcatcaag 2040

ggtcagattc caccaaagca catatccctt gtccattcaa ctatttcttt gaatatagag 2100

gaaagcaaat atacaattga gattgtgagg agtggacagg gtagctacag attgagactg 2160

aatggatcac ttattgaagc caatgtacaa acattatgtg atggaggcct tttaatgcag 2220

ctggatggaa atagccatgt tatttatgct gaagaagaag cgggtggtac acggcttctt 2280

attgatggaa aaacatgctt gctacagaat gaccatgatc cgtcaaggtt attagctgag 2340

acaccctgca aacttcttcg tttcttgatt gccgatggtg ctcatgttga tgctgatgta 2400

ccatacgcgg aagttgaggt tatgaagatg tgcatgcccc tcttgtcgcc tgctgctggt 2460

gtcattaatg ttttgttgtc tgagggccag gcgatgcagg ctggtgatct tatagcgaga 2520

cttgatctcg atgacccttc tgctgtgaag agagccgagc catttgaagg atcttttcca 2580

gaaatgagcc ttcctattgc tgcttctggc caagttcaca aaagatgtgc tgcaagtttg 2640

aacgctgctc gaatggtcct tgcaggatat gaccatgcgg ccaacaaagt tgtgcaagat 2700

ttggtatggt gccttgatac acctgctctt cctttcctac aatgggaaga gcttatgtct 2760

gttttagcaa ctagacttcc aagacgtctt aagagcgagt tggagggcaa atacaatgaa 2820

tacaagttaa atgttgacca tgtgaagatc aaggatttcc ctaccgagat gcttagagag 2880

acaatcgagg aaaatcttgc atgtgtttcc gagaaggaaa tggtgacaat tgagaggctt 2940

gttgaccctc tgatgagcct gctgaagtca tacgagggtg ggagagaaag ccatgcccac 3000

tttattgtca agtccctttt tgaggagtat ctctcggttg aggaactatt cagtgatggc 3060

attcagtctg acgtgattga acgcctgcgc ctacaatata gtaaagacct ccagaaggtt 3120

gtagacattg ttttgtctca ccagggtgtg agaaacaaaa caaagctgat actcgcgctc 3180

atggagaaac tggtctatcc aaaccctgct gcctacagag atcagttgat tcgcttttct 3240

tccctcaacc ataaaagata ttataagttg gctcttaaag ctagtgaact tcttgaacaa 3300

accaagctca gcgaactccg cacaagcatt gcaaggaacc tttcagcgct ggatatgttc 3360

accgaggaaa aggcagattt ctccttgcaa gacagaaaat tggccattaa tgagagcatg 3420

ggagatttag tcactgcccc actgccagtt gaagatgcac ttgtttcttt gtttgattgt 3480

actgatcaaa ctcttcagca gagagtgatt cagacataca tatctcgatt ataccagcct 3540

caacttgtga aggatagcat ccagctgaaa tatcaggatt ctggtgttat tgctttatgg 3600

gaattcactg aaggaaatca tgagaagaga ttgggtgcta tggttatcct gaagtcacta 3660

gaatctgtgt caacagccat tggagctgct ctaaaggatg catcacatta tgcaagctct 3720

gcgggcaaca cggtgcatat tgctttgttg gatgctgata cccaactgaa tacaactgaa 3780

gatagtggtg ataatgacca agctcaagac aagatggata aactttcttt tgtactgaaa 3840

caagatgttg tcatggctga tctacgtgct gctgatgtca aggttgttag ttgcattgtt 3900

caaagagatg gagcaatcat gcctatgcgc cgtaccttcc tcttgtcaga ggaaaaactt 3960

tgttacgagg aagagccgat tcttcggcat gtggagcctc cactttctgc acttcttgag 4020

ttggataaat tgaaagtgaa aggatacaat gagatgaagt atacaccgtc acgtgatcgt 4080

cagtggcata tatacacact tagaaatact gaaaatccaa aaatgctgca cagggtattt 4140

ttccgaacac ttgtcagaca acccagtgca ggcaacaggt ttacatcaga ccatatcact 4200

gatgttgaag taggacacgc agaggaacct ctttcattta cttcaagcag catattaaaa 4260

tcgttgaaga ttgctaaaga agaattggag cttcacgcga tcaggactgg ccattctcat 4320

atgtacttgt gcatattgaa agagcaaaag cttcttgacc ttgttcctgt ttcagggaac 4380

actgttgtgg atgttggtca agatgaagct actgcatgct ctcttttgaa agaaatggct 4440

ttaaagatac atgaacttgt tggtgcaaga atgcatcatc tttctgtatg ccagtgggaa 4500

gtgaaactta agttggtgag cgatggccct gccagtggta gctggagagt tgtaacaacc 4560

aatgttactg gtcacacctg cactgtggat atctaccggg aggtcgaaga tacagaatca 4620

cagaaactag tataccactc caccgcattg tcatctggtc ctttgcatgg tgttgcactg 4680

aatacttcgt atcagccttt gagtgttatt gatttaaaac gttgctctgc caggaacaac 4740

aaaactacat actgctatga ttttccattg acatttgaag ctgcagtgca gaagtcgtgg 4800

tctaacattt ccagtgaaaa caaccaatgt tatgttaaag cgacagagct tgtgtttgct 4860

gaaaagaatg ggtcgtgggg cactcctata attcctatgc agcgtgctgc tgggctgaat 4920

gacattggta tggtagcctg gatcttggac atgtccactc ctgaatttcc cagcggcaga 4980

cagatcattg ttatcgcaaa tgatattaca tttagagctg gatcatttgg cccaagggaa 5040

gatgcatttt tcgaagctgt aaccaacctg gcttgtgaga agaagcttcc acttatctac 5100

ttggctgcaa actctggtgc tcggattggc attgctgatg aagtaaaatc ttgcttccgt 5160

gttggatgga ctgatgatag cagccctgaa cgtggattta ggtacattta tatgactgac 5220

gaagaccatg atcgtattgg ctcttcagtt atagcacaca agatgcagct agatagtggc 5280

gagatcaggt gggttattga ttctgttgtg ggaaaagagg atggactagg tgtggagaac 5340

atacatggaa gtgctgctat tgccagtgcc tattctaggg cgtacgagga gacatttaca 5400

cttacattcg ttactggacg aactgttgga atcggagcct atcttgctcg acttggcata 5460

cggtgcatac agcgtattga ccagcccatt attttgaccg ggttttctgc cctgaacaag 5520

cttcttgggc gggaggtgta cagctcccac atgcagttgg gtggtcccaa aatcatggcg 5580

acgaatggtg ttgtccatct gactgttcca gatgaccttg aaggtgtttc taatatattg 5640

aggtggctca gctatgttcc tgcaaacatt ggtggacctc ttcctattac aaaatctttg 5700

gacccaatag acagacccgt tgcatacatc cctgagaata catgtgatcc tcgtgcagcc 5760

atcagtggca ttgatgacag ccaagggaaa tggttgggtg gcatgtttga caaagacagt 5820

tttgtggaga catttgaagg atgggcgaag acagtagtta ctggcagagc aaaacttgga 5880

gggattcctg ttggtgttat agctgtggag acacagacca tgatgcagct cgtccccgct 5940

gatccaggcc agcctgattc ccacgagcgg tctgttcctc gtgctgggca agtttggttt 6000

ccagattctg ctaccaagac agcgcaggcg atgttggact tcaaccgtga aggattacct 6060

ctgttcatac ttgctaactg gagaggcttc tctggagggc aaagagatct ttttgaagga 6120

attctgcagg ctgggtcaac aattgttgag aaccttagga catacaatca gcctgccttt 6180

gtatatatcc ccaaggctgc agagctacgt ggaggagcct gggtcgtgat tgatagcaag 6240

ataaacccag atcgcatcga gtgctatgct gagaggactg caaagggtaa tgttctcgaa 6300

cctcaagggt tgattgagat caagttcagg tcagaggaac tcaaagaatg catgggtagg 6360

cttgatccag aattgataga tctgaaagca agactccagg gagcaaatgg aagcctatct 6420

gatggagaat cccttcagaa gagcatagaa gctcggaaga aacagttgct gcctctgtac 6480

acccaaatcg cggtacgttt tgcggaattg cacgacactt cccttagaat ggctgctaaa 6540

ggtgtgatca ggaaagttgt agactgggaa gactctcggt ctttcttcta caagagatta 6600

cggaggaggc tatccgagga cgttctggca aaggagatta gaggtgtaat tggtgagaag 6660

tttcctcaca aatcagcgat cgagctgatc aagaaatggt acttggcttc tgaggcagct 6720

gcagcaggaa gcaccgactg ggatgacgac gatgcttttg tcgcctggag ggagaaccct 6780

gaaaactata aggagtatat caaagagctt agggctcaaa gggtatctcg gttgctctca 6840

gatgttgcag gctccagttc ggatttacaa gccttgccgc agggtctttc catgctacta 6900

gataagatgg atccctctaa gagagcacag tttatcgagg aggtcatgaa ggtcctgaaa 6960

tga 6963

<210> 5
<211> 11927
<212> DNA
<213> Oryza sativa

<400> 5
atgacatcca cacatgtggc gacattggga gttggtgccc aggcacctcc tcgtcaccag 60

aaaaagtcag ctggcactgc atttgtatca tctgggtcat caagaccctc ataccgaaag 120

aatggtcagc gtactcggtc acttagggaa gaaagcaatg gaggagtgtc tgattccaaa 180

aagcttaacc actctattcg ccaaggtgac cactagctac tttacatatg ctataatttg 240

tgccaaacat aaacatgcaa tggctgctat tatttaaacg ttaatgttga aatagctgct 300

ataggataca gcaaaaatat ataattgact gggcaagatg caacaattgt ttttcactaa 360

agttagttat cttttgctgt aaaagacaac tgttttttac ataaaatggt attaataacc 420

ttgtaatatt caatgcaaca tgttctcaag taaaaaaaaa cattgcctgg ttgtataagc 480

aaatgtgtcg ttgtagacat cttattaaac ctttttgtga tatctattac cgtagggaac 540

aggggagctg tttaaatctg ttatcataga gtaatatgag aaaagtggat tgtgcgactt 600

tggcatgtat acctgctcaa tttcaaatat atgtctatgt gcaggtcttg ctggcatcat 660

tgacctccca aatgacgcag cttcagaagt tgatatttca cagtaaggac tttatatttt 720

ataataatta ttatataatt ttctgacatg ttttgagaac ctcaaaacat gtgattgcac 780

cttccttttt tatgtctggt tcagaaactg ataagttttg acagtgttta ggatggatct 840

ttgatgcgca cagtgctttc taatgttttc atttttgaaa gtaatgtttt aggaagaaat 900

atctgattaa atttatactt tatctttaca aaagtcaaat gcgttctgta tcaattgcgg 960

tttgtaatat ggcaagaaca tgctttcaga atttgttcat acaatgcttt ctttctatta 1020

ttatgtagaa caaataccta atactttgtt caccttttat agtggacacc tctcacagct 1080

ttttcagtaa gtgatgcaat tttgtacatt tgtaagatgt gttccagaaa ccttttctcc 1140

tgcaattcta atgtacccac tcaaactggt atcaccaaag atctccatct gattgaaaaa 1200

aagctgcgtg aagtatgctt atttatgcta accatacatg atttatactg ttttatagta 1260

caatgcttat ttatgctaac catacataat tttattctgt tttctagtac attatttgtg 1320

cccctgacca taaatgatcc tttcttttac agtggttccg aagatcccag ggggcctacg 1380

gtcccaggtt cctaccaaat gaatgggatt atcaatgaaa cacataatgg gaggcatgct 1440

tcagtctcca aggttgttga gttttgtacg gcacttggtg gcaaaacacc aattcacagt 1500

gtattagtgg ccaacaatgg aatggcagca gctaagttca tgcggagtgt ccgaacatgg 1560

gctaatgata cttttggatc agagaaggca attcagctga tagctatggc aactccggag 1620

gatctgagga taaatgcaga gcacatcaga attgccgatc aatttgtaga ggtacctggt 1680

ggaacaaaca acaacaacta tgcaaatgtc caactcatag tggaggttag ttcagctcat 1740

ccctcaacac aacattttcg tttctattta agttagggaa aaatctctac gaccctccaa 1800

tttctgaaca tccaattttc accatcaact gcaatcacag atagcagaga gaacaggtgt 1860

ttctgctgtt tggcctggtt ggggtcatgc atctgagaat cctgaacttc cagatgcgct 1920

gactgcaaaa ggaattgttt ttcttgggcc accagcatca tcaatgcatg cattaggaga 1980

caaggttggc tcagctctca ttgctcaagc agctggagtt ccaacacttg cttggagtgg 2040

atcacatgtg agccttgtct tctctttttt agcttatcat cttatctttt cggtgatgca 2100

ttatcccaat gacactaaac cataggtgga agttcctctg gagtgttgct tggactcaat 2160

acctgatgag atgtatagaa aagcttgtgt tactaccaca gaggaagcag ttgcaagttg 2220

tcaggtggtt ggttatcctg ccatgattaa ggcatcttgg ggtggtggtg gtaaaggaat 2280

aaggaaggtt tgttcttctt gtagttatca agagattgtt tggattgcaa gtgtttagtg 2340

cccatagtta actctggtct ttctaacatg agtaactcaa ctttcttgca ggttcataat 2400

gatgatgagg ttaggacatt atttaagcaa gttcaaggcg aagtacctgg ttccccaata 2460

tttatcatga ggctagctgc tcaggtgggg ccttttatgg aagttacacc ttttccctta 2520

atgttgagtt attccggagt tattatggtt atgttctgta tgtttgatct gtaaattatt 2580

gaaattcacc tccattggtt ctccagatta gcagacctac aattctacat atggtttata 2640

ctttataaat actaggattt agggatcttc atatagttta tacatggtat ttagatttca 2700

tttgtaaccc tattgaagac atcctgattg ttgtcttatg tagagtcgac atcttgaagt 2760

tcagttgctt tgtgatcaat atggcaacgt agcagcactt cacagtcgag attgcagtgt 2820

acaacggcga caccaaaagg tctgctgtct cagttaaatc acccctctga atgatctact 2880

tcttgcctgc tgcgttggtc agaggaataa tggttgtatt ctactgaaca gataatcgag 2940

gaaggaccag ttactgttgc tcctcgtgag actgtgaaag agcttgagca ggcagcacgg 3000

aggcttgcta aagctgtggg ttatgttggt gctgctactg ttgaatacct ttacagcatg 3060

gaaactggtg aatattattt tctggaactt aatccacggc tacaggtcgg ctcctttgac 3120

attcttcagg aattaatttc tgttgaccac atgatttaca ttgtcaaatg gtctcacagg 3180

ttgagcatcc tgtcactgag tggatagctg aagtaaattt gcctgcggct caagttgctg 3240

ttggaatggg tatacccctt tggcagattc caggtaatgc ttcttcattt agttcctgct 3300

ctttgttaat tgaatgagct cttatacaga ccatgagaca cattctactg ttaattcata 3360

gtatcccctg acttgttagt gttagagata cagagatgta tcacaaattc attgtatctc 3420

ctcaaggact gtaaaaatcc tataattaaa tttctgaaaa tttgttcttt taagcagaaa 3480

aaaaatctct aaattatctc cctgtataca gagatcaggc gcttctacgg aatgaaccat 3540

ggaggaggct atgacctttg gaggaaaaca gcagctctag cgactccatt taactttgat 3600

gaagtagatt ctaaatggcc aaaaggccac tgcgtagctg ttagaataac tagcgaggat 3660

ccagatgatg ggtttaagcc tactggtgga aaagtaaagg tgcggtttcc tgatgttagg 3720

tgtatgaatt gaacacattg ctatattgca gctagtgaaa tgactggatc atggttctct 3780

tattttcagg agataagttt caagagtaaa ccaaatgttt gggcctattt ctcagtaaag 3840

gtagtcctca atattgttgc actgccacat tatttgagtt gtcctaacaa ttgtgctgca 3900

attgttagtt ttcaactatt tgttgttctg tttggttgac tggtaccctc tctttgcagt 3960

ctggtggagg catccatgaa ttcgctgatt ctcagttcgg tatgtaaagt taaaagagta 4020

atattgtctt tgctatttat gtttgtcctc acttttaaaa gatattgcct tccattacag 4080

gacatgtttt tgcgtatgga actactagat cggcagcaat aactaccatg gctcttgcac 4140

taaaagaggt tcaaattcgt ggagaaattc attcaaacgt agactacaca gttgacctat 4200

taaatgtaag gactaaatat ctgcttattg aaccttgctt tttggttccc taatgccatt 4260

ttagtctggc tactgaagaa cttatccatc atgccatttc tgttatctta aattcaggcc 4320

tcagatttta gagaaaataa gattcatact ggttggctgg ataccaggat agccatgcgt 4380

gttcaagctg agaggcctcc atggtatatt tcagtcgttg gaggggcttt atatgtaaga 4440

caaactatgc cactcattag catttatgtg aagcaaatgc ggaaaacatg atcaatatgt 4500

cgtcttattt aaatttattt atttttgtgc tgcagaaaac agtaactgcc aacacggcca 4560

ctgtttctga ttatgttggt tatcttacca agggccagat tccaccaaag gtactattct 4620

gttttttcag gatatgaatg ctgtttgaat gtgaaaacca ttgaccataa atccttgttt 4680

gcagcatata tcccttgtct atacgactgt tgctttgaat atagatggga aaaaatatac 4740

agtaagtgtg acattcttaa tggggaaact taatttgttg taaataatca atatcatatt 4800

gactcgtgta tgctgcatca tagatcgata ctgtgaggag tggacatggt agctacagat 4860

tgcgaatgaa tggatcaacg gttgacgcaa atgtacaaat attatgtgat ggtgggcttt 4920

taatgcaggt aatatcttct tcctagttaa agaagatata tcttgttcaa agaattctga 4980

ttattgatct tttaatgttt tcagctggat ggaaacagcc atgtaattta tgctgaagaa 5040

gaggccagtg gtacacgact tcttattgat ggaaagacat gcatgttaca ggtaatgata 5100

gccttgttct ttttagttct agtcacggtg tttgcttgct atttgttgta tctatttaat 5160

gcattcacta attactatat tagtttgcat catcaagtta aaatggaact tctttcttgc 5220

agaatgacca tgacccatca aagttattag ctgagacacc atgcaaactt cttcgtttct 5280

tggttgctga tggtgctcat gttgatgctg atgtaccata tgcggaagtt gaggttatga 5340

agatgtgcat gcccctctta tcacccgctt ctggtgtcat acatgttgta atgtctgagg 5400

gccaagcaat gcaggtacat tcctacattc cattcattgt gctgtgctga catgaacatt 5460

tcaagtaaat acctgtaact tgtttattat tctaggctgg tgatcttata gctaggctgg 5520

atcttgatga cccttctgct gttaagagag ctgagccgtt cgaagatact tttccacaaa 5580

tgggtctccc tattgctgct tctggccaag ttcacaaatt atgtgctgca agtctgaatg 5640

cttgtcgaat gatccttgcg gggtatgagc atgatattga caaggtaaac atcatgtcct 5700

cttgtttttt cttttgttta tcatgcattc ttatgttcat catgtcctct ggcaaatcta 5760

gattccgctg tcgtttcaca cagatttttc tcattctcat aatggtgcca aacataaata 5820

tgctgctata ttcatcaatg ttttcactcg atttctaatt ttgcttttga gttttaaact 5880

ttagtacaat ccatatctaa tctcctttgg caacagtgaa tccattatat atatttttat 5940

taaactgctt tctttttcag gttgtgccag agttggtata ctgcctagac actccggagc 6000

ttcctttcct gcagtgggag gagcttatgt ctgttttagc aactagactt ccaagaaatc 6060

ttaaaagtga ggtatattat ggttgacaag atagctagtc tcatgctcta aggacttgta 6120

catttcgcca cataggttaa ttttccatat caagttctaa tgtacgatat aaaagtagta 6180

ctggcctaaa acagtattgg tggttgacta tctttgttgt gtaagatcaa gtatttcttt 6240

ttcatgctta gtttgtcaat acttcacatt tatcactgac ttgtcgagct aaatgagatt 6300

ttatttgatt tctgtgctcc attatttttg tatatatata tatatattta actatgacta 6360

tatgttatgc ctcaaacgtt tcaaactctt tcagttggag ggcaaatatg aggaatacaa 6420

agtaaaattt gactctggga taatcaatga tttccctgcc aatatgctac gagtgataat 6480

tgaggtcagt tattcaattt gttgtgataa tcactgcctt aactgttcgt tcttttaaca 6540

agcggtttta taggaaaatc ttgcatgtgg ttctgagaag gagaaggcta caaatgagag 6600

gcttgttgag cctcttatga gcctactgaa gtcatatgag ggtgggagag aaagtcatgc 6660

tcactttgtt gtcaagtccc tttttgagga gtatctctat gttgaagaat tgttcagtga 6720

tggaattcag gttaacttac ctattcgcat taaacaaatc atcagttgtt ttatgataaa 6780

gtcaaaatgt ttatatttcc cattcttctg tggatcaaat atatcacgga catgatatag 6840

tttccttagg ctatataatg gttcttcatc aaataatatt gcaggaaaca gtatagcaaa 6900

ctatttgtat atactcgaga tggaaattgt tagaaacatc attgactaaa tctgtccttt 6960

gttacgctgt ttttgtagtc tgatgtgatt gagcgtctgc gccttcaaca tagtaaagac 7020

ctacagaagg tcgtagacat tgtgttgtcc caccaggtaa atttcttcat ggtctgatga 7080

cttcactgcg aatggttact gaactgtctt cttgttctga caatgtgact tttctttgta 7140

gagtgttaga aataaaacta agctgatact aaaactcatg gagagtctgg tctatccaaa 7200

tcctgctgcc tacagggatc aattgattcg cttttcttcc cttaatcaca aagcgtatta 7260

caaggtgacc aggataaaca taaataaacg tgaatttttc aatgaccttt tcttctgaca 7320

tctgaatctg atgaatttct tgcatattaa tacagttggc acttaaagct agtgaacttc 7380

ttgaacaaac aaaacttagt gagctccgtg caagaatagc aaggagcctt tcagagctgg 7440

agatgtttac tgaggaaagc aagggtctct ccatgcataa gcgagaaatt gccattaagg 7500

agagcatgga agatttagtc actgctccac tgccagttga agatgcgctc atttctttat 7560

ttgattgtag tgatacaact gttcaacaga gagtgattga gacttatata gctcgattat 7620

accaggtatg agaagaaaga ccttttgaaa ttatttatat taacatatcc tagtaaaaca 7680

gcatgctcat catttcttaa aaaaagttta cagcacctga tgtttggtta ctgaccgcat 7740

cattaaaata aagttacttg ttgtggagag atgtattttg gaacttgtgg cacatgcagt 7800

aacatgctac tgctcgatat gtttgctaac ttgacaacaa tatttttcag cctcatcttg 7860

taaaggacag tatcaaaatg aaatggatag aatcgggtgt tattgcttta tgggaatttc 7920

ctgaagggca ttttgatgca agaaatggag gagcggttct tggtgacaaa agatggggtg 7980

ccatggtcat tgtcaagtct cttgaatcac tttcaatggc cattagattt gcactaaagg 8040

agacatcaca ctacactagc tctgagggca atatgatgca tattgctttg ttgggtgctg 8100

ataataagat gcatataatt caagaaaggt atgttcatat gctatgttgg tgctgaaata 8160

gttatatatg tagttagctg gtggagttct ggtaattaac ctatcccatt gttcagtggt 8220

gatgatgctg acagaatagc caaacttccc ttgatactaa aggataatgt aaccgatctg 8280

catgcctctg gtgtgaaaac aataagtttc attgttcaaa gagatgaagc acggatgaca 8340

atgcgtcgta ccttcctttg gtctgatgaa aagctttctt atgaggaaga gccaattctc 8400

cggcatgtgg aacctcctct ttctgcactt cttgagttgg tacgtgatat catcaaaatg 8460

ataatgtttt ggtatggcat tgattatctt ctatgctctt tgtatttatt cagcctattg 8520

tggatacagg acaagttgaa agtgaaagga tacaatgaaa tgaagtatac cccatcacgg 8580

gatcgtcaat ggcatatcta cacacttaga aatactgaaa accccaaaat gttgcaccgg 8640

gtatttttcc gaacccttgt caggcaaccc agtgtatcca acaagttttc ttcgggccag 8700

attggtgaca tggaagttgg gagtgctgaa gaacctctgt catttacatc aaccagcata 8760

ttaagatctt tgatgactgc tatagaggaa ttggagcttc acgcaattag aactggccat 8820

tcacacatgt atttgcatgt attgaaagaa caaaagcttc ttgatcttgt tccagtttca 8880

gggtaagtgc gcatatttct ttttgggaac atatgcttgc ttatgaggtt ggtcttctca 8940

atgatcttct tatcttactc aggaatacag ttttggatgt tggtcaagat gaagctactg 9000

catattcact tttaaaagaa atggctatga agatacatga acttgttggt gcaagaatgc 9060

accatctttc tgtatgccaa tgggaagtga aacttaagtt ggactgcgat ggtcctgcca 9120

gtggtacctg gaggattgta acaaccaatg ttactagtca cacttgcact gtggatgtaa 9180

gtttaatcct ctagcatttt gttttctttg gaaaagcatg tgattttaag ccggctggtc 9240

ctcataccca gacctagtga tctttatata gtgtagacat ttttctaact gcttttaatt 9300

gttttagatc taccgtgaga tggaagataa agaatcacgg aagttagtat accatcccgc 9360

cactccggcg gctggtcctc tgcatggtgt ggcactgaat aatccatatc agcctttgag 9420

tgtcattgat ctcaaacgct gttctgctag gaataataga actacatact gctatgattt 9480

tccactggtg agttgactgc tcccttatat tcaatgcatt accatagcaa attcatattc 9540

gttcatgttg tcaaaataag ccgatgaaaa ttcaaaactg taggcatttg aaactgcagt 9600

gaggaagtca tggtcctcta gtacctctgg tgcttctaaa ggtgttgaaa atgcccaatg 9660

ttatgttaaa gctacagagt tggtatttgc ggacaaacat gggtcatggg gcactccttt 9720

agttcaaatg gaccggcctg ctgggctcaa tgacattggt atggtagctt ggaccttgaa 9780

gatgtccact cctgaatttc ctagtggtag ggagattatt gttgttgcaa atgatattac 9840

gttcagagct ggatcatttg gcccaaggga agatgcattt tttgaagctg ttaccaacct 9900

agcctgtgag aagaaacttc ctcttattta tttggcagca aattctggtg ctcgaattgg 9960

catagcagat gaagtgaaat cttgcttccg tgttgggtgg tctgatgatg gcagccctga 10020

acgtgggttt cagtacattt atctaagcga agaagactat gctcgtattg gcacttctgt 10080

catagcacat aagatgcagc tagacagtgg tgaaattagg tgggttattg attctgttgt 10140

gggcaaggaa gatggacttg gtgtggagaa tatacatgga agtgctgcta ttgccagtgc 10200

ttattctagg gcatataagg agacatttac acttacattt gtgactggaa gaactgttgg 10260

aataggagct tatcttgctc gacttggcat ccggtgcata cagcgtcttg accagcctat 10320

tattcttaca ggctattctg cactgaacaa gcttcttggg cgggaagtgt acagctccca 10380

catgcagttg ggtggtccca aaatcatggc aactaatggt gttgtccatc ttactgtttc 10440

agatgacctt gaaggcgttt ctaatatatt gaggtggctc agttatgttc ctgcctacat 10500

tggtggacca cttccagtaa caacaccgtt ggacccaccg gacagacctg ttgcatacat 10560

tcctgagaac tcgtgtgatc ctcgagcggc tatccgtggt gttgatgaca gccaagggaa 10620

atggttaggt ggtatgtttg ataaagacag ctttgtggaa acatttgaag gttgggctaa 10680

gacagtggtt actggcagag caaagcttgg tggaattcca gtgggtgtga tagctgtgga 10740

gactcagacc atgatgcaaa ctatccctgc tgaccctggt cagcttgatt cccgtgagca 10800

atctgttcct cgtgctggac aagtgtggtt tccagattct gcaaccaaga ctgcgcaggc 10860

attgctggac ttcaaccgtg aaggattacc tctgttcatc ctcgctaact ggagaggctt 10920

ctctggtgga caaagagatc tttttgaagg aattcttcag gctggctcga ctattgttga 10980

gaaccttagg acatacaatc agcctgcctt tgtctacatt cccatggctg cagagctacg 11040

aggaggggct tgggttgtgg ttgatagcaa gataaaccca gaccgcattg agtgctatgc 11100

tgagaggact gcaaaaggca atgttctgga accgcaaggg ttaattgaga tcaagttcag 11160

gtcagaggaa ctccaggatt gcatgagtcg gcttgaccca acattaattg atctgaaagc 11220

aaaactcgaa gtagcaaata aaaatggaag tgctgacaca aaatcgcttc aagaaaatat 11280

agaagctcga acaaaacagt tgatgcctct atatactcag attgcgatac ggtttgctga 11340

attgcatgat acatccctca gaatggctgc gaaaggtgtg attaagaaag ttgtggactg 11400

ggaagaatca cgatctttct tctataagag attacggagg aggatctctg aggatgttct 11460

tgcaaaagaa attagagctg tagcaggtga gcagttttcc caccaaccag caatcgagct 11520

gatcaagaaa tggtattcag cttcacatgc agctgaatgg gatgatgacg atgcttttgt 11580

tgcttggatg gataaccctg aaaactacaa ggattatatt caatatctta aggctcaaag 11640

agtatcccaa tccctctcaa gtctttcaga ttccagctca gatttgcaag ccctgccaca 11700

gggtctttcc atgttactag ataaggtaat tagcttactg atgcttatat aaattctttt 11760

tcattacata tggctggaga actatctaat caaataatga ttataattcc aatcgttctt 11820

tttatgccat tatgatcttc tgaaatttcc ttctttggac acttattcag atggatccct 11880

ctagaagagc tcaacttgtt gaagaaatca ggaaggtcct tggttga 11927

<210> 6
<211> 6984
<212> DNA
<213> Oryza sativa

<400> 6
atgacatcca cacatgtggc gacattggga gttggtgccc aggcacctcc tcgtcaccag 60

aaaaagtcag ctggcactgc atttgtatca tctgggtcat caagaccctc ataccgaaag 120

aatggtcagc gtactcggtc acttagggaa gaaagcaatg gaggagtgtc tgattccaaa 180

aagcttaacc actctattcg ccaaggtctt gctggcatca ttgacctccc aaatgacgca 240

gcttcagaag ttgatatttc acatggttcc gaagatccca gggggcctac ggtcccaggt 300

tcctaccaaa tgaatgggat tatcaatgaa acacataatg ggaggcatgc ttcagtctcc 360

aaggttgttg agttttgtac ggcacttggt ggcaaaacac caattcacag tgtattagtg 420

gccaacaatg gaatggcagc agctaagttc atgcggagtg tccgaacatg ggctaatgat 480

acttttggat cagagaaggc aattcagctg atagctatgg caactccgga ggatctgagg 540

ataaatgcag agcacatcag aattgccgat caatttgtag aggtacctgg tggaacaaac 600

aacaacaact atgcaaatgt ccaactcata gtggagatag cagagagaac aggtgtttct 660

gctgtttggc ctggttgggg tcatgcatct gagaatcctg aacttccaga tgcgctgact 720

gcaaaaggaa ttgtttttct tgggccacca gcatcatcaa tgcatgcatt aggagacaag 780

gttggctcag ctctcattgc tcaagcagct ggagttccaa cacttgcttg gagtggatca 840

catgtggaag ttcctctgga gtgttgcttg gactcaatac ctgatgagat gtatagaaaa 900

gcttgtgtta ctaccacaga ggaagcagtt gcaagttgtc aggtggttgg ttatcctgcc 960

atgattaagg catcttgggg tggtggtggt aaaggaataa ggaaggttca taatgatgat 1020

gaggttagga cattatttaa gcaagttcaa ggcgaagtac ctggttcccc aatatttatc 1080

atgaggctag ctgctcagag tcgacatctt gaagttcagt tgctttgtga tcaatatggc 1140

aacgtagcag cacttcacag tcgagattgc agtgtacaac ggcgacacca aaagataatc 1200

gaggaaggac cagttactgt tgctcctcgt gagactgtga aagagcttga gcaggcagca 1260

cggaggcttg ctaaagctgt gggttatgtt ggtgctgcta ctgttgaata cctttacagc 1320

atggaaactg gtgaatatta ttttctggaa cttaatccac ggctacaggt tgagcatcct 1380

gtcactgagt ggatagctga agtaaatttg cctgcggctc aagttgctgt tggaatgggt 1440

ataccccttt ggcagattcc agagatcagg cgcttctacg gaatgaacca tggaggaggc 1500

tatgaccttt ggaggaaaac agcagctcta gcgactccat ttaactttga tgaagtagat 1560

tctaaatggc caaaaggcca ctgcgtagct gttagaataa ctagcgagga tccagatgat 1620

gggtttaagc ctactggtgg aaaagtaaag gagataagtt tcaagagtaa accaaatgtt 1680

tgggcctatt tctcagtaaa gtctggtgga ggcatccatg aattcgctga ttctcagttc 1740

ggacatgttt ttgcgtatgg aactactaga tcggcagcaa taactaccat ggctcttgca 1800

ctaaaagagg ttcaaattcg tggagaaatt cattcaaacg tagactacac agttgaccta 1860

ttaaatgcct cagattttag agaaaataag attcatactg gttggctgga taccaggata 1920

gccatgcgtg ttcaagctga gaggcctcca tggtatattt cagtcgttgg aggggcttta 1980

tataaaacag taactgccaa cacggccact gtttctgatt atgttggtta tcttaccaag 2040

ggccagattc caccaaagca tatatccctt gtctatacga ctgttgcttt gaatatagat 2100

gggaaaaaat atacaatcga tactgtgagg agtggacatg gtagctacag attgcgaatg 2160

aatggatcaa cggttgacgc aaatgtacaa atattatgtg atggtgggct tttaatgcag 2220

ctggatggaa acagccatgt aatttatgct gaagaagagg ccagtggtac acgacttctt 2280

attgatggaa agacatgcat gttacagaat gaccatgacc catcaaagtt attagctgag 2340

acaccatgca aacttcttcg tttcttggtt gctgatggtg ctcatgttga tgctgatgta 2400

ccatatgcgg aagttgaggt tatgaagatg tgcatgcccc tcttatcacc cgcttctggt 2460

gtcatacatg ttgtaatgtc tgagggccaa gcaatgcagg ctggtgatct tatagctagg 2520

ctggatcttg atgacccttc tgctgttaag agagctgagc cgttcgaaga tacttttcca 2580

caaatgggtc tccctattgc tgcttctggc caagttcaca aattatgtgc tgcaagtctg 2640

aatgcttgtc gaatgatcct tgcggggtat gagcatgata ttgacaaggt tgtgccagag 2700

ttggtatact gcctagacac tccggagctt cctttcctgc agtgggagga gcttatgtct 2760

gttttagcaa ctagacttcc aagaaatctt aaaagtgagt tggagggcaa atatgaggaa 2820

tacaaagtaa aatttgactc tgggataatc aatgatttcc ctgccaatat gctacgagtg 2880

ataattgagg aaaatcttgc atgtggttct gagaaggaga aggctacaaa tgagaggctt 2940

gttgagcctc ttatgagcct actgaagtca tatgagggtg ggagagaaag tcatgctcac 3000

tttgttgtca agtccctttt tgaggagtat ctctatgttg aagaattgtt cagtgatgga 3060

attcagtctg atgtgattga gcgtctgcgc cttcaacata gtaaagacct acagaaggtc 3120

gtagacattg tgttgtccca ccagagtgtt agaaataaaa ctaagctgat actaaaactc 3180

atggagagtc tggtctatcc aaatcctgct gcctacaggg atcaattgat tcgcttttct 3240

tcccttaatc acaaagcgta ttacaagttg gcacttaaag ctagtgaact tcttgaacaa 3300

acaaaactta gtgagctccg tgcaagaata gcaaggagcc tttcagagct ggagatgttt 3360

actgaggaaa gcaagggtct ctccatgcat aagcgagaaa ttgccattaa ggagagcatg 3420

gaagatttag tcactgctcc actgccagtt gaagatgcgc tcatttcttt atttgattgt 3480

agtgatacaa ctgttcaaca gagagtgatt gagacttata tagctcgatt ataccagcct 3540

catcttgtaa aggacagtat caaaatgaaa tggatagaat cgggtgttat tgctttatgg 3600

gaatttcctg aagggcattt tgatgcaaga aatggaggag cggttcttgg tgacaaaaga 3660

tggggtgcca tggtcattgt caagtctctt gaatcacttt caatggccat tagatttgca 3720

ctaaaggaga catcacacta cactagctct gagggcaata tgatgcatat tgctttgttg 3780

ggtgctgata ataagatgca tataattcaa gaaagtggtg atgatgctga cagaatagcc 3840

aaacttccct tgatactaaa ggataatgta accgatctgc atgcctctgg tgtgaaaaca 3900

ataagtttca ttgttcaaag agatgaagca cggatgacaa tgcgtcgtac cttcctttgg 3960

tctgatgaaa agctttctta tgaggaagag ccaattctcc ggcatgtgga acctcctctt 4020

tctgcacttc ttgagttgga caagttgaaa gtgaaaggat acaatgaaat gaagtatacc 4080

ccatcacggg atcgtcaatg gcatatctac acacttagaa atactgaaaa ccccaaaatg 4140

ttgcaccggg tatttttccg aacccttgtc aggcaaccca gtgtatccaa caagttttct 4200

tcgggccaga ttggtgacat ggaagttggg agtgctgaag aacctctgtc atttacatca 4260

accagcatat taagatcttt gatgactgct atagaggaat tggagcttca cgcaattaga 4320

actggccatt cacacatgta tttgcatgta ttgaaagaac aaaagcttct tgatcttgtt 4380

ccagtttcag ggaatacagt tttggatgtt ggtcaagatg aagctactgc atattcactt 4440

ttaaaagaaa tggctatgaa gatacatgaa cttgttggtg caagaatgca ccatctttct 4500

gtatgccaat gggaagtgaa acttaagttg gactgcgatg gtcctgccag tggtacctgg 4560

aggattgtaa caaccaatgt tactagtcac acttgcactg tggatatcta ccgtgagatg 4620

gaagataaag aatcacggaa gttagtatac catcccgcca ctccggcggc tggtcctctg 4680

catggtgtgg cactgaataa tccatatcag cctttgagtg tcattgatct caaacgctgt 4740

tctgctagga ataatagaac tacatactgc tatgattttc cactggcatt tgaaactgca 4800

gtgaggaagt catggtcctc tagtacctct ggtgcttcta aaggtgttga aaatgcccaa 4860

tgttatgtta aagctacaga gttggtattt gcggacaaac atgggtcatg gggcactcct 4920

ttagttcaaa tggaccggcc tgctgggctc aatgacattg gtatggtagc ttggaccttg 4980

aagatgtcca ctcctgaatt tcctagtggt agggagatta ttgttgttgc aaatgatatt 5040

acgttcagag ctggatcatt tggcccaagg gaagatgcat tttttgaagc tgttaccaac 5100

ctagcctgtg agaagaaact tcctcttatt tatttggcag caaattctgg tgctcgaatt 5160

ggcatagcag atgaagtgaa atcttgcttc cgtgttgggt ggtctgatga tggcagccct 5220

gaacgtgggt ttcagtacat ttatctaagc gaagaagact atgctcgtat tggcacttct 5280

gtcatagcac ataagatgca gctagacagt ggtgaaatta ggtgggttat tgattctgtt 5340

gtgggcaagg aagatggact tggtgtggag aatatacatg gaagtgctgc tattgccagt 5400

gcttattcta gggcatataa ggagacattt acacttacat ttgtgactgg aagaactgtt 5460

ggaataggag cttatcttgc tcgacttggc atccggtgca tacagcgtct tgaccagcct 5520

attattctta caggctattc tgcactgaac aagcttcttg ggcgggaagt gtacagctcc 5580

cacatgcagt tgggtggtcc caaaatcatg gcaactaatg gtgttgtcca tcttactgtt 5640

tcagatgacc ttgaaggcgt ttctaatata ttgaggtggc tcagttatgt tcctgcctac 5700

attggtggac cacttccagt aacaacaccg ttggacccac cggacagacc tgttgcatac 5760

attcctgaga actcgtgtga tcctcgagcg gctatccgtg gtgttgatga cagccaaggg 5820

aaatggttag gtggtatgtt tgataaagac agctttgtgg aaacatttga aggttgggct 5880

aagacagtgg ttactggcag agcaaagctt ggtggaattc cagtgggtgt gatagctgtg 5940

gagactcaga ccatgatgca aactatccct gctgaccctg gtcagcttga ttcccgtgag 6000

caatctgttc ctcgtgctgg acaagtgtgg tttccagatt ctgcaaccaa gactgcgcag 6060

gcattgctgg acttcaaccg tgaaggatta cctctgttca tcctcgctaa ctggagaggc 6120

ttctctggtg gacaaagaga tctttttgaa ggaattcttc aggctggctc gactattgtt 6180

gagaacctta ggacatacaa tcagcctgcc tttgtctaca ttcccatggc tgcagagcta 6240

cgaggagggg cttgggttgt ggttgatagc aagataaacc cagaccgcat tgagtgctat 6300

gctgagagga ctgcaaaagg caatgttctg gaaccgcaag ggttaattga gatcaagttc 6360

aggtcagagg aactccagga ttgcatgagt cggcttgacc caacattaat tgatctgaaa 6420

gcaaaactcg aagtagcaaa taaaaatgga agtgctgaca caaaatcgct tcaagaaaat 6480

atagaagctc gaacaaaaca gttgatgcct ctatatactc agattgcgat acggtttgct 6540

gaattgcatg atacatccct cagaatggct gcgaaaggtg tgattaagaa agttgtggac 6600

tgggaagaat cacgatcttt cttctataag agattacgga ggaggatctc tgaggatgtt 6660

cttgcaaaag aaattagagc tgtagcaggt gagcagtttt cccaccaacc agcaatcgag 6720

ctgatcaaga aatggtattc agcttcacat gcagctgaat gggatgatga cgatgctttt 6780

gttgcttgga tggataaccc tgaaaactac aaggattata ttcaatatct taaggctcaa 6840

agagtatccc aatccctctc aagtctttca gattccagct cagatttgca agccctgcca 6900

cagggtcttt ccatgttact agataagatg gatccctcta gaagagctca acttgttgaa 6960

gaaatcagga aggtccttgg ttga 6984

<210> 7
<211> 25
<212> DNA
<213> Artificial Sequence

<220>
<221> source
<223> /note="Description of Artificial Sequence: Synthetic
      primer"

<400> 7
gcaaatgata ttacgttcag agctg 25

<210> 8
<211> 24
<212> DNA
<213> Artificial Sequence

<220>
<221> source
<223> /note="Description of Artificial Sequence: Synthetic
      primer"

<400> 8
gttaccaacc tagcctgtga gaag 24

<210> 9
<211> 26
<212> DNA
<213> Artificial Sequence

<220>
<221> source
<223> /note="Description of Artificial Sequence: Synthetic
      primer"

<400> 9
gatttcttca acaagttgag ctcttc                                          26


<210> 10
<211> 24
<212> DNA
<213> Artificial Sequence

<220>
<221> source
<223> /note="Description of Artificial Sequence: Synthetic
      primer"

<400> 10
agtaacatgg aaagaccctg tggc                                            24


<210> 11
<211> 6978
<212> DNA
<213> Zea mays

<400> 11
atgtcacagc ttggattagc cgcagctgcc tcaaaggcct tgccactact ccctaatcgc      60

cagagaagtt cagctgggac tacattctca tcatcttcat tatcgaggcc cttaaacaga     120

aggaaaagcc gtactcgttc actccgtgat ggcggagatg gggtatcaga tgccaaaaag     180

cacagccagt ctgttcgtca aggtcttgct ggcattatcg acctcccaag tgaggcacct     240

tccgaagtgg atatttcaca tggatctgag gatcctaggg ggccaacaga ttcttatcaa     300

atgaatggga ttatcaatga aacacataat ggaagacatg cctcagtgtc caaggttgtt     360

gaattttgtg cggcactagg tggcaaaaca ccaattcaca gtatattagt ggccaacaat     420

ggaatggcag cagcaaaatt tatgaggagt gtccggacat gggctaatga tacttttgga     480

tctgagaagg caattcaact catagctatg gcaactccgg aagacatgag gataaatgca     540

gaacacatta gaattgctga ccaattcgta gaggtgcctg gtggaacaaa caataataac     600

tacgccaatg ttcaactcat agtggagatg gcacaaaaac taggtgtttc tgctgtttgg     660

cctggttggg gtcatgcttc tgagaatcct gaactgccag atgcattgac cgcaaaaggg     720

atcgtttttc ttggcccacc tgcatcatca atgaatgctt tgggagataa ggtcggctca     780

gctctcattg ctcaagcagc cggggtccca actcttgctc ggagtggatc acatgttgaa     840

gttccattag agtgctgctt agacgcgata cctgaggaga tgtatagaaa agcttgcgtt     900

actaccacag aggaagcagt tgcaagttgt caagtggttg gttatcctgc catgattaag 960

gcatcctggg gaggtggtgg taaaggaata agaaaggttc ataatgatga tgaggttaga 1020

gcgctgttta agcaagtaca aggtgaagtc cctggctccc caatatttgt catgaggctt 1080

gcatcccaga gtcggcatct tgaagttcag ttgctttgtg atcaatatgg taatgtagca 1140

gcacttcaca gtcgtgattg cagtgtgcaa cggcgacacc agaagattat tgaagaaggt 1200

ccagttactg ttgctcctcg tgagacagtt aaagcacttg agcaggcagc aaggaggctt 1260

gctaaggctg tgggttatgt tggtgctgct actgttgagt atctttacag catggaaact 1320

ggagactact attttctgga acttaatccc cgactacagg ttgagcatcc agtcaccgag 1380

tggatagctg aagtaaatct gcctgcagct caagttgctg ttggaatggg catacctctt 1440

tggcagattc cagaaatcag acgtttctat ggaatggact atggaggagg gtatgacatt 1500

tggaggaaaa cagcagctct tgctacacca tttaattttg atgaagtaga ttctcaatgg 1560

ccaaagggcc attgtgtagc agttagaatt actagtgagg acccagatga tggtttcaaa 1620

cctactggtg ggaaagtgaa ggagataagt tttaaaagca agcctaatgt ttgggcctac 1680

ttctcagtaa agtctggtgg aggcattcat gaatttgctg attctcagtt cggacatgtt 1740

tttgcatatg ggctctctag atcagcagca ataacaaaca tgactcttgc attaaaagag 1800

attcaaattc gtggagaaat tcattcaaat gttgattaca cagttgacct cttaaatgct 1860

tcagacttta gagaaaacaa gattcatact ggttggctcg acaccagaat agctatgcgt 1920

gttcaagctg agaggccccc atggtatatt tcagtggttg gaggtgcttt atataaaaca 1980

gtaaccacca atgcagccac tgtttctgaa tatgttagtt atctcaccaa gggccagatt 2040

ccaccaaagc atatatccct tgtcaattct acagttaatt tgaatataga agggagcaaa 2100

tacacaattg aaactgtaag gactggacat ggtagctaca ggttgagaat gaatgattca 2160

acagttgaag cgaatgtaca atctttatgt gatggtggcc tcttaatgca gttggatgga 2220

aacagccatg taatttatgc agaagaagaa gctggtggta cacggcttca gattgatgga 2280

aagacatgtt tattgcagaa tgaccatgat ccatcaaagt tattagctga gacaccctgc 2340

aaacttcttc gtttcttggt tgctgatggt gctcatgttg atgcggatgt accatacgcg 2400

gaagttgagg ttatgaagat gtgcatgcct ctcttgtcac ctgcttctgg tgtcattcat 2460

tgtatgatgt ctgagggcca ggcattgcag gctggtgatc ttatagcaag gttggatctt 2520

gatgaccctt ctgctgtgaa aagagctgag ccatttgatg gaatatttcc acaaatggag 2580

ctccctgttg ctgtctctag tcaagtacac aaaagatatg ctgcaagttt gaatgctgct 2640

cgaatggtcc ttgcaggata tgagcacaat attaatgaag tcgttcaaga tttggtatgc 2700

tgcctggaca accctgagct tcctttccta cagtgggatg aacttatgtc tgttctagca 2760

acgaggcttc caagaaatct caagagtgag ttagaggata aatacaagga atacaagttg 2820

aatttttacc atggaaaaaa cgaggacttt ccatccaagt tgctaagaga catcattgag 2880

gaaaatcttt cttatggttc agagaaggaa aaggctacaa atgagaggct tgttgagcct 2940

cttatgaacc tactgaagtc atatgagggt gggagagaga gccatgcaca ttttgttgtc 3000

aagtctcttt tcgaggagta tcttacagtg gaagaacttt ttagtgatgg cattcagtct 3060

gacgtgattg aaacattgcg gcatcagcac agtaaagacc tgcagaaggt tgtagacatt 3120

gtgttgtctc accagggtgt gaggaacaaa gctaagcttg taacggcact tatggaaaag 3180

ctggtttatc caaatcctgg tggttacagg gatctgttag ttcgcttttc ttccctcaat 3240

cataaaagat attataagtt ggcccttaaa gcaagtgaac ttcttgaaca aaccaaacta 3300

agtgaactcc gtgcaagcgt tgcaagaagc ctttcggatc tggggatgca taagggagaa 3360

atgagtatta aggataacat ggaagattta gtctctgccc cattacctgt tgaagatgct 3420

ctgatttctt tgtttgatta cagtgatcga actgttcagc agaaagtgat tgagacatac 3480

atatcacgat tgtaccagcc tcatcttgta aaggatagca tccaaatgaa attcaaggaa 3540

tctggtgcta ttacttttgg ggaattttat gaagggcatg ttgatactag aaatggacat 3600

ggggctatta ttggtgggaa gcgatggggt gccatggtcg ttctcaaatc acttgaatct 3660

gcgtcaacag ccattgtggc tgcattaaag gattcggcac agttcaacag ctctgagggc 3720

aacatgatgc acattgcatt attgagtgct gaaaatgaaa gtaatataag tggaataagc 3780

agtgatgatc aagctcaaca taagatggaa aagcttagca agatactgaa ggatactagc 3840

gttgcaagtg atctccaagc tgctggtttg aaggttataa gttgcattgt tcaaagagat 3900

gaagctcgca tgccaatgcg ccacacattc ctctggttgg atgacaagag ttgttatgaa 3960

gaagagcaga ttctccggca tgtggagcct cccctctcta cacttcttga attggataag 4020

ttgaaggtga aaggatacaa tgaaatgaag tatactcctt cgcgtgaccg ccaatggcat 4080

atctacacac taagaaatac tgaaaacccc aaaatgttgc atagggtgtt tttccgaact 4140

attgtcaggc aacccaatgc aggcaacaag tttacatcgg ctcagatcag cgacgctgaa 4200

gtaggatgtc ccgaagaatc tctttcattt acatcaaata gcatcttaag atcattgatg 4260

actgctattg aagaattaga gcttcatgca attaggacag gtcattctca catgtatttg 4320

tgcatactga aagagcaaaa gcttcttgac ctcattccat tttcagggag tacaattgtt 4380

gatgttggcc aagatgaagc taccgcttgt tcacttttaa aatcaatggc tttgaagata 4440

catgagcttg ttggtgcaag gatgcatcat ctgtctgtat gccagtggga ggtgaaactc 4500

aagttggact gtgatggccc tgcaagtggt acctggagag ttgtaactac aaatgttact 4560

ggtcacacct gcaccattga tatataccga gaagtggagg aaatagaatc gcagaagtta 4620

gtgtaccatt cagccacttc gtcagctgga ccattgcatg gtgttgcact gaataatcca 4680

tatcaacctt tgagtgtgat tgatctaaag cgctgctctg ctaggaacaa cagaacaaca 4740

tattgctatg attttccgct ggcctttgaa actgcactgc agaagtcatg gcagtccaat 4800

ggctctactg tttctgaagg caatgaaaat agtaaatcct acgtgaaggc aactgagcta 4860

gtgtttgctg aaaaacatgg gtcctggggc actcctataa ttccgatgga acgccctgct 4920

gggctcaacg acattggtat ggtcgcttgg atcatggaga tgtcaacacc tgaatttccc 4980

aatggcaggc agattattgt tgtagcaaat gatatcactt tcagagctgg atcatttggc 5040

ccaagggaag atgcattttt tgaaactgtc actaacctgg cttgcgaaag gaaacttcct 5100

cttatatact tggcagcaaa ctctggtgct aggattggca tagctgatga agtaaaatct 5160

tgcttccgtg ttggatggtc tgacgaaggc agtcctgaac gagggtttca gtacatctat 5220

ctgactgaag aagactatgc tcgcattagc tcttctgtta tagcacataa gctggagcta 5280

gatagtggtg aaattaggtg gattattgac tctgttgtgg gcaaggagga tgggcttggt 5340

gtcgagaaca tacatggaag tgctgctatt gccagtgctt attctagggc atatgaggag 5400

acatttacac ttacatttgt gactgggcgg actgtaggaa taggagctta tcttgctcga 5460

cttggtatac ggtgcataca gcgtcttgac cagcctatta ttttaacagg gttttctgcc 5520

ctgaacaagc tccttgggcg ggaagtgtac agctcccaca tgcagcttgg tggtcctaag 5580

atcatggcga ctaatggtgt tgtccacctc actgttccag atgaccttga aggtgtttcc 5640

aatatattga ggtggctcag ctatgttcct gcaaacattg gtggacctct tcctattacc 5700

aaacctctgg accctccaga cagacctgtt gcttacatcc ctgagaacac atgcgatcca 5760

cgtgcagcta tctgtggtgt agatgacagc caaggaaaat ggttgggtgg tatgtttgac 5820

aaagacagct ttgtggagac atttgaagga tgggcaaaaa cagtggttac tggcagagca 5880

aagcttggag gaattcctgt gggcgtcata gctgtggaga cacagaccat gatgcagatc 5940

atccctgctg atccaggtca gcttgattcc catgagcgat ctgtccctcg tgctggacaa 6000

gtgtggttcc cagattctgc aaccaagacc gctcaggcat tattagactt caaccgtgaa 6060

ggattgcctc tgttcatcct ggctaattgg agaggcttct ctggtggaca aagagatctc 6120

tttgaaggaa ttcttcaggc tgggtcaaca attgtcgaga accttaggac atctaatcag 6180

cctgcttttg tgtacattcc tatggctgga gagcttcgtg gaggagcttg ggttgtggtc 6240

gatagcaaaa taaatccaga ccgcattgag tgttatgctg aaaggactgc caaaggtaat 6300

gttctcgaac ctcaagggtt aattgaaatc aagttcaggt cagaggaact ccaagactgt 6360

atgggtaggc ttgacccaga gttgataaat ctgaaagcaa aactccaaga tgtaaatcat 6420

ggaaatggaa gtctaccaga catagaaggg attcggaaga gtatagaagc acgtacgaaa 6480

cagttgctgc ctttatatac ccagattgca atacggtttg ctgaattgca tgatacttcc 6540

ctaagaatgg cagctaaagg tgtgattaag aaagttgtag actgggaaga atcacgctcg 6600

ttcttctata aaaggctacg gaggaggatc gcagaagatg ttcttgcaaa agaaataagg 6660

cagatagtcg gtgataaatt tacgcaccaa ttagcaatgg agctcatcaa ggaatggtac 6720

cttgcttctc aggccacaac aggaagcact ggatgggatg acgatgatgc ttttgttgcc 6780

tggaaggaca gtcctgaaaa ctacaagggg catatccaaa agcttagggc tcaaaaagtg 6840

tctcattcgc tctctgatct tgctgactcc agttcagatc tgcaagcatt ctcgcagggt 6900

ctttctacgc tattagataa gatggatccc tctcagagag cgaagtttgt tcaggaagtc 6960

aagaaggtcc ttgattga 6978

<210> 12
<211> 2325
<212> PRT
<213> Zea mays

<400> 12
Met Ser Gln Leu Gly Leu Ala Ala Ala Ala Ser Lys Ala Leu Pro Leu
1               5                   10                  15

Leu Pro Asn Arg Gln Arg Ser Ser Ala Gly Thr Thr Phe Ser Ser Ser
            20                  25                  30

Ser Leu Ser Arg Pro Leu Asn Arg Arg Lys Ser Arg Thr Arg Ser Leu
        35                  40                  45

Arg Asp Gly Gly Asp Gly Val Ser Asp Ala Lys Lys His Ser Gln Ser
    50                  55                  60

Val Arg Gln Gly Leu Ala Gly Ile Ile Asp Leu Pro Ser Glu Ala Pro
65                  70                  75                  80

Ser Glu Val Asp Ile Ser His Gly Ser Glu Asp Pro Arg Gly Pro Thr
                85                  90                  95

Asp Ser Tyr Gln Met Asn Gly Ile Ile Asn Glu Thr His Asn Gly Arg
            100                 105                 110

His Ala Ser Val Ser Lys Val Val Glu Phe Cys Ala Ala Leu Gly Gly
        115                 120                 125

Lys Thr Pro Ile His Ser Ile Leu Val Ala Asn Asn Gly Met Ala Ala
    130                 135                 140

Ala Lys Phe Met Arg Ser Val Arg Thr Trp Ala Asn Asp Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Met Ala Gln Lys Leu Gly Val Ser Ala Val Trp Pro Gly Trp Gly
    210                 215                 220

His Ala Ser Glu Asn Pro Glu Leu Pro Asp Ala Leu Thr Ala Lys Gly
225                 230                 235                 240

Ile Val Phe Leu Gly Pro Pro Ala Ser Ser Met Asn Ala Leu Gly Asp
                245                 250                 255

Lys Val Gly Ser Ala Leu Ile Ala Gln Ala Ala Gly Val Pro Thr Leu
            260                 265                 270

Ala Arg Ser Gly Ser His Val Glu Val Pro Leu Glu Cys Cys Leu Asp
        275                 280                 285

Ala Ile Pro Glu Glu Met Tyr Arg Lys Ala Cys Val Thr Thr Thr Glu
    290                 295                 300

Glu Ala Val Ala Ser Cys Gln Val Val Gly Tyr Pro Ala Met Ile Lys
305                 310                 315                 320

Ala Ser Trp Gly Gly Gly Gly Lys Gly Ile Arg Lys Val His Asn Asp
                325                 330                 335

Asp Glu Val Arg Ala Leu Phe Lys Gln Val Gln Gly Glu Val Pro Gly
            340                 345                 350

Ser Pro Ile Phe Val Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Ala Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Asp Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Ile Pro Glu Ile Arg Arg Phe Tyr Gly Met Asp Tyr Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Leu Ser Arg Ser Ala Ala Ile Thr
            580                 585                 590

Asn Met Thr Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Arg
    610                 615                 620

Glu Asn Lys Ile His Thr Gly Trp Leu Asp Thr Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Val Thr Thr Asn Ala Ala Thr Val Ser Glu Tyr Val
            660                 665                 670

Ser Tyr Leu Thr Lys Gly Gln Ile Pro Pro Lys His Ile Ser Leu Val
        675                 680                 685

Asn Ser Thr Val Asn Leu Asn Ile Glu Gly Ser Lys Tyr Thr Ile Glu
    690                 695                 700

Thr Val Arg Thr Gly His Gly Ser Tyr Arg Leu Arg Met Asn Asp Ser
705                 710                 715                 720

Thr Val Glu Ala Asn Val Gln Ser Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Gln Ile Asp Gly Lys Thr Cys Leu Leu Gln Asn Asp
        755                 760                 765

His Asp Pro Ser Lys Leu Leu Ala Glu Thr Pro Cys Lys Leu Leu Arg
    770                 775                 780

Phe Leu Val Ala Asp Gly Ala His Val Asp Ala Asp Val Pro Tyr Ala
785                 790                 795                 800

Glu Val Glu Val Met Lys Met Cys Met Pro Leu Leu Ser Pro Ala Ser
                805                 810                 815

Gly Val Ile His Cys Met Met Ser Glu Gly Gln Ala Leu Gln Ala Gly
            820                 825                 830

Asp Leu Ile Ala Arg Leu Asp Leu Asp Asp Pro Ser Ala Val Lys Arg
        835                 840                 845

Ala Glu Pro Phe Asp Gly Ile Phe Pro Gln Met Glu Leu Pro Val Ala
    850                 855                 860

Val Ser Ser Gln Val His Lys Arg Tyr Ala Ala Ser Leu Asn Ala Ala
865                 870                 875                 880

Arg Met Val Leu Ala Gly Tyr Glu His Asn Ile Asn Glu Val Val Gln
                885                 890                 895

Asp Leu Val Cys Cys Leu Asp Asn Pro Glu Leu Pro Phe Leu Gln Trp
            900                 905                 910

Asp Glu Leu Met Ser Val Leu Ala Thr Arg Leu Pro Arg Asn Leu Lys

        915                 920                 925

Ser Glu Leu Glu Asp Lys Tyr Lys Glu Tyr Lys Leu Asn Phe Tyr His
    930                 935                 940

Gly Lys Asn Glu Asp Phe Pro Ser Lys Leu Leu Arg Asp Ile Ile Glu
945                 950                 955                 960

Glu Asn Leu Ser Tyr Gly Ser Glu Lys Glu Lys Ala Thr Asn Glu Arg
                965                 970                 975

Leu Val Glu Pro Leu Met Asn Leu Leu Lys Ser Tyr Glu Gly Gly Arg
            980                 985                 990

Glu Ser His Ala His Phe Val Val Lys Ser Leu Phe Glu Glu Tyr Leu
        995                 1000                1005

Thr Val Glu Glu Leu Phe Ser Asp Gly Ile Gln Ser Asp Val Ile
    1010                1015                1020

Glu Thr Leu Arg His Gln His Ser Lys Asp Leu Gln Lys Val Val
    1025                1030                1035

Asp Ile Val Leu Ser His Gln Gly Val Arg Asn Lys Ala Lys Leu
    1040                1045                1050

Val Thr Ala Leu Met Glu Lys Leu Val Tyr Pro Asn Pro Gly Gly
    1055                1060                1065

Tyr Arg Asp Leu Leu Val Arg Phe Ser Ser Leu Asn His Lys Arg
    1070                1075                1080

Tyr Tyr Lys Leu Ala Leu Lys Ala Ser Glu Leu Leu Glu Gln Thr
    1085                1090                1095

Lys Leu Ser Glu Leu Arg Ala Ser Val Ala Arg Ser Leu Ser Asp
    1100                1105                1110

Leu Gly Met His Lys Gly Glu Met Ser Ile Lys Asp Asn Met Glu
    1115                1120                1125

Asp Leu Val Ser Ala Pro Leu Pro Val Glu Asp Ala Leu Ile Ser
    1130                1135                1140

Leu Phe Asp Tyr Ser Asp Arg Thr Val Gln Gln Lys Val Ile Glu
    1145                1150                1155

Thr Tyr Ile Ser Arg Leu Tyr Gln Pro His Leu Val Lys Asp Ser
    1160                1165                1170

Ile Gln Met Lys Phe Lys Glu Ser Gly Ala Ile Thr Phe Trp Glu
    1175                1180                1185

Phe Tyr Glu Gly His Val Asp Thr Arg Asn Gly His Gly Ala Ile
    1190                1195                1200

Ile Gly Gly Lys Arg Trp Gly Ala Met Val Val Leu Lys Ser Leu
    1205                1210                1215

Glu Ser Ala Ser Thr Ala Ile Val Ala Ala Leu Lys Asp Ser Ala
    1220                1225                1230

Gln Phe Asn Ser Ser Glu Gly Asn Met Met His Ile Ala Leu Leu
    1235                1240                1245

Ser Ala Glu Asn Glu Ser Asn Ile Ser Gly Ile Ser Ser Asp Asp
    1250                1255                1260

Gln Ala Gln His Lys Met Glu Lys Leu Ser Lys Ile Leu Lys Asp
    1265                1270                1275

Thr Ser Val Ala Ser Asp Leu Gln Ala Ala Gly Leu Lys Val Ile
    1280                1285                1290

Ser Cys Ile Val Gln Arg Asp Glu Ala Arg Met Pro Met Arg His
    1295                1300                1305

Thr Phe Leu Trp Leu Asp Asp Lys Ser Cys Tyr Glu Glu Glu Gln
    1310                1315                1320

Ile Leu Arg His Val Glu Pro Pro Leu Ser Thr Leu Leu Glu Leu
    1325                1330                1335

Asp Lys Leu Lys Val Lys Gly Tyr Asn Glu Met Lys Tyr Thr Pro
    1340                1345                1350

Ser Arg Asp Arg Gln Trp His Ile Tyr Thr Leu Arg Asn Thr Glu
    1355                1360                1365

Asn Pro Lys Met Leu His Arg Val Phe Phe Arg Thr Ile Val Arg
    1370                1375                1380

Gln Pro Asn Ala Gly Asn Lys Phe Thr Ser Ala Gln Ile Ser Asp
    1385                1390                1395

Ala Glu Val Gly Cys Pro Glu Glu Ser Leu Ser Phe Thr Ser Asn
    1400                1405                1410

Ser Ile Leu Arg Ser Leu Met Thr Ala Ile Glu Glu Leu Glu Leu
    1415                1420                1425

His Ala Ile Arg Thr Gly His Ser His Met Tyr Leu Cys Ile Leu
    1430                1435                1440

Lys Glu Gln Lys Leu Leu Asp Leu Ile Pro Phe Ser Gly Ser Thr
    1445                1450                1455

Ile Val Asp Val Gly Gln Asp Glu Ala Thr Ala Cys Ser Leu Leu
    1460                1465                1470

Lys Ser Met Ala Leu Lys Ile His Glu Leu Val Gly Ala Arg Met
    1475                1480                1485

His His Leu Ser Val Cys Gln Trp Glu Val Lys Leu Lys Leu Asp
    1490                1495                1500

Cys Asp Gly Pro Ala Ser Gly Thr Trp Arg Val Val Thr Thr Asn
    1505                1510                1515

Val Thr Gly His Thr Cys Thr Ile Asp Ile Tyr Arg Glu Val Glu
    1520                1525                1530

Glu Ile Glu Ser Gln Lys Leu Val Tyr His Ser Ala Thr Ser Ser
    1535                1540                1545

Ala Gly Pro Leu His Gly Val Ala Leu Asn Asn Pro Tyr Gln Pro
    1550                1555                1560

Leu Ser Val Ile Asp Leu Lys Arg Cys Ser Ala Arg Asn Asn Arg
    1565                1570                1575

Thr Thr Tyr Cys Tyr Asp Phe Pro Leu Ala Phe Glu Thr Ala Leu
    1580                1585                1590

Gln Lys Ser Trp Gln Ser Asn Gly Ser Thr Val Ser Glu Gly Asn
    1595                1600                1605

Glu Asn Ser Lys Ser Tyr Val Lys Ala Thr Glu Leu Val Phe Ala
    1610                1615                1620

Glu Lys His Gly Ser Trp Gly Thr Pro Ile Ile Pro Met Glu Arg
    1625                1630                1635

Pro Ala Gly Leu Asn Asp Ile Gly Met Val Ala Trp Ile Met Glu
    1640                1645                1650

Met Ser Thr Pro Glu Phe Pro Asn Gly Arg Gln Ile Ile Val Val
    1655                1660                1665

Ala Asn Asp Ile Thr Phe Arg Ala Gly Ser Phe Gly Pro Arg Glu
    1670                1675                1680

Asp Ala Phe Phe Glu Thr Val Thr Asn Leu Ala Cys Glu Arg Lys
    1685                1690                1695

Leu Pro Leu Ile Tyr Leu Ala Ala Asn Ser Gly Ala Arg Ile Gly
    1700                1705                1710

Ile Ala Asp Glu Val Lys Ser Cys Phe Arg Val Gly Trp Ser Asp
    1715                1720                1725

Glu Gly Ser Pro Glu Arg Gly Phe Gln Tyr Ile Tyr Leu Thr Glu
    1730                1735                1740

Glu Asp Tyr Ala Arg Ile Ser Ser Ser Val Ile Ala His Lys Leu
    1745                1750                1755

Glu Leu Asp Ser Gly Glu Ile Arg Trp Ile Ile Asp Ser Val Val
    1760                1765                1770

Gly Lys Glu Asp Gly Leu Gly Val Glu Asn Ile His Gly Ser Ala
    1775                1780                1785

Ala Ile Ala Ser Ala Tyr Ser Arg Ala Tyr Glu Glu Thr Phe Thr
    1790                1795                1800

Leu Thr Phe Val Thr Gly Arg Thr Val Gly Ile Gly Ala Tyr Leu
    1805                1810                1815

Ala Arg Leu Gly Ile Arg Cys Ile Gln Arg Leu Asp Gln Pro Ile
    1820                1825                1830

Ile Leu Thr Gly Phe Ser Ala Leu Asn Lys Leu Leu Gly Arg Glu
    1835                1840                1845

Val Tyr Ser Ser His Met Gln Leu Gly Gly Pro Lys Ile Met Ala
    1850                1855                1860

Thr Asn Gly Val Val His Leu Thr Val Pro Asp Asp Leu Glu Gly

1865                1870                1875

Val Ser Asn Ile Leu Arg Trp Leu Ser Tyr Val Pro Ala Asn Ile
    1880                1885                1890

Gly Gly Pro Leu Pro Ile Thr Lys Pro Leu Asp Pro Pro Asp Arg
    1895                1900                1905

Pro Val Ala Tyr Ile Pro Glu Asn Thr Cys Asp Pro Arg Ala Ala
    1910                1915                1920

Ile Cys Gly Val Asp Asp Ser Gln Gly Lys Trp Leu Gly Gly Met
    1925                1930                1935

Phe Asp Lys Asp Ser Phe Val Glu Thr Phe Glu Gly Trp Ala Lys
    1940                1945                1950

Thr Val Val Thr Gly Arg Ala Lys Leu Gly Gly Ile Pro Val Gly
    1955                1960                1965

Val Ile Ala Val Glu Thr Gln Thr Met Met Gln Ile Ile Pro Ala
    1970                1975                1980

Asp Pro Gly Gln Leu Asp Ser His Glu Arg Ser Val Pro Arg Ala
    1985                1990                1995

Gly Gln Val Trp Phe Pro Asp Ser Ala Thr Lys Thr Ala Gln Ala
    2000                2005                2010

Leu Leu Asp Phe Asn Arg Glu Gly Leu Pro Leu Phe Ile Leu Ala
    2015                2020                2025

Asn Trp Arg Gly Phe Ser Gly Gly Gln Arg Asp Leu Phe Glu Gly
    2030                2035                2040

Ile Leu Gln Ala Gly Ser Thr Ile Val Glu Asn Leu Arg Thr Ser
    2045                2050                2055

Asn Gln Pro Ala Phe Val Tyr Ile Pro Met Ala Gly Glu Leu Arg
    2060                2065                2070

Gly Gly Ala Trp Val Val Val Asp Ser Lys Ile Asn Pro Asp Arg
    2075                2080                2085

Ile Glu Cys Tyr Ala Glu Arg Thr Ala Lys Gly Asn Val Leu Glu
    2090                2095                2100

Pro Gln Gly Leu Ile Glu Ile Lys Phe Arg Ser Glu Glu Leu Gln
    2105                2110                2115

Asp Cys Met Gly Arg Leu Asp Pro Glu Leu Ile Asn Leu Lys Ala
    2120                2125                2130

Lys Leu Gln Asp Val Asn His Gly Asn Gly Ser Leu Pro Asp Ile
    2135                2140                2145

Glu Gly Ile Arg Lys Ser Ile Glu Ala Arg Thr Lys Gln Leu Leu
    2150                2155                2160

Pro Leu Tyr Thr Gln Ile Ala Ile Arg Phe Ala Glu Leu His Asp
    2165                2170                2175

Thr Ser Leu Arg Met Ala Ala Lys Gly Val Ile Lys Lys Val Val
    2180                2185                2190

Asp Trp Glu Glu Ser Arg Ser Phe Phe Tyr Lys Arg Leu Arg Arg
    2195                2200                2205

Arg Ile Ala Glu Asp Val Leu Ala Lys Glu Ile Arg Gln Ile Val
    2210                2215                2220

Gly Asp Lys Phe Thr His Gln Leu Ala Met Glu Leu Ile Lys Glu
    2225                2230                2235

Trp Tyr Leu Ala Ser Gln Ala Thr Thr Gly Ser Thr Gly Trp Asp
    2240                2245                2250

Asp Asp Asp Ala Phe Val Ala Trp Lys Asp Ser Pro Glu Asn Tyr
    2255                2260                2265

Lys Gly His Ile Gln Lys Leu Arg Ala Gln Lys Val Ser His Ser
    2270                2275                2280

Leu Ser Asp Leu Ala Asp Ser Ser Ser Asp Leu Gln Ala Phe Ser
    2285                2290                2295

Gln Gly Leu Ser Thr Leu Leu Asp Lys Met Asp Pro Ser Gln Arg
    2300                2305                2310

Ala Lys Phe Val Gln Glu Val Lys Lys Val Leu Asp
    2315                2320                2325

<210> 13
<211> 6975
<212> DNA

<213> Zea mays

<400> 13
atgtcacagc ttggattagc cgcagctgcc tcaaaggcct tgccactact ccctaatcgc 60

cagagaagtt cagctgggac tacattctca tcatcttcat tatcgaggcc cttaaacaga 120

aggaaaagcc gtactcgttc actccgtgat ggcggagatg gggtatcaga tgccaaaaag 180

cacagccagt ctgttcgtca aggtcttgct ggcattatcg acctcccaag tgaggcacct 240

tccgaagtgg atatttcaca tggatctgag gatcctaggg ggccaacaga ttcttatcaa 300

atgaatggga ttatcaatga aacacataat ggaagacatg cctcagtgtc caaggttgtt 360

gaattttgtg cggcactagg tggcaaaaca ccaattcaca gtatattagt ggccaacaat 420

ggaatggcag cagcaaaatt tatgaggagt gtccggacat gggctaatga tacttttgga 480

tctgagaagg caattcaact catagctatg gcaactccgg aagacatgag gataaatgca 540

gaacacatta gaattgctga ccaattcgta gaggtgcctg gtggaacaaa caataataac 600

tacgccaatg ttcaactcat agtggagatg gcacaaaaac taggtgtttc tgctgtttgg 660

cctggttggg gtcatgcttc tgagaatcct gaactgccag atgcattgac cgcaaaaggg 720

atcgtttttc ttggcccacc tgcatcatca atgaatgctt tgggagataa ggtcggctca 780

gctctcattg ctcaagcagc cggggtccca actcttgctt ggagtggatc acatgttgaa 840

gttccattag agtgctgctt agacgcgata cctgaggaga tgtatagaaa agcttgcgtt 900

actaccacag aggaagcagt tgcaagttgt caagtggttg gttatcctgc catgattaag 960

gcatcctggg gaggtggtgg taaaggaata agaaaggttc ataatgatga tgaggttaga 1020

gcgctgttta agcaagtaca aggtgaagtc cctggctccc caatatttgt catgaggctt 1080

gcatcccaga gtcggcatct tgaagttcag ttgctttgtg atcaatatgg taatgtagca 1140

gcacttcaca gtcgtgattg cagtgtgcaa cggcgacacc agaagattat tgaagaaggt 1200

ccagttactg ttgctcctcg tgagacagtt aaagcacttg agcaggcagc aaggaggctt 1260

gctaaggctg tgggttatgt tggtgctgct actgttgagt atctttacag catggaaact 1320

ggagactact attttctgga acttaatccc cgactacagg ttgagcatcc agtcaccgag 1380

tggatagctg aagtaaatct gcctgcagct caagttgctg ttggaatggg catacctctt 1440

tggcagattc cagaaatcag acgtttctat ggaatggact atggaggagg gtatgacatt 1500

tggaggaaaa cagcagctct tgctacacca tttaattttg atgaagtaga ttctcaatgg 1560

ccaaagggcc attgtgtagc agttagaatt actagtgagg acccagatga tggtttcaaa 1620

cctactggtg ggaaagtgaa ggagataagt tttaaaagca agcctaatgt ttgggcctac 1680

ttctcagtaa agtctggtgg aggcattcat gaatttgctg attctcagtt cggacatgtt 1740

tttgcatatg ggctctctag atcagcagca ataacaaaca tgactcttgc attaaaagag 1800

attcaaattc gtggagaaat tcattcaaat gttgattaca cagttgacct cttaaatgct 1860

tcagacttta gagaaaacaa gattcatact ggttggctcg acaccagaat agctatgcgt 1920

gttcaagctg agaggccccc atggtatatt tcagtggttg ggggtgcttt atataaaaca 1980

gtaaccacca atgcagccac tgtttctgaa tatgttagtt atctcaccaa gggccagatt 2040

ccaccaaagc atatatccct tgtcaattct acagttaatt tgaatataga agggagcaaa 2100

tacacaattg aaactgtaag gactggacat ggtagctaca ggttgagaat gaatgattca 2160

acagttgaag cgaatgtaca atctttatgt gatggtggcc tcttaatgca gttggatgga 2220

aacagccatg taatttatgc agaagaagaa gctggtggta cacggcttca gattgatgga 2280

aagacatgtt tattgcagaa tgaccatgat ccatcaaagt tattagctga gacaccctgc 2340

aaacttcttc gtttcttggt tgctgatggt gctcatgttg atgcggatgt accatacgcg 2400

gaagttgagg ttatgaagat gtgcatgcct ctcttgtcgc ctgcttctgg tgtcattcat 2460

tgtatgatgt ctgagggcca ggcattgcag gctggtgatc ttatagcaag gttggatctt 2520

gatgaccctt ctgctgtgaa aagagctgag ccatttgatg gaatatttcc acaaatggag 2580

ctccctgttg ctgtctctag tcaagtacac aaaagatatg ctgcaagttt gaatgctgct 2640

cgaatggtcc ttgcaggata tgagcacaat attaatgaag tcgttcaaga tttggtatgc 2700

tgcctggaca accctgagct tcctttccta cagtgggatg aacttatgtc tgttctagca 2760

acgaggcttc caagaaatct caagagtgag ttagaggata aatacaagga atacaagttg 2820

aatttttacc atggaaaaaa cgaggacttt ccatccaagt tgctaagaga catcattgag 2880

gaaaatcttt cttatggttc agagaaggaa aaggctacaa atgagaggct tgttgagcct 2940

cttatgaacc tactgaagtc atatgagggt gggagagaga gccatgcaca ttttgttgtc 3000

aagtctcttt tcgaggagta tcttacagtg gaagaacttt ttagtgatgg cattcagtct 3060

gacgtgattg aaacattgcg gcatcagcac agtaaagacc tgcagaaggt tgtagacatt 3120

gtgttgtctc accagggtgt gaggaacaaa gctaagcttg taacggcact tatggaaaag 3180

ctggtttatc caaatcctgg tggttacagg gatctgttag ttcgcttttc ttccctcaat 3240

cataaaagat attataagtt ggcccttaaa gcaagtgaac ttcttgaaca aaccaaacta 3300

agtgaactcc gtgcaagcgt tgcaagaagc ctttcggatc tggggatgca taagggagaa 3360

atgagtatta aggataacat ggaagattta gtctctgccc cattacctgt tgaagatgct 3420

ctgatttctt tgtttgatta cagtgatcga actgttcagc agaaagtgat tgagacatac 3480

atatcacgat tgtaccagcc tcatcttgta aaggatagca tccaaatgaa attcaaggaa 3540

tctggtgcta ttactttttg ggaattttat gaagggcatg ttgatactag aaatggacat 3600

ggggctatta ttggtgggaa gcgatggggt gccatggtcg ttctcaaatc acttgaatct 3660

gcgtcaacag ccattgtggc tgcattaaag gattcggcac agttcaacag ctctgagggc 3720

aacatgatgc acattgcatt attgagtgct gaaaatgaaa gtaatataag tggaataagt 3780

gatgatcaag ctcaacataa gatggaaaag cttagcaaga tactgaagga tactagcgtt 3840

gcaagtgatc tccaagctgc tggtttgaag gttataagtt gcattgttca aagagatgaa 3900

gctcgcatgc caatgcgcca cacattcctc tggttggatg acaagagttg ttatgaagaa 3960

gagcagattc tccggcatgt ggagcctccc ctctctacac ttcttgaatt ggataagttg 4020

aaggtgaaag gatacaatga aatgaagtat actccttcgc gtgaccgcca atggcatatc 4080

tacacactaa gaaatactga aaaccccaaa atgttgcata gggtgttttt ccgaactatt 4140

gtcaggcaac ccaatgcagg caacaagttt acatcggctc agatcagcga cgctgaagta 4200

ggatgtcccg aagaatctct ttcatttaca tcaaatagca tcttaagatc attgatgact 4260

gctattgaag aattagagct tcatgcaatt aggacaggtc attctcacat gtatttgtgc 4320

atactgaaag agcaaaagct tcttgacctc attccatttt cagggagtac aattgttgat 4380

gttggccaag atgaagctac cgcttgttca cttttaaaat caatggcttt gaagatacat 4440

gagcttgttg gtgcaaggat gcatcatctg tctgtatgcc agtgggaggt gaaactcaag 4500

ttggactgtg atggccctgc aagtggtacc tggagagttg taactacaaa tgttactggt 4560

cacacctgca ccattgatat ataccgagaa gtggaggaaa tagaatcgca gaagttagtg 4620

taccattcag ccacttcgtc agctggacca ttgcatggtg ttgcactgaa taatccatat 4680

caacctttga gtgtgattga tctaaagcgc tgctctgcta ggaacaacag aacaacatat 4740

tgctatgatt ttccgctggc ctttgaaact gcactgcaga agtcatggca gaccaatggc 4800

tctactgttt ctgaaggcaa tgaaaatagt aaatcctacg tgaaggcaac tgagctagtg 4860

tttgctgaaa aacatgggtc ctggggcact cctataattc cgatggaacg ccctgctggg 4920

ctcaacgaca ttggtatggt cgcttggatc atggagatgt caacacctga atttcccaat 4980

ggcaggcaga ttattgttgt agcaaatgat atcactttca gagctggatc atttggccca 5040

agggaagatg cattttttga aactgtcact aacctggctt gcgaaaggaa acttcctctt 5100

atatacttgg cagcaaactc tggtgctagg attggcatag ctgatgaagt aaaatcttgc 5160

ttccgtgttg gatggtctga cgaaggcagt cctgaacgag ggtttcagta catctatctg 5220

actgaagaag actatgctcg cattagctct tctgttatag cacataagct ggagctagat 5280

agtggtgaaa ttaggtggat tattgactct gttgtgggca aggaggatgg gcttggtgtc 5340

gagaacatac atggaagtgc tgctattgcc agtgcttatt ctagggcata tgaggagaca 5400

tttacactta catttgtgac tgggcggact gtaggaatag gagcttatct tgctcgactt 5460

ggtatacggt gcatacagcg tcttgaccag cctattattt taacagggtt ttctgccctg 5520

aacaagctcc ttgggcggga agtgtacagc tcccacatgc agcttggtgg tcctaagatc 5580

atggcgacta atggtgttgt ccacctcact gttccagatg accttgaagg tgtttccaat 5640

atattgaggt ggctcagcta tgttcctgca aacattggtg gacctcttcc tattaccaaa 5700

cctctggacc ctccagacag acctgttgct tacatccctg agaacacatg cgatccacgt 5760

gcagctatct gtggtgtaga tgacagccaa gggaaatggt tgggtggtat gtttgacaaa 5820

gacagctttg tggagacatt tgaaggatgg gcaaaaacag tggttactgg cagagcaaag 5880

cttggaggaa ttcctgtggg cgtcatagct gtggagacac agaccatgat gcagatcatc 5940

cctgctgatc caggtcagct tgattcccat gagcgatctg tccctcgtgc tggacaagtg 6000

tggttcccag attctgcaac caagaccgct caggcattat tagacttcaa ccgtgaagga 6060

ttgcctctgt tcatcctggc taattggaga ggcttctctg gtggacaaag agatctcttt 6120

gaaggaattc ttcaggctgg gtcaacaatt gtcgagaacc ttaggacata taatcagcct 6180

gcttttgtgt acattcctat ggctggagag cttcgtggag gagcttgggt tgtggtcgat 6240

agcaaaataa atccagaccg cattgagtgt tatgctgaaa ggactgccaa aggtaatgtt 6300

ctcgaacctc aagggttaat tgaaatcaag ttcaggtcag aggaactcca agactgtatg 6360

ggtaggcttg acccagagtt gataaatctg aaagcaaaac tccaagatgt aaatcatgga 6420

aatggaagtc taccagacat agaagggatt cggaagagta tagaagcacg tacgaaacag 6480

ttgctgcctt tatataccca gattgcaata cggtttgctg aattgcatga tacttcccta 6540

agaatggcag ctaaaggtgt gattaagaaa gttgtagact gggaagaatc acgctcgttc 6600

ttctataaaa ggctacggag gaggatcgca gaagatgttc ttgcaaaaga aataaggcag 6660

atagtcggtg ataaatttac gcaccaatta gcaatggagc tcatcaagga atggtacctt 6720

gcttctcagg ccacaacagg aagcactgga tgggatgacg atgatgcttt tgttgcctgg 6780

aaggacagtc ctgaaaacta caaggggcat atccaaaagc ttagggctca aaaagtgtct 6840

cattcgctct ctgatcttgc tgactccagt tcagatctgc aagcattctc gcagggtctt 6900

tctacgctat tagataagat ggatccctct cagagagcga agtttgttca ggaagtcaag 6960

aaggtccttg attga 6975

<210> 14
<211> 2324
<212> PRT
<213> Zea mays

<400> 14
Met Ser Gln Leu Gly Leu Ala Ala Ala Ala Ser Lys Ala Leu Pro Leu
1               5                   10                  15

Leu Pro Asn Arg Gln Arg Ser Ser Ala Gly Thr Thr Phe Ser Ser Ser
            20                  25                  30

Ser Leu Ser Arg Pro Leu Asn Arg Arg Lys Ser Arg Thr Arg Ser Leu
        35                  40                  45

Arg Asp Gly Gly Asp Gly Val Ser Asp Ala Lys Lys His Ser Gln Ser
    50                  55                  60

Val Arg Gln Gly Leu Ala Gly Ile Ile Asp Leu Pro Ser Glu Ala Pro
65                  70                  75                  80

Ser Glu Val Asp Ile Ser His Gly Ser Glu Asp Pro Arg Gly Pro Thr
                85                  90                  95

Asp Ser Tyr Gln Met Asn Gly Ile Ile Asn Glu Thr His Asn Gly Arg
            100                 105                 110

His Ala Ser Val Ser Lys Val Val Glu Phe Cys Ala Ala Leu Gly Gly
        115                 120                 125

Lys Thr Pro Ile His Ser Ile Leu Val Ala Asn Asn Gly Met Ala Ala
    130                 135                 140

Ala Lys Phe Met Arg Ser Val Arg Thr Trp Ala Asn Asp Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Met Ala Gln Lys Leu Gly Val Ser Ala Val Trp Pro Gly Trp Gly
    210                 215                 220

His Ala Ser Glu Asn Pro Glu Leu Pro Asp Ala Leu Thr Ala Lys Gly
225                 230                 235                 240

Ile Val Phe Leu Gly Pro Pro Ala Ser Ser Met Asn Ala Leu Gly Asp
                245                 250                 255

Lys Val Gly Ser Ala Leu Ile Ala Gln Ala Ala Gly Val Pro Thr Leu
            260                 265                 270

Ala Trp Ser Gly Ser His Val Glu Val Pro Leu Glu Cys Cys Leu Asp
        275                 280                 285

Ala Ile Pro Glu Glu Met Tyr Arg Lys Ala Cys Val Thr Thr Thr Glu
    290                 295                 300

Glu Ala Val Ala Ser Cys Gln Val Val Gly Tyr Pro Ala Met Ile Lys
305                 310                 315                 320

Ala Ser Trp Gly Gly Gly Gly Lys Gly Ile Arg Lys Val His Asn Asp
                325                 330                 335

Asp Glu Val Arg Ala Leu Phe Lys Gln Val Gln Gly Glu Val Pro Gly
            340                 345                 350

Ser Pro Ile Phe Val Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Ala Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Asp Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Ile Pro Glu Ile Arg Arg Phe Tyr Gly Met Asp Tyr Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Leu Ser Arg Ser Ala Ala Ile Thr
            580                 585                 590

Asn Met Thr Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Arg
    610                 615                 620

Glu Asn Lys Ile His Thr Gly Trp Leu Asp Thr Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Val Thr Thr Asn Ala Ala Thr Val Ser Glu Tyr Val
            660                 665                 670

Ser Tyr Leu Thr Lys Gly Gln Ile Pro Pro Lys His Ile Ser Leu Val
        675                 680                 685

Asn Ser Thr Val Asn Leu Asn Ile Glu Gly Ser Lys Tyr Thr Ile Glu
    690                 695                 700

Thr Val Arg Thr Gly His Gly Ser Tyr Arg Leu Arg Met Asn Asp Ser
705                 710                 715                 720

Thr Val Glu Ala Asn Val Gln Ser Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Gln Ile Asp Gly Lys Thr Cys Leu Leu Gln Asn Asp
        755                 760                 765

His Asp Pro Ser Lys Leu Leu Ala Glu Thr Pro Cys Lys Leu Leu Arg
    770                 775                 780

Phe Leu Val Ala Asp Gly Ala His Val Asp Ala Asp Val Pro Tyr Ala

785                 790                 795                 800

Glu Val Glu Val Met Lys Met Cys Met Pro Leu Leu Ser Pro Ala Ser
                805                 810                 815

Gly Val Ile His Cys Met Met Ser Glu Gly Gln Ala Leu Gln Ala Gly
            820                 825                 830

Asp Leu Ile Ala Arg Leu Asp Leu Asp Asp Pro Ser Ala Val Lys Arg
        835                 840                 845

Ala Glu Pro Phe Asp Gly Ile Phe Pro Gln Met Glu Leu Pro Val Ala
    850                 855                 860

Val Ser Ser Gln Val His Lys Arg Tyr Ala Ala Ser Leu Asn Ala Ala
865                 870                 875                 880

Arg Met Val Leu Ala Gly Tyr Glu His Asn Ile Asn Glu Val Val Gln
                885                 890                 895

Asp Leu Val Cys Cys Leu Asp Asn Pro Glu Leu Pro Phe Leu Gln Trp
            900                 905                 910

Asp Glu Leu Met Ser Val Leu Ala Thr Arg Leu Pro Arg Asn Leu Lys
        915                 920                 925

Ser Glu Leu Glu Asp Lys Tyr Lys Glu Tyr Lys Leu Asn Phe Tyr His
    930                 935                 940

Gly Lys Asn Glu Asp Phe Pro Ser Lys Leu Leu Arg Asp Ile Ile Glu
945                 950                 955                 960

Glu Asn Leu Ser Tyr Gly Ser Glu Lys Glu Lys Ala Thr Asn Glu Arg
                965                 970                 975

Leu Val Glu Pro Leu Met Asn Leu Leu Lys Ser Tyr Glu Gly Gly Arg
            980                 985                 990

Glu Ser His Ala His Phe Val Val  Lys Ser Leu Phe Glu  Glu Tyr Leu
        995                 1000                1005

Thr Val  Glu Glu Leu Phe Ser  Asp Gly Ile Gln Ser  Asp Val Ile
    1010                1015                1020

Glu Thr  Leu Arg His Gln His  Ser Lys Asp Leu Gln  Lys Val Val
    1025                1030                1035

Asp Ile Val Leu Ser His Gln Gly Val Arg Asn Lys Ala Lys Leu
    1040                1045                1050

Val Thr Ala Leu Met Glu Lys Leu Val Tyr Pro Asn Pro Gly Gly
    1055                1060                1065

Tyr Arg Asp Leu Leu Val Arg Phe Ser Ser Leu Asn His Lys Arg
    1070                1075                1080

Tyr Tyr Lys Leu Ala Leu Lys Ala Ser Glu Leu Leu Glu Gln Thr
    1085                1090                1095

Lys Leu Ser Glu Leu Arg Ala Ser Val Ala Arg Ser Leu Ser Asp
    1100                1105                1110

Leu Gly Met His Lys Gly Glu Met Ser Ile Lys Asp Asn Met Glu
    1115                1120                1125

Asp Leu Val Ser Ala Pro Leu Pro Val Glu Asp Ala Leu Ile Ser
    1130                1135                1140

Leu Phe Asp Tyr Ser Asp Arg Thr Val Gln Gln Lys Val Ile Glu
    1145                1150                1155

Thr Tyr Ile Ser Arg Leu Tyr Gln Pro His Leu Val Lys Asp Ser
    1160                1165                1170

Ile Gln Met Lys Phe Lys Glu Ser Gly Ala Ile Thr Phe Trp Glu
    1175                1180                1185

Phe Tyr Glu Gly His Val Asp Thr Arg Asn Gly His Gly Ala Ile
    1190                1195                1200

Ile Gly Gly Lys Arg Trp Gly Ala Met Val Val Leu Lys Ser Leu
    1205                1210                1215

Glu Ser Ala Ser Thr Ala Ile Val Ala Ala Leu Lys Asp Ser Ala
    1220                1225                1230

Gln Phe Asn Ser Ser Glu Gly Asn Met Met His Ile Ala Leu Leu
    1235                1240                1245

Ser Ala Glu Asn Glu Ser Asn Ile Ser Gly Ile Ser Asp Asp Gln
    1250                1255                1260

Ala Gln His Lys Met Glu Lys Leu Ser Lys Ile Leu Lys Asp Thr
    1265                1270                1275

Ser Val Ala Ser Asp Leu Gln Ala Ala Gly Leu Lys Val Ile Ser
    1280                1285                1290

Cys Ile Val Gln Arg Asp Glu Ala Arg Met Pro Met Arg His Thr
    1295                1300                1305

Phe Leu Trp Leu Asp Asp Lys Ser Cys Tyr Glu Glu Glu Gln Ile
    1310                1315                1320

Leu Arg His Val Glu Pro Pro Leu Ser Thr Leu Leu Glu Leu Asp
    1325                1330                1335

Lys Leu Lys Val Lys Gly Tyr Asn Glu Met Lys Tyr Thr Pro Ser
    1340                1345                1350

Arg Asp Arg Gln Trp His Ile Tyr Thr Leu Arg Asn Thr Glu Asn
    1355                1360                1365

Pro Lys Met Leu His Arg Val Phe Phe Arg Thr Ile Val Arg Gln
    1370                1375                1380

Pro Asn Ala Gly Asn Lys Phe Thr Ser Ala Gln Ile Ser Asp Ala
    1385                1390                1395

Glu Val Gly Cys Pro Glu Glu Ser Leu Ser Phe Thr Ser Asn Ser
    1400                1405                1410

Ile Leu Arg Ser Leu Met Thr Ala Ile Glu Glu Leu Glu Leu His
    1415                1420                1425

Ala Ile Arg Thr Gly His Ser His Met Tyr Leu Cys Ile Leu Lys
    1430                1435                1440

Glu Gln Lys Leu Leu Asp Leu Ile Pro Phe Ser Gly Ser Thr Ile
    1445                1450                1455

Val Asp Val Gly Gln Asp Glu Ala Thr Ala Cys Ser Leu Leu Lys
    1460                1465                1470

Ser Met Ala Leu Lys Ile His Glu Leu Val Gly Ala Arg Met His
    1475                1480                1485

His Leu Ser Val Cys Gln Trp Glu Val Lys Leu Lys Leu Asp Cys
    1490                1495                1500

Asp Gly Pro Ala Ser Gly Thr Trp Arg Val Val Thr Thr Asn Val
    1505                1510                1515

Thr Gly His Thr Cys Thr Ile Asp Ile Tyr Arg Glu Val Glu Glu
    1520                1525                1530

Ile Glu Ser Gln Lys Leu Val Tyr His Ser Ala Thr Ser Ser Ala
    1535                1540                1545

Gly Pro Leu His Gly Val Ala Leu Asn Asn Pro Tyr Gln Pro Leu
    1550                1555                1560

Ser Val Ile Asp Leu Lys Arg Cys Ser Ala Arg Asn Asn Arg Thr
    1565                1570                1575

Thr Tyr Cys Tyr Asp Phe Pro Leu Ala Phe Glu Thr Ala Leu Gln
    1580                1585                1590

Lys Ser Trp Gln Thr Asn Gly Ser Thr Val Ser Glu Gly Asn Glu
    1595                1600                1605

Asn Ser Lys Ser Tyr Val Lys Ala Thr Glu Leu Val Phe Ala Glu
    1610                1615                1620

Lys His Gly Ser Trp Gly Thr Pro Ile Ile Pro Met Glu Arg Pro
    1625                1630                1635

Ala Gly Leu Asn Asp Ile Gly Met Val Ala Trp Ile Met Glu Met
    1640                1645                1650

Ser Thr Pro Glu Phe Pro Asn Gly Arg Gln Ile Ile Val Val Ala
    1655                1660                1665

Asn Asp Ile Thr Phe Arg Ala Gly Ser Phe Gly Pro Arg Glu Asp
    1670                1675                1680

Ala Phe Phe Glu Thr Val Thr Asn Leu Ala Cys Glu Arg Lys Leu
    1685                1690                1695

Pro Leu Ile Tyr Leu Ala Ala Asn Ser Gly Ala Arg Ile Gly Ile
    1700                1705                1710

Ala Asp Glu Val Lys Ser Cys Phe Arg Val Gly Trp Ser Asp Glu
    1715                1720                1725

Gly Ser Pro Glu Arg Gly Phe Gln Tyr Ile Tyr Leu Thr Glu Glu
    1730                1735                1740

Asp Tyr Ala Arg Ile Ser Ser Ser Val Ile Ala His Lys Leu Glu

1745                1750                1755

Leu Asp Ser Gly Glu Ile Arg Trp Ile Ile Asp Ser Val Val Gly
    1760                1765                1770

Lys Glu Asp Gly Leu Gly Val Glu Asn Ile His Gly Ser Ala Ala
    1775                1780                1785

Ile Ala Ser Ala Tyr Ser Arg Ala Tyr Glu Glu Thr Phe Thr Leu
    1790                1795                1800

Thr Phe Val Thr Gly Arg Thr Val Gly Ile Gly Ala Tyr Leu Ala
    1805                1810                1815

Arg Leu Gly Ile Arg Cys Ile Gln Arg Leu Asp Gln Pro Ile Ile
    1820                1825                1830

Leu Thr Gly Phe Ser Ala Leu Asn Lys Leu Leu Gly Arg Glu Val
    1835                1840                1845

Tyr Ser Ser His Met Gln Leu Gly Gly Pro Lys Ile Met Ala Thr
    1850                1855                1860

Asn Gly Val Val His Leu Thr Val Pro Asp Asp Leu Glu Gly Val
    1865                1870                1875

Ser Asn Ile Leu Arg Trp Leu Ser Tyr Val Pro Ala Asn Ile Gly
    1880                1885                1890

Gly Pro Leu Pro Ile Thr Lys Pro Leu Asp Pro Pro Asp Arg Pro
    1895                1900                1905

Val Ala Tyr Ile Pro Glu Asn Thr Cys Asp Pro Arg Ala Ala Ile
    1910                1915                1920

Cys Gly Val Asp Asp Ser Gln Gly Lys Trp Leu Gly Gly Met Phe
    1925                1930                1935

Asp Lys Asp Ser Phe Val Glu Thr Phe Glu Gly Trp Ala Lys Thr
    1940                1945                1950

Val Val Thr Gly Arg Ala Lys Leu Gly Gly Ile Pro Val Gly Val
    1955                1960                1965

Ile Ala Val Glu Thr Gln Thr Met Met Gln Ile Ile Pro Ala Asp
    1970                1975                1980

Pro Gly Gln Leu Asp Ser His Glu Arg Ser Val Pro Arg Ala Gly
    1985                1990                1995

Gln Val Trp Phe Pro Asp Ser Ala Thr Lys Thr Ala Gln Ala Leu
    2000                2005                2010

Leu Asp Phe Asn Arg Glu Gly Leu Pro Leu Phe Ile Leu Ala Asn
    2015                2020                2025

Trp Arg Gly Phe Ser Gly Gly Gln Arg Asp Leu Phe Glu Gly Ile
    2030                2035                2040

Leu Gln Ala Gly Ser Thr Ile Val Glu Asn Leu Arg Thr Tyr Asn
    2045                2050                2055

Gln Pro Ala Phe Val Tyr Ile Pro Met Ala Gly Glu Leu Arg Gly
    2060                2065                2070

Gly Ala Trp Val Val Val Asp Ser Lys Ile Asn Pro Asp Arg Ile
    2075                2080                2085

Glu Cys Tyr Ala Glu Arg Thr Ala Lys Gly Asn Val Leu Glu Pro
    2090                2095                2100

Gln Gly Leu Ile Glu Ile Lys Phe Arg Ser Glu Glu Leu Gln Asp
    2105                2110                2115

Cys Met Gly Arg Leu Asp Pro Glu Leu Ile Asn Leu Lys Ala Lys
    2120                2125                2130

Leu Gln Asp Val Asn His Gly Asn Gly Ser Leu Pro Asp Ile Glu
    2135                2140                2145

Gly Ile Arg Lys Ser Ile Glu Ala Arg Thr Lys Gln Leu Leu Pro
    2150                2155                2160

Leu Tyr Thr Gln Ile Ala Ile Arg Phe Ala Glu Leu His Asp Thr
    2165                2170                2175

Ser Leu Arg Met Ala Ala Lys Gly Val Ile Lys Lys Val Val Asp
    2180                2185                2190

Trp Glu Glu Ser Arg Ser Phe Phe Tyr Lys Arg Leu Arg Arg Arg
    2195                2200                2205

Ile Ala Glu Asp Val Leu Ala Lys Glu Ile Arg Gln Ile Val Gly
    2210                2215                2220

Asp Lys Phe Thr His Gln Leu Ala Met Glu Leu Ile Lys Glu Trp
    2225                2230                2235

Tyr Leu Ala Ser Gln Ala Thr Thr Gly Ser Thr Gly Trp Asp Asp
    2240                2245                2250

Asp Asp Ala Phe Val Ala Trp Lys Asp Ser Pro Glu Asn Tyr Lys
    2255                2260                2265

Gly His Ile Gln Lys Leu Arg Ala Gln Lys Val Ser His Ser Leu
    2270                2275                2280

Ser Asp Leu Ala Asp Ser Ser Ser Asp Leu Gln Ala Phe Ser Gln
    2285                2290                2295

Gly Leu Ser Thr Leu Leu Asp Lys Met Asp Pro Ser Gln Arg Ala
    2300                2305                2310

Lys Phe Val Gln Glu Val Lys Lys Val Leu Asp
    2315                2320

<210> 15
<211> 6936
<212> DNA
<213> Triticum aestivum

<400> 15
atgggatcca cacatttgcc cattgtcggc cttaatgcct cgacaacacc atcgctatcc      60

actattcgcc cggtaaattc agccggtgct gcattccaac catctgcccc ttctagaacc     120

tccaagaaga aaagtcgtcg tgttcagtca ttaagggatg gaggcgatgg aggcgtgtca     180

gaccctaacc agtctattcg ccaaggtctt gccggcatca ttgacctccc aaaggagggc     240

acatcagctc cggaagtgga tatttcacat gggtccgaag aacccagggg ctcctaccaa     300

atgaatggga tactgaatga agcacataat gggaggcatg cttcgctgtc taaggttgtc     360

gaattttgta tggcattggg cggcaaaaca ccaattcaca gtgtattagt tgcgaacaat     420

ggaatggcag cagctaagtt catgcggagt gtccgaacat gggctaatga aacatttggg     480

tcagagaagg caattcagtt gatagctatg gctactccag aagacatgag gataaatgca     540

gagcacatta gaattgctga tcaatttgtt gaagtacccg gtggaacaaa caataacaac     600

tatgcaaatg tccaactcat agtggagata gcagtgagaa ccggtgtttc tgctgtttgg     660

cctggttggg gccatgcatc tgagaatcct gaacttccag atgcactaaa tgcaaacgga     720

attgtttttc ttgggccacc atcatcatca atgaacgcac taggtgacaa ggttggttca     780

gctctcattg ctcaagcagc aggggttccg actcttcctt ggagtggatc acaggtggaa     840

attccattag aagtttgttt ggactcgata cccgcggaga tgtataggaa agcttgtgtt 900

agtactacgg aggaagcact tgcgagttgt cagatgattg ggtatcccgc catgattaaa 960

gcatcatggg gtggtggtgg taaagggatc cgaaaggtta ataatgacga tgatgtcaga 1020

gcactgttta agcaagtgca aggtgaagtt cctggctccc caatatttat catgagactt 1080

gcatctcaga gtcgacatct tgaagttcag ttgctttgtg atcaatatgg caatgtagct 1140

gcgcttcaca gtcgtgactg cagtgtgcaa cggcgacacc aaaagattat tgaggaagga 1200

ccagttactg ttgctcctcg cgagacagtg aaagagctag agcaagcagc aaggaggctt 1260

gctaaggctg tgggttatgt tggtgctgct actgttgaat atctctacag catggagact 1320

ggtgaatact atttctggaa acttaatcca cggttgcagg ttgagcatcc agtcaccgag 1380

tggatagctg aagtaaactt gcctgcagct caagttgcag ttggaatggg tatacccctt 1440

tggcaggttc cagagatcag acgtttctat ggaatggaca atggaggagg ctatgacatt 1500

tggaggaaaa cagcagctct tgctactcca tttaacttcg atgaagtgga ttctcaatgg 1560

ccaaagggtc attgtgtagc agttaggata accagtgagg atccagatga cggattcaag 1620

cctaccggtg gaaaagtaaa ggagatcagt tttaaaagca agccaaatgt ttgggcctat 1680

ttctctgtta agtccggtgg aggcattcat gaatttgctg attctcagtt tggacatgtt 1740

tttgcatatg gagtgtctag agcagcagca ataaccaaca tgtctcttgc gctaaaagag 1800

attcaaattc gtggagaaat tcattcaaat gttgattaca cagttgatct cttgaatgcc 1860

tcagacttca aagaaaacag gattcatact ggctggctgg ataacagaat agcaatgcga 1920

gtccaagctg agagacctcc gtggtatatt tcagtggttg gaggagctct atataaaaca 1980

ataacgagca acacagacac tgtttctgaa tatgttagct atctcgtcaa gggtcagatt 2040

ccaccgaagc atatatccct tgtccattca actgtttctt tgaatataga ggaaagcaaa 2100

tatacaattg aaactataag gagcggacag ggtagctaca gattgcgaat gaatggatca 2160

gttattgaag caaatgtcca aacattatgt gatggtggac ttttaatgca gttggatgga 2220

aacagccatg taatttatgc tgaagaagag gccggtggta cacggcttct aattgatgga 2280

aagacatgct tgttacagaa tgatcacgat ccttcaaggt tattagctga gacaccctgc 2340

aaacttcttc gtttcttggt tgccgatggt gctcatgttg aagctgatgt accatatgcg 2400

gaagttgagg ttatgaagat gtgcatgccc ctcttgtcac ctgctgctgg tgtcattaat 2460

gttttgttgt ctgagggcca gcctatgcag gctggtgatc ttatagcaag acttgatctt 2520

gatgaccctt ctgctgtgaa gagagctgag ccatttaacg gatctttccc agaaatgagc 2580

cttcctattg ctgcttctgg ccaagttcac aaaagatgtg ccacaagctt gaatgctgct 2640

cggatggtcc ttgcaggata tgatcacccg atcaacaaag ttgtacaaga tctggtatcc 2700

tgtctagatg ctcctgagct tcctttccta caatgggaag agcttatgtc tgttttagca 2760

actagacttc caaggcttct taagagcgag ttggagggta aatacagtga atataagtta 2820

aatgttggcc atgggaagag caaggatttc ccttccaaga tgctaagaga gataatcgag 2880

gaaaatcttg cacatggttc tgagaaggaa attgctacaa atgagaggct tgttgagcct 2940

cttatgagcc tactgaagtc atatgagggt ggcagagaaa gccatgcaca ctttattgtg 3000

aagtcccttt tcgaggacta tctctcggtt gaggaactat tcagtgatgg cattcagtct 3060

gatgtgattg aacgcctgcg ccaacaacat agtaaagatc tccagaaggt tgtagacatt 3120

gtgttgtctc accagggtgt gagaaacaaa actaagctga tactaacact catggagaaa 3180

ctggtctatc caaaccctgc tgtctacaag gatcagttga ctcgcttttc ctccctcaat 3240

cacaaaagat attataagtt ggcccttaaa gctagcgagc ttcttgaaca aaccaagctt 3300

agtgagctcc gcacaagcat tgcaaggagc ctttcagaac ttgagatgtt tactgaagaa 3360

aggacggcca ttagtgagat catgggagat ttagtgactg ccccactgcc agttgaagat 3420

gcactggttt ctttgtttga ttgtagtgat caaactcttc agcagagggt gatcgagacg 3480

tacatatctc gattatacca gcctcatctt gtcaaggata gtatccagct gaaatatcag 3540

gaatctggtg ttattgcttt atgggaattc gctgaagcgc attcagagaa gagattgggt 3600

gctatggtta ttgtgaagtc gttagaatct gtatcagcag caattggagc tgcactaaag 3660

ggtacatcac gctatgcaag ctctgagggt aacataatgc atattgcttt attgggtgct 3720

gataatcaaa tgcatggaac tgaagacagt ggtgataacg atcaagctca agtcaggata 3780

gacaaacttt ctgcgacact ggaacaaaat actgtcacag ctgatctccg tgctgctggt 3840

gtgaaggtta ttagttgcat tgttcaaagg gatggagcac tcatgcctat gcgccatacc 3900

ttcctcttgt cggatgaaaa gctttgttat gaggaagagc cggttctccg gcatgtggag 3960

cctcctcttt ctgctcttct tgagttgggt aagttgaaag tgaaaggata caatgaggtg 4020

aagtatacac cgtcacgtga tcgtcagtgg aacatataca cacttagaaa tacagagaac 4080

cccaaaatgt tgcacagggt gtttttccga actcttgtca ggcaacccgg tgcttccaac 4140

aaattcacat caggcaacat cagtgatgtt gaagtgggag gagctgagga atctctttca 4200

tttacatcga gcagcatatt aagatcgctg atgactgcta tagaagagtt ggagcttcac 4260

gcgattagga caggtcactc tcatatgttt ttgtgcatat tgaaagagca aaagcttctt 4320

gatcttgttc ccgtttcagg gaacaaagtt gtggatattg gccaagatga agctactgca 4380

tgcttgcttc tgaaagaaat ggctctacag atacatgaac ttgtgggtgc aaggatgcat 4440

catctttctg tatgccaatg ggaggtgaaa cttaagttgg acagcgatgg gcctgccagt 4500

ggtacctgga gagttgtaac aaccaatgtt actagtcaca cctgcactgt ggatatctac 4560

cgtgaggtcg aagatacaga atcacagaaa ctagtgtacc actctgctcc atcgtcatct 4620

ggtcctttgc atggcgttgc actgaatact ccatatcagc ctttgagtgt tattgatctg 4680

aaacgttgct ccgctagaaa taacagaact acatactgct atgattttcc gttggcattt 4740

gaaactgcag tgcagaagtc atggtctaac atttctagtg acactaaccg atgttatgtt 4800

aaagcgacgg agctggtgtt tgctcacaag aacgggtcat ggggcactcc tgtaattcct 4860

atggagcgtc ctgctgggct caatgacatt ggtatggtag cttggatctt ggacatgtcc 4920

actcctgaat atcccaatgg caggcagatt gttgtcatcg caaatgatat tacttttaga 4980

gctggatcgt ttggtccaag ggaagatgca ttttttgaaa ctgttaccaa cctagcttgt 5040

gagaggaagc ttcctctcat ctacttggca gcaaactctg gtgctcggat cggcatagca 5100

gatgaagtaa aatcttgctt ccgtgttgga tggtctgatg atggcagccc tgaacgtggg 5160

tttcaatata tttatctgac tgaagaagac catgctcgta ttagcgcttc tgttatagcg 5220

cacaagatgc agcttgataa tggtgaaatt aggtgggtta ttgattctgt tgtagggaag 5280

gaggatgggc taggtgtgga gaacatacat ggaagtgctg ctattgccag tgcctattct 5340

agggcctatg aggagacatt tacgcttaca tttgtgactg gaaggactgt tggaatagga 5400

gcatatcttg ctcgacttgg catacggtgc atacagcgta ctgaccagcc cattatccta 5460

actgggttct ctgccttgaa caagcttctt ggccgggaag tttacagctc ccacatgcag 5520

ttgggtggcc ccaaaattat ggcgacaaac ggtgttgtcc atctgacagt ttcagatgac 5580

cttgaaggtg tatctaatat attgaggtgg ctcagctatg ttcctgccaa cattggtgga 5640

cctcttccta ttacaaaatc tttggaccca cctgacagac ccgttgctta catccctgag 5700

aatacatgcg atcctcgtgc tgccatcagt ggcattgatg atagccaagg gaaatggttg 5760

gggggcatgt tcgacaaaga cagttttgtg gagacatttg aaggatgggc gaagtcagtt 5820

gttactggca gagcgaaact cggagggatt ccggtgggtg ttatagctgt ggagacacag 5880

actatgatgc agctcatccc tgctgatcca ggccagcttg attcccatga gcgatctgtt 5940

cctcgtgctg ggcaagtctg gtttccagat tcagctacta agacagcgca ggcaatgctg 6000

gacttcaacc gtgaaggatt acctctgttc atccttgcta actggagagg cttctctggt 6060

ggacaaagag atctttttga aggaatcctt caggctgggt caacaattgt tgagaacctt 6120

aggacataca atcagcctgc ctttgtatat atccccaagg ctgcagagct acgtggaggg 6180

gcttgggtcg tgattgatag caagataaat ccagatcgca ttgagttcta tgctgagagg 6240

actgcaaagg gcaatgttct cgaacctcaa gggttgatcg agatcaagtt caggtcagag 6300

gaactccaag agtgcatggg taggcttgat ccagaattga taaatctgaa ggcaaagctc 6360

cagggagtaa agcatgaaaa tggaagtcta cctgagtcag aatcccttca gaagagcata 6420

gaagcccgga agaaacagtt gttgcctttg tatactcaaa ttgcggtacg gttcgctgaa 6480

ttgcatgaca cttcccttag aatggctgct aagggtgtga ttaagaaggt tgtagactgg 6540

gaagattcta ggtcgttctt ctacaagaga ttacggagga ggatatccga ggatgttctt 6600

gcgaaggaaa ttagaggtgt aagtggcaag cagttttctc accaatcggc aatcgagctg 6660

atccagaaat ggtacttggc ctctaaggga gctgaaacag gaagcactga atgggatgat 6720

gacgatgctt ttgttgcctg gagggaaaac cctgaaaact accaggagta tatcaaagaa 6780

ctcagggctc aaagggtatc tcagttgctc tcagatgttg cagactccag tccagatcta 6840

gaagccttgc cacagggtct ttctatgcta ttagagaaga tggatccctc aaggagagca 6900

cagtttgttg aggaagtcaa gaaagtcctt aaatga 6936

<210> 16
<211> 2311
<212> PRT
<213> Triticum aestivum

<400> 16
Met Gly Ser Thr His Leu Pro Ile Val Gly Leu Asn Ala Ser Thr Thr
1               5                   10                  15

Pro Ser Leu Ser Thr Ile Arg Pro Val Asn Ser Ala Gly Ala Ala Phe
            20                  25                  30

Gln Pro Ser Ala Pro Ser Arg Thr Ser Lys Lys Lys Ser Arg Arg Val
        35                  40                  45

Gln Ser Leu Arg Asp Gly Gly Asp Gly Gly Val Ser Asp Pro Asn Gln
    50                  55                  60

Ser Ile Arg Gln Gly Leu Ala Gly Ile Ile Asp Leu Pro Lys Glu Gly
65                  70                  75                  80

Thr Ser Ala Pro Glu Val Asp Ile Ser His Gly Ser Glu Glu Pro Arg
                85                  90                  95

Gly Ser Tyr Gln Met Asn Gly Ile Leu Asn Glu Ala His Asn Gly Arg
            100                 105                 110

His Ala Ser Leu Ser Lys Val Val Glu Phe Cys Met Ala Leu Gly Gly
        115                 120                 125

Lys Thr Pro Ile His Ser Val Leu Val Ala Asn Asn Gly Met Ala Ala
    130                 135                 140

Ala Lys Phe Met Arg Ser Val Arg Thr Trp Ala Asn Glu Thr Phe Gly
145                 150                 155                 160

Ser Glu Lys Ala Ile Gln Leu Ile Ala Met Ala Thr Pro Glu Asp Met

165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Ile Ala Val Arg Thr Gly Val Ser Ala Val Trp Pro Gly Trp Gly
    210                 215                 220

His Ala Ser Glu Asn Pro Glu Leu Pro Asp Ala Leu Asn Ala Asn Gly
225                 230                 235                 240

Ile Val Phe Leu Gly Pro Pro Ser Ser Ser Met Asn Ala Leu Gly Asp
                245                 250                 255

Lys Val Gly Ser Ala Leu Ile Ala Gln Ala Ala Gly Val Pro Thr Leu
            260                 265                 270

Pro Trp Ser Gly Ser Gln Val Glu Ile Pro Leu Glu Val Cys Leu Asp
        275                 280                 285

Ser Ile Pro Ala Glu Met Tyr Arg Lys Ala Cys Val Ser Thr Thr Glu
    290                 295                 300

Glu Ala Leu Ala Ser Cys Gln Met Ile Gly Tyr Pro Ala Met Ile Lys
305                 310                 315                 320

Ala Ser Trp Gly Gly Gly Gly Lys Gly Ile Arg Lys Val Asn Asn Asp
                325                 330                 335

Asp Asp Val Arg Ala Leu Phe Lys Gln Val Gln Gly Glu Val Pro Gly
            340                 345                 350

Ser Pro Ile Phe Ile Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Glu Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Glu Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Val Pro Glu Ile Arg Arg Phe Tyr Gly Met Asp Asn Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Val Ser Arg Ala Ala Ala Ile Thr
            580                 585                 590

Asn Met Ser Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Lys
    610                 615                 620

Glu Asn Arg Ile His Thr Gly Trp Leu Asp Asn Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Ile Thr Ser Asn Thr Asp Thr Val Ser Glu Tyr Val
            660                 665                 670

Ser Tyr Leu Val Lys Gly Gln Ile Pro Pro Lys His Ile Ser Leu Val
        675                 680                 685

His Ser Thr Val Ser Leu Asn Ile Glu Glu Ser Lys Tyr Thr Ile Glu
    690                 695                 700

Thr Ile Arg Ser Gly Gln Gly Ser Tyr Arg Leu Arg Met Asn Gly Ser
705                 710                 715                 720

Val Ile Glu Ala Asn Val Gln Thr Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Leu Ile Asp Gly Lys Thr Cys Leu Leu Gln Asn Asp
        755                 760                 765

His Asp Pro Ser Arg Leu Leu Ala Glu Thr Pro Cys Lys Leu Leu Arg
    770                 775                 780

Phe Leu Val Ala Asp Gly Ala His Val Glu Ala Asp Val Pro Tyr Ala
785                 790                 795                 800

Glu Val Glu Val Met Lys Met Cys Met Pro Leu Leu Ser Pro Ala Ala
                805                 810                 815

Gly Val Ile Asn Val Leu Leu Ser Glu Gly Gln Pro Met Gln Ala Gly
            820                 825                 830

Asp Leu Ile Ala Arg Leu Asp Leu Asp Asp Pro Ser Ala Val Lys Arg
        835                 840                 845

Ala Glu Pro Phe Asn Gly Ser Phe Pro Glu Met Ser Leu Pro Ile Ala
    850                 855                 860

Ala Ser Gly Gln Val His Lys Arg Cys Ala Thr Ser Leu Asn Ala Ala
865                 870                 875                 880

Arg Met Val Leu Ala Gly Tyr Asp His Pro Ile Asn Lys Val Val Gln
                885                 890                 895

Asp Leu Val Ser Cys Leu Asp Ala Pro Glu Leu Pro Phe Leu Gln Trp
            900                 905                 910

Glu Glu Leu Met Ser Val Leu Ala Thr Arg Leu Pro Arg Leu Leu Lys
        915                 920                 925

Ser Glu Leu Glu Gly Lys Tyr Ser Glu Tyr Lys Leu Asn Val Gly His
    930                 935                 940

Gly Lys Ser Lys Asp Phe Pro Ser Lys Met Leu Arg Glu Ile Ile Glu
945                 950                 955                 960

Glu Asn Leu Ala His Gly Ser Glu Lys Glu Ile Ala Thr Asn Glu Arg
                965                 970                 975

Leu Val Glu Pro Leu Met Ser Leu Leu Lys Ser Tyr Glu Gly Gly Arg
            980                 985                 990

Glu Ser His Ala His Phe Ile Val  Lys Ser Leu Phe Glu  Asp Tyr Leu
        995                 1000                 1005

Ser Val  Glu Glu Leu Phe Ser  Asp Gly Ile Gln Ser  Asp Val Ile
    1010                 1015                 1020

Glu Arg  Leu Arg Gln Gln His  Ser Lys Asp Leu Gln  Lys Val Val
    1025                 1030                 1035

Asp Ile  Val Leu Ser His Gln  Gly Val Arg Asn Lys  Thr Lys Leu
    1040                 1045                 1050

Ile Leu  Thr Leu Met Glu Lys  Leu Val Tyr Pro Asn  Pro Ala Val
    1055                 1060                 1065

Tyr Lys  Asp Gln Leu Thr Arg  Phe Ser Ser Leu Asn  His Lys Arg
    1070                 1075                 1080

Tyr Tyr  Lys Leu Ala Leu Lys  Ala Ser Glu Leu Leu  Glu Gln Thr
    1085                 1090                 1095

Lys Leu  Ser Glu Leu Arg Thr  Ser Ile Ala Arg Ser  Leu Ser Glu
    1100                 1105                 1110

Leu Glu  Met Phe Thr Glu Glu  Arg Thr Ala Ile Ser  Glu Ile Met
    1115                 1120                 1125

Gly Asp  Leu Val Thr Ala Pro  Leu Pro Val Glu Asp  Ala Leu Val
    1130                 1135                 1140

Ser Leu  Phe Asp Cys Ser Asp  Gln Thr Leu Gln Gln  Arg Val Ile
    1145                 1150                 1155

Glu Thr  Tyr Ile Ser Arg Leu  Tyr Gln Pro His Leu  Val Lys Asp

1160                 1165                 1170

Ser Ile Gln Leu Lys Tyr Gln Glu Ser Gly Val Ile Ala Leu Trp
    1175                 1180                 1185

Glu Phe Ala Glu Ala His Ser Glu Lys Arg Leu Gly Ala Met Val
    1190                 1195                 1200

Ile Val Lys Ser Leu Glu Ser Val Ser Ala Ala Ile Gly Ala Ala
    1205                 1210                 1215

Leu Lys Gly Thr Ser Arg Tyr Ala Ser Ser Glu Gly Asn Ile Met
    1220                 1225                 1230

His Ile Ala Leu Leu Gly Ala Asp Asn Gln Met His Gly Thr Glu
    1235                 1240                 1245

Asp Ser Gly Asp Asn Asp Gln Ala Gln Val Arg Ile Asp Lys Leu
    1250                 1255                 1260

Ser Ala Thr Leu Glu Gln Asn Thr Val Thr Ala Asp Leu Arg Ala
    1265                 1270                 1275

Ala Gly Val Lys Val Ile Ser Cys Ile Val Gln Arg Asp Gly Ala
    1280                 1285                 1290

Leu Met Pro Met Arg His Thr Phe Leu Leu Ser Asp Glu Lys Leu
    1295                 1300                 1305

Cys Tyr Glu Glu Glu Pro Val Leu Arg His Val Glu Pro Pro Leu
    1310                 1315                 1320

Ser Ala Leu Leu Glu Leu Gly Lys Leu Lys Val Lys Gly Tyr Asn
    1325                 1330                 1335

Glu Val Lys Tyr Thr Pro Ser Arg Asp Arg Gln Trp Asn Ile Tyr
    1340                 1345                 1350

Thr Leu Arg Asn Thr Glu Asn Pro Lys Met Leu His Arg Val Phe
    1355                 1360                 1365

Phe Arg Thr Leu Val Arg Gln Pro Gly Ala Ser Asn Lys Phe Thr
    1370                 1375                 1380

Ser Gly Asn Ile Ser Asp Val Glu Val Gly Gly Ala Glu Glu Ser
    1385                 1390                 1395

Leu Ser Phe Thr Ser Ser Ser Ile Leu Arg Ser Leu Met Thr Ala
    1400                1405                1410

Ile Glu Glu Leu Glu Leu His Ala Ile Arg Thr Gly His Ser His
    1415                1420                1425

Met Phe Leu Cys Ile Leu Lys Glu Gln Lys Leu Leu Asp Leu Val
    1430                1435                1440

Pro Val Ser Gly Asn Lys Val Val Asp Ile Gly Gln Asp Glu Ala
    1445                1450                1455

Thr Ala Cys Leu Leu Leu Lys Glu Met Ala Leu Gln Ile His Glu
    1460                1465                1470

Leu Val Gly Ala Arg Met His His Leu Ser Val Cys Gln Trp Glu
    1475                1480                1485

Val Lys Leu Lys Leu Asp Ser Asp Gly Pro Ala Ser Gly Thr Trp
    1490                1495                1500

Arg Val Val Thr Thr Asn Val Thr Ser His Thr Cys Thr Val Asp
    1505                1510                1515

Ile Tyr Arg Glu Val Glu Asp Thr Glu Ser Gln Lys Leu Val Tyr
    1520                1525                1530

His Ser Ala Pro Ser Ser Ser Gly Pro Leu His Gly Val Ala Leu
    1535                1540                1545

Asn Thr Pro Tyr Gln Pro Leu Ser Val Ile Asp Leu Lys Arg Cys
    1550                1555                1560

Ser Ala Arg Asn Asn Arg Thr Thr Tyr Cys Tyr Asp Phe Pro Leu
    1565                1570                1575

Ala Phe Glu Thr Ala Val Gln Lys Ser Trp Ser Asn Ile Ser Ser
    1580                1585                1590

Asp Thr Asn Arg Cys Tyr Val Lys Ala Thr Glu Leu Val Phe Ala
    1595                1600                1605

His Lys Asn Gly Ser Trp Gly Thr Pro Val Ile Pro Met Glu Arg
    1610                1615                1620

Pro Ala Gly Leu Asn Asp Ile Gly Met Val Ala Trp Ile Leu Asp
    1625                1630                1635

Met Ser Thr Pro Glu Tyr Pro Asn Gly Arg Gln Ile Val Val Ile
    1640                1645                1650

Ala Asn Asp Ile Thr Phe Arg Ala Gly Ser Phe Gly Pro Arg Glu
    1655                1660                1665

Asp Ala Phe Phe Glu Thr Val Thr Asn Leu Ala Cys Glu Arg Lys
    1670                1675                1680

Leu Pro Leu Ile Tyr Leu Ala Ala Asn Ser Gly Ala Arg Ile Gly
    1685                1690                1695

Ile Ala Asp Glu Val Lys Ser Cys Phe Arg Val Gly Trp Ser Asp
    1700                1705                1710

Asp Gly Ser Pro Glu Arg Gly Phe Gln Tyr Ile Tyr Leu Thr Glu
    1715                1720                1725

Glu Asp His Ala Arg Ile Ser Ala Ser Val Ile Ala His Lys Met
    1730                1735                1740

Gln Leu Asp Asn Gly Glu Ile Arg Trp Val Ile Asp Ser Val Val
    1745                1750                1755

Gly Lys Glu Asp Gly Leu Gly Val Glu Asn Ile His Gly Ser Ala
    1760                1765                1770

Ala Ile Ala Ser Ala Tyr Ser Arg Ala Tyr Glu Glu Thr Phe Thr
    1775                1780                1785

Leu Thr Phe Val Thr Gly Arg Thr Val Gly Ile Gly Ala Tyr Leu
    1790                1795                1800

Ala Arg Leu Gly Ile Arg Cys Ile Gln Arg Thr Asp Gln Pro Ile
    1805                1810                1815

Ile Leu Thr Gly Phe Ser Ala Leu Asn Lys Leu Leu Gly Arg Glu
    1820                1825                1830

Val Tyr Ser Ser His Met Gln Leu Gly Gly Pro Lys Ile Met Ala
    1835                1840                1845

Thr Asn Gly Val Val His Leu Thr Val Ser Asp Asp Leu Glu Gly
    1850                1855                1860

Val Ser Asn Ile Leu Arg Trp Leu Ser Tyr Val Pro Ala Asn Ile
    1865                1870                1875

Gly Gly Pro Leu Pro Ile Thr Lys Ser Leu Asp Pro Pro Asp Arg
    1880                1885                1890

Pro Val Ala Tyr Ile Pro Glu Asn Thr Cys Asp Pro Arg Ala Ala
    1895                1900                1905

Ile Ser Gly Ile Asp Asp Ser Gln Gly Lys Trp Leu Gly Gly Met
    1910                1915                1920

Phe Asp Lys Asp Ser Phe Val Glu Thr Phe Glu Gly Trp Ala Lys
    1925                1930                1935

Ser Val Val Thr Gly Arg Ala Lys Leu Gly Gly Ile Pro Val Gly
    1940                1945                1950

Val Ile Ala Val Glu Thr Gln Thr Met Met Gln Leu Ile Pro Ala
    1955                1960                1965

Asp Pro Gly Gln Leu Asp Ser His Glu Arg Ser Val Pro Arg Ala
    1970                1975                1980

Gly Gln Val Trp Phe Pro Asp Ser Ala Thr Lys Thr Ala Gln Ala
    1985                1990                1995

Met Leu Asp Phe Asn Arg Glu Gly Leu Pro Leu Phe Ile Leu Ala
    2000                2005                2010

Asn Trp Arg Gly Phe Ser Gly Gly Gln Arg Asp Leu Phe Glu Gly
    2015                2020                2025

Ile Leu Gln Ala Gly Ser Thr Ile Val Glu Asn Leu Arg Thr Tyr
    2030                2035                2040

Asn Gln Pro Ala Phe Val Tyr Ile Pro Lys Ala Ala Glu Leu Arg
    2045                2050                2055

Gly Gly Ala Trp Val Val Ile Asp Ser Lys Ile Asn Pro Asp Arg
    2060                2065                2070

Ile Glu Phe Tyr Ala Glu Arg Thr Ala Lys Gly Asn Val Leu Glu
    2075                2080                2085

Pro Gln Gly Leu Ile Glu Ile Lys Phe Arg Ser Glu Glu Leu Gln
    2090                2095                2100

Glu Cys Met Gly Arg Leu Asp Pro Glu Leu Ile Asn Leu Lys Ala

2105                    2110                    2115

Lys Leu Gln Gly Val Lys His Glu Asn Gly Ser Leu Pro Glu Ser
    2120                    2125                    2130

Glu Ser Leu Gln Lys Ser Ile Glu Ala Arg Lys Lys Gln Leu Leu
    2135                    2140                    2145

Pro Leu Tyr Thr Gln Ile Ala Val Arg Phe Ala Glu Leu His Asp
    2150                    2155                    2160

Thr Ser Leu Arg Met Ala Ala Lys Gly Val Ile Lys Lys Val Val
    2165                    2170                    2175

Asp Trp Glu Asp Ser Arg Ser Phe Phe Tyr Lys Arg Leu Arg Arg
    2180                    2185                    2190

Arg Ile Ser Glu Asp Val Leu Ala Lys Glu Ile Arg Gly Val Ser
    2195                    2200                    2205

Gly Lys Gln Phe Ser His Gln Ser Ala Ile Glu Leu Ile Gln Lys
    2210                    2215                    2220

Trp Tyr Leu Ala Ser Lys Gly Ala Glu Thr Gly Ser Thr Glu Trp
    2225                    2230                    2235

Asp Asp Asp Asp Ala Phe Val Ala Trp Arg Glu Asn Pro Glu Asn
    2240                    2245                    2250

Tyr Gln Glu Tyr Ile Lys Glu Leu Arg Ala Gln Arg Val Ser Gln
    2255                    2260                    2265

Leu Leu Ser Asp Val Ala Asp Ser Ser Pro Asp Leu Glu Ala Leu
    2270                    2275                    2280

Pro Gln Gly Leu Ser Met Leu Leu Glu Lys Met Asp Pro Ser Arg
    2285                    2290                    2295

Arg Ala Gln Phe Val Glu Glu Val Lys Lys Val Leu Lys
    2300                    2305                    2310

<210> 17
<211> 6966
<212> DNA
<213> Setaria italica

<400> 17
atgtcgcaac ttggattagc tgcagctgcc tcaaaggcgc tgccactact tcctaatcgc        60

catagaactt cagctggaac tacattccca tcacctgtat catcgcggcc ctcaaaccga 120
aggaaaagcc gcactcgttc acttcgtgat ggaggagatg gggtatcaga tgccaaaaag 180
cacaaccagt ctgtccgtca aggtcttgct ggcatcatcg acctcccaaa tgaggcaaca 240
tcggaagtgg atatttctca tggatccgag gatcccaggg ggccaaccga ttcatatcaa 300
atgaatggga ttgtaagtga agcacataat ggcagacatg cctcagtgtc caaggttgtt 360
gaattttgtg cggcgctagg tggcaaaaca ccaattcaca gtatactagt ggccaacaat 420
ggaatggcag cagcaaagtt catgaggagt gtccggacat gggctaatga tacttttgga 480
tcggagaagg cgattcagct catagctatg gcaactccag aagacatgag gataaatgca 540
gaacacatta gaattgctga tcaatttgtg gaggtgcctg gtggaacaaa caataacaac 600
tatgcaaatg ttcaactcat agtggaggta gcagaaagaa taggtgtttc tgctgtttgg 660
cctggttggg gtcatgcttc tgagaatcct gaacttccag atgcattgac cgcaaaagga 720
gttgttttcc ttgggccacc tgcggcatca atgaatgcat tgggagataa ggtcggttca 780
gctctcattg ctcaagcagc tggggtcccg accctttcgt ggagtggatc acatgttgaa 840
gttccattag agtgctgctt agatgcgata cctgaggaaa tgtatagaaa agcttgtgtt 900
actaccacag aagaagctgt tgcgagttgt caggtggttg gttatcctgc catgattaag 960
gcatcctggg gaggtggtgg taaaggaata agaaaggttc ataatgacga tgaggttaga 1020
gcactgttta agcaagtaca aggtgaagtc cctggctccc caatatttat catgaggctt 1080
gcatcccaga gtcgtcatct tgaagttcag ttgctttgtg atcaatatgg caatgtggca 1140
gcacttcaca gtcgtgattg cagtgtgcaa cggcgacacc aaaagattat tgaggaaggc 1200
ccagttactg ttgctcctcg tgagacagtt aaagcgcttg agcaggcagc aaggaggctt 1260
gctaaggctg tgggttatgt tggtgctgct actgttgaat acctttacag catggagact 1320
ggggaatact attttctgga gcttaatccc agattacagg tcgagcatcc agtcactgag 1380
tggattgctg aagtaaatct tcctgcagct caagttgcag ttggaatggg catacctctt 1440
tggcagattc cagaaatcag acgtttcgat ggaatggact atggaggagg atatgacatt 1500
tggaggaaaa cagcagctct tgccacacca tttaattttg atgaagtaga ttctcaatgg 1560
ccaaagggcc attgtgtagc agttagaatt actagcgagg atccagatga tggtttcaaa 1620
cctactggtg ggaaagtgaa ggagataagt tttaaaagca agcctaatgt ttgggcctac 1680
ttctcagtaa agtctggtgg aggcattcat gaatttgttg attctcagtt tgggcatgtt 1740
tttgcatatg ggctctctag atcagcagca ataacgaaca tggctcttgc attaaaagag 1800
attcaaattc gtggagaaat tcattcaaat gttgattaca cagttgatct cttaaatgct 1860
tcagacttca gagaaaataa gattcatact ggctggcttg ataccagaat agctatgcgt 1920
gttcaagctg agaggccccc atggtatatt tcagtggttg gaggagctct atataaaaca 1980

gtaactgcca atgcagccac tgtttctgat tatgtcagtt atctcaccaa gggccagatt 2040

ccaccaaagc atatatccct tgtcagttca acagttaatc tgaatatcga agggagcaaa 2100

tacacagttg aaactgtaag gactggacat ggtagctaca gattacgaat gaatgattca 2160

gcaattgaag cgaatgtaca atccttatgt gatggaggcc tcttaatgca gttggatgga 2220

aatagccatg taatttacgc ggaagaagaa gctggtggta cacgacttct gattgatgga 2280

aagacatgct tgttacagaa tgatcatgat ccatcaaagt tattagctga gacaccctgc 2340

aaacttcttc ggttcttggt tgctgatggt gcccatgttg atgctgatgt accatatgcg 2400

gaagttgagg ttatgaaaat gtgcatgcct ctcttgtcgc ctgcttctgg tgtcattcat 2460

gttatgatgt ctgagggcca ggcattgcag gctggtgatc ttatagcaag gctggatctt 2520

gatgaccctt ctgctgtgaa aagagctgaa ccatttcatg gaatatttcc acaaatggac 2580

cttcctgttg ctgcctctag ccaagtacac aaaagatatg ctgcaagttg gaatgctgct 2640

cgaatggtcc ttgcaggata cgagcataat atcaatgaag ttgtacaaga tttggtatgc 2700

tgcctggatg atcccgagct tcccttccta cagtgggatg aacttatgtc agttctagca 2760

actaggcttc caagaaatct taagagtgag ttagaggata aatacatgga atacaagttg 2820

aacttttacc atgggaaaaa caaggacttc ccgtccaagc tgctgagaga catcattgag 2880

gcaaatcttg catatggttc agagaaggaa aaagctacga atgagaggct tattgagcct 2940

cttatgagcc tacttaagtc atatgagggt gggagagaaa gccatgctca ttttgttgtc 3000

aagtcccttt tcaaggagta ccttgctgtg gaagaacttt tcagtgatgg gattcagtct 3060

gatgtgattg aaaccctgcg tcatcagcac agtaaagact tgcagaaggt tgtagacatt 3120

gtgttgtctc accagggtgt gaggaacaaa gctaagcttg taacagcact tatggaaaag 3180

ctggtttatc caaatcctgc tgcttacagg gatctgttgg ttcgcttttc ttcactcaat 3240

cataaaagat attataagtt ggcccttaaa gcaagcgaac ttcttgaaca aactaaacta 3300

agtgaactcc gtgcaagcat cgcaagaagc ctttctgatc tggggatgca taagggagaa 3360

atgactattg aagatagcat ggaagattta gtctctgccc cattacctgt cgaagatgca 3420

cttatttctt tgtttgatta cagtgatcca actgttcagc agaaagtgat cgagacatac 3480

atatctcgat tgtatcagcc tcttcttgtg aaagatagca tccaagtgaa atttaaggaa 3540

tctggtgcct ttgctttatg ggaattttct gaagggcatg ttgatactaa aaatggacaa 3600

gggaccgttc ttggtcgaac aagatggggt gccatggtag ctgtcaaatc agttgaatct 3660

gcacgaacag ccattgtagc tgcattaaag gattcggcac agcatgccag ctctgagggc 3720

aacatgatgc acattgcctt attgagtgct gaaaatgaaa ataatatcag tgatgatcaa 3780

gctcaacata ggatggaaaa acttaacaag atactcaagg atactagtgt cgcaaatgat 3840

cttcgagctg ctggtttgaa ggttataagt tgcattgttc aaagagatga agcacgcatg 3900

ccaatgcgcc acacattact ctggtcagat gaaaagagtt gttatgagga agagcagatt 3960

cttcggcatg tggagcctcc cctctccatg cttcttgaaa tggataagtt gaaagtgaaa 4020

ggatacaatg aaatgaagta tactccatca cgtgatcgtc aatggcatat ctacacacta 4080

agaaatactg aaaaccccaa aatgttgcat agggtatttt tccgaactat tgtcaggcaa 4140

cccaatgcag gcaacaagtt tatatcagcc caaattggcg acactgaagt aggaggtcct 4200

gaggaatctt tgtcatttac atctaatagc attttaagag ccttgatgac tgctattgaa 4260

gaattagagc ttcatgcaat taggactgat cattctcaca tgtatttgtg catattgaaa 4320

gaacaaaagc ttcttgatct cattccgttt tcagggagca caatcgtcga tgttgtccaa 4380

gacgaagcta ctgcttgttc acttttaaaa tcaatggctt tgaagataca cgaacttgtt 4440

ggtgcacaga tgcatcatct ttctgtatgc cagtgggagg tgaaactcaa gttgtactgc 4500

gatgggcctg ccagtggcac ctggagagtt gtaactacaa atgttactag tcacacttgc 4560

accgttgata tctaccggga agtggaagat actgaatcgc agaagttagt ataccattca 4620

gcttctccgt cagctagtcc tttgcatggt gtggccctgg ataatccgta tcaacctttg 4680

agtgtcattg atctaaaaca ctgctctgct aggaacaaca gaactacata ttgctatgat 4740

tttccactgg catttgaaac tgccctgcag aagtcatggc agtccaatgg ctccagtgtt 4800

tctgaaggca gtgaaaatag taggtcttat gtgaaagcaa cagagctggt gtttgctgaa 4860

aaacatgggt cctggggcac tcctataatt tccatggagc gtcccgctgg gctcaatgac 4920

attggcatgg tagcttggat cttagagatg tccactcctg aatttcccaa tggcaggcag 4980

attattgtca tagcaaatga tattactttc agagctggat catttggccc aagggaagat 5040

gcgttttttg aagctgtcac gaacctggcc tgcgagagga agcttcctct tatatacttg 5100

gcagcaaact ccggtgctag gattggcata gccgatgaag tgaaatcttg cttccgtgtt 5160

gggtggtccg atgaaggcag ccctgaacgg ggttttcagt acatttatct gactgacgaa 5220

gactatgccc gtattagctt gtctgttata gcacacaagc tgcagctgga taatggtgaa 5280

attaggtgga ttattgactc tgttgtgggc aaggaggatg ggcttggtgt tgagaatata 5340

catggaagtg ctgctattgc cagtgcttat tctagggcat atgaggagac atttacactt 5400

acatttgtga ctgggcggac tgttggaata ggagcatatc ttgctcggct cggtatacgg 5460

tgcatacagc gtcttgacca gcctattatt ttaactgggt tttctgccct gaacaagctt 5520

cttgggcggg aagtgtacag ctcccacatg cagttgggtg gtcctaagat catggcgacc 5580

aatggtgttg tccacttgac tgtttcagat gaccttgaag gtgtttccaa tatattgagg 5640

tggctcagct atgttcctgc caacattggt ggacctcttc ctattacaaa acctttggac 5700

ccaccagaca gacctgttgc atacatccct gagaacacat gtgatccgcg cgcagccatt 5760

cgtggtgtag atgacagcca agggaaatgg ttgggtggta tgtttgacaa agacagcttt 5820

gtcgagacat ttgaaggatg ggcgaaaaca gtggttacgg gcagagcaaa gcttggagga 5880

attcctgttg gcgtcatagc tgtggagaca caaaccatga tgcagcttat ccctgctgat 5940

ccaggccagc ttgattccca tgagcgatct gttcctcggg ctggacaagt gtggttccca 6000

gattctgcaa ccaagacagc tcaggcattg ttggacttca accgtgaagg attgccgctg 6060

ttcatccttg ctaactggag aggattctct ggtggacaaa gagatctgtt tgaaggaatt 6120

cttcaggctg ggtcaacaat tgttgagaac cttaggacat acaatcagcc tgcttttgtc 6180

tacattccta tggctggaga gctgcgtgga ggagcttggg ttgtggttga tagcaaaata 6240

aatccagacc gaattgagtg ttatgctgag aggactgcta aaggcaatgt tctggaacct 6300

caagggttaa ttgaaatcaa attcagatca gaggagctcc aagactgtat gggtaggctt 6360

gacccagggt tgataaatct gaaagcaaaa ctccaaggtg caaagcttgg aaatggaagc 6420

ctaacagatg tagaatccct tcagaagagt atagatgctc gtacgaaaca gttgttgcct 6480

ttatacaccc agattgcaat acggtttgct gaattgcatg atacttccct cagaatggca 6540

gctaaaggtg tgattaagaa agttgtagat tgggaagaat cacgttcttt cttctacaga 6600

aggctacgga ggaggatctc tgaagatgtt cttgcaaaag aaataagagg aatagctggt 6660

gaccacttca ctcaccaatc agcagttgag ctgatcaagg aatggtactt ggcttctcaa 6720

gccacaacag gaagcactga atgggatgat gatgatgctt ttgttgcctg gaaggagaat 6780

cctgaaaact ataagggata tatccaagag ttaagggctc aaaaggtgtc tcagtcgctc 6840

tccgatcttg cagactccag ttcagatcta gaagcattct cacagggtct ttccacatta 6900

ttagataaga tggatccctc tcagagagcc aagttcattc aggaagtcaa gaaggtcctg 6960

ggttga 6966

<210> 18
<211> 2321
<212> PRT
<213> Setaria italica

<400> 18
Met Ser Gln Leu Gly Leu Ala Ala Ala Ala Ser Lys Ala Leu Pro Leu
1               5                   10                  15

Leu Pro Asn Arg His Arg Thr Ser Ala Gly Thr Thr Phe Pro Ser Pro
            20                  25                  30

Val Ser Ser Arg Pro Ser Asn Arg Arg Lys Ser Arg Thr Arg Ser Leu
        35                  40                  45

Arg Asp Gly Gly Asp Gly Val Ser Asp Ala Lys Lys His Asn Gln Ser

50                  55                  60

Val Arg Gln Gly Leu Ala Gly Ile Ile Asp Leu Pro Asn Glu Ala Thr
65                  70                  75                  80

Ser Glu Val Asp Ile Ser His Gly Ser Glu Asp Pro Arg Gly Pro Thr
                85                  90                  95

Asp Ser Tyr Gln Met Asn Gly Ile Val Ser Glu Ala His Asn Gly Arg
            100                 105                 110

His Ala Ser Val Ser Lys Val Val Glu Phe Cys Ala Ala Leu Gly Gly
        115                 120                 125

Lys Thr Pro Ile His Ser Ile Leu Val Ala Asn Asn Gly Met Ala Ala
    130                 135                 140

Ala Lys Phe Met Arg Ser Val Arg Thr Trp Ala Asn Asp Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Val Ala Glu Arg Ile Gly Val Ser Ala Val Trp Pro Gly Trp Gly
    210                 215                 220

His Ala Ser Glu Asn Pro Glu Leu Pro Asp Ala Leu Thr Ala Lys Gly
225                 230                 235                 240

Val Val Phe Leu Gly Pro Pro Ala Ala Ser Met Asn Ala Leu Gly Asp
                245                 250                 255

Lys Val Gly Ser Ala Leu Ile Ala Gln Ala Ala Gly Val Pro Thr Leu
            260                 265                 270

Ser Trp Ser Gly Ser His Val Glu Val Pro Leu Glu Cys Cys Leu Asp
        275                 280                 285

Ala Ile Pro Glu Glu Met Tyr Arg Lys Ala Cys Val Thr Thr Thr Glu
    290                 295                 300

Glu Ala Val Ala Ser Cys Gln Val Val Gly Tyr Pro Ala Met Ile Lys
305                 310                 315                 320

Ala Ser Trp Gly Gly Gly Gly Lys Gly Ile Arg Lys Val His Asn Asp
                325                 330                 335

Asp Glu Val Arg Ala Leu Phe Lys Gln Val Gln Gly Glu Val Pro Gly
            340                 345                 350

Ser Pro Ile Phe Ile Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Ala Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Glu Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Ile Pro Glu Ile Arg Arg Phe Asp Gly Met Asp Tyr Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Val Asp Ser Gln
                565                 570                 575

Phe Gly His Val Phe Ala Tyr Gly Leu Ser Arg Ser Ala Ala Ile Thr
            580                 585                 590

Asn Met Ala Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Arg
    610                 615                 620

Glu Asn Lys Ile His Thr Gly Trp Leu Asp Thr Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Val Thr Ala Asn Ala Ala Thr Val Ser Asp Tyr Val
            660                 665                 670

Ser Tyr Leu Thr Lys Gly Gln Ile Pro Pro Lys His Ile Ser Leu Val
        675                 680                 685

Ser Ser Thr Val Asn Leu Asn Ile Glu Gly Ser Lys Tyr Thr Val Glu
    690                 695                 700

Thr Val Arg Thr Gly His Gly Ser Tyr Arg Leu Arg Met Asn Asp Ser
705                 710                 715                 720

Ala Ile Glu Ala Asn Val Gln Ser Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Leu Ile Asp Gly Lys Thr Cys Leu Leu Gln Asn Asp
        755                 760                 765

His Asp Pro Ser Lys Leu Leu Ala Glu Thr Pro Cys Lys Leu Leu Arg
    770                 775                 780

Phe Leu Val Ala Asp Gly Ala His Val Asp Ala Asp Val Pro Tyr Ala
785                 790                 795                 800

Glu Val Glu Val Met Lys Met Cys Met Pro Leu Leu Ser Pro Ala Ser
                805                 810                 815

Gly Val Ile His Val Met Met Ser Glu Gly Gln Ala Leu Gln Ala Gly
            820                 825                 830

Asp Leu Ile Ala Arg Leu Asp Leu Asp Asp Pro Ser Ala Val Lys Arg
        835                 840                 845

Ala Glu Pro Phe His Gly Ile Phe Pro Gln Met Asp Leu Pro Val Ala
    850                 855                 860

Ala Ser Ser Gln Val His Lys Arg Tyr Ala Ala Ser Trp Asn Ala Ala
865                 870                 875                 880

Arg Met Val Leu Ala Gly Tyr Glu His Asn Ile Asn Glu Val Val Gln
                885                 890                 895

Asp Leu Val Cys Cys Leu Asp Asp Pro Glu Leu Pro Phe Leu Gln Trp
            900                 905                 910

Asp Glu Leu Met Ser Val Leu Ala Thr Arg Leu Pro Arg Asn Leu Lys
        915                 920                 925

Ser Glu Leu Glu Asp Lys Tyr Met Glu Tyr Lys Leu Asn Phe Tyr His
    930                 935                 940

Gly Lys Asn Lys Asp Phe Pro Ser Lys Leu Leu Arg Asp Ile Ile Glu
945                 950                 955                 960

Ala Asn Leu Ala Tyr Gly Ser Glu Lys Glu Lys Ala Thr Asn Glu Arg
                965                 970                 975

Leu Ile Glu Pro Leu Met Ser Leu Leu Lys Ser Tyr Glu Gly Gly Arg
            980                 985                 990

Glu Ser His Ala His Phe Val Val  Lys Ser Leu Phe Lys  Glu Tyr Leu
        995                 1000                 1005

Ala Val  Glu Glu Leu Phe Ser  Asp Gly Ile Gln Ser  Asp Val Ile
    1010                 1015                 1020

Glu Thr  Leu Arg His Gln His  Ser Lys Asp Leu Gln  Lys Val Val
    1025                 1030                 1035

Asp Ile  Val Leu Ser His Gln  Gly Val Arg Asn Lys  Ala Lys Leu
    1040                 1045                 1050

Val Thr  Ala Leu Met Glu Lys  Leu Val Tyr Pro Asn  Pro Ala Ala

1055                1060                1065

Tyr Arg  Asp Leu Leu Val Arg  Phe Ser Ser Leu Asn  His Lys Arg
    1070                1075                1080

Tyr Tyr  Lys Leu Ala Leu Lys  Ala Ser Glu Leu Leu  Glu Gln Thr
    1085                1090                1095

Lys Leu  Ser Glu Leu Arg Ala  Ser Ile Ala Arg Ser  Leu Ser Asp
    1100                1105                1110

Leu Gly  Met His Lys Gly Glu  Met Thr Ile Glu Asp  Ser Met Glu
    1115                1120                1125

Asp Leu  Val Ser Ala Pro Leu  Pro Val Glu Asp Ala  Leu Ile Ser
    1130                1135                1140

Leu Phe  Asp Tyr Ser Asp Pro  Thr Val Gln Gln Lys  Val Ile Glu
    1145                1150                1155

Thr Tyr  Ile Ser Arg Leu Tyr  Gln Pro Leu Leu Val  Lys Asp Ser
    1160                1165                1170

Ile Gln  Val Lys Phe Lys Glu  Ser Gly Ala Phe Ala  Leu Trp Glu
    1175                1180                1185

Phe Ser  Glu Gly His Val Asp  Thr Lys Asn Gly Gln  Gly Thr Val
    1190                1195                1200

Leu Gly  Arg Thr Arg Trp Gly  Ala Met Val Ala Val  Lys Ser Val
    1205                1210                1215

Glu Ser  Ala Arg Thr Ala Ile  Val Ala Ala Leu Lys  Asp Ser Ala
    1220                1225                1230

Gln His  Ala Ser Ser Glu Gly  Asn Met Met His Ile  Ala Leu Leu
    1235                1240                1245

Ser Ala  Glu Asn Glu Asn Asn  Ile Ser Asp Asp Gln  Ala Gln His
    1250                1255                1260

Arg Met  Glu Lys Leu Asn Lys  Ile Leu Lys Asp Thr  Ser Val Ala
    1265                1270                1275

Asn Asp  Leu Arg Ala Ala Gly  Leu Lys Val Ile Ser  Cys Ile Val
    1280                1285                1290

Gln Arg Asp Glu Ala Arg Met Pro Met Arg His Thr Leu Leu Trp
    1295                1300                1305

Ser Asp Glu Lys Ser Cys Tyr Glu Glu Glu Gln Ile Leu Arg His
    1310                1315                1320

Val Glu Pro Pro Leu Ser Met Leu Leu Glu Met Asp Lys Leu Lys
    1325                1330                1335

Val Lys Gly Tyr Asn Glu Met Lys Tyr Thr Pro Ser Arg Asp Arg
    1340                1345                1350

Gln Trp His Ile Tyr Thr Leu Arg Asn Thr Glu Asn Pro Lys Met
    1355                1360                1365

Leu His Arg Val Phe Phe Arg Thr Ile Val Arg Gln Pro Asn Ala
    1370                1375                1380

Gly Asn Lys Phe Ile Ser Ala Gln Ile Gly Asp Thr Glu Val Gly
    1385                1390                1395

Gly Pro Glu Glu Ser Leu Ser Phe Thr Ser Asn Ser Ile Leu Arg
    1400                1405                1410

Ala Leu Met Thr Ala Ile Glu Glu Leu Glu Leu His Ala Ile Arg
    1415                1420                1425

Thr Asp His Ser His Met Tyr Leu Cys Ile Leu Lys Glu Gln Lys
    1430                1435                1440

Leu Leu Asp Leu Ile Pro Phe Ser Gly Ser Thr Ile Val Asp Val
    1445                1450                1455

Val Gln Asp Glu Ala Thr Ala Cys Ser Leu Leu Lys Ser Met Ala
    1460                1465                1470

Leu Lys Ile His Glu Leu Val Gly Ala Gln Met His His Leu Ser
    1475                1480                1485

Val Cys Gln Trp Glu Val Lys Leu Lys Leu Tyr Cys Asp Gly Pro
    1490                1495                1500

Ala Ser Gly Thr Trp Arg Val Val Thr Thr Asn Val Thr Ser His
    1505                1510                1515

Thr Cys Thr Val Asp Ile Tyr Arg Glu Val Glu Asp Thr Glu Ser
    1520                1525                1530

Gln Lys Leu Val Tyr His Ser Ala Ser Pro Ser Ala Ser Pro Leu
    1535                1540                1545

His Gly Val Ala Leu Asp Asn Pro Tyr Gln Pro Leu Ser Val Ile
    1550                1555                1560

Asp Leu Lys His Cys Ser Ala Arg Asn Asn Arg Thr Thr Tyr Cys
    1565                1570                1575

Tyr Asp Phe Pro Leu Ala Phe Glu Thr Ala Leu Gln Lys Ser Trp
    1580                1585                1590

Gln Ser Asn Gly Ser Ser Val Ser Glu Gly Ser Glu Asn Ser Arg
    1595                1600                1605

Ser Tyr Val Lys Ala Thr Glu Leu Val Phe Ala Glu Lys His Gly
    1610                1615                1620

Ser Trp Gly Thr Pro Ile Ile Ser Met Glu Arg Pro Ala Gly Leu
    1625                1630                1635

Asn Asp Ile Gly Met Val Ala Trp Ile Leu Glu Met Ser Thr Pro
    1640                1645                1650

Glu Phe Pro Asn Gly Arg Gln Ile Ile Val Ile Ala Asn Asp Ile
    1655                1660                1665

Thr Phe Arg Ala Gly Ser Phe Gly Pro Arg Glu Asp Ala Phe Phe
    1670                1675                1680

Glu Ala Val Thr Asn Leu Ala Cys Glu Arg Lys Leu Pro Leu Ile
    1685                1690                1695

Tyr Leu Ala Ala Asn Ser Gly Ala Arg Ile Gly Ile Ala Asp Glu
    1700                1705                1710

Val Lys Ser Cys Phe Arg Val Gly Trp Ser Asp Glu Gly Ser Pro
    1715                1720                1725

Glu Arg Gly Phe Gln Tyr Ile Tyr Leu Thr Asp Glu Asp Tyr Ala
    1730                1735                1740

Arg Ile Ser Leu Ser Val Ile Ala His Lys Leu Gln Leu Asp Asn
    1745                1750                1755

Gly Glu Ile Arg Trp Ile Ile Asp Ser Val Val Gly Lys Glu Asp
    1760                1765                1770

Gly Leu Gly Val Glu Asn Ile His Gly Ser Ala Ala Ile Ala Ser
    1775                1780                1785

Ala Tyr Ser Arg Ala Tyr Glu Glu Thr Phe Thr Leu Thr Phe Val
    1790                1795                1800

Thr Gly Arg Thr Val Gly Ile Gly Ala Tyr Leu Ala Arg Leu Gly
    1805                1810                1815

Ile Arg Cys Ile Gln Arg Leu Asp Gln Pro Ile Ile Leu Thr Gly
    1820                1825                1830

Phe Ser Ala Leu Asn Lys Leu Leu Gly Arg Glu Val Tyr Ser Ser
    1835                1840                1845

His Met Gln Leu Gly Gly Pro Lys Ile Met Ala Thr Asn Gly Val
    1850                1855                1860

Val His Leu Thr Val Ser Asp Asp Leu Glu Gly Val Ser Asn Ile
    1865                1870                1875

Leu Arg Trp Leu Ser Tyr Val Pro Ala Asn Ile Gly Gly Pro Leu
    1880                1885                1890

Pro Ile Thr Lys Pro Leu Asp Pro Pro Asp Arg Pro Val Ala Tyr
    1895                1900                1905

Ile Pro Glu Asn Thr Cys Asp Pro Arg Ala Ala Ile Arg Gly Val
    1910                1915                1920

Asp Asp Ser Gln Gly Lys Trp Leu Gly Gly Met Phe Asp Lys Asp
    1925                1930                1935

Ser Phe Val Glu Thr Phe Glu Gly Trp Ala Lys Thr Val Val Thr
    1940                1945                1950

Gly Arg Ala Lys Leu Gly Gly Ile Pro Val Gly Val Ile Ala Val
    1955                1960                1965

Glu Thr Gln Thr Met Met Gln Leu Ile Pro Ala Asp Pro Gly Gln
    1970                1975                1980

Leu Asp Ser His Glu Arg Ser Val Pro Arg Ala Gly Gln Val Trp
    1985                1990                1995

Phe Pro Asp Ser Ala Thr Lys Thr Ala Gln Ala Leu Leu Asp Phe

2000                2005                2010

Asn Arg Glu Gly Leu Pro Leu Phe Ile Leu Ala Asn Trp Arg Gly
    2015                2020                2025

Phe Ser Gly Gly Gln Arg Asp Leu Phe Glu Gly Ile Leu Gln Ala
    2030                2035                2040

Gly Ser Thr Ile Val Glu Asn Leu Arg Thr Tyr Asn Gln Pro Ala
    2045                2050                2055

Phe Val Tyr Ile Pro Met Ala Gly Glu Leu Arg Gly Gly Ala Trp
    2060                2065                2070

Val Val Val Asp Ser Lys Ile Asn Pro Asp Arg Ile Glu Cys Tyr
    2075                2080                2085

Ala Glu Arg Thr Ala Lys Gly Asn Val Leu Glu Pro Gln Gly Leu
    2090                2095                2100

Ile Glu Ile Lys Phe Arg Ser Glu Glu Leu Gln Asp Cys Met Gly
    2105                2110                2115

Arg Leu Asp Pro Gly Leu Ile Asn Leu Lys Ala Lys Leu Gln Gly
    2120                2125                2130

Ala Lys Leu Gly Asn Gly Ser Leu Thr Asp Val Glu Ser Leu Gln
    2135                2140                2145

Lys Ser Ile Asp Ala Arg Thr Lys Gln Leu Leu Pro Leu Tyr Thr
    2150                2155                2160

Gln Ile Ala Ile Arg Phe Ala Glu Leu His Asp Thr Ser Leu Arg
    2165                2170                2175

Met Ala Ala Lys Gly Val Ile Lys Lys Val Val Asp Trp Glu Glu
    2180                2185                2190

Ser Arg Ser Phe Phe Tyr Arg Arg Leu Arg Arg Arg Ile Ser Glu
    2195                2200                2205

Asp Val Leu Ala Lys Glu Ile Arg Gly Ile Ala Gly Asp His Phe
    2210                2215                2220

Thr His Gln Ser Ala Val Glu Leu Ile Lys Glu Trp Tyr Leu Ala
    2225                2230                2235

Ser Gln Ala Thr Thr Gly Ser Thr Glu Trp Asp Asp Asp Asp Ala
    2240                2245                2250

Phe Val Ala Trp Lys Glu Asn Pro Glu Asn Tyr Lys Gly Tyr Ile
    2255                2260                2265

Gln Glu Leu Arg Ala Gln Lys Val Ser Gln Ser Leu Ser Asp Leu
    2270                2275                2280

Ala Asp Ser Ser Ser Asp Leu Glu Ala Phe Ser Gln Gly Leu Ser
    2285                2290                2295

Thr Leu Leu Asp Lys Met Asp Pro Ser Gln Arg Ala Lys Phe Ile
    2300                2305                2310

Gln Glu Val Lys Lys Val Leu Gly
    2315                2320

<210> 19
<211> 6966
<212> DNA
<213> Setaria italica

<400> 19
atgtcgcaac ttggattagc tgcagctgcc tcaaaggcgc tgccactact tcctaatcgc      60

catagaactt cagctggaac tacattccca tcacctgtat catcgcggcc ctcaaaccga     120

aggaaaagcc gcactcgttc acttcgtgat ggaggagatg gggtatcaga tgccaaaaag     180

cacaaccagt ctgtccgtca aggtcttgct ggcatcatcg acctcccaaa tgaggcaaca     240

tcggaagtgg atatttctca tggatccgag gatcccaggg ggccaaccga ttcatatcaa     300

atgaatggga ttgtaaatga agcacataat ggcagacatg cctcagtgtc caaggttgtt     360

gaattttgtg cggcgctagg tggcaaaaca ccaattcaca gtatactagt ggccaacaat     420

ggaatggcag cagcaaagtt catgaggagt gtccggacat gggctaatga tacttttgga     480

tcggagaagg cgattcagct catagctatg gcaactccag aagacatgag gataaatgca     540

gaacacatta gaattgctga tcaatttgta gaggtgcctg gtggaacaaa caataacaac     600

tatgcaaatg ttcaactcat agtggaggta gcagaaagaa taggtgtttc tgctgtttgg     660

cctggttggg gtcatgcttc tgagaatcct gaacttccag atgcattgac cgcaaaagga     720

attgttttcc ttgggccacc tgcggcatca atgaatgcat tgggagataa ggtcggttca     780

gctctcattg ctcaagcagc tggggtcccg accctttcgt ggagtggatc acatgttgaa     840

gttccattag agtgctgctt agatgcgata cctgaggaaa tgtatagaaa agcttgtgtt     900

actaccacag aagaagctgt tgcgagttgt caggtggttg gttatcctgc catgattaag     960

gcatcctggg gaggtggtgg taaaggaata agaaaggttc ataatgacga tgaggttaga    1020

gcactgttta agcaagtaca aggtgaagtc cctggctccc caatatttat catgaggctt 1080

gcatcccaga gtcgtcatct tgaagttcag ttgctttgtg atcaatatgg caatgtggca 1140

gcacttcaca gtcgtgattg cagtgtgcaa cggcgacacc aaaagattat tgaggaaggc 1200

ccagttactg ttgctcctcg tgagacagtt aaagcgcttg agcaggcagc aaggaggctt 1260

gctaaggctg tgggttatgt tggtgctgct actgttgaat acctttacag catggagact 1320

ggggaatact atttctggga gcttaatccc agattacagg tcgagcatcc agtcactgag 1380

tggattgctg aagtaaatct tcctgcagct caagttgcag ttggaatggg catacctctt 1440

tggcagattc cagaaatcag acgtttctat ggaatggact atggaggagg atatgacatt 1500

tggaggaaaa cagcagctct tgccacacca tttaattttg atgaagtaga ttctcaatgg 1560

ccaaagggcc attgtgtagc agttagaatt actagcgagg atccagatga tggtttcaaa 1620

cctactggtg ggaaagtgaa ggagataagt tttaaaagca agcctaatgt ttgggcctac 1680

ttctcagtaa agtctggtgg aggcattcat gaatttgctg attctcagtt tgggcatgtt 1740

tttgcatatg ggctctctag atcagcagca ataacgaaca tggctcttgc attaaaagag 1800

attcaaattc gtggagaaat tcattcaaat gttgattaca cagttgatct cttaaatgct 1860

tcagacttca gagaaaataa gattcatact ggctggcttg ataccagaat agctatgcgt 1920

gttcaagctg agaggccccc atggtatatt tcagtggttg gaggagctct atataaaaca 1980

gtaactgcca atgcagccac tgtttctgat tatgtcagtt atctcaccaa gggccagatt 2040

ccaccaaagc atatatccct tgtcagttca acagttaatc tgaatatcga agggagcaaa 2100

tacacagttg aaactgtaag gactggacat ggtagctaca gattacgaat gaatgattca 2160

gcaattgaag cgaatgtaca atctttatgt gatggaggcc tcttaatgca gttggatgga 2220

aatagccatg taatttacgc ggaagaagaa gctggtggta cacgacttct gattgatgga 2280

aagacatgct tgttacagaa tgatcatgat ccatcaaagt tattagctga gacaccctgc 2340

aaacttcttc ggttcttggt tgctgatggt gctcatgttg atgctgatgt accatatgcg 2400

gaagttgagg ttatgaaaat gtgcatgcct ctcttgtcgc ctgcttctgg tgtcattcat 2460

gttatgatgt ctgagggcca ggcattgcag gctggtgatc ttatagcaag gctggatctt 2520

gatgaccctt ctgctgtgaa aagagctgaa ccatttcatg gaatatttcc acaaatggac 2580

cttcctgttg ctgcctctag ccaagtacac aaaagatatg ctgcaagttt gaatgctgct 2640

cgaatggtcc ttgcaggata cgagcataat atcaatgaag ttgtacaaga tttggtatgc 2700

tgcctggatg atcccgagct tcccttccta cagtgggatg aacttatgtc agttctagca 2760

actaggcttc caagaaatct taagagtgag ttagaggata aatacatgga atacaagttg 2820

aacttttacc atgggaaaaa caaggacttc ccgtccaagc tgctgagaga catcattgag 2880

gcaaatcttg catatggttc agagaaggaa aaagctacga atgagaggct tattgagcct 2940

cttatgagcc tacttaagtc atatgagggt gggagagaaa gccatgctca ttttgttgtc 3000

aagtcccttt tcaaggagta ccttgctgtg gaagaacttt tcagtgatgg gattcagtct 3060

gatgtgattg aaaccctgcg tcatcagcac agtaaagact tgcagaaggt tgtagacatt 3120

gtgttgtctc accagggtgt gaggaacaaa gctaagcttg taacagcact tatggaaaag 3180

ctggtttatc caaatcctgc tgcttacagg gatctgttgg ttcgcttttc ttcactcaat 3240

cataaaagat attataagtt ggcccttaaa gcaagcgaac ttcttgaaca aactaaacta 3300

agtgaactcc gtgcaagcat cgcaagaagc ctttctgatc tggggatgca taagggagaa 3360

atgactattg aagatagcat ggaagattta gtctctgccc cattacctgt cgaagatgca 3420

cttatttctt tgtttgatta cagtgatcca actgttcagc agaaagtgat cgagacatac 3480

atatctcgat tgtatcagcc tcttcttgtg aaagatagca tccaagtgaa atttaaggaa 3540

tctggtgcct ttgctttatg ggaattttct gaagggcatg ttgatactaa aaatggacaa 3600

gggaccgttc ttggtcgaac aagatggggt gccatggtag ctgtcaaatc agttgaatct 3660

gcacgaacag ccattgtagc tgcattaaag gattcggcac agcatgccag ctctgagggc 3720

aacatgatgc acattgcctt attgagtgct gaaaatgaaa ataatatcag tgatgatcaa 3780

gctcaacata ggatggaaaa acttaacaag atactcaagg atactagtgt cgcaaatgat 3840

cttcgagctg ctggtttgaa ggttataagt tgcattgttc aaagagatga agcacgcatg 3900

ccaatgcgcc acacattact ctggtcagat gaaaagagtt gttatgagga agagcagatt 3960

cttcggcatg tggagcctcc cctctccatg cttcttgaaa tggataagtt gaaagtgaaa 4020

ggatacaatg aaatgaagta tactccatca cgtgatcgtc aatggcatat ctacacacta 4080

agaaatactg aaaaccccaa aatgttgcat agggtatttt tccgaactat tgtcaggcaa 4140

cccaatgcag gcaacaagtt tatatcagcc caaattggcg acactgaagt aggaggtcct 4200

gaggaatctt tgtcatttac atctaatagc attttaagag ccttgatgac tgctattgaa 4260

gaattagagc ttcatgcaat taggactggt cattctcaca tgtatttgtg catattgaaa 4320

gaacaaaagc ttcttgatct cattccgttt tcagggagca caatcgtcga tgttggccaa 4380

gacgaagcta ctgcttgttc acttttaaaa tcaatggctt tgaagataca cgaacttgtt 4440

ggtgcacaga tgcatcatct ttctgtatgc cagtgggagg tgaaactcaa gttgtactgc 4500

gatgggcctg ccagtggcac ctggagagtt gtaactacaa atgttactag tcacacttgc 4560

accattgata tctaccggga agtggaagat actgaatcgc agaagttagt ataccattca 4620

gcttctccgt cagctagtcc tttgcatggt gtggccctgg ataatccgta tcaacctttg 4680

agtgtcattg atctaaaacg ctgctctgct aggaacaaca gaactacata ttgctatgat 4740

tttccactgg catttgaaac tgccctgcag aagtcatggc agtccaatgg ctccagtgtt 4800

tctgaaggca gtgaaaatag taggtcttat gtgaaagcaa cagagctggt gtttgctgaa 4860

aaacatgggt cctggggcac tcctataatt tccatggagc gtcccgctgg gctcaatgac 4920

attggcatgg tagcttggat cttagagatg tccactcctg aatttcccaa tggcaggcag 4980

attattgtca tagcaaatga tattactttc agagctggat catttggccc aagggaagat 5040

gcgttttttg aagctgtcac gaacctggcc tgcgagagga agcttcctct tatatacttg 5100

gcagcaaact ccggtgctag gattggcata gccgatgaag tgaaatcttg cttccgtgtt 5160

gggtggtccg atgaaggcag ccctgaacgg ggttttcagt acatttatct gactgacgaa 5220

gactatgccc gtattagctt gtctgttata gcacacaagc tgcagctgga taatggtgaa 5280

attaggtgga ttattgactc tgttgtgggc aaggaggatg ggcttggtgt tgagaatcta 5340

catggaagtg ctgctattgc cagtgcttat tctagggcat atgaggagac atttacactt 5400

acatttgtga ctgggcggac tgttggaata ggagcatatc tcgctcggct cggtatacgg 5460

tgcatacagc gtcttgacca gcctattatt ttaactgggt tttctgccct gaacaagctt 5520

cttgggcggg aagtgtacag ctcccacatg cagttgggtg gtcctaagat catggcgacc 5580

aatggtgttg tccacttgac tgtttcagat gaccttgaag gtgtttccaa tatattgagg 5640

tggctcagct atgttcctgc caacattggt ggacctcttc ctattacaaa acctttggac 5700

ccaccagaca gacctgttgc atacatccct gagaacacat gtgatccgcg cgcagccatt 5760

cgtggtgtag atgacagcca agggaaatgg ttgggtggta tgtttgacaa agacagcttt 5820

gtcgagacat ttgaaggatg ggcgaaaaca gtggttacgg gcagagcaaa gcttggagga 5880

attcctgttg gtgtcatagc tgtggagaca caaaccatga tgcagcttat ccctgctgat 5940

ccaggccagc ttgattccca tgagcgatct gttcctcggg ctggacaagt gtggttccca 6000

gattctgcaa ccaagacagc tcaggcattg ttggacttca accgtgaagg attgccgctg 6060

ttcatccttg ctaactggag aggattctct ggtggacaaa gagatctgtt tgaaggaatt 6120

cttcaggctg ggtcaacaat tgttgagaac cttaggacat acaatcagcc tgcttttgtc 6180

tacattccta tggctggaga gctgcgtgga ggagcttggg ttgtggttga tagcaaaata 6240

aatccagacc gaattgagtg ttatgctgag aggactgcta aaggcaatgt tcttgaacct 6300

caagggttaa ttgaaatcaa attcagatca gaggagctcc aagactgtat gggtaggctt 6360

gacccagagt tgataaatct gaaagcaaaa ctccaaggtg caaagcttgg aaatggaagc 6420

ctaacagatg tagaatccct tcagaagagt atagatgctc gtacgaaaca gttgttgcct 6480

ttatacaccc agattgcaat acggtttgct gaattgcatg atacttccct cagaatggca 6540

gctaaaggtg tgattaagaa agttgtagat tgggaagaat cacgttcttt cttctacaga 6600

aggctacgga ggaggatctc tgaagatgtt cttgcaaaag aaataagagg aatagctggt 6660

gaccacttca ctcaccaatc agcagttgag ctgatcaagg aatggtactt ggcttctcaa 6720

gccacaacag gaagcactga atgggatgat gatgatgctt ttgttgcctg gaaggagaat 6780

cctgaaaact ataagggata tatccaagag ttaagggctc aaaaggtgtc tcagtcgctc 6840

tccgatcttg cagactccag ttcagatcta gaagcattct cacagggtct ttccacatta 6900

ttagataaga tggatccctc tcagagagcc aagttcattc aggaagtcaa gaaggtcctg 6960

ggttga 6966

<210> 20
<211> 2321
<212> PRT
<213> Setaria italica

<400> 20
Met Ser Gln Leu Gly Leu Ala Ala Ala Ala Ser Lys Ala Leu Pro Leu
1               5                   10                  15

Leu Pro Asn Arg His Arg Thr Ser Ala Gly Thr Thr Phe Pro Ser Pro
            20                  25                  30

Val Ser Ser Arg Pro Ser Asn Arg Arg Lys Ser Arg Thr Arg Ser Leu
        35                  40                  45

Arg Asp Gly Gly Asp Gly Val Ser Asp Ala Lys Lys His Asn Gln Ser
    50                  55                  60

Val Arg Gln Gly Leu Ala Gly Ile Ile Asp Leu Pro Asn Glu Ala Thr
65                  70                  75                  80

Ser Glu Val Asp Ile Ser His Gly Ser Glu Asp Pro Arg Gly Pro Thr
                85                  90                  95

Asp Ser Tyr Gln Met Asn Gly Ile Val Asn Glu Ala His Asn Gly Arg
            100                 105                 110

His Ala Ser Val Ser Lys Val Val Glu Phe Cys Ala Ala Leu Gly Gly
        115                 120                 125

Lys Thr Pro Ile His Ser Ile Leu Val Ala Asn Asn Gly Met Ala Ala
    130                 135                 140

Ala Lys Phe Met Arg Ser Val Arg Thr Trp Ala Asn Asp Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Val Ala Glu Arg Ile Gly Val Ser Ala Val Trp Pro Gly Trp Gly
    210                 215                 220

His Ala Ser Glu Asn Pro Glu Leu Pro Asp Ala Leu Thr Ala Lys Gly
225                 230                 235                 240

Ile Val Phe Leu Gly Pro Pro Ala Ala Ser Met Asn Ala Leu Gly Asp
                245                 250                 255

Lys Val Gly Ser Ala Leu Ile Ala Gln Ala Ala Gly Val Pro Thr Leu
            260                 265                 270

Ser Trp Ser Gly Ser His Val Glu Val Pro Leu Glu Cys Cys Leu Asp
        275                 280                 285

Ala Ile Pro Glu Glu Met Tyr Arg Lys Ala Cys Val Thr Thr Thr Glu
    290                 295                 300

Glu Ala Val Ala Ser Cys Gln Val Val Gly Tyr Pro Ala Met Ile Lys
305                 310                 315                 320

Ala Ser Trp Gly Gly Gly Gly Lys Gly Ile Arg Lys Val His Asn Asp
                325                 330                 335

Asp Glu Val Arg Ala Leu Phe Lys Gln Val Gln Gly Glu Val Pro Gly
            340                 345                 350

Ser Pro Ile Phe Ile Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Ala Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Glu Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Ile Pro Glu Ile Arg Arg Phe Tyr Gly Met Asp Tyr Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Leu Ser Arg Ser Ala Ala Ile Thr
            580                 585                 590

Asn Met Ala Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Arg
    610                 615                 620

Glu Asn Lys Ile His Thr Gly Trp Leu Asp Thr Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Val Thr Ala Asn Ala Ala Thr Val Ser Asp Tyr Val
            660                 665                 670

Ser Tyr Leu Thr Lys Gly Gln Ile Pro Pro Lys His Ile Ser Leu Val
        675                 680                 685

Ser Ser Thr Val Asn Leu Asn Ile Glu Gly Ser Lys Tyr Thr Val Glu
    690                 695                 700

Thr Val Arg Thr Gly His Gly Ser Tyr Arg Leu Arg Met Asn Asp Ser
705                 710                 715                 720

Ala Ile Glu Ala Asn Val Gln Ser Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Leu Ile Asp Gly Lys Thr Cys Leu Leu Gln Asn Asp
        755                 760                 765

His Asp Pro Ser Lys Leu Leu Ala Glu Thr Pro Cys Lys Leu Leu Arg
    770                 775                 780

Phe Leu Val Ala Asp Gly Ala His Val Asp Ala Asp Val Pro Tyr Ala
785                 790                 795                 800

Glu Val Glu Val Met Lys Met Cys Met Pro Leu Leu Ser Pro Ala Ser
                805                 810                 815

Gly Val Ile His Val Met Met Ser Glu Gly Gln Ala Leu Gln Ala Gly
            820                 825                 830

Asp Leu Ile Ala Arg Leu Asp Leu Asp Asp Pro Ser Ala Val Lys Arg
        835                 840                 845

Ala Glu Pro Phe His Gly Ile Phe Pro Gln Met Asp Leu Pro Val Ala
    850                 855                 860

Ala Ser Ser Gln Val His Lys Arg Tyr Ala Ala Ser Leu Asn Ala Ala
865                 870                 875                 880

Arg Met Val Leu Ala Gly Tyr Glu His Asn Ile Asn Glu Val Val Gln
                885                 890                 895

Asp Leu Val Cys Cys Leu Asp Asp Pro Glu Leu Pro Phe Leu Gln Trp
            900                 905                 910

Asp Glu Leu Met Ser Val Leu Ala Thr Arg Leu Pro Arg Asn Leu Lys
        915                 920                 925

Ser Glu Leu Glu Asp Lys Tyr Met Glu Tyr Lys Leu Asn Phe Tyr His

930                 935                 940

Gly Lys Asn Lys Asp Phe Pro Ser Lys Leu Leu Arg Asp Ile Ile Glu
945                 950                 955                 960

Ala Asn Leu Ala Tyr Gly Ser Glu Lys Glu Lys Ala Thr Asn Glu Arg
                965                 970                 975

Leu Ile Glu Pro Leu Met Ser Leu Leu Lys Ser Tyr Glu Gly Gly Arg
            980                 985                 990

Glu Ser His Ala His Phe Val Val  Lys Ser Leu Phe Lys  Glu Tyr Leu
        995                 1000                 1005

Ala Val  Glu Glu Leu Phe Ser  Asp Gly Ile Gln Ser  Asp Val Ile
    1010                 1015                 1020

Glu Thr  Leu Arg His Gln His  Ser Lys Asp Leu Gln  Lys Val Val
    1025                 1030                 1035

Asp Ile  Val Leu Ser His Gln  Gly Val Arg Asn Lys  Ala Lys Leu
    1040                 1045                 1050

Val Thr  Ala Leu Met Glu Lys  Leu Val Tyr Pro Asn  Pro Ala Ala
    1055                 1060                 1065

Tyr Arg  Asp Leu Leu Val Arg  Phe Ser Ser Leu Asn  His Lys Arg
    1070                 1075                 1080

Tyr Tyr  Lys Leu Ala Leu Lys  Ala Ser Glu Leu Leu  Glu Gln Thr
    1085                 1090                 1095

Lys Leu  Ser Glu Leu Arg Ala  Ser Ile Ala Arg Ser  Leu Ser Asp
    1100                 1105                 1110

Leu Gly  Met His Lys Gly Glu  Met Thr Ile Glu Asp  Ser Met Glu
    1115                 1120                 1125

Asp Leu  Val Ser Ala Pro Leu  Pro Val Glu Asp Ala  Leu Ile Ser
    1130                 1135                 1140

Leu Phe  Asp Tyr Ser Asp Pro  Thr Val Gln Gln Lys  Val Ile Glu
    1145                 1150                 1155

Thr Tyr  Ile Ser Arg Leu Tyr  Gln Pro Leu Leu Val  Lys Asp Ser
    1160                 1165                 1170

Ile Gln Val Lys Phe Lys Glu Ser Gly Ala Phe Ala Leu Trp Glu
    1175                1180                1185

Phe Ser Glu Gly His Val Asp Thr Lys Asn Gly Gln Gly Thr Val
    1190                1195                1200

Leu Gly Arg Thr Arg Trp Gly Ala Met Val Ala Val Lys Ser Val
    1205                1210                1215

Glu Ser Ala Arg Thr Ala Ile Val Ala Ala Leu Lys Asp Ser Ala
    1220                1225                1230

Gln His Ala Ser Ser Glu Gly Asn Met Met His Ile Ala Leu Leu
    1235                1240                1245

Ser Ala Glu Asn Glu Asn Asn Ile Ser Asp Asp Gln Ala Gln His
    1250                1255                1260

Arg Met Glu Lys Leu Asn Lys Ile Leu Lys Asp Thr Ser Val Ala
    1265                1270                1275

Asn Asp Leu Arg Ala Ala Gly Leu Lys Val Ile Ser Cys Ile Val
    1280                1285                1290

Gln Arg Asp Glu Ala Arg Met Pro Met Arg His Thr Leu Leu Trp
    1295                1300                1305

Ser Asp Glu Lys Ser Cys Tyr Glu Glu Glu Gln Ile Leu Arg His
    1310                1315                1320

Val Glu Pro Pro Leu Ser Met Leu Leu Glu Met Asp Lys Leu Lys
    1325                1330                1335

Val Lys Gly Tyr Asn Glu Met Lys Tyr Thr Pro Ser Arg Asp Arg
    1340                1345                1350

Gln Trp His Ile Tyr Thr Leu Arg Asn Thr Glu Asn Pro Lys Met
    1355                1360                1365

Leu His Arg Val Phe Phe Arg Thr Ile Val Arg Gln Pro Asn Ala
    1370                1375                1380

Gly Asn Lys Phe Ile Ser Ala Gln Ile Gly Asp Thr Glu Val Gly
    1385                1390                1395

Gly Pro Glu Glu Ser Leu Ser Phe Thr Ser Asn Ser Ile Leu Arg
    1400                1405                1410

Ala Leu Met Thr Ala Ile Glu Glu Leu Glu Leu His Ala Ile Arg
    1415                1420                1425

Thr Gly His Ser His Met Tyr Leu Cys Ile Leu Lys Glu Gln Lys
    1430                1435                1440

Leu Leu Asp Leu Ile Pro Phe Ser Gly Ser Thr Ile Val Asp Val
    1445                1450                1455

Gly Gln Asp Glu Ala Thr Ala Cys Ser Leu Leu Lys Ser Met Ala
    1460                1465                1470

Leu Lys Ile His Glu Leu Val Gly Ala Gln Met His His Leu Ser
    1475                1480                1485

Val Cys Gln Trp Glu Val Lys Leu Lys Leu Tyr Cys Asp Gly Pro
    1490                1495                1500

Ala Ser Gly Thr Trp Arg Val Val Thr Thr Asn Val Thr Ser His
    1505                1510                1515

Thr Cys Thr Ile Asp Ile Tyr Arg Glu Val Glu Asp Thr Glu Ser
    1520                1525                1530

Gln Lys Leu Val Tyr His Ser Ala Ser Pro Ser Ala Ser Pro Leu
    1535                1540                1545

His Gly Val Ala Leu Asp Asn Pro Tyr Gln Pro Leu Ser Val Ile
    1550                1555                1560

Asp Leu Lys Arg Cys Ser Ala Arg Asn Asn Arg Thr Thr Tyr Cys
    1565                1570                1575

Tyr Asp Phe Pro Leu Ala Phe Glu Thr Ala Leu Gln Lys Ser Trp
    1580                1585                1590

Gln Ser Asn Gly Ser Ser Val Ser Glu Gly Ser Glu Asn Ser Arg
    1595                1600                1605

Ser Tyr Val Lys Ala Thr Glu Leu Val Phe Ala Glu Lys His Gly
    1610                1615                1620

Ser Trp Gly Thr Pro Ile Ile Ser Met Glu Arg Pro Ala Gly Leu
    1625                1630                1635

Asn Asp Ile Gly Met Val Ala Trp Ile Leu Glu Met Ser Thr Pro
    1640                1645                1650

Glu Phe Pro Asn Gly Arg Gln Ile Ile Val Ile Ala Asn Asp Ile
    1655                1660                1665

Thr Phe Arg Ala Gly Ser Phe Gly Pro Arg Glu Asp Ala Phe Phe
    1670                1675                1680

Glu Ala Val Thr Asn Leu Ala Cys Glu Arg Lys Leu Pro Leu Ile
    1685                1690                1695

Tyr Leu Ala Ala Asn Ser Gly Ala Arg Ile Gly Ile Ala Asp Glu
    1700                1705                1710

Val Lys Ser Cys Phe Arg Val Gly Trp Ser Asp Glu Gly Ser Pro
    1715                1720                1725

Glu Arg Gly Phe Gln Tyr Ile Tyr Leu Thr Asp Glu Asp Tyr Ala
    1730                1735                1740

Arg Ile Ser Leu Ser Val Ile Ala His Lys Leu Gln Leu Asp Asn
    1745                1750                1755

Gly Glu Ile Arg Trp Ile Ile Asp Ser Val Val Gly Lys Glu Asp
    1760                1765                1770

Gly Leu Gly Val Glu Asn Leu His Gly Ser Ala Ala Ile Ala Ser
    1775                1780                1785

Ala Tyr Ser Arg Ala Tyr Glu Glu Thr Phe Thr Leu Thr Phe Val
    1790                1795                1800

Thr Gly Arg Thr Val Gly Ile Gly Ala Tyr Leu Ala Arg Leu Gly
    1805                1810                1815

Ile Arg Cys Ile Gln Arg Leu Asp Gln Pro Ile Ile Leu Thr Gly
    1820                1825                1830

Phe Ser Ala Leu Asn Lys Leu Leu Gly Arg Glu Val Tyr Ser Ser
    1835                1840                1845

His Met Gln Leu Gly Gly Pro Lys Ile Met Ala Thr Asn Gly Val
    1850                1855                1860

Val His Leu Thr Val Ser Asp Asp Leu Glu Gly Val Ser Asn Ile
    1865                1870                1875

Leu Arg Trp Leu Ser Tyr Val Pro Ala Asn Ile Gly Gly Pro Leu

1880                1885                1890

Pro Ile Thr Lys Pro Leu Asp Pro Pro Asp Arg Pro Val Ala Tyr
    1895                1900                1905

Ile Pro Glu Asn Thr Cys Asp Pro Arg Ala Ala Ile Arg Gly Val
    1910                1915                1920

Asp Asp Ser Gln Gly Lys Trp Leu Gly Gly Met Phe Asp Lys Asp
    1925                1930                1935

Ser Phe Val Glu Thr Phe Glu Gly Trp Ala Lys Thr Val Val Thr
    1940                1945                1950

Gly Arg Ala Lys Leu Gly Gly Ile Pro Val Gly Val Ile Ala Val
    1955                1960                1965

Glu Thr Gln Thr Met Met Gln Leu Ile Pro Ala Asp Pro Gly Gln
    1970                1975                1980

Leu Asp Ser His Glu Arg Ser Val Pro Arg Ala Gly Gln Val Trp
    1985                1990                1995

Phe Pro Asp Ser Ala Thr Lys Thr Ala Gln Ala Leu Leu Asp Phe
    2000                2005                2010

Asn Arg Glu Gly Leu Pro Leu Phe Ile Leu Ala Asn Trp Arg Gly
    2015                2020                2025

Phe Ser Gly Gly Gln Arg Asp Leu Phe Glu Gly Ile Leu Gln Ala
    2030                2035                2040

Gly Ser Thr Ile Val Glu Asn Leu Arg Thr Tyr Asn Gln Pro Ala
    2045                2050                2055

Phe Val Tyr Ile Pro Met Ala Gly Glu Leu Arg Gly Gly Ala Trp
    2060                2065                2070

Val Val Val Asp Ser Lys Ile Asn Pro Asp Arg Ile Glu Cys Tyr
    2075                2080                2085

Ala Glu Arg Thr Ala Lys Gly Asn Val Leu Glu Pro Gln Gly Leu
    2090                2095                2100

Ile Glu Ile Lys Phe Arg Ser Glu Glu Leu Gln Asp Cys Met Gly
    2105                2110                2115

Arg Leu Asp Pro Glu Leu Ile Asn Leu Lys Ala Lys Leu Gln Gly
    2120                2125                2130

Ala Lys Leu Gly Asn Gly Ser Leu Thr Asp Val Glu Ser Leu Gln
    2135                2140                2145

Lys Ser Ile Asp Ala Arg Thr Lys Gln Leu Leu Pro Leu Tyr Thr
    2150                2155                2160

Gln Ile Ala Ile Arg Phe Ala Glu Leu His Asp Thr Ser Leu Arg
    2165                2170                2175

Met Ala Ala Lys Gly Val Ile Lys Lys Val Val Asp Trp Glu Glu
    2180                2185                2190

Ser Arg Ser Phe Phe Tyr Arg Arg Leu Arg Arg Arg Ile Ser Glu
    2195                2200                2205

Asp Val Leu Ala Lys Glu Ile Arg Gly Ile Ala Gly Asp His Phe
    2210                2215                2220

Thr His Gln Ser Ala Val Glu Leu Ile Lys Glu Trp Tyr Leu Ala
    2225                2230                2235

Ser Gln Ala Thr Thr Gly Ser Thr Glu Trp Asp Asp Asp Asp Ala
    2240                2245                2250

Phe Val Ala Trp Lys Glu Asn Pro Glu Asn Tyr Lys Gly Tyr Ile
    2255                2260                2265

Gln Glu Leu Arg Ala Gln Lys Val Ser Gln Ser Leu Ser Asp Leu
    2270                2275                2280

Ala Asp Ser Ser Ser Asp Leu Glu Ala Phe Ser Gln Gly Leu Ser
    2285                2290                2295

Thr Leu Leu Asp Lys Met Asp Pro Ser Gln Arg Ala Lys Phe Ile
    2300                2305                2310

Gln Glu Val Lys Lys Val Leu Gly
    2315                2320

<210> 21
<211> 6966
<212> DNA
<213> Setaria italica

<400> 21
atgtcgcaac ttggattagc tgcagctgcc tcaaaggcgc tgccactact tcctaatcgc 60

catagaactt cagctggaac tacattccca tcacctgtat catcgcggcc ctcaaaccga 120

aggaaaagcc gcactcgttc acttcgtgat ggaggagatg gggtatcaga tgccaaaaag 180

cacaaccagt ctgtccgtca aggtcttgct ggcatcatcg acctcccaaa tgaggcaaca 240

tcggaagtgg atatttctca tggatccgag gatcccaggg ggccaaccga ttcatatcaa 300

atgaatggga ttgtaaatga agcacataat ggcagacatg cctcagtgtc caaggttgtt 360

gaattttgtg cggcgctagg tggcaaaaca ccaattcaca gtatactagt ggccaacaat 420

ggaatggcag cagcaaagtt catgaggagt gtccggacat gggctaatga tacttttgga 480

tcggagaagg cgattcagct catagctatg gcaactccag aagacatgag gataaatgca 540

gaacacatta gaattgctga tcaatttgta gaggtgcctg gtggaacaaa caataacaac 600

tatgcaaatg ttcaactcat agtggaggta gcagaaagaa taggtgtttc tgctgtttgg 660

cctggttggg gtcatgcttc tgagaatcct gaacttccag atgcattgac cgcaaaagga 720

attgttttcc ttgggccacc tgcggcatca atgaatgcat tgggagataa ggtcggttca 780

gctctcattg ctcaagcagc tggggtcccg accctttcgt ggagtggatc acatgttgaa 840

gttccattag agtgctgctt agatgcgata cctgaggaaa tgtatagaaa agcttgtgtt 900

actaccacag aagaagctgt tgcgagttgt caggtggttg gttatcctgc catgattaag 960

gcatcctggg gaggtggtgg taaaggaata agaaaggttc ataatgacga tgaggttaga 1020

gcactgttta agcaagtaca aggtgaagtc cctggctccc caatatttat catgaggctt 1080

gcatcccaga gtcgtcatct tgaagttcag ttgctttgtg atcaatatgg caatgtggca 1140

gcacttcaca gtcgtgattg cagtgtgcaa cggcgacacc aaaagattat tgaggaaggc 1200

ccagttactg ttgctcctcg tgagacagtt aaagcgcttg agcaggcagc aaggaggctt 1260

gctaaggctg tgggttatgt tggtgctgct actgttgaat acctttacag catggagact 1320

ggggaatact attttctgga gcttaatccc agattacagg tcgagcatcc agtcactgag 1380

tggattgctg aagtaaatct tcctgcagct caagttgcag ttggaatggg catacctctt 1440

tggcagattc cagaaatcag acgtttctat ggaatggact atggaggagg atatgacatt 1500

tggaggaaaa cagcagctct tgccacacca tttaattttg atgaagtaga ttctcaatgg 1560

ccaaagggcc attgtgtagc agttagaatt actagcgagg atccagatga tggtttcaaa 1620

cctactggtg ggaaagtgaa ggagataagt tttaaaagca agcctaatgt ttgggcctac 1680

ttctcagtaa agtctggtgg aggcattcat gaatttgctg attctcagtt tgggcatgtt 1740

tttgcatatg ggctctctag atcagcagca ataacgaaca tggctcttgc attaaaagag 1800

attcaaattc gtggagaaat tcattcaaat gttgattaca cagttgatct cttaaatgct 1860

tcagacttca gagaaaataa gattcatact ggctggcttg ataccagaat agctatgcgt 1920

gttcaagctg agaggccccc atggtatatt tcagtggttg gaggagctct atataaaaca 1980
gtaactgcca atgcagccac tgtttctgat tatgtcagtt atctcaccaa gggccagatt 2040
ccaccaaagc atatatccct tgtcagttca acagttaatc tgaatatcga agggagcaaa 2100
tacacagttg aaactgtaag gactggacat ggtagctaca gattacgaat gaatgattca 2160
gcaattgaag cgaatgtaca atctttatgt gatggaggcc tcttaatgca gttggatgga 2220
aatagccatg taatttacgc ggaagaagaa gctggtggta cacgacttct gattgatgga 2280
aagacatgct tgttacagaa tgatcatgat ccatcaaagt tattagctga gacaccctgc 2340
aaacttcttc ggttcttggt tgctgatggt gctcatgttg atgctgatgt accatatgcg 2400
gaagttgagg ttatgaaaat gtgcatgcct ctcttgtcgc ctgcttctgg tgtcattcat 2460
gttatgatgt ctgagggcca ggcattgcag gctggtgatc ttatagcaag gctggatctt 2520
gatgaccctt ctgctgtgaa aagagctgaa ccatttcatg gaatatttcc acaaatggac 2580
cttcctgttg ctgcctctag ccaagtacac aaaagatatg ctgcaagttt gaatgctgct 2640
cgaatggtcc ttgcaggata cgagcataat atcaatgaag ttgtacaaga tttggtatgc 2700
tgcctggatg atcccgagct tcccttccta cagtgggatg aacttatgtc agttctagca 2760
actaggcttc caagaaatct taagagtgag ttagaggata aatacatgga atacaagttg 2820
aacttttacc atgggaaaaa caaggacttc ccgtccaagc tgctgagaga catcattgag 2880
gcaaatcttg catatggttc agagaaggaa aaagctacga atgagaggct tattgagcct 2940
cttatgagcc tacttaagtc atatgagggt gggagagaaa gccatgctca ttttgttgtc 3000
aagtcccttt tcaaggagta ccttgctgtg gaagaacttt tcagtgatgg gattcagtct 3060
gatgtgattg aaaccctgcg tcatcagcac agtaaagact tgcagaaggt tgtagacatt 3120
gtgttgtctc accagggtgt gaggaacaaa gctaagcttg taacagcact tatggaaaag 3180
ctggtttatc caaatcctgc tgcttacagg gatctgttgg ttcgcttttc ttcactcaat 3240
cataaaagat attataagtt ggcccttaaa gcaagcgaac ttcttgaaca aactaaacta 3300
agtgaactcc gtgcaagcat cgcaagaagc ctttctgatc tggggatgca taagggagaa 3360
atgactattg aagatagcat ggaagattta gtctctgccc cattacctgt cgaagatgca 3420
cttatttctt tgtttgatta cagtgatcca actgttcagc agaaagtgat cgagacatac 3480
atatctcgat tgtatcagcc tcttcttgtg aaagatagca tccaagtgaa atttaaggaa 3540
tctggtgcct ttgctttatg ggaattttct gaagggcatg ttgatactaa aaatggacaa 3600
gggaccgttc ttggtcgaac aagatggggt gccatggtag ctgtcaaatc agttgaatct 3660
gcacgaacag ccattgtagc tgcattaaag gattcggcac agcatgccag ctctgagggc 3720
aacatgatgc acattgcctt attgagtgct gaaaatgaaa ataatatcag tgatgatcaa 3780
gctcaacata ggatggaaaa acttaacaag atactcaagg atactagtgt cgcaaatgat 3840

cttcgagctg ctggtttgaa ggttataagt tgcattgttc aaagagatga agcacgcatg 3900

ccaatgcgcc acacattact ctggtcagat gaaaagagtt gttatgagga agagcagatt 3960

cttcggcatg tggagcctcc cctctccatg cttcttgaaa tggataagtt gaaagtgaaa 4020

ggatacaatg aaatgaagta tactccatca cgtgatcgtc aatggcatat ctacacacta 4080

agaaatactg aaaaccccaa aatgttgcat agggtatttt tccgaactat tgtcaggcaa 4140

cccaatgcag gcaacaagtt tatatcagcc caaattggcg acactgaagt aggaggtcct 4200

gaggaatctt tgtcatttac atctaatagc attttaagag ccttgatgac tgctattgaa 4260

gaattagagc ttcatgcaat taggactggt cattctcaca tgtatttgtg catattgaaa 4320

gaacaaaagc ttcttgatct cattccgttt tcagggagca caatcgtcga tgttggccaa 4380

gacgaagcta ctgcttgttc acttttaaaa tcaatggctt tgaagataca cgaacttgtt 4440

ggtgcacaga tgcatcatct ttctgtatgc cagtgggagg tgaaactcaa gttgtactgc 4500

gatgggcctg ccagtggcac ctggagagtt gtaactacaa atgttactag tcacacttgc 4560

accgttgata tctaccggga agtggaagat actgaatcgc agaagttagt ataccattca 4620

gcttctccgt cagctagtcc tttgcatggt gtggccctgg ataatccgta tcaacctttg 4680

agtgtcattg atctaaaacg ctgctctgct aggaacaaca gaactacata ttgctatgat 4740

tttccactgg catttgaaac tgccctgcag aagtcatggc agtccaatgg ctccagtgtt 4800

tctgaaggca gtgaaaatag taggtcttat gtgaaagcaa cagagctggt gtttgctgaa 4860

aaacatgggt cctggggcac tcctataatt tccatggagc gtcccgctgg gctcaatgac 4920

attggcatgg tagcttggat cttagagatg tccactcctg aatttcccaa tggcaggcag 4980

attattgtca tagcaaatga tattactttc agagctggat catttggccc aagggaagat 5040

gcgttttttg aagctgtcac gaacctggcc tgcgagagga agcttcctct tatatacttg 5100

gcagcaaact ccggtgctag gattggcata gccgatgaag tgaaatcttg cttccgtgtt 5160

gggtggtccg atgaaggcag ccctgaacgg ggttttcagt acatttatct gactgacgaa 5220

gactatgccc gtattagctt gtctgttata gcacacaagc tgcagctgga taatggtgaa 5280

attaggtgga ttattgactc tgttgtgggc aaggaggatg ggcttggtgt tgagaatata 5340

catggaagtg ctgctattgc cagtgcttat tctagggcat atgaggagac atttacactt 5400

acatttgtga ctgggcggac tgttggaata ggagcatatc ttgctcggct cggtatacgg 5460

tgcatacagc gtcttgacca gcctattatt ttaactgggt tttctgccct gaacaagctt 5520

cttgggcggg aagtgtacag ctcccacatg cagttgggtg gtcctaagat catggcgacc 5580

aatggtgttg tccacttgac tgtttcagat gaccttgaag gtgtttccaa tatattgagg 5640

tggctcagct atgttcctgc caacattggt ggacctcttc ctattacaaa acctttggac 5700

ccaccagaca gacctgttgc atacatccct gagaacacat gtgatccgcg cgcagccatt 5760

cgtggtgtag atgacagcca agggaaatgg ttgggtggta tgtttgacaa agacagcttt 5820

gtcgagacat ttgaaggatg ggcgaaaaca gtggttacgg gcagagcaaa gcttggagga 5880

attcctgttg gtgtcatagc tgtggagaca caaaccatga tgcagcttat ccctgctgat 5940

ccaggccagc ttgattccca tgagcgatct gttcctcggg ctggacaagt gtggttccca 6000

gattctgcaa ccaagacagc tcaggcattg ttggacttca accgtgaagg attgccgctg 6060

ttcatccttg ctaactggag aggattctct ggtggacaaa gagatctgtt tgaaggaatt 6120

cttcaggctg ggtcaacaat tgttgagaac cttaggacat acaatcagcc tgcttttgtc 6180

tacattccta tggctggaga gctgcgtgga ggagcttggg ttgtggttga tagcaaaata 6240

aatccagacc gaattgagtg ttatgctgag aggactgcta aaggcaatgt tctggaacct 6300

caagggttaa ttgaaatcaa attcagatca gaggagctcc aagactgtat gggtaggctt 6360

gacccagagt tgataaatct gaaagcaaaa ctccaaggtg caaagcttgg aaatggaagc 6420

ctaacagatg tagaatccct tcagaagagt atagatgctc gtacgaaaca gttgttgcct 6480

ttatacaccc agattgcaat acggtttgct gaattgcatg atacttccct cagaatggca 6540

gctaaaggtg tgattaagaa agttgtagat tgggaagaat tacgttcttt cttctacaga 6600

aggctacgga ggaggatctc tgaagatgtt cttgcaaaag aaataagagg aatagctggt 6660

gaccacttca ctcaccaatc agcagttgag ctgatcaagg aatggtactt ggcttctcaa 6720

gccacaacag gaagcactga atgggatgat gatgatgctt ttgttgcctg gaaggagaat 6780

cctgaaaact ataagggata tatccaagag ttaagggctc aaaaggtgtc tcagtcgctc 6840

tccgatcttg cagactccag ttcagatcta gaagcattct cacagggtct ttccacatta 6900

ttagataaga tggatccctc tcagagagcc aagttcattc aggaagtcaa gaaggtcctg 6960

ggttga 6966

<210> 22
<211> 2321
<212> PRT
<213> Setaria italica

<400> 22
Met Ser Gln Leu Gly Leu Ala Ala Ala Ala Ser Lys Ala Leu Pro Leu
1               5                   10                  15

Leu Pro Asn Arg His Arg Thr Ser Ala Gly Thr Thr Phe Pro Ser Pro
            20                  25                  30

Val Ser Ser Arg Pro Ser Asn Arg Arg Lys Ser Arg Thr Arg Ser Leu
        35                  40                  45

Arg Asp Gly Gly Asp Gly Val Ser Asp Ala Lys Lys His Asn Gln Ser
    50                  55                  60

Val Arg Gln Gly Leu Ala Gly Ile Ile Asp Leu Pro Asn Glu Ala Thr
65                  70                  75                  80

Ser Glu Val Asp Ile Ser His Gly Ser Glu Asp Pro Arg Gly Pro Thr
                85                  90                  95

Asp Ser Tyr Gln Met Asn Gly Ile Val Asn Glu Ala His Asn Gly Arg
            100                 105                 110

His Ala Ser Val Ser Lys Val Val Glu Phe Cys Ala Ala Leu Gly Gly
        115                 120                 125

Lys Thr Pro Ile His Ser Ile Leu Val Ala Asn Asn Gly Met Ala Ala
    130                 135                 140

Ala Lys Phe Met Arg Ser Val Arg Thr Trp Ala Asn Asp Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Val Ala Glu Arg Ile Gly Val Ser Ala Val Trp Pro Gly Trp Gly
    210                 215                 220

His Ala Ser Glu Asn Pro Glu Leu Pro Asp Ala Leu Thr Ala Lys Gly
225                 230                 235                 240

Ile Val Phe Leu Gly Pro Pro Ala Ala Ser Met Asn Ala Leu Gly Asp
                245                 250                 255

Lys Val Gly Ser Ala Leu Ile Ala Gln Ala Ala Gly Val Pro Thr Leu
            260                 265                 270

Ser Trp Ser Gly Ser His Val Glu Val Pro Leu Glu Cys Cys Leu Asp
        275                 280                 285

Ala Ile Pro Glu Glu Met Tyr Arg Lys Ala Cys Val Thr Thr Thr Glu
    290                 295                 300

Glu Ala Val Ala Ser Cys Gln Val Val Gly Tyr Pro Ala Met Ile Lys
305                 310                 315                 320

Ala Ser Trp Gly Gly Gly Gly Lys Gly Ile Arg Lys Val His Asn Asp
                325                 330                 335

Asp Glu Val Arg Ala Leu Phe Lys Gln Val Gln Gly Glu Val Pro Gly
            340                 345                 350

Ser Pro Ile Phe Ile Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Ala Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Glu Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Ile Pro Glu Ile Arg Arg Phe Tyr Gly Met Asp Tyr Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Leu Ser Arg Ser Ala Ala Ile Thr
            580                 585                 590

Asn Met Ala Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Arg
    610                 615                 620

Glu Asn Lys Ile His Thr Gly Trp Leu Asp Thr Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Val Thr Ala Asn Ala Ala Thr Val Ser Asp Tyr Val
            660                 665                 670

Ser Tyr Leu Thr Lys Gly Gln Ile Pro Pro Lys His Ile Ser Leu Val
        675                 680                 685

Ser Ser Thr Val Asn Leu Asn Ile Glu Gly Ser Lys Tyr Thr Val Glu
    690                 695                 700

Thr Val Arg Thr Gly His Gly Ser Tyr Arg Leu Arg Met Asn Asp Ser
705                 710                 715                 720

Ala Ile Glu Ala Asn Val Gln Ser Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Leu Ile Asp Gly Lys Thr Cys Leu Leu Gln Asn Asp
        755                 760                 765

His Asp Pro Ser Lys Leu Leu Ala Glu Thr Pro Cys Lys Leu Leu Arg
    770                 775                 780

Phe Leu Val Ala Asp Gly Ala His Val Asp Ala Asp Val Pro Tyr Ala
785                 790                 795                 800

Glu Val Glu Val Met Lys Met Cys Met Pro Leu Leu Ser Pro Ala Ser

                805                 810                 815

Gly Val Ile His Val Met Met Ser Glu Gly Gln Ala Leu Gln Ala Gly
            820                 825                 830

Asp Leu Ile Ala Arg Leu Asp Leu Asp Asp Pro Ser Ala Val Lys Arg
        835                 840                 845

Ala Glu Pro Phe His Gly Ile Phe Pro Gln Met Asp Leu Pro Val Ala
    850                 855                 860

Ala Ser Ser Gln Val His Lys Arg Tyr Ala Ala Ser Leu Asn Ala Ala
865                 870                 875                 880

Arg Met Val Leu Ala Gly Tyr Glu His Asn Ile Asn Glu Val Val Gln
                885                 890                 895

Asp Leu Val Cys Cys Leu Asp Asp Pro Glu Leu Pro Phe Leu Gln Trp
            900                 905                 910

Asp Glu Leu Met Ser Val Leu Ala Thr Arg Leu Pro Arg Asn Leu Lys
        915                 920                 925

Ser Glu Leu Glu Asp Lys Tyr Met Glu Tyr Lys Leu Asn Phe Tyr His
    930                 935                 940

Gly Lys Asn Lys Asp Phe Pro Ser Lys Leu Leu Arg Asp Ile Ile Glu
945                 950                 955                 960

Ala Asn Leu Ala Tyr Gly Ser Glu Lys Glu Lys Ala Thr Asn Glu Arg
                965                 970                 975

Leu Ile Glu Pro Leu Met Ser Leu Leu Lys Ser Tyr Glu Gly Gly Arg
            980                 985                 990

Glu Ser His Ala His Phe Val Val Lys Ser Leu Phe Lys Glu Tyr Leu
        995                 1000                1005

Ala Val Glu Glu Leu Phe Ser Asp Gly Ile Gln Ser Asp Val Ile
    1010                1015                1020

Glu Thr Leu Arg His Gln His Ser Lys Asp Leu Gln Lys Val Val
    1025                1030                1035

Asp Ile Val Leu Ser His Gln Gly Val Arg Asn Lys Ala Lys Leu
    1040                1045                1050

Val Thr Ala Leu Met Glu Lys Leu Val Tyr Pro Asn Pro Ala Ala
    1055                1060                1065

Tyr Arg Asp Leu Leu Val Arg Phe Ser Ser Leu Asn His Lys Arg
    1070                1075                1080

Tyr Tyr Lys Leu Ala Leu Lys Ala Ser Glu Leu Leu Glu Gln Thr
    1085                1090                1095

Lys Leu Ser Glu Leu Arg Ala Ser Ile Ala Arg Ser Leu Ser Asp
    1100                1105                1110

Leu Gly Met His Lys Gly Glu Met Thr Ile Glu Asp Ser Met Glu
    1115                1120                1125

Asp Leu Val Ser Ala Pro Leu Pro Val Glu Asp Ala Leu Ile Ser
    1130                1135                1140

Leu Phe Asp Tyr Ser Asp Pro Thr Val Gln Gln Lys Val Ile Glu
    1145                1150                1155

Thr Tyr Ile Ser Arg Leu Tyr Gln Pro Leu Leu Val Lys Asp Ser
    1160                1165                1170

Ile Gln Val Lys Phe Lys Glu Ser Gly Ala Phe Ala Leu Trp Glu
    1175                1180                1185

Phe Ser Glu Gly His Val Asp Thr Lys Asn Gly Gln Gly Thr Val
    1190                1195                1200

Leu Gly Arg Thr Arg Trp Gly Ala Met Val Ala Val Lys Ser Val
    1205                1210                1215

Glu Ser Ala Arg Thr Ala Ile Val Ala Ala Leu Lys Asp Ser Ala
    1220                1225                1230

Gln His Ala Ser Ser Glu Gly Asn Met Met His Ile Ala Leu Leu
    1235                1240                1245

Ser Ala Glu Asn Glu Asn Asn Ile Ser Asp Asp Gln Ala Gln His
    1250                1255                1260

Arg Met Glu Lys Leu Asn Lys Ile Leu Lys Asp Thr Ser Val Ala
    1265                1270                1275

Asn Asp Leu Arg Ala Ala Gly Leu Lys Val Ile Ser Cys Ile Val
    1280                1285                1290

Gln Arg Asp Glu Ala Arg Met Pro Met Arg His Thr Leu Leu Trp
    1295                1300                1305

Ser Asp Glu Lys Ser Cys Tyr Glu Glu Glu Gln Ile Leu Arg His
    1310                1315                1320

Val Glu Pro Pro Leu Ser Met Leu Leu Glu Met Asp Lys Leu Lys
    1325                1330                1335

Val Lys Gly Tyr Asn Glu Met Lys Tyr Thr Pro Ser Arg Asp Arg
    1340                1345                1350

Gln Trp His Ile Tyr Thr Leu Arg Asn Thr Glu Asn Pro Lys Met
    1355                1360                1365

Leu His Arg Val Phe Phe Arg Thr Ile Val Arg Gln Pro Asn Ala
    1370                1375                1380

Gly Asn Lys Phe Ile Ser Ala Gln Ile Gly Asp Thr Glu Val Gly
    1385                1390                1395

Gly Pro Glu Glu Ser Leu Ser Phe Thr Ser Asn Ser Ile Leu Arg
    1400                1405                1410

Ala Leu Met Thr Ala Ile Glu Glu Leu Glu Leu His Ala Ile Arg
    1415                1420                1425

Thr Gly His Ser His Met Tyr Leu Cys Ile Leu Lys Glu Gln Lys
    1430                1435                1440

Leu Leu Asp Leu Ile Pro Phe Ser Gly Ser Thr Ile Val Asp Val
    1445                1450                1455

Gly Gln Asp Glu Ala Thr Ala Cys Ser Leu Leu Lys Ser Met Ala
    1460                1465                1470

Leu Lys Ile His Glu Leu Val Gly Ala Gln Met His His Leu Ser
    1475                1480                1485

Val Cys Gln Trp Glu Val Lys Leu Lys Leu Tyr Cys Asp Gly Pro
    1490                1495                1500

Ala Ser Gly Thr Trp Arg Val Val Thr Thr Asn Val Thr Ser His
    1505                1510                1515

Thr Cys Thr Val Asp Ile Tyr Arg Glu Val Glu Asp Thr Glu Ser
    1520                1525                1530

Gln Lys Leu Val Tyr His Ser Ala Ser Pro Ser Ala Ser Pro Leu
    1535                1540                1545

His Gly Val Ala Leu Asp Asn Pro Tyr Gln Pro Leu Ser Val Ile
    1550                1555                1560

Asp Leu Lys Arg Cys Ser Ala Arg Asn Asn Arg Thr Thr Tyr Cys
    1565                1570                1575

Tyr Asp Phe Pro Leu Ala Phe Glu Thr Ala Leu Gln Lys Ser Trp
    1580                1585                1590

Gln Ser Asn Gly Ser Ser Val Ser Glu Gly Ser Glu Asn Ser Arg
    1595                1600                1605

Ser Tyr Val Lys Ala Thr Glu Leu Val Phe Ala Glu Lys His Gly
    1610                1615                1620

Ser Trp Gly Thr Pro Ile Ile Ser Met Glu Arg Pro Ala Gly Leu
    1625                1630                1635

Asn Asp Ile Gly Met Val Ala Trp Ile Leu Glu Met Ser Thr Pro
    1640                1645                1650

Glu Phe Pro Asn Gly Arg Gln Ile Ile Val Ile Ala Asn Asp Ile
    1655                1660                1665

Thr Phe Arg Ala Gly Ser Phe Gly Pro Arg Glu Asp Ala Phe Phe
    1670                1675                1680

Glu Ala Val Thr Asn Leu Ala Cys Glu Arg Lys Leu Pro Leu Ile
    1685                1690                1695

Tyr Leu Ala Ala Asn Ser Gly Ala Arg Ile Gly Ile Ala Asp Glu
    1700                1705                1710

Val Lys Ser Cys Phe Arg Val Gly Trp Ser Asp Glu Gly Ser Pro
    1715                1720                1725

Glu Arg Gly Phe Gln Tyr Ile Tyr Leu Thr Asp Glu Asp Tyr Ala
    1730                1735                1740

Arg Ile Ser Leu Ser Val Ile Ala His Lys Leu Gln Leu Asp Asn
    1745                1750                1755

Gly Glu Ile Arg Trp Ile Ile Asp Ser Val Val Gly Lys Glu Asp

1760                1765                1770

Gly Leu Gly Val Glu Asn Ile His Gly Ser Ala Ala Ile Ala Ser
    1775                1780                1785

Ala Tyr Ser Arg Ala Tyr Glu Glu Thr Phe Thr Leu Thr Phe Val
    1790                1795                1800

Thr Gly Arg Thr Val Gly Ile Gly Ala Tyr Leu Ala Arg Leu Gly
    1805                1810                1815

Ile Arg Cys Ile Gln Arg Leu Asp Gln Pro Ile Ile Leu Thr Gly
    1820                1825                1830

Phe Ser Ala Leu Asn Lys Leu Leu Gly Arg Glu Val Tyr Ser Ser
    1835                1840                1845

His Met Gln Leu Gly Gly Pro Lys Ile Met Ala Thr Asn Gly Val
    1850                1855                1860

Val His Leu Thr Val Ser Asp Asp Leu Glu Gly Val Ser Asn Ile
    1865                1870                1875

Leu Arg Trp Leu Ser Tyr Val Pro Ala Asn Ile Gly Gly Pro Leu
    1880                1885                1890

Pro Ile Thr Lys Pro Leu Asp Pro Pro Asp Arg Pro Val Ala Tyr
    1895                1900                1905

Ile Pro Glu Asn Thr Cys Asp Pro Arg Ala Ala Ile Arg Gly Val
    1910                1915                1920

Asp Asp Ser Gln Gly Lys Trp Leu Gly Gly Met Phe Asp Lys Asp
    1925                1930                1935

Ser Phe Val Glu Thr Phe Glu Gly Trp Ala Lys Thr Val Val Thr
    1940                1945                1950

Gly Arg Ala Lys Leu Gly Gly Ile Pro Val Gly Val Ile Ala Val
    1955                1960                1965

Glu Thr Gln Thr Met Met Gln Leu Ile Pro Ala Asp Pro Gly Gln
    1970                1975                1980

Leu Asp Ser His Glu Arg Ser Val Pro Arg Ala Gly Gln Val Trp
    1985                1990                1995

Phe Pro Asp Ser Ala Thr Lys Thr Ala Gln Ala Leu Leu Asp Phe
    2000                2005                2010

Asn Arg Glu Gly Leu Pro Leu Phe Ile Leu Ala Asn Trp Arg Gly
    2015                2020                2025

Phe Ser Gly Gly Gln Arg Asp Leu Phe Glu Gly Ile Leu Gln Ala
    2030                2035                2040

Gly Ser Thr Ile Val Glu Asn Leu Arg Thr Tyr Asn Gln Pro Ala
    2045                2050                2055

Phe Val Tyr Ile Pro Met Ala Gly Glu Leu Arg Gly Gly Ala Trp
    2060                2065                2070

Val Val Val Asp Ser Lys Ile Asn Pro Asp Arg Ile Glu Cys Tyr
    2075                2080                2085

Ala Glu Arg Thr Ala Lys Gly Asn Val Leu Glu Pro Gln Gly Leu
    2090                2095                2100

Ile Glu Ile Lys Phe Arg Ser Glu Glu Leu Gln Asp Cys Met Gly
    2105                2110                2115

Arg Leu Asp Pro Glu Leu Ile Asn Leu Lys Ala Lys Leu Gln Gly
    2120                2125                2130

Ala Lys Leu Gly Asn Gly Ser Leu Thr Asp Val Glu Ser Leu Gln
    2135                2140                2145

Lys Ser Ile Asp Ala Arg Thr Lys Gln Leu Leu Pro Leu Tyr Thr
    2150                2155                2160

Gln Ile Ala Ile Arg Phe Ala Glu Leu His Asp Thr Ser Leu Arg
    2165                2170                2175

Met Ala Ala Lys Gly Val Ile Lys Lys Val Val Asp Trp Glu Glu
    2180                2185                2190

Leu Arg Ser Phe Phe Tyr Arg Arg Leu Arg Arg Arg Ile Ser Glu
    2195                2200                2205

Asp Val Leu Ala Lys Glu Ile Arg Gly Ile Ala Gly Asp His Phe
    2210                2215                2220

Thr His Gln Ser Ala Val Glu Leu Ile Lys Glu Trp Tyr Leu Ala
    2225                2230                2235

Ser Gln Ala Thr Thr Gly Ser Thr Glu Trp Asp Asp Asp Asp Ala
    2240                2245                2250

Phe Val Ala Trp Lys Glu Asn Pro Glu Asn Tyr Lys Gly Tyr Ile
    2255                2260                2265

Gln Glu Leu Arg Ala Gln Lys Val Ser Gln Ser Leu Ser Asp Leu
    2270                2275                2280

Ala Asp Ser Ser Ser Asp Leu Glu Ala Phe Ser Gln Gly Leu Ser
    2285                2290                2295

Thr Leu Leu Asp Lys Met Asp Pro Ser Gln Arg Ala Lys Phe Ile
    2300                2305                2310

Gln Glu Val Lys Lys Val Leu Gly
    2315                2320

<210> 23
<211> 6963
<212> DNA
<213> Alopecurus myosuroides

<400> 23
atgggatcca cacatctgcc cattgtcggg tttaatgcat ccacaacacc atcgctatcc      60

actcttcgcc agataaactc agctgctgct gcattccaat cttcgtcccc ttcaaggtca     120

tccaagaaga aaagccgacg tgttaagtca ataagggatg atggcgatgg aagcgtgcca     180

gaccctgcag gccatggcca gtctattcgc caaggtctcg ctggcatcat cgacctccca     240

aaggagggcg catcagctcc agatgtggac atttcacatg ggtctgaaga ccacaaggcc     300

tcctaccaaa tgaatgggat actgaatgaa tcacataacg ggaggcacgc ctctctgtct     360

aaagtttatg aattttgcac ggaattgggt ggaaaaacac caattcacag tgtattagtc     420

gccaacaatg gaatggcagc agctaagttc atgcggagtg tccggacatg ggctaatgat     480

acatttgggt cagagaaggc gattcagttg atagctatgg caactccgga agacatgaga     540

ataaatgcag agcacattag aattgctgat cagtttgttg aagtacctgg tggaacaaac     600

aataacaact atgcaaatgt ccaactcata gtggagatag cagagagaac tggtgtctcc     660

gccgtttggc ctggttgggg ccatgcatct gagaatcctg aacttccaga tgcactaact     720

gcaaaaggaa ttgtttttct tgggccacca gcatcatcaa tgaacgcact aggcgacaag     780

gttggttcag ctctcattgc tcaagcagca ggggttccca ctcttgcttg gagtggatca     840

catgtggaaa ttccattaga actttgtttg gactcgatac ctgaggagat gtataggaaa     900

gcctgtgtta caaccgctga tgaagcagtt gcaagttgtc agatgattgg ttaccctgcc     960

atgatcaagg catcctgggg tggtggtggt aaagggatta gaaaggttaa taatgatgac 1020

gaggtgaaag cactgtttaa gcaagtacag ggtgaagttc ctggctcccc gatatttatc 1080

atgagacttg catctcagag tcgtcatctt gaagtccagc tgctttgtga tgaatatggc 1140

aatgtagcag cacttcacag tcgtgattgc agtgtgcaac gacgacacca aaagattatc 1200

gaggaaggac cagttactgt tgctcctcgt gaaacagtga aagagctaga gcaagcagca 1260

aggaggcttg ctaaggccgt gggttacgtc ggtgctgcta ctgttgaata tctctacagc 1320

atggagactg gtgaatacta ttttctggag cttaatccac ggttgcaggt tgagcaccca 1380

gtcaccgagt cgatagctga agtaaatttg cctgcagccc aagttgcagt tgggatgggt 1440

ataccccttt ggcagattcc agagatcaga cgtttctacg gaatggacaa tggaggaggc 1500

tatgatattt ggaggaaaac agcagctctc gctactccat tcaactttga tgaagtagat 1560

tctcaatggc cgaagggtca ttgtgtggca gttaggataa ccagtgagaa tccagatgat 1620

ggattcaagc ctactggtgg aaaagtaaag gagataagtt ttaaaagtaa gccaaatgtc 1680

tggggatatt tctcagttaa gtctggtgga ggcattcatg aatttgcgga ttctcagttt 1740

ggacacgttt ttgcctatgg agagactaga tcagcagcaa taaccagcat gtctcttgca 1800

ctaaaagaga ttcaaattcg tggagaaatt catacaaacg ttgattacac ggttgatctc 1860

ttgaatgccc cagacttcag agaaaacacg atccataccg gttggctgga taccagaata 1920

gctatgcgtg ttcaagctga gaggcctccc tggtatattt cagtggttgg aggagctcta 1980

tataaaacaa taaccaccaa tgcggagacc gtttctgaat atgttagcta tctcatcaag 2040

ggtcagattc caccaaagca catatccctt gtccattcaa ctatttcttt gaatatagag 2100

gaaagcaaat atacaattga gattgtgagg agtggacagg gtagctacag attgagactg 2160

aatggatcac ttattgaagc caatgtacaa acattatgtg atggaggcct tttaatgcag 2220

ctggatggaa atagccatgt tatttatgct gaagaagaag cgggtggtac acggcttctt 2280

attgatggaa aaacatgctt gctacagaat gaccatgatc cgtcaaggtt attagctgag 2340

acaccctgca aacttcttcg tttcttgatt gccgatggtg ctcatgttga tgctgatgta 2400

ccatacgcgg aagttgaggt tatgaagatg tgcatgcccc tcttgtcgcc tgctgctggt 2460

gtcattaatg ttttgttgtc tgagggccag gcgatgcagg ctggtgatct tatagcgaga 2520

cttgatctcg atgacccttc tgctgtgaag agagccgagc catttgaagg atcttttcca 2580

gaaatgagcc ttcctattgc tgcttctggc caagttcaca aaagatgtgc tgcaagtttg 2640

aacgctgctc gaatggtcct tgcaggatat gaccatgcgg ccaacaaagt tgtgcaagat 2700

ttggtatggt gccttgatac acctgctctt cctttcctac aatgggaaga gcttatgtct 2760

gttttagcaa ctagacttcc aagacgtctt aagagcgagt tggagggcaa atacaatgaa 2820

tacaagttaa atgttgacca tgtgaagatc aaggatttcc ctaccgagat gcttagagag 2880

acaatcgagg aaaatcttgc atgtgtttcc gagaaggaaa tggtgacaat tgagaggctt 2940

gttgaccctc tgatgagcct gctgaagtca tacgagggtg ggagagaaag ccatgcccac 3000

tttattgtca agtccctttt tgaggagtat ctctcggttg aggaactatt cagtgatggc 3060

attcagtctg acgtgattga acgcctgcgc ctacaatata gtaaagacct ccagaaggtt 3120

gtagacattg ttttgtctca ccagggtgtg agaaacaaaa caaagctgat actcgcgctc 3180

atggagaaac tggtctatcc aaaccctgct gcctacagag atcagttgat tcgcttttct 3240

tccctcaacc ataaaagata ttataagttg gctcttaaag ctagtgaact tcttgaacaa 3300

accaagctca gcgaactccg cacaagcatt gcaaggaacc tttcagcgct ggatatgttc 3360

accgaggaaa aggcagattt ctccttgcaa gacagaaaat tggccattaa tgagagcatg 3420

ggagatttag tcactgcccc actgccagtt gaagatgcac ttgtttcttt gtttgattgt 3480

actgatcaaa ctcttcagca gagagtgatt cagacataca tatctcgatt ataccagcct 3540

caacttgtga aggatagcat ccagctgaaa tatcaggatt ctggtgttat tgctttatgg 3600

gaattcactg aaggaaatca tgagaagaga ttgggtgcta tggttatcct gaagtcacta 3660

gaatctgtgt caacagccat tggagctgct ctaaaggatg catcacatta tgcaagctct 3720

gcgggcaaca cggtgcatat tgctttgttg gatgctgata cccaactgaa tacaactgaa 3780

gatagtggtg ataatgacca agctcaagac aagatggata aactttcttt tgtactgaaa 3840

caagatgttg tcatggctga tctacgtgct gctgatgtca aggttgttag ttgcattgtt 3900

caaagagatg gagcaatcat gcctatgcgc cgtaccttcc tcttgtcaga ggaaaaactt 3960

tgttacgagg aagagccgat tcttcggcat gtggagcctc cactttctgc acttcttgag 4020

ttggataaat tgaaagtgaa aggatacaat gagatgaagt atacaccgtc acgtgatcgt 4080

cagtggcata tatacacact tagaaatact gaaaatccaa aaatgctgca cagggtattt 4140

ttccgaacac ttgtcagaca acccagtgca ggcaacaggt ttacatcaga ccatatcact 4200

gatgttgaag taggacacgc agaggaacct ctttcattta cttcaagcag catattaaaa 4260

tcgttgaaga ttgctaaaga agaattggag cttcacgcga tcaggactgg ccattctcat 4320

atgtacttgt gcatattgaa agagcaaaag cttcttgacc ttgttcctgt ttcagggaac 4380

actgttgtgg atgttggtca agatgaagct actgcatgct ctcttttgaa agaaatggct 4440

ttaaagatac atgaacttgt tggtgcaaga atgcatcatc tttctgtatg ccagtgggaa 4500

gtgaaactta agttggtgag cgatgggcct gccagtggta gctggagagt tgtaacaacc 4560

aatgttactg gtcacacctg cactgtggat atctaccggg aggtcgaaga tacagaatca 4620

cagaaactag tataccactc caccgcattg tcatctggtc ctttgcatgg tgttgcactg 4680

aatacttcgt atcagccttt gagtgttatt gatttaaaac gttgctctgc caggaacaac 4740

aaaactacat actgctatga ttttccattg acatttgaag ctgcagtgca gaagtcgtgg 4800

tctaacattt ccagtgaaaa caaccaatgt tatgttaaag cgacagagct tgtgtttgct 4860

gaaaagaatg ggtcgtgggg cactcctata attcctatgc agcgtgctgc tgggctgaat 4920

gacattggta tggtagcctg gatcttggac atgtccactc ctgaatttcc cagcggcaga 4980

cagatcattg ttatcgcaaa tgatattaca tttagagctg gatcatttgg cccaagggaa 5040

gatgcatttt tcgaagctgt aaccaacctg gcttgtgaga agaagcttcc acttatctac 5100

ttggctgcaa actctggtgc tcggattggc attgctgatg aagtaaaatc ttgcttccgt 5160

gttggatgga ctgatgatag cagccctgaa cgtggattta ggtacattta tatgactgac 5220

gaagaccatg atcgtattgg ctcttcagtt atagcacaca agatgcagct agatagtggc 5280

gagatcaggt gggttattga ttctgttgtg ggaaaagagg atggactagg tgtggagaac 5340

atacatggaa gtgctgctat tgccagtgcc tattctaggg cgtacgagga gacatttaca 5400

cttacattcg ttactggacg aactgttgga atcggagcct atcttgctcg acttggcata 5460

cggtgcatac agcgtattga ccagcccatt attttgaccg ggttttctgc cctgaacaag 5520

cttcttgggc gggaggtgta cagctcccac atgcagttgg gtggtcccaa aatcatggcg 5580

acgaatggtg ttgtccatct gactgttcca gatgaccttg aaggtgtttc taatatattg 5640

aggtggctca gctatgttcc tgcaaacatt ggtggacctc ttcctattac aaaatctttg 5700

gacccaatag acagacccgt tgcatacatc cctgagaata catgtgatcc tcgtgcagcc 5760

atcagtggca ttgatgacag ccaagggaaa tggttgggtg gcatgtttga caaagacagt 5820

tttgtggaga catttgaagg atgggcgaag acagtagtta ctggcagagc aaaacttgga 5880

gggattcctg ttggtgttat agctgtggag acacagacca tgatgcagct cgtccccgct 5940

gatccaggcc agcctgattc ccacgagcgg tctgttcctc gtgctgggca agtttggttt 6000

ccagattctg ctaccaagac agcgcaggcg atgttggact tcaaccgtga aggattacct 6060

ctgttcatac ttgctaactg gagaggcttc tctggagggc aaagagatct ttttgaagga 6120

attctgcagg ctgggtcaac aattgttgag aaccttagga catacaatca gcctgccttt 6180

gtatatatcc ccaaggctgc agagctacgt ggaggagcct gggtcgtgat tgatagcaag 6240

ataaacccag atcgcatcga gtgctatgct gagaggactg caaagggtaa tgttctcgaa 6300

cctcaagggt tgattgagat caagttcagg tcagaggaac tcaaagaatg catgggtagg 6360

cttgatccag aattgataga tctgaaagca agactccagg gagcaaatgg aagcctatct 6420

gatggagaat cccttcagaa gagcatagaa gctcggaaga aacagttgct gcctctgtac 6480

acccaaatcg cggtacgttt tgcggaattg cacgacactt cccttagaat ggctgctaaa 6540

ggtgtgatca ggaaagttgt agactgggaa gactctcggt ctttcttcta caagagatta 6600

cggaggaggc tatccgagga cgttctggca aaggagatta gaggtgtaat tggtgagaag 6660

tttcctcaca aatcagcgat cgagctgatc aagaaatggt acttggcttc tgaggcagct     6720

gcagcaggaa gcaccgactg ggatgacgac gatgcttttg tcgcctggag ggagaaccct     6780

gaaaactata aggagtatat caaagagctt agggctcaaa gggtatctcg gttgctctca     6840

gatgttgcag gctccagttc ggatttacaa gccttgccgc agggtctttc catgctacta     6900

gataagatgg atccctctaa gagagcacag tttatcgagg aggtcatgaa ggtcctgaaa     6960

tga                                                                   6963

<210> 24
<211> 2320
<212> PRT
<213> Alopecurus myosuroides

<400> 24
Met Gly Ser Thr His Leu Pro Ile Val Gly Phe Asn Ala Ser Thr Thr
1               5                   10                  15

Pro Ser Leu Ser Thr Leu Arg Gln Ile Asn Ser Ala Ala Ala Ala Phe
            20                  25                  30

Gln Ser Ser Ser Pro Ser Arg Ser Ser Lys Lys Lys Ser Arg Arg Val
        35                  40                  45

Lys Ser Ile Arg Asp Asp Gly Asp Gly Ser Val Pro Asp Pro Ala Gly
    50                  55                  60

His Gly Gln Ser Ile Arg Gln Gly Leu Ala Gly Ile Ile Asp Leu Pro
65                  70                  75                  80

Lys Glu Gly Ala Ser Ala Pro Asp Val Asp Ile Ser His Gly Ser Glu
                85                  90                  95

Asp His Lys Ala Ser Tyr Gln Met Asn Gly Ile Leu Asn Glu Ser His
            100                 105                 110

Asn Gly Arg His Ala Ser Leu Ser Lys Val Tyr Glu Phe Cys Thr Glu
        115                 120                 125

Leu Gly Gly Lys Thr Pro Ile His Ser Val Leu Val Ala Asn Asn Gly
    130                 135                 140

Met Ala Ala Ala Lys Phe Met Arg Ser Val Arg Thr Trp Ala Asn Asp
145                 150                 155                 160

Thr Phe Gly Ser Glu Lys Ala Ile Gln Leu Ile Ala Met Ala Thr Pro
                165                 170                 175

Glu Asp Met Arg Ile Asn Ala Glu His Ile Arg Ile Ala Asp Gln Phe
            180                 185                 190

Val Glu Val Pro Gly Gly Thr Asn Asn Asn Asn Tyr Ala Asn Val Gln
        195                 200                 205

Leu Ile Val Glu Ile Ala Glu Arg Thr Gly Val Ser Ala Val Trp Pro
    210                 215                 220

Gly Trp Gly His Ala Ser Glu Asn Pro Glu Leu Pro Asp Ala Leu Thr
225                 230                 235                 240

Ala Lys Gly Ile Val Phe Leu Gly Pro Pro Ala Ser Ser Met Asn Ala
                245                 250                 255

Leu Gly Asp Lys Val Gly Ser Ala Leu Ile Ala Gln Ala Ala Gly Val
            260                 265                 270

Pro Thr Leu Ala Trp Ser Gly Ser His Val Glu Ile Pro Leu Glu Leu
        275                 280                 285

Cys Leu Asp Ser Ile Pro Glu Glu Met Tyr Arg Lys Ala Cys Val Thr
    290                 295                 300

Thr Ala Asp Glu Ala Val Ala Ser Cys Gln Met Ile Gly Tyr Pro Ala
305                 310                 315                 320

Met Ile Lys Ala Ser Trp Gly Gly Gly Gly Lys Gly Ile Arg Lys Val
                325                 330                 335

Asn Asn Asp Asp Glu Val Lys Ala Leu Phe Lys Gln Val Gln Gly Glu
            340                 345                 350

Val Pro Gly Ser Pro Ile Phe Ile Met Arg Leu Ala Ser Gln Ser Arg
        355                 360                 365

His Leu Glu Val Gln Leu Leu Cys Asp Glu Tyr Gly Asn Val Ala Ala
    370                 375                 380

Leu His Ser Arg Asp Cys Ser Val Gln Arg Arg His Gln Lys Ile Ile
385                 390                 395                 400

Glu Glu Gly Pro Val Thr Val Ala Pro Arg Glu Thr Val Lys Glu Leu
                405                 410                 415

Glu Gln Ala Ala Arg Arg Leu Ala Lys Ala Val Gly Tyr Val Gly Ala
            420                 425                 430

Ala Thr Val Glu Tyr Leu Tyr Ser Met Glu Thr Gly Glu Tyr Tyr Phe
        435                 440                 445

Leu Glu Leu Asn Pro Arg Leu Gln Val Glu His Pro Val Thr Glu Ser
    450                 455                 460

Ile Ala Glu Val Asn Leu Pro Ala Ala Gln Val Ala Val Gly Met Gly
465                 470                 475                 480

Ile Pro Leu Trp Gln Ile Pro Glu Ile Arg Arg Phe Tyr Gly Met Asp
                485                 490                 495

Asn Gly Gly Gly Tyr Asp Ile Trp Arg Lys Thr Ala Ala Leu Ala Thr
            500                 505                 510

Pro Phe Asn Phe Asp Glu Val Asp Ser Gln Trp Pro Lys Gly His Cys
        515                 520                 525

Val Ala Val Arg Ile Thr Ser Glu Asn Pro Asp Asp Gly Phe Lys Pro
    530                 535                 540

Thr Gly Gly Lys Val Lys Glu Ile Ser Phe Lys Ser Lys Pro Asn Val
545                 550                 555                 560

Trp Gly Tyr Phe Ser Val Lys Ser Gly Gly Gly Ile His Glu Phe Ala
                565                 570                 575

Asp Ser Gln Phe Gly His Val Phe Ala Tyr Gly Glu Thr Arg Ser Ala
            580                 585                 590

Ala Ile Thr Ser Met Ser Leu Ala Leu Lys Glu Ile Gln Ile Arg Gly
        595                 600                 605

Glu Ile His Thr Asn Val Asp Tyr Thr Val Asp Leu Leu Asn Ala Pro
    610                 615                 620

Asp Phe Arg Glu Asn Thr Ile His Thr Gly Trp Leu Asp Thr Arg Ile
625                 630                 635                 640

Ala Met Arg Val Gln Ala Glu Arg Pro Pro Trp Tyr Ile Ser Val Val
                645                 650                 655

Gly Gly Ala Leu Tyr Lys Thr Ile Thr Thr Asn Ala Glu Thr Val Ser
            660                 665                 670

Glu Tyr Val Ser Tyr Leu Ile Lys Gly Gln Ile Pro Pro Lys His Ile

675                 680                 685

Ser Leu Val His Ser Thr Ile Ser Leu Asn Ile Glu Glu Ser Lys Tyr
    690                 695                 700

Thr Ile Glu Ile Val Arg Ser Gly Gln Gly Ser Tyr Arg Leu Arg Leu
705                 710                 715                 720

Asn Gly Ser Leu Ile Glu Ala Asn Val Gln Thr Leu Cys Asp Gly Gly
                725                 730                 735

Leu Leu Met Gln Leu Asp Gly Asn Ser His Val Ile Tyr Ala Glu Glu
            740                 745                 750

Glu Ala Gly Gly Thr Arg Leu Leu Ile Asp Gly Lys Thr Cys Leu Leu
        755                 760                 765

Gln Asn Asp His Asp Pro Ser Arg Leu Leu Ala Glu Thr Pro Cys Lys
    770                 775                 780

Leu Leu Arg Phe Leu Ile Ala Asp Gly Ala His Val Asp Ala Asp Val
785                 790                 795                 800

Pro Tyr Ala Glu Val Glu Val Met Lys Met Cys Met Pro Leu Leu Ser
                805                 810                 815

Pro Ala Ala Gly Val Ile Asn Val Leu Leu Ser Glu Gly Gln Ala Met
            820                 825                 830

Gln Ala Gly Asp Leu Ile Ala Arg Leu Asp Leu Asp Asp Pro Ser Ala
        835                 840                 845

Val Lys Arg Ala Glu Pro Phe Glu Gly Ser Phe Pro Glu Met Ser Leu
    850                 855                 860

Pro Ile Ala Ala Ser Gly Gln Val His Lys Arg Cys Ala Ala Ser Leu
865                 870                 875                 880

Asn Ala Ala Arg Met Val Leu Ala Gly Tyr Asp His Ala Ala Asn Lys
                885                 890                 895

Val Val Gln Asp Leu Val Trp Cys Leu Asp Thr Pro Ala Leu Pro Phe
            900                 905                 910

Leu Gln Trp Glu Glu Leu Met Ser Val Leu Ala Thr Arg Leu Pro Arg
        915                 920                 925

Arg Leu Lys Ser Glu Leu Glu Gly Lys Tyr Asn Glu Tyr Lys Leu Asn
    930                 935                 940

Val Asp His Val Lys Ile Lys Asp Phe Pro Thr Glu Met Leu Arg Glu
945                 950                 955                 960

Thr Ile Glu Glu Asn Leu Ala Cys Val Ser Glu Lys Glu Met Val Thr
                965                 970                 975

Ile Glu Arg Leu Val Asp Pro Leu Met Ser Leu Leu Lys Ser Tyr Glu
            980                 985                 990

Gly Gly Arg Glu Ser His Ala His Phe Ile Val Lys Ser Leu Phe Glu
        995                 1000                1005

Glu Tyr Leu Ser Val Glu Glu Leu Phe Ser Asp Gly Ile Gln Ser
    1010                1015                1020

Asp Val Ile Glu Arg Leu Arg Leu Gln Tyr Ser Lys Asp Leu Gln
    1025                1030                1035

Lys Val Val Asp Ile Val Leu Ser His Gln Gly Val Arg Asn Lys
    1040                1045                1050

Thr Lys Leu Ile Leu Ala Leu Met Glu Lys Leu Val Tyr Pro Asn
    1055                1060                1065

Pro Ala Ala Tyr Arg Asp Gln Leu Ile Arg Phe Ser Ser Leu Asn
    1070                1075                1080

His Lys Arg Tyr Tyr Lys Leu Ala Leu Lys Ala Ser Glu Leu Leu
    1085                1090                1095

Glu Gln Thr Lys Leu Ser Glu Leu Arg Thr Ser Ile Ala Arg Asn
    1100                1105                1110

Leu Ser Ala Leu Asp Met Phe Thr Glu Glu Lys Ala Asp Phe Ser
    1115                1120                1125

Leu Gln Asp Arg Lys Leu Ala Ile Asn Glu Ser Met Gly Asp Leu
    1130                1135                1140

Val Thr Ala Pro Leu Pro Val Glu Asp Ala Leu Val Ser Leu Phe
    1145                1150                1155

Asp Cys Thr Asp Gln Thr Leu Gln Gln Arg Val Ile Gln Thr Tyr
    1160                1165                1170

Ile Ser Arg Leu Tyr Gln Pro Gln Leu Val Lys Asp Ser Ile Gln
    1175                1180                1185

Leu Lys Tyr Gln Asp Ser Gly Val Ile Ala Leu Trp Glu Phe Thr
    1190                1195                1200

Glu Gly Asn His Glu Lys Arg Leu Gly Ala Met Val Ile Leu Lys
    1205                1210                1215

Ser Leu Glu Ser Val Ser Thr Ala Ile Gly Ala Ala Leu Lys Asp
    1220                1225                1230

Ala Ser His Tyr Ala Ser Ser Ala Gly Asn Thr Val His Ile Ala
    1235                1240                1245

Leu Leu Asp Ala Asp Thr Gln Leu Asn Thr Thr Glu Asp Ser Gly
    1250                1255                1260

Asp Asn Asp Gln Ala Gln Asp Lys Met Asp Lys Leu Ser Phe Val
    1265                1270                1275

Leu Lys Gln Asp Val Val Met Ala Asp Leu Arg Ala Ala Asp Val
    1280                1285                1290

Lys Val Val Ser Cys Ile Val Gln Arg Asp Gly Ala Ile Met Pro
    1295                1300                1305

Met Arg Arg Thr Phe Leu Leu Ser Glu Glu Lys Leu Cys Tyr Glu
    1310                1315                1320

Glu Glu Pro Ile Leu Arg His Val Glu Pro Pro Leu Ser Ala Leu
    1325                1330                1335

Leu Glu Leu Asp Lys Leu Lys Val Lys Gly Tyr Asn Glu Met Lys
    1340                1345                1350

Tyr Thr Pro Ser Arg Asp Arg Gln Trp His Ile Tyr Thr Leu Arg
    1355                1360                1365

Asn Thr Glu Asn Pro Lys Met Leu His Arg Val Phe Phe Arg Thr
    1370                1375                1380

Leu Val Arg Gln Pro Ser Ala Gly Asn Arg Phe Thr Ser Asp His
    1385                1390                1395

Ile Thr Asp Val Glu Val Gly His Ala Glu Glu Pro Leu Ser Phe
    1400                1405                1410

Thr Ser Ser Ser Ile Leu Lys Ser Leu Lys Ile Ala Lys Glu Glu
    1415                1420                1425

Leu Glu Leu His Ala Ile Arg Thr Gly His Ser His Met Tyr Leu
    1430                1435                1440

Cys Ile Leu Lys Glu Gln Lys Leu Leu Asp Leu Val Pro Val Ser
    1445                1450                1455

Gly Asn Thr Val Val Asp Val Gly Gln Asp Glu Ala Thr Ala Cys
    1460                1465                1470

Ser Leu Leu Lys Glu Met Ala Leu Lys Ile His Glu Leu Val Gly
    1475                1480                1485

Ala Arg Met His His Leu Ser Val Cys Gln Trp Glu Val Lys Leu
    1490                1495                1500

Lys Leu Val Ser Asp Gly Pro Ala Ser Gly Ser Trp Arg Val Val
    1505                1510                1515

Thr Thr Asn Val Thr Gly His Thr Cys Thr Val Asp Ile Tyr Arg
    1520                1525                1530

Glu Val Glu Asp Thr Glu Ser Gln Lys Leu Val Tyr His Ser Thr
    1535                1540                1545

Ala Leu Ser Ser Gly Pro Leu His Gly Val Ala Leu Asn Thr Ser
    1550                1555                1560

Tyr Gln Pro Leu Ser Val Ile Asp Leu Lys Arg Cys Ser Ala Arg
    1565                1570                1575

Asn Asn Lys Thr Thr Tyr Cys Tyr Asp Phe Pro Leu Thr Phe Glu
    1580                1585                1590

Ala Ala Val Gln Lys Ser Trp Ser Asn Ile Ser Ser Glu Asn Asn
    1595                1600                1605

Gln Cys Tyr Val Lys Ala Thr Glu Leu Val Phe Ala Glu Lys Asn
    1610                1615                1620

Gly Ser Trp Gly Thr Pro Ile Ile Pro Met Gln Arg Ala Ala Gly
    1625                1630                1635

Leu Asn Asp Ile Gly Met Val Ala Trp Ile Leu Asp Met Ser Thr

1640                1645                1650

Pro Glu Phe Pro Ser Gly Arg Gln Ile Ile Val Ile Ala Asn Asp
    1655                1660                1665

Ile Thr Phe Arg Ala Gly Ser Phe Gly Pro Arg Glu Asp Ala Phe
    1670                1675                1680

Phe Glu Ala Val Thr Asn Leu Ala Cys Glu Lys Lys Leu Pro Leu
    1685                1690                1695

Ile Tyr Leu Ala Ala Asn Ser Gly Ala Arg Ile Gly Ile Ala Asp
    1700                1705                1710

Glu Val Lys Ser Cys Phe Arg Val Gly Trp Thr Asp Asp Ser Ser
    1715                1720                1725

Pro Glu Arg Gly Phe Arg Tyr Ile Tyr Met Thr Asp Glu Asp His
    1730                1735                1740

Asp Arg Ile Gly Ser Ser Val Ile Ala His Lys Met Gln Leu Asp
    1745                1750                1755

Ser Gly Glu Ile Arg Trp Val Ile Asp Ser Val Val Gly Lys Glu
    1760                1765                1770

Asp Gly Leu Gly Val Glu Asn Ile His Gly Ser Ala Ala Ile Ala
    1775                1780                1785

Ser Ala Tyr Ser Arg Ala Tyr Glu Glu Thr Phe Thr Leu Thr Phe
    1790                1795                1800

Val Thr Gly Arg Thr Val Gly Ile Gly Ala Tyr Leu Ala Arg Leu
    1805                1810                1815

Gly Ile Arg Cys Ile Gln Arg Ile Asp Gln Pro Ile Ile Leu Thr
    1820                1825                1830

Gly Phe Ser Ala Leu Asn Lys Leu Leu Gly Arg Glu Val Tyr Ser
    1835                1840                1845

Ser His Met Gln Leu Gly Gly Pro Lys Ile Met Ala Thr Asn Gly
    1850                1855                1860

Val Val His Leu Thr Val Pro Asp Asp Leu Glu Gly Val Ser Asn
    1865                1870                1875

Ile Leu Arg Trp Leu Ser Tyr Val Pro Ala Asn Ile Gly Gly Pro
    1880                1885                1890

Leu Pro Ile Thr Lys Ser Leu Asp Pro Ile Asp Arg Pro Val Ala
    1895                1900                1905

Tyr Ile Pro Glu Asn Thr Cys Asp Pro Arg Ala Ala Ile Ser Gly
    1910                1915                1920

Ile Asp Asp Ser Gln Gly Lys Trp Leu Gly Gly Met Phe Asp Lys
    1925                1930                1935

Asp Ser Phe Val Glu Thr Phe Glu Gly Trp Ala Lys Thr Val Val
    1940                1945                1950

Thr Gly Arg Ala Lys Leu Gly Gly Ile Pro Val Gly Val Ile Ala
    1955                1960                1965

Val Glu Thr Gln Thr Met Met Gln Leu Val Pro Ala Asp Pro Gly
    1970                1975                1980

Gln Pro Asp Ser His Glu Arg Ser Val Pro Arg Ala Gly Gln Val
    1985                1990                1995

Trp Phe Pro Asp Ser Ala Thr Lys Thr Ala Gln Ala Met Leu Asp
    2000                2005                2010

Phe Asn Arg Glu Gly Leu Pro Leu Phe Ile Leu Ala Asn Trp Arg
    2015                2020                2025

Gly Phe Ser Gly Gly Gln Arg Asp Leu Phe Glu Gly Ile Leu Gln
    2030                2035                2040

Ala Gly Ser Thr Ile Val Glu Asn Leu Arg Thr Tyr Asn Gln Pro
    2045                2050                2055

Ala Phe Val Tyr Ile Pro Lys Ala Ala Glu Leu Arg Gly Gly Ala
    2060                2065                2070

Trp Val Val Ile Asp Ser Lys Ile Asn Pro Asp Arg Ile Glu Cys
    2075                2080                2085

Tyr Ala Glu Arg Thr Ala Lys Gly Asn Val Leu Glu Pro Gln Gly
    2090                2095                2100

Leu Ile Glu Ile Lys Phe Arg Ser Glu Glu Leu Lys Glu Cys Met
    2105                2110                2115

Gly Arg Leu Asp Pro Glu Leu Ile Asp Leu Lys Ala Arg Leu Gln
    2120                2125                2130

Gly Ala Asn Gly Ser Leu Ser Asp Gly Glu Ser Leu Gln Lys Ser
    2135                2140                2145

Ile Glu Ala Arg Lys Lys Gln Leu Leu Pro Leu Tyr Thr Gln Ile
    2150                2155                2160

Ala Val Arg Phe Ala Glu Leu His Asp Thr Ser Leu Arg Met Ala
    2165                2170                2175

Ala Lys Gly Val Ile Arg Lys Val Val Asp Trp Glu Asp Ser Arg
    2180                2185                2190

Ser Phe Phe Tyr Lys Arg Leu Arg Arg Arg Leu Ser Glu Asp Val
    2195                2200                2205

Leu Ala Lys Glu Ile Arg Gly Val Ile Gly Glu Lys Phe Pro His
    2210                2215                2220

Lys Ser Ala Ile Glu Leu Ile Lys Lys Trp Tyr Leu Ala Ser Glu
    2225                2230                2235

Ala Ala Ala Ala Gly Ser Thr Asp Trp Asp Asp Asp Asp Ala Phe
    2240                2245                2250

Val Ala Trp Arg Glu Asn Pro Glu Asn Tyr Lys Glu Tyr Ile Lys
    2255                2260                2265

Glu Leu Arg Ala Gln Arg Val Ser Arg Leu Leu Ser Asp Val Ala
    2270                2275                2280

Gly Ser Ser Ser Asp Leu Gln Ala Leu Pro Gln Gly Leu Ser Met
    2285                2290                2295

Leu Leu Asp Lys Met Asp Pro Ser Lys Arg Ala Gln Phe Ile Glu
    2300                2305                2310

Glu Val Met Lys Val Leu Lys
    2315                2320

<210> 25
<211> 6936
<212> DNA
<213> Aegilops tauschii

<400> 25

atgggatcca cacatttgcc cattgtcggc cttaatgcct cgacaacacc atcgctatcc 60

actattcgcc cggtaaattc agccggtgct gcattccaac catctgcccc ttctagaacc 120

tccaagaaga aaagtcgtcg tgttcagtca ttaagggatg gaggcgatgg aggcgtgtca 180

gaccctaacc agtctattcg ccaaggtctt gccggcatca ttgacctccc aaaggagggc 240

acatcagctc cggaagtgga tatttcacat gggtccgaag aacccagggg ctcctaccaa 300

atgaatggga tactgaatga agcacataat gggaggcatg cttcgctgtc taaggttgtc 360

gaattttgta tggcattggg cggcaaaaca ccaattcata gtgtattagt tgcgaacaat 420

ggaatggcag cagctaagtt catgcggagt gtccgaacat gggctaatga aacatttggg 480

tcagagaagg caattcagtt gatagctatg gctactccag aagacatgag gataaatgca 540

gagcacatta gaattgctga tcaatttgtt gaagtacccg gtggaacaaa caataacaac 600

tatgcaaatg tccaactcat agtggagata gcagtgagaa ccggtgtttc tgctgtttgg 660

cctggttggg gccatgcatc tgagaatcct gaacttccag atgcactaaa tgcaaacgga 720

attgtttttc ttgggccacc atcatcatca atgaacgcac taggtgacaa ggttggttca 780

gctctcattg ctcaagcagc aggggttccg actcttcctt ggagtggatc acaggtggaa 840

attccattag aagtttgttt ggactcgata cctgcggata tgtataggaa agcttgtgtt 900

agtactacgg aggaagcact tgcgagttgt cagatgattg ggtatccagc catgattaaa 960

gcatcatggg gtggtggtgg taaagggatc cgaaaggtta ataacgacga tgatgtcaga 1020

gcactgttta agcaagtgca aggtgaagtt cctggctccc caatatttat catgagactt 1080

gcatctcaga gtcgacatct tgaagttcag ttgctttgtg atcaatatgg caatgtagct 1140

gcgcttcaca gtcgtgactg cagtgtgcaa cggcgacacc aaaagattat tgaggaagga 1200

ccagttactg ttgctcctcg cgagacagtg aaagagctag agcaagcagc aaggaggctt 1260

gctaaggctg tgggttatgt tggtgctgct actgttgaat atctctacag catggagact 1320

ggtgaatact attttctgga acttaatcca cggttgcagg ttgagcatcc agtcaccgag 1380

tggatagctg aagtaaactt gcctgcagct caagttgcag ttggaatggg tatacccctt 1440

tggcaggttc cagagatcag acgtttctat ggaatggaca atggaggagg ctatgacatt 1500

tggaggaaaa cagcagctct tgctacccca tttaactttg atgaagtgga ttctcaatgg 1560

ccaaagggtc attgtgtagc agttaggata accagtgagg atccagatga cggattcaag 1620

cctaccggtg gaaaagtaaa ggagatcagt tttaaaagca agccaaatgt ttgggcctat 1680

ttctctgtta agtccggtgg aggcattcat gaatttgctg attctcagtt tggacatgtt 1740

tttgcatatg gagtgtctag agcagcagca ataaccaaca tgtctcttgc gctaaaagag 1800

attcaaattc gtggagaaat tcattcaaat gttgattaca cagttgatct cttgaatgcc 1860

tcagacttca aagaaaacag gattcatact ggctggctgg ataacagaat agcaatgcga 1920

gtccaagctg agagacctcc gtggtatatt tcagtggttg gaggagctct atataaaaca 1980

ataacgagca acacagacac tgtttctgaa tatgttagct atctcgtcaa gggtcagatt 2040

ccaccgaagc atatatccct tgtccattca actgtttctt tgaatataga ggaaagcaaa 2100

tatacaattg aaactataag gagcggacag ggtagctaca gattgcgaat gaatggatca 2160

gttattgaag caaatgtcca aacattatgt gatggtggac ttttaatgca gttggatgga 2220

aacagccatg taatttatgc tgaagaagag gccggtggta cacggcttct aattgatgga 2280

aagacatgct tgttacagaa tgatcacgat ccttcaaggt tattagctga gacaccctgc 2340

aaacttcttc gtttcttggt tgccgatggt gctcatgttg aagctgatgt accatatgcg 2400

gaagttgagg ttatgaagat gtgcatgccc ctcttgtcac ctgctgctgg tgtcattaat 2460

gttttgttgt ctgagggcca gcctatgcag gctggtgatc ttatagcaag acttgatctt 2520

gatgaccctt ctgctgtgaa gagagctgag ccgtttaacg gatctttccc agaaatgagc 2580

cttcctattg ctgcttctgg ccaagttcac aaaagatgtg ccacaagctt gaatgctgct 2640

cggatggtcc ttgcaggata tgatcacccg atcaacaaag ttgtacaaga tctggtatcc 2700

tgtctagatg ctcctgagct tcctttccta caatgggaag agcttatgtc tgttttagca 2760

actagacttc caaggcttct taagagcgag ttggagggta aatacagtga atataagtta 2820

aatgttggcc atggaaagag caaggatttc ccttccaaga tgctaagaga gataatcgag 2880

gaaaatcttg cacatggttc tgagaaggaa attgctacaa atgagaggct tgttgagcct 2940

cttatgagcc tactgaagtc atatgagggt ggcagagaaa gccatgcaca ctttattgtg 3000

aagtcccttt tcgaggacta tctctcggtt gaggaactat tcagtgatgg cattcagtct 3060

gatgtgattg aacgcctgcg ccaacaacat agtaaagatc tccagaaggt tgtagacatt 3120

gtgttgtctc accagggtgt gagaaacaaa actaagctga tactaacact catggagaaa 3180

ctggtctatc caaaccctgc tgcctacaag gatcagttga ctcgcttttc ctccctcaat 3240

cacaaaagat attataagtt ggcccttaaa gctagcgagc ttcttgaaca aaccaagctt 3300

agtgagctcc gcacaagcat tgcaaggagc ctttcagaac ttgagatgtt tactgaagaa 3360

aggacggcca ttagtgagat catgggagat ttagtgactg ccccactgcc agttgaagat 3420

gcactggttt ctttgtttga ttgtagtgat caaactcttc agcagagggt gatcgagacg 3480

tacatatctc gattatacca gcctcatctt gtcaaggata gtatccagct gaaatatcag 3540

gaatctggtg ttattgcttt atgggaattc gctgaagcgc attcagagaa gagattgggt 3600

gctatggtta ttgtgaagtc gttagaatct gtatcagcag caattggagc tgcactaaag 3660

ggtacatcac gctatgcaag ctctgagggt aacataatgc atattgcttt attgggtgct 3720

gataatcaaa tgcatggaac tgaagacagt ggtgataacg atcaagctca agtcaggata 3780

gacaaacttt ctgcgacact ggaacaaaat actgtcacag ctgatctccg tgctgctggt 3840
gtgaaggtta ttagttgcat tgttcaaagg gatggagcac tcatgcctat gcgccatacc 3900
ttcctcttgt cggatgaaaa gctttgttat gaggaagagc cggttctccg gcatgtggag 3960
cctcctcttt ctgctcttct tgagttgggt aagttgaaag tgaaaggata caatgaggtg 4020
aagtatacac cgtcacgtga tcgtcagtgg aacatataca cacttagaaa tacagagaac 4080
cccaaaatgt tgcacagggt gtttttccga actcttgtca ggcaacccgg tgcttccaac 4140
aaattcacat caggcaacat cagtgatgtt gaagtgggag gagctgagga atctctttca 4200
tttacatcga gcagcatatt aagatcgctg atgactgcta tagaagagtt ggagcttcac 4260
gcgattagga caggtcactc tcatatgttt ttgtgcatat tgaaagagca aagcttcttg 4320
gatcttgttc ccgtttcagg gaacaaagtt gtggatattg gccaagatga agctactgca 4380
tgcttgcttc tgaaagaaat ggctctacag atacatgaac ttgtgggtgc aaggatgcat 4440
catctttctg tatgccaatg ggaggtgaaa cttaagttgg acagcgatgg gcctgccagt 4500
ggtacctgga gagttgtaac aaccaatgtt actagtcaca cctgcactgt ggatatctac 4560
cgtgaggttg aagatacaga atcacagaaa ctagtgtacc actctgctcc atcgtcatct 4620
ggtcctttgc atggcgttgc actgaatact ccatatcagc ctttgagtgt tattgatctg 4680
aaacgttgct ccgctagaaa taacagaact acatactgct atgatttccc gttggcattt 4740
gaaactgcag tgcagaagtc atggtctaac atttctagtg acactaaccg atgttatgtt 4800
aaagcgacgg agctggtgtt tgctcacaag aacgggtcat ggggcactcc tgtaattcct 4860
atggagcgtc ctgctgggct caatgacatt ggtatggtag cttggatctt ggacatgtcc 4920
actcctgaat atcccaatgg caggcagatt gttgtcatcg caaatgatat tacttttaga 4980
gctggatcgt ttggtccaag ggaagatgca ttttttgaaa ctgttaccaa cctagcttgt 5040
gagaggaagc ttcctctcat ctacttggca gcaaactctg gtgctcggat cggcatagca 5100
gatgaagtaa aatcttgctt ccgtgttgga tggtctgatg atggcagccc tgaacgtggg 5160
tttcaatata tttatctgac tgaagaagac catgctcgta ttagcgcttc tgttatagcg 5220
cacaagatgc agcttgataa tggtgaaatt aggtgggtta ttgattctgt tgtagggaag 5280
gaggatgggc taggtgtgga gaacatacat ggaagtgctg ctattgccag tgcctattct 5340
agggcctatg aggagacatt tacgcttaca tttgtgactg gaaggactgt tggaatagga 5400
gcatatcttg ctcgacttgg catacggtgc attcagcgta ctgaccagcc cattatccta 5460
actgggttct ctgccttgaa caagcttctt ggccgggaag tgtacagctc ccacatgcag 5520
ttgggtggcc ccaaaattat ggccacaaac ggtgttgtcc atctgacagt ttcagatgac 5580
cttgaaggtg tatctaatat attgaggtgg ctcagctatg ttcctgccaa cattggtgga 5640
cctcttccta ttacaaaatc tttggaccca cctgacagac ccgttgctta catccctgag 5700

aatacatgtg atcctcgtgc agccatcagt ggcattgatg atagccaagg gaaatggttg 5760

gggggtatgt tcgacaaaga cagttttgtg gagacatttg aaggatgggc gaagtcagta 5820

gttactggca gagcgaaact cggagggatt ccggtgggtg ttatagctgt ggagacacag 5880

actatgatgc agctcatccc tgctgatcca ggtcagcttg attcccatga gcggtctgtt 5940

cctcgtgctg ggcaagtctg gtttccagat tcagctacta agacagcgca ggcaatgctg 6000

gacttcaacc gtgaaggatt acctctgttc atccttgcta actggagagg cttctctggt 6060

gggcaaagag atctttttga aggaatcctt caggctgggt caacaattgt tgagaacctt 6120

aggacataca atcagcctgc ctttgtatat atccccaagg ctgcagagct acgtggaggg 6180

gcttgggtcg tgattgatag caagataaat ccagatcgca ttgagttcta tgctgagagg 6240

actgcaaagg gcaatgttct tgaacctcaa gggttgattg agatcaagtt caggtcagag 6300

gaactccaag agtgcatggg caggcttgac ccagaattga taaatttgaa ggcaaaactc 6360

ctgggagcaa agcatgaaaa tggaagtcta tctgagtcag aatcccttca gaagagcata 6420

gaagcccgga agaaacagtt gttgcctttg tatactcaaa ttgcggtacg gttcgctgaa 6480

ttgcatgaca cttcccttag aatggctgct aagggtgtga ttaagaaggt tgtagactgg 6540

gaagattcta ggtctttctt ctacaagaga ttacggagga ggatatccga ggatgttctt 6600

gcaaaggaaa ttagaggtgt aagtggcaag cagttttctc accaatcggc aatcgagctg 6660

atccagaaat ggtacttggc ctctaaggga gctgaaacgg gaaacactga atgggatgat 6720

gacgatgctt ttgttgcctg gagggaaaac cctgaaaact accaggagta tatcaaagaa 6780

ctcagggctc aaagggtatc tcagttgctc tcagatgttg cagactccag tccagatcta 6840

gaagccttgc cacagggtct ttctatgcta ctagagaaga tggatccctc aaggagagca 6900

cagtttgttg aggaagtcaa gaaggccctt aaatga 6936

<210> 26
<211> 2311
<212> PRT
<213> Aegilops tauschii

<400> 26
Met Gly Ser Thr His Leu Pro Ile Val Gly Leu Asn Ala Ser Thr Thr
1               5                   10                  15

Pro Ser Leu Ser Thr Ile Arg Pro Val Asn Ser Ala Gly Ala Ala Phe
            20                  25                  30

Gln Pro Ser Ala Pro Ser Arg Thr Ser Lys Lys Lys Ser Arg Arg Val
        35                  40                  45

Gln Ser Leu Arg Asp Gly Gly Asp Gly Gly Val Ser Asp Pro Asn Gln

50                  55                  60

Ser Ile Arg Gln Gly Leu Ala Gly Ile Ile Asp Leu Pro Lys Glu Gly
65                  70                  75                  80

Thr Ser Ala Pro Glu Val Asp Ile Ser His Gly Ser Glu Glu Pro Arg
                85                  90                  95

Gly Ser Tyr Gln Met Asn Gly Ile Leu Asn Glu Ala His Asn Gly Arg
            100                 105                 110

His Ala Ser Leu Ser Lys Val Val Glu Phe Cys Met Ala Leu Gly Gly
        115                 120                 125

Lys Thr Pro Ile His Ser Val Leu Val Ala Asn Asn Gly Met Ala Ala
    130                 135                 140

Ala Lys Phe Met Arg Ser Val Arg Thr Trp Ala Asn Glu Thr Phe Gly
145                 150                 155                 160

Ser Glu Lys Ala Ile Gln Leu Ile Ala Met Ala Thr Pro Glu Asp Met
                165                 170                 175

Arg Ile Asn Ala Glu His Ile Arg Ile Ala Asp Gln Phe Val Glu Val
            180                 185                 190

Pro Gly Gly Thr Asn Asn Asn Asn Tyr Ala Asn Val Gln Leu Ile Val
        195                 200                 205

Glu Ile Ala Val Arg Thr Gly Val Ser Ala Val Trp Pro Gly Trp Gly
    210                 215                 220

His Ala Ser Glu Asn Pro Glu Leu Pro Asp Ala Leu Asn Ala Asn Gly
225                 230                 235                 240

Ile Val Phe Leu Gly Pro Pro Ser Ser Ser Met Asn Ala Leu Gly Asp
                245                 250                 255

Lys Val Gly Ser Ala Leu Ile Ala Gln Ala Ala Gly Val Pro Thr Leu
            260                 265                 270

Pro Trp Ser Gly Ser Gln Val Glu Ile Pro Leu Glu Val Cys Leu Asp
        275                 280                 285

Ser Ile Pro Ala Asp Met Tyr Arg Lys Ala Cys Val Ser Thr Thr Glu
    290                 295                 300

Glu Ala Leu Ala Ser Cys Gln Met Ile Gly Tyr Pro Ala Met Ile Lys
305                 310                 315                 320

Ala Ser Trp Gly Gly Gly Gly Lys Gly Ile Arg Lys Val Asn Asn Asp
                325                 330                 335

Asp Asp Val Arg Ala Leu Phe Lys Gln Val Gln Gly Glu Val Pro Gly
            340                 345                 350

Ser Pro Ile Phe Ile Met Arg Leu Ala Ser Gln Ser Arg His Leu Glu
        355                 360                 365

Val Gln Leu Leu Cys Asp Gln Tyr Gly Asn Val Ala Ala Leu His Ser
    370                 375                 380

Arg Asp Cys Ser Val Gln Arg Arg His Gln Lys Ile Ile Glu Glu Gly
385                 390                 395                 400

Pro Val Thr Val Ala Pro Arg Glu Thr Val Lys Glu Leu Glu Gln Ala
                405                 410                 415

Ala Arg Arg Leu Ala Lys Ala Val Gly Tyr Val Gly Ala Ala Thr Val
            420                 425                 430

Glu Tyr Leu Tyr Ser Met Glu Thr Gly Glu Tyr Tyr Phe Leu Glu Leu
        435                 440                 445

Asn Pro Arg Leu Gln Val Glu His Pro Val Thr Glu Trp Ile Ala Glu
    450                 455                 460

Val Asn Leu Pro Ala Ala Gln Val Ala Val Gly Met Gly Ile Pro Leu
465                 470                 475                 480

Trp Gln Val Pro Glu Ile Arg Arg Phe Tyr Gly Met Asp Asn Gly Gly
                485                 490                 495

Gly Tyr Asp Ile Trp Arg Lys Thr Ala Ala Leu Ala Thr Pro Phe Asn
            500                 505                 510

Phe Asp Glu Val Asp Ser Gln Trp Pro Lys Gly His Cys Val Ala Val
        515                 520                 525

Arg Ile Thr Ser Glu Asp Pro Asp Asp Gly Phe Lys Pro Thr Gly Gly
    530                 535                 540

Lys Val Lys Glu Ile Ser Phe Lys Ser Lys Pro Asn Val Trp Ala Tyr
545                 550                 555                 560

Phe Ser Val Lys Ser Gly Gly Gly Ile His Glu Phe Ala Asp Ser Gln
                565                 570                 575

Phe Gly His Val Phe Ala Tyr Gly Val Ser Arg Ala Ala Ala Ile Thr
            580                 585                 590

Asn Met Ser Leu Ala Leu Lys Glu Ile Gln Ile Arg Gly Glu Ile His
        595                 600                 605

Ser Asn Val Asp Tyr Thr Val Asp Leu Leu Asn Ala Ser Asp Phe Lys
    610                 615                 620

Glu Asn Arg Ile His Thr Gly Trp Leu Asp Asn Arg Ile Ala Met Arg
625                 630                 635                 640

Val Gln Ala Glu Arg Pro Pro Trp Tyr Ile Ser Val Val Gly Gly Ala
                645                 650                 655

Leu Tyr Lys Thr Ile Thr Ser Asn Thr Asp Thr Val Ser Glu Tyr Val
            660                 665                 670

Ser Tyr Leu Val Lys Gly Gln Ile Pro Pro Lys His Ile Ser Leu Val
        675                 680                 685

His Ser Thr Val Ser Leu Asn Ile Glu Glu Ser Lys Tyr Thr Ile Glu
    690                 695                 700

Thr Ile Arg Ser Gly Gln Gly Ser Tyr Arg Leu Arg Met Asn Gly Ser
705                 710                 715                 720

Val Ile Glu Ala Asn Val Gln Thr Leu Cys Asp Gly Gly Leu Leu Met
                725                 730                 735

Gln Leu Asp Gly Asn Ser His Val Ile Tyr Ala Glu Glu Glu Ala Gly
            740                 745                 750

Gly Thr Arg Leu Leu Ile Asp Gly Lys Thr Cys Leu Leu Gln Asn Asp
        755                 760                 765

His Asp Pro Ser Arg Leu Leu Ala Glu Thr Pro Cys Lys Leu Leu Arg
    770                 775                 780

Phe Leu Val Ala Asp Gly Ala His Val Glu Ala Asp Val Pro Tyr Ala
785                 790                 795                 800

Glu Val Glu Val Met Lys Met Cys Met Pro Leu Leu Ser Pro Ala Ala
                805                 810                 815

Gly Val Ile Asn Val Leu Leu Ser Glu Gly Gln Pro Met Gln Ala Gly
            820                 825                 830

Asp Leu Ile Ala Arg Leu Asp Leu Asp Asp Pro Ser Ala Val Lys Arg
        835                 840                 845

Ala Glu Pro Phe Asn Gly Ser Phe Pro Glu Met Ser Leu Pro Ile Ala
    850                 855                 860

Ala Ser Gly Gln Val His Lys Arg Cys Ala Thr Ser Leu Asn Ala Ala
865                 870                 875                 880

Arg Met Val Leu Ala Gly Tyr Asp His Pro Ile Asn Lys Val Val Gln
                885                 890                 895

Asp Leu Val Ser Cys Leu Asp Ala Pro Glu Leu Pro Phe Leu Gln Trp
            900                 905                 910

Glu Glu Leu Met Ser Val Leu Ala Thr Arg Leu Pro Arg Leu Leu Lys
        915                 920                 925

Ser Glu Leu Glu Gly Lys Tyr Ser Glu Tyr Lys Leu Asn Val Gly His
    930                 935                 940

Gly Lys Ser Lys Asp Phe Pro Ser Lys Met Leu Arg Glu Ile Ile Glu
945                 950                 955                 960

Glu Asn Leu Ala His Gly Ser Glu Lys Glu Ile Ala Thr Asn Glu Arg
                965                 970                 975

Leu Val Glu Pro Leu Met Ser Leu Leu Lys Ser Tyr Glu Gly Gly Arg
            980                 985                 990

Glu Ser His Ala His Phe Ile Val  Lys Ser Leu Phe Glu  Asp Tyr Leu
        995                 1000                1005

Ser Val  Glu Glu Leu Phe Ser  Asp Gly Ile Gln Ser  Asp Val Ile
    1010                1015                1020

Glu Arg  Leu Arg Gln Gln His  Ser Lys Asp Leu Gln  Lys Val Val
    1025                1030                1035

Asp Ile  Val Leu Ser His Gln  Gly Val Arg Asn Lys  Thr Lys Leu
    1040                1045                1050

Ile Leu  Thr Leu Met Glu Lys  Leu Val Tyr Pro Asn  Pro Ala Ala

1055                1060                1065

Tyr Lys  Asp Gln Leu Thr Arg  Phe Ser Ser Leu Asn  His Lys Arg
    1070                1075                1080

Tyr Tyr  Lys Leu Ala Leu Lys  Ala Ser Glu Leu Leu  Glu Gln Thr
    1085                1090                1095

Lys Leu  Ser Glu Leu Arg Thr  Ser Ile Ala Arg Ser  Leu Ser Glu
    1100                1105                1110

Leu Glu  Met Phe Thr Glu Glu  Arg Thr Ala Ile Ser  Glu Ile Met
    1115                1120                1125

Gly Asp  Leu Val Thr Ala Pro  Leu Pro Val Glu Asp  Ala Leu Val
    1130                1135                1140

Ser Leu  Phe Asp Cys Ser Asp  Gln Thr Leu Gln Gln  Arg Val Ile
    1145                1150                1155

Glu Thr  Tyr Ile Ser Arg Leu  Tyr Gln Pro His Leu  Val Lys Asp
    1160                1165                1170

Ser Ile  Gln Leu Lys Tyr Gln  Glu Ser Gly Val Ile  Ala Leu Trp
    1175                1180                1185

Glu Phe  Ala Glu Ala His Ser  Glu Lys Arg Leu Gly  Ala Met Val
    1190                1195                1200

Ile Val  Lys Ser Leu Glu Ser  Val Ser Ala Ala Ile  Gly Ala Ala
    1205                1210                1215

Leu Lys  Gly Thr Ser Arg Tyr  Ala Ser Ser Glu Gly  Asn Ile Met
    1220                1225                1230

His Ile  Ala Leu Leu Gly Ala  Asp Asn Gln Met His  Gly Thr Glu
    1235                1240                1245

Asp Ser  Gly Asp Asn Asp Gln  Ala Gln Val Arg Ile  Asp Lys Leu
    1250                1255                1260

Ser Ala  Thr Leu Glu Gln Asn  Thr Val Thr Ala Asp  Leu Arg Ala
    1265                1270                1275

Ala Gly  Val Lys Val Ile Ser  Cys Ile Val Gln Arg  Asp Gly Ala
    1280                1285                1290

Leu Met Pro Met Arg His Thr Phe Leu Leu Ser Asp Glu Lys Leu
    1295                1300                1305

Cys Tyr Glu Glu Glu Pro Val Leu Arg His Val Glu Pro Pro Leu
    1310                1315                1320

Ser Ala Leu Leu Glu Leu Gly Lys Leu Lys Val Lys Gly Tyr Asn
    1325                1330                1335

Glu Val Lys Tyr Thr Pro Ser Arg Asp Arg Gln Trp Asn Ile Tyr
    1340                1345                1350

Thr Leu Arg Asn Thr Glu Asn Pro Lys Met Leu His Arg Val Phe
    1355                1360                1365

Phe Arg Thr Leu Val Arg Gln Pro Gly Ala Ser Asn Lys Phe Thr
    1370                1375                1380

Ser Gly Asn Ile Ser Asp Val Glu Val Gly Gly Ala Glu Glu Ser
    1385                1390                1395

Leu Ser Phe Thr Ser Ser Ser Ile Leu Arg Ser Leu Met Thr Ala
    1400                1405                1410

Ile Glu Glu Leu Glu Leu His Ala Ile Arg Thr Gly His Ser His
    1415                1420                1425

Met Phe Leu Cys Ile Leu Lys Glu Gln Lys Leu Leu Asp Leu Val
    1430                1435                1440

Pro Val Ser Gly Asn Lys Val Val Asp Ile Gly Gln Asp Glu Ala
    1445                1450                1455

Thr Ala Cys Leu Leu Leu Lys Glu Met Ala Leu Gln Ile His Glu
    1460                1465                1470

Leu Val Gly Ala Arg Met His His Leu Ser Val Cys Gln Trp Glu
    1475                1480                1485

Val Lys Leu Lys Leu Asp Ser Asp Gly Pro Ala Ser Gly Thr Trp
    1490                1495                1500

Arg Val Val Thr Thr Asn Val Thr Ser His Thr Cys Thr Val Asp
    1505                1510                1515

Ile Tyr Arg Glu Val Glu Asp Thr Glu Ser Gln Lys Leu Val Tyr
    1520                1525                1530

His Ser Ala Pro Ser Ser Ser Gly Pro Leu His Gly Val Ala Leu
    1535                1540                1545

Asn Thr Pro Tyr Gln Pro Leu Ser Val Ile Asp Leu Lys Arg Cys
    1550                1555                1560

Ser Ala Arg Asn Asn Arg Thr Thr Tyr Cys Tyr Asp Phe Pro Leu
    1565                1570                1575

Ala Phe Glu Thr Ala Val Gln Lys Ser Trp Ser Asn Ile Ser Ser
    1580                1585                1590

Asp Thr Asn Arg Cys Tyr Val Lys Ala Thr Glu Leu Val Phe Ala
    1595                1600                1605

His Lys Asn Gly Ser Trp Gly Thr Pro Val Ile Pro Met Glu Arg
    1610                1615                1620

Pro Ala Gly Leu Asn Asp Ile Gly Met Val Ala Trp Ile Leu Asp
    1625                1630                1635

Met Ser Thr Pro Glu Tyr Pro Asn Gly Arg Gln Ile Val Val Ile
    1640                1645                1650

Ala Asn Asp Ile Thr Phe Arg Ala Gly Ser Phe Gly Pro Arg Glu
    1655                1660                1665

Asp Ala Phe Phe Glu Thr Val Thr Asn Leu Ala Cys Glu Arg Lys
    1670                1675                1680

Leu Pro Leu Ile Tyr Leu Ala Ala Asn Ser Gly Ala Arg Ile Gly
    1685                1690                1695

Ile Ala Asp Glu Val Lys Ser Cys Phe Arg Val Gly Trp Ser Asp
    1700                1705                1710

Asp Gly Ser Pro Glu Arg Gly Phe Gln Tyr Ile Tyr Leu Thr Glu
    1715                1720                1725

Glu Asp His Ala Arg Ile Ser Ala Ser Val Ile Ala His Lys Met
    1730                1735                1740

Gln Leu Asp Asn Gly Glu Ile Arg Trp Val Ile Asp Ser Val Val
    1745                1750                1755

Gly Lys Glu Asp Gly Leu Gly Val Glu Asn Ile His Gly Ser Ala
    1760                1765                1770

Ala Ile Ala Ser Ala Tyr Ser Arg Ala Tyr Glu Glu Thr Phe Thr
    1775                1780                1785

Leu Thr Phe Val Thr Gly Arg Thr Val Gly Ile Gly Ala Tyr Leu
    1790                1795                1800

Ala Arg Leu Gly Ile Arg Cys Ile Gln Arg Thr Asp Gln Pro Ile
    1805                1810                1815

Ile Leu Thr Gly Phe Ser Ala Leu Asn Lys Leu Leu Gly Arg Glu
    1820                1825                1830

Val Tyr Ser Ser His Met Gln Leu Gly Gly Pro Lys Ile Met Ala
    1835                1840                1845

Thr Asn Gly Val Val His Leu Thr Val Ser Asp Asp Leu Glu Gly
    1850                1855                1860

Val Ser Asn Ile Leu Arg Trp Leu Ser Tyr Val Pro Ala Asn Ile
    1865                1870                1875

Gly Gly Pro Leu Pro Ile Thr Lys Ser Leu Asp Pro Pro Asp Arg
    1880                1885                1890

Pro Val Ala Tyr Ile Pro Glu Asn Thr Cys Asp Pro Arg Ala Ala
    1895                1900                1905

Ile Ser Gly Ile Asp Asp Ser Gln Gly Lys Trp Leu Gly Gly Met
    1910                1915                1920

Phe Asp Lys Asp Ser Phe Val Glu Thr Phe Glu Gly Trp Ala Lys
    1925                1930                1935

Ser Val Val Thr Gly Arg Ala Lys Leu Gly Gly Ile Pro Val Gly
    1940                1945                1950

Val Ile Ala Val Glu Thr Gln Thr Met Met Gln Leu Ile Pro Ala
    1955                1960                1965

Asp Pro Gly Gln Leu Asp Ser His Glu Arg Ser Val Pro Arg Ala
    1970                1975                1980

Gly Gln Val Trp Phe Pro Asp Ser Ala Thr Lys Thr Ala Gln Ala
    1985                1990                1995

Met Leu Asp Phe Asn Arg Glu Gly Leu Pro Leu Phe Ile Leu Ala

2000                2005                2010

Asn Trp Arg Gly Phe Ser Gly Gly Gln Arg Asp Leu Phe Glu Gly
2015                2020                2025

Ile Leu Gln Ala Gly Ser Thr Ile Val Glu Asn Leu Arg Thr Tyr
2030                2035                2040

Asn Gln Pro Ala Phe Val Tyr Ile Pro Lys Ala Ala Glu Leu Arg
2045                2050                2055

Gly Gly Ala Trp Val Val Ile Asp Ser Lys Ile Asn Pro Asp Arg
2060                2065                2070

Ile Glu Phe Tyr Ala Glu Arg Thr Ala Lys Gly Asn Val Leu Glu
2075                2080                2085

Pro Gln Gly Leu Ile Glu Ile Lys Phe Arg Ser Glu Glu Leu Gln
2090                2095                2100

Glu Cys Met Gly Arg Leu Asp Pro Glu Leu Ile Asn Leu Lys Ala
2105                2110                2115

Lys Leu Leu Gly Ala Lys His Glu Asn Gly Ser Leu Ser Glu Ser
2120                2125                2130

Glu Ser Leu Gln Lys Ser Ile Glu Ala Arg Lys Lys Gln Leu Leu
2135                2140                2145

Pro Leu Tyr Thr Gln Ile Ala Val Arg Phe Ala Glu Leu His Asp
2150                2155                2160

Thr Ser Leu Arg Met Ala Ala Lys Gly Val Ile Lys Lys Val Val
2165                2170                2175

Asp Trp Glu Asp Ser Arg Ser Phe Phe Tyr Lys Arg Leu Arg Arg
2180                2185                2190

Arg Ile Ser Glu Asp Val Leu Ala Lys Glu Ile Arg Gly Val Ser
2195                2200                2205

Gly Lys Gln Phe Ser His Gln Ser Ala Ile Glu Leu Ile Gln Lys
2210                2215                2220

Trp Tyr Leu Ala Ser Lys Gly Ala Glu Thr Gly Asn Thr Glu Trp
2225                2230                2235

Asp Asp Asp Asp Ala Phe Val Ala Trp Arg Glu Asn Pro Glu Asn
    2240                2245                2250

Tyr Gln Glu Tyr Ile Lys Glu Leu Arg Ala Gln Arg Val Ser Gln
    2255                2260                2265

Leu Leu Ser Asp Val Ala Asp Ser Ser Pro Asp Leu Glu Ala Leu
    2270                2275                2280

Pro Gln Gly Leu Ser Met Leu Leu Glu Lys Met Asp Pro Ser Arg
    2285                2290                2295

Arg Ala Gln Phe Val Glu Glu Val Lys Lys Ala Leu Lys
    2300                2305                2310

## Description

### BACKGROUND OF THE INVENTION

Rice is one of the most important food crops in the world, particularly in Asia. Rice is a cereal grain produced by plants in the genus *Oryza.* The two most frequently cultivated species are *Oryza sativa* and *Oryza glaberrima*, with *O. sativa* being the most frequently cultivated domestic rice. In addition to the two domestic species, the genus *Oryza* contains more than 20 wild species. One of these wild species, *Oryza rufipogon* ("red rice" also referred to as *Oryza sativa* subsp. *rufipogon*) presents a major problem in commercial cultivation. Red rice produces red coated seeds. After harvest, rice seeds are milled to remove their hull. After milling, domestic rice is white while wild red rice appears discolored. The presence of discolored seeds reduces the value of the rice crop. Since red rice belongs to the same species as cultivated rice (*Oryza sativa*), their genetic makeup is very similar. This genetic similarity has made herbicidal control of red rice difficult.

Domestic rice tolerant to imidazolinone herbicides have been developed and are currently marketed under the tradename CLEARFIELD^{®}. Imidazolinone herbicides inhibit a plant's acetohydroxyacid synthase (AHAS) enzyme. When cultivating CLEARFIELD^{®} rice, it is possible to control red rice and other weeds by application of imidazolinone herbicides. Unfortunately, imidazolinone herbicide-tolerant red rice and weeds have developed.

Acetyl-Coenzyme A carboxylase (ACCase; EC 6.4.1.2) enzymes synthesize malonyl-CoA as the start of the de novo fatty acid synthesis pathway in plant chloroplasts. ACCase in grass chloroplasts is a multifunctional, nuclear-genome-encoded, very large, single polypeptide, transported into the plastid via an N-terminal transit peptide. The active form in grass chloroplasts is a homomeric protein, likely a homodimer.

ACCase enzymes in grasses are inhibited by three classes of herbicidal active ingredients. The two most prevalent classes are aryloxyphenoxypropanoates ("FOPs") and cyclohexanediones ("DIMs"). In addition to these two classes, a third class phenylpyrazolines ("DENs") has been described.

A number of ACCase-inhibitor-tolerance (AIT) mutations have been found in monocot weed species exhibiting tolerance toward one or more DIM or FOP herbicides. Further, an AIT maize has been marketed by BASF. All such mutations are found in the carboxyltransferase domain of the ACCase enzyme, and these appear to be located in a substrate binding pocket, altering access to the catalytic site.

DIMs and FOPs are important herbicides and it would be advantageous if rice could be provided that exhibits tolerance to these classes of herbicide. Currently, these classes of herbicide are of limited value in rice agriculture. In some cases, herbicide-tolerance-inducing mutations create a severe fitness penalty in the tolerant plant. Therefore, there remains a need in the art for an AIT rice that also exhibits no fitness penalty. This need and others are met by the present invention.

### BRIEF SUMMARY OF THE INVENTION

The present invention relates to herbicide-tolerant plants and methods of producing and treating herbicide-tolerant plants. In one embodiment, the present invention provides a rice plant tolerant to at least one herbicide that inhibits acetyl-Coenzyme A carboxylase activity at levels of herbicide that would normally inhibit the growth of a rice plant. Typically, an herbicide-tolerant rice plant of the invention expresses an acetyl-Coenzyme A carboxylase (ACCase) in which the amino acid sequence differs from an amino acid sequence of an acetyl-Coenzyme A carboxylase of a wild-type rice plant. By convention, mutations within monocot ACCase amino acid residues are typically referred to in reference to their position in the *Alopecurus myosuroides* (blackgrass) plastidic monomeric ACCase sequence (Genbank CAC84161.1) and denoted with an *(Am).* Examples of amino acid positions at which an acetyl-Coenzyme A carboxylase of an herbicide-tolerant plant of the invention differs from the acetyl-Coenzyme A carboxylase of the corresponding wild-type plant include, but are not limited to, one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098*(Am)*. Examples of differences at these amino acid positions include, but are not limited to, one or more of the following: the amino acid at position 1,781 *(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, the present invention provides a rice plant expressing an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position *2,095(Am)* is glutamic acid; the amino acid at position 2,096*(Am)* is alanine, or serine; or the amino acid at position 2,098*(Am)* is alanine, glycine, proline, histidine, or serine.

The present invention also provides methods of producing herbicide-tolerant plants and plants produced by such methods. An example of a plant produced by the methods of the invention is an herbicide-tolerant rice plant which is tolerant to at least one herbicide that inhibits acetyl-Coenzyme A carboxylase activity at levels of herbicide that would normally inhibit the growth of said plant, wherein the herbicide-tolerant plant is produced by: a) obtaining cells from a plant that is not tolerant to the herbicide; b) contacting the cells with a medium comprising one or more acetyl-Coenzyme A carboxylase inhibitors; and c) generating an herbicide-tolerant plant from the cells. Herbicide-tolerant plants produced by methods of the invention include, but are not limited to, herbicide-tolerant plants generated by performing a), b) and c) above and progeny of a plant generated by performing a), b), and c) above. In one embodiment, cells used to practice methods of this type will be in the form of a callus.

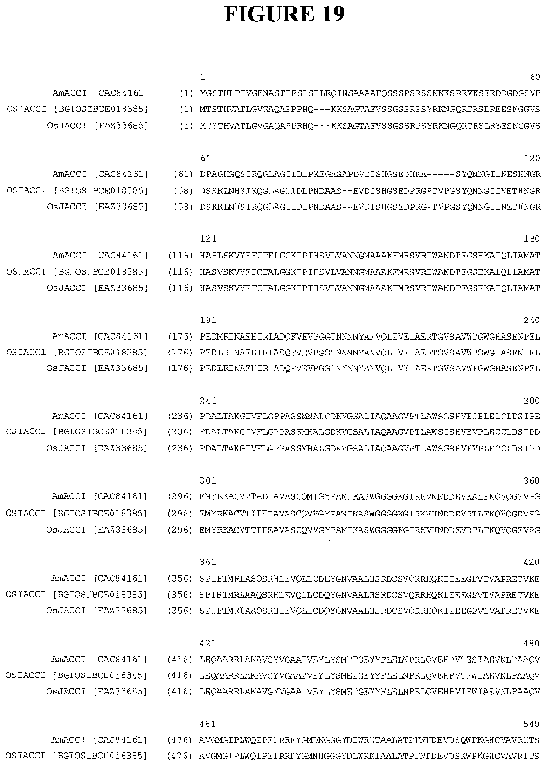

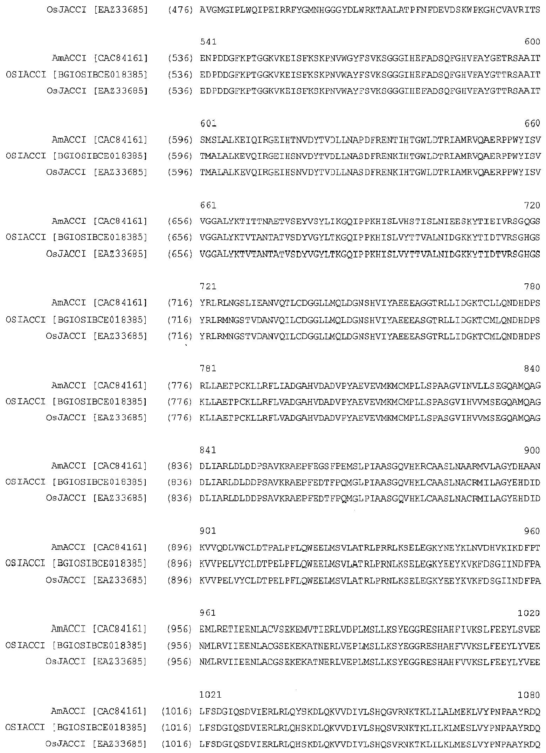

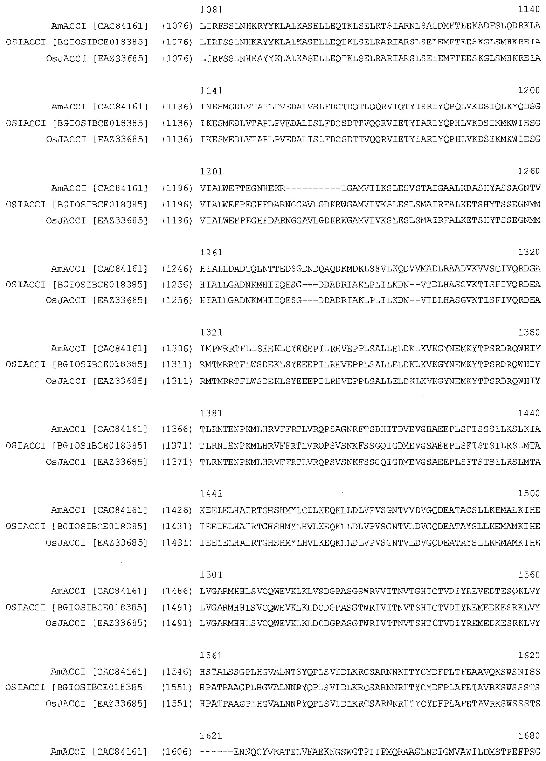

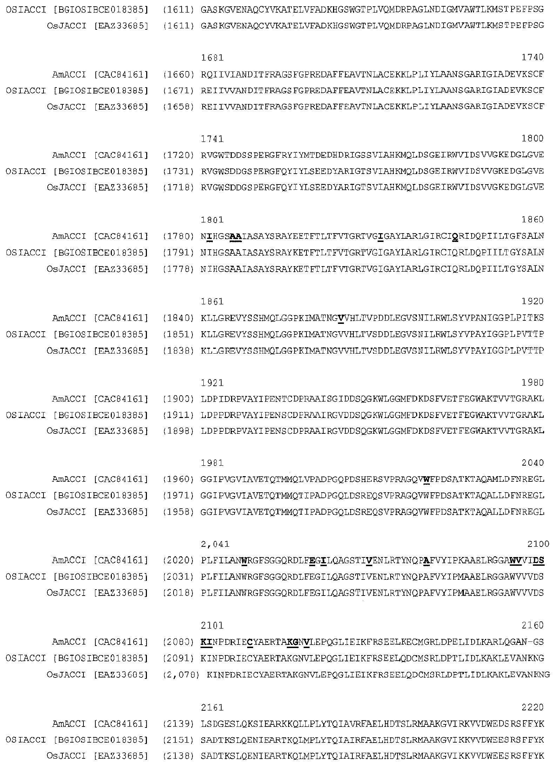

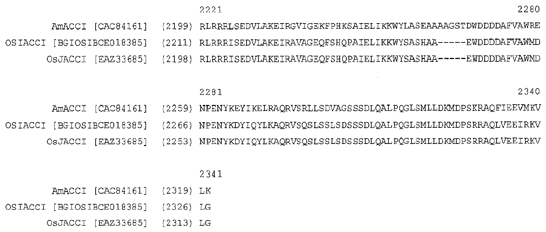

The present invention provides plants expressing acetyl-Coenzyme A carboxylase enzymes comprising defined amino acid sequences. For example, the present invention provides a rice plant, wherein one or more of the genomes of said rice plant encode a protein comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position *2,078(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Figure 19 below provides an alignment of the *Alopecurus myosuroides* acetyl-Coenzyme A carboxylase sequence (SEQ ID NO:1), the *Oryza sativa* Indical acetyl-Coenzyme A carboxylase sequence (SEQ ID NO:2) and the *Oryza sativa* Japonica acetyl-Coenzyme A carboxylase sequence (SEQ ID NO:3) with examples of positions where the wild type sequences may differ with sequences of the invention indicated.

In another embodiment, the present invention comprises seeds deposited in an acceptable depository in accordance with the Budapest Treaty, cells derived from such seeds, plants grown from such seeds and cells derived from such plants, progeny of plants grown from such seed and cells derived from such progeny. The growth of plants produced from deposited seed and progeny of such plants will typically be tolerant to acetyl-Coenzyme A carboxylase-inhibiting herbicides at levels of herbicide that would normally inhibit the growth of a corresponding wild-type plant. In one embodiment, the present invention provides a rice plant grown from a seed produced from a plant of any one of lines OsHPHI2, OsARWI1, OsARWI3, OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively. The present invention also encompasses mutants, recombinants, and/or genetically engineered derivatives prepared from a plant of any one of lines OsHPHI2, OsARWI1, OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, as well as any progeny of the plant grown or bred from a plant of any one of lines OsHPHI2, OsARWI1, OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, so long as such plants or progeny have the herbicide tolerance characteristics of the plant grown from a a plant of any one of lines OsHPHI2, OsARWI1, OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively. The present invention also encompasses cells cultured from such seeds and plants and their progeny produced from the cultured cells.

An herbicide-tolerant plant of the invention may be a member of the species *O*. *sativa.* Herbicide-tolerant plants of the invention are typically tolerant to aryloxyphenoxypropionate herbicides, cyclohexanedione herbicides, phenylpyrazoline herbicides or combinations thereof at levels of herbicide that would normally inhibit the growth of a corresponding wild-type plant, for example, a rice plant. In some embodiments, an herbicide-tolerant plant of the invention is not a GMO-plant. The present invention also provides an herbicide-tolerant plant that is mutagenized, for example, a mutagenized rice plant. The present invention also encompasses cells derived from the plants and seeds of the herbicide-tolerant plants described above.

The present invention provides methods for controlling growth of weeds. In one embodiment, the present invention provides a method of controlling growth of weeds in vicinity to rice plants. Such methods may comprise applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide. Methods of the invention may be practiced with any herbicide that interferes with acetyl-Coenzyme A carboxylase activity including, but not limited to, aryloxyphenoxypropionate herbicides, cyclohexanedione herbicides, phenylpyrazoline herbicides or combinations thereof.

The present invention provides a method for controlling growth of weeds in vicinity to rice plants. One example of such methods may comprise applying one or more herbicides to the weeds and to the rice plants at levels of herbicide that would normally inhibit the growth of a rice plant, wherein at least one herbicide inhibits acetyl-Coenzyme A carboxylase activity. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

The present invention encompasses a method for controlling growth of weeds. One example of such methods may comprise (a) crossing an herbicide-tolerant rice plant with other rice germplasm, and harvesting the resulting hybrid rice seed; (b) planting the hybrid rice seed; and (c) applying one or more acetyl-Coenzyme A carboxylase-inhibiting herbicides to the hybrid rice and to the weeds in vicinity to the hybrid rice at levels of herbicide that would normally inhibit the growth of a rice plant. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

In another embodiment, the present invention includes a method for selecting herbicide-tolerant rice plants. One example of such methods may comprise (a) crossing an herbicide-tolerant rice plant with other rice germplasm, and harvesting the resulting hybrid rice seed; (b) planting the hybrid rice seed; (c) applying one or more herbicides to the hybrid rice at levels of herbicide that would normally inhibit the growth of a rice plant, wherein at least one of the herbicides inhibits acetyl-Coenzyme A carboxylase; and (d) harvesting seeds from the rice plants to which herbicide has been applied. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

The present invention also encompasses a method for growing herbicide-tolerant rice plants. One example of such a method comprises (a) planting rice seeds; (b) allowing the rice seeds to sprout; (c) applying one or more herbicides to the rice sprouts at levels of herbicide that would normally inhibit the growth of a rice plant, wherein at least one of the herbicides inhibits acetyl-Coenzyme A carboxylase. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

In one embodiment, the present invention provides a seed of an herbicide-tolerant rice plant. Such seed may be used to grow herbicide-tolerant rice plants, wherein a plant grown from the seed is tolerant to at least one herbicide that inhibits acetyl-Coenzyme A carboxylase activity at levels of herbicide that would normally inhibit the growth of a rice plant. Examples of herbicides to which plants grown from seeds of the invention would be tolerant include but are not limited to, aryloxyphenoxypropionate herbicides, cyclohexanedione herbicides, phenylpyrazoline herbicides or combinations thereof.

In another embodiment, the present invention provides a seed of a rice plant, wherein a plant grown from the seed expresses an acetyl-Coenzyme A carboxylase (ACCase) in which the amino acid sequence differs from an amino acid sequence of an acetyl-Coenzyme A carboxylase of a wild-type rice plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, *2*,*088(Am)*, *2*,*095(Am)*, *2*,*096(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, a plant grown from the seed may express an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

The present invention encompasses seeds of specific herbicide-tolerant cultivars. One example of such seeds is a seed of rice cultivar Indical, wherein a representative sample of seed of said cultivar was deposited under ATCC Accession No. PTA-10267, PTA-10568, PTA-10569, or PTA-10570. Another example of such seeds are those of an herbicide-tolerant Nipponbare cultivar, wherein a representative sample of seed of said cultivar was deposited under ATCC Accession No. PTA-10571. The present invention also encompasses a rice plant, or a part thereof, produced by growing the seeds as well as a tissue culture of cells produced from the seed. Tissue cultures of cells may be produced from a seed directly or from a part of a plant grown from a seed, for example, from the leaves, pollen, embryos, cotyledons, hypocotyls, meristematic cells, roots, root tips, pistils, anthers, flowers and/or stems. The present invention also includes plants and their progeny that have been generated from tissue cultures of cells. Such plants will typically have all the morphological and physiological characteristics of cultivar Indical.

The present invention also provides methods for producing rice seed. Such methods may comprise crossing an herbicide-tolerant rice plant with other rice germplasm; and harvesting the resulting hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

The present method also comprises methods of producing F1 hybrid rice seed. Such methods may comprise crossing an herbicide-tolerant rice plant with a different rice plant; and harvesting the resultant F1 hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

The present method also comprises methods of producing F1 hybrid plants. Such methods may comprise crossing an herbicide-tolerant plant with a different plant; and harvesting the resultant F1 hybrid seed and growing the resultant F1 hybrid plant, wherein the herbicide-tolerant plant is tolerant to aryloxyphenoxypropionate herbicides, cyclohexanedione herbicides, phenylpyrazoline herbicides or combinations thereof at levels of herbicide that would normally inhibit the growth of a plant.

The present invention also provides methods of producing herbicide-tolerant rice plants that may also comprise a transgene. One example of such a method may comprise transforming a cell of a rice plant with a transgene, wherein the transgene encodes an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods of the invention, for example, the cell may be in the form of a callus. In some embodiments, the transgene may comprise a nucleic acid sequence encoding an amino acid sequence comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. The present invention also encompasses plants produced by such methods. Another example of a method of producing an herbicide-tolerant plant comprising a transgene may comprise transforming a cell of a rice plant with a transgene encoding an enzyme that confers herbicide tolerance, wherein the cell was produced from a rice plant or seed thereof expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods of the invention, for example, the cell may be in the form of a callus. The present invention also encompasses herbicide-tolerant plants produced by such methods.

In one embodiment, the present invention comprises methods of producing recombinant plants. An example of a method for producing a recombinant rice plant may comprise transforming a cell of a rice plant with a transgene, wherein the cell was produced from a rice plant expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods of the invention, for example, the cell may be in the form of a callus. A transgene for use in the methods of the invention may comprise any desired nucleic acid sequence, for example, the transgene may encode a protein. In one example, the transgene may encode an enzyme, for example, an enzyme that modifies fatty acid metabolism and/or carbohydrate metabolism. Examples of suitable enzymes include but are not limited to, fructosyltransferase, levansucrase, alpha-amylase, invertase and starch branching enzyme or encoding an antisense of stearyl-ACP desaturase. The present invention also encompasses recombinant plants produced by methods of the invention.

Methods of the invention may be used to produce a plant, e.g., a rice plant, having any desired traits. An example of such a method may comprise: (a) crossing a rice plant that is tolerant to aryloxyphenoxypropionate herbicides, cyclohexanedione herbicides, phenylpyrazoline herbicides or combinations thereof at levels of herbicide that would normally inhibit the growth of a rice plant with a plant of another rice cultivar that comprises the desired trait to produce progeny plants; (b) selecting one or more progeny plants that have the desired trait to produce selected progeny plants; (c) crossing the selected progeny plants with the herbicide-tolerant plants to produce backcross progeny plants; (d) selecting for backcross progeny plants that have the desired trait and herbicide tolerance; and (e) repeating steps (c) and (d) three or more times in succession to produce selected fourth or higher backcross progeny plants that comprise the desired trait and herbicide tolerance. Any desired trait may be introduced using the methods of the invention. Examples of traits that may be desired include, but are not limited to, male sterility, herbicide tolerance, drought tolerance insect resistance, modified fatty acid metabolism, modified carbohydrate metabolism and resistance to bacterial disease, fungal disease or viral disease. An example of a method for producing a male sterile rice plant may comprise transforming a rice plant tolerant to at least one herbicide that inhibits acetyl-Coenzyme A carboxylase activity at levels of herbicide that would normally inhibit the growth of a rice plant with a nucleic acid molecule that confers male sterility. The present invention also encompasses male sterile plants produced by such methods.

The present invention provides compositions comprising plant cells, for example, cells from a rice plant. One example of such a composition comprises one or more cells of a rice plant; and an aqueous medium, wherein the medium comprises a compound that inhibits acetyl-Coenzyme A carboxylase activity. In some embodiments, the cells may be derived from a rice plant tolerant to aryloxyphenoxypropionate herbicides, cyclohexanedione herbicides, phenylpyrazoline herbicides or combinations thereof at levels of herbicide that would normally inhibit the growth of a rice plant. Any compound that inhibits acetyl-Coenzyme A carboxylase activity may be used in the compositions of the invention, for example, one or more of aryloxyphenoxypropionate herbicides, cyclohexanedione herbicides, phenylpyrazoline herbicides and combinations thereof.

The present invention comprises nucleic acid molecules encoding all or a portion of an acetyl-Coenzyme A carboxylase enzyme. In some embodiments, the invention comprises a recombinant, mutagenized, synthetic, and/or isolated nucleic acid molecule encoding a rice acetyl-Coenzyme A carboxylase (ACCase) in which the amino acid sequence differs from an amino acid sequence of an acetyl-Coenzyme A carboxylase of a wild-type rice plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, a nucleic acid molecule of the invention may encode an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. In some embodiments, the invention comprises a recombinant, mutagenized, synthetic, and/or isolated nuceleic acid encoding a protein comprising all or a portion of a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is Arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

In one embodiment, the present invention provides an herbicide-tolerant, BEP clade plant. Typically such a plant is one having increased tolerance to an ACCase-inhibitor (ACCI) as compared to a wild-type variety of the plant. Such plants may be produced by a process comprising either:
(I) the steps of
   (a) providing BEP clade plant cells having a first, zero or non-zero level of ACCI tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant;
thereby obtaining an herbicide-tolerant, BEP clade plant.

In one embodiment, an herbicide-tolerant BEP clade plant of the invention is a BET subclade plant.

In one embodiment, an herbicide-tolerant BET subclade plant of the invention is a BET crop plant.

In some embodiments, an herbicide-tolerant plant of the invention may be a member of the Bambusoideae - Ehrhartoideae subclade. Any suitable medium for growing plant cells may be used in the practice of the invention. In some embodiments, the medium may comprise a mutagen while in other embodiments the medium does not comprise a mutagen. In some embodiments, an herbicide-tolerant plant of the invention may be a member of the subfamily Ehrhartoideae. Any suitable cells may be used in the practice of the methods of the invention, for example, the cells may be in the form of a callus. In some embodiments, an herbicide-tolerant plant of the invention may be a member of the genus *Oryza*, for example, may be a member of the species *O*. *sativa.*

The present invention includes herbicide-tolerant BEP clade plants produced by the above method. Such herbicide-tolerant plants may express an acetyl-Coenzyme A carboxylase (ACCase) in which the amino acid sequence differs from an amino acid sequence of an acetyl-Coenzyme A carboxylase of a corresponding wild-type BEP clade plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am),* 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. In some embodiments, the an herbicide-tolerant BEP clade plant of the invention may expresses an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is Arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

In one embodiment, the present invention also includes rice plants that are tolerant to ACCase inhibitors by virtue of having only one substitution in its plastidic ACCase as compared to the corresponding wild-type ACCase. In yet another embodiment, the invention includes rice plants that are tolerant to ACCase inhibitors by virtue of having two or more substitutions in its plastidic ACCase as compared to the corresponding wild-type ACCase.

In one embodiment, the present invention provides rice plants that are tolerant to ACCase inhibitors, by virtue of having two or more substitution in its plastidic ACCase as compared to the corresponding wild-type ACCase, wherein the substitutions are at amino acid positions selected from the group consisting of 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am),* or 2,098*(Am)*.

In one embodiment, the present invention provides rice plants wherein the rice plants comprise plastidic ACCase that is not transgenic. In one embodiment, the present invention provides plants wherein the plants comprise a rice plastidic ACCase that is transgenic.

In one embodiment, the present invention provides method for controlling growth of weeds within the vicinity of a rice plant as described herein, comprising applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide.

In one embodiment, the present invention provides methods for producing seed comprising: (i) planting seed produced from a plant of the invention, (ii) growing plants from the seed and (ii) harvesting seed from the plants.

The present invention also encompasses herbicide-tolerant BEP clade plants produced by the process of (a) crossing or back-crossing a plant grown from a seed of an herbicide-tolerant BEP clade plant produced as described above with other germplasm; (b) growing the plants resulting from said crossing or back-crossing in the presence of at least one herbicide that normally inhibits acetyl-Coenzyme A carboxylase, at levels of the herbicide that would normally inhibit the growth of a plant; and (c) selecting for further propagation plants resulting from said crossing or back-crossing, wherein the plants selected are plants that grow without significant injury in the presence of the herbicide.

The present invention also encompasses a recombinant, mutagenized, synthetic, and/or isolated nucleic acid molecule comprising a nucleotide sequence encoding a mutagenized acetyl-Coenzyme A carboxylase of a plant in the BEP clade of the Family Poaceae, in which the amino acid sequence of the mutagenized acetyl-Coenzyme A carboxylase differs from an amino acid sequence of an acetyl-Coenzyme A carboxylase of the corresponding wild-type plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098*(Am).* Such a nucleic acid molecule may b produced by a process comprising either:
(I) the steps of
   (a) providing BEP clade plant cells having a first, zero or non-zero level of ACCase-inhibitor (ACCI) tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant;
thereby obtaining an herbicide-tolerant, BEP clade plant; and isolating a nucleic acid from the herbicide-tolerant BEP clade plant.

In one embodiment, the invention encompasses methods of screening, isolating, identifying, and/or characterizing herbicide tolerant mutations in monocot plastidic ACCases. In one embodiment, the invention encompasses the use of calli, or plant cell lines. In other embodiments, the invention encompasses performing the culturing of plant material or cells in a tissue culture environment. In yet other embodiments, the invention encompasses the presence of a nylon membrane in the tissue culture environment. In other embodiments, the tissue culture environment comprises liquid phase media while in other embodiments, the environment comprises semi-solid media. In yet other embodiments, the invention encompasses culturing plant material in the presence of herbicide (e.g., cycloxydim) in liquid media followed by culturing in semi-solid media with herbicide. In yet other embodiments, the invention encompasses culturing plant material in the presence of herbicide in semi-solid media followed by culturing in liquid media with herbicide.

In some embodiments, the invention encompasses the direct application of a lethal dose of herbicide (e.g., cycloxydim). In other embodiment, the invention encompasses the step-wise increase in herbicide dose, starting with a sub-lethal dose. In other embodiments, the invention encompasses at least one, at least two, at least three, at least four, at least five, at least six, at least seven, at least eight, or more herbicides in one step, or concurrently.

In other embodiments, the mutational frequency is determined by the number of mutant herbicide-tolerant clones as a fraction of the number of the individual calli used in the experiment. In some embodiments, the invention encompasses a mutational frequency of at least 0.03% or higher. In some embodiments, the invention encompasses mutational frequencies of at least 0.03%, at least 0.05%, at least 0.10%, at least 0.15%, at least 0.20%, at least 0.25%, at least 0.30%, at least 0.35%, at least 0.40% or higher. In other embodiments, the invention encompasses mutational frequencies that are at least 2 fold, at least 3 fold, at least 4 fold, at least 5 fold, at least 6 fold, at least 7 fold, at least 8 fold, at least 9 fold, at least 10 fold or higher than other methods of screening, isolating, identifying, and/or characterizing herbicide tolerant mutations in monocot plastidic ACCases.

In some embodiments, the methods of the invention encompass identifying the herbicide tolerant mutation(s) in the ACCase. In further embodiments, the invention comprises recapitulating the herbicide tolerant mutation(s) in monocot plant cells.

In some embodiments, the invention encompasses an isolated cell or tissue said cell or tissue of plant origin having: a) a deficiency in ACCase activity derived from a host ACCase (i.e., endogenous) gene; and b) an ACCase activity from a monocot-derived plastidic ACCase gene.

### Monocot sources of ACCase

In other embodiments, the invention encompasses plastidic ACCases or portions thereof from the monocot family of plants as described herein.

In other embodiments, the invention encompasses screening for herbicide-tolerant mutants of monocot plastidic ACCase in host plant cells.

In other embodiments, the invention encompasses the use of prepared host cells to screen for herbicide-tolerant mutants of monocot plastidic ACCase. In some embodiments, the invention provides a host cell which is devoid of plastidic ACCase activity. In other embodiments, the host cells of the invention express a monocot plastidic ACCase which is herbicide sensitive.

In other embodiments, methods of the invention comprise host cells deficient in ACCase activity due to a mutation of the genomic plastidic ACCase gene which include a single point mutation, multiple point mutations, a partial deletion, a partial knockout, a complete deletion and a complete knockout. In another embodiment, genomic plastidic ACCase activity is reduced or ablated using other molecular biology techniques such as RNAi, siRNA or antisense RNA. Such molecular biology techniques are well known in the art. In yet other embodiments, genomic ACCase derived activity may be reduced or ablated by a metabolic inhibitor of ACCase.

In some embodiments, the host cell is a monocot plant host cell.

In yet other embodiments, the invention encompasses a method of making a transgenic plant cell comprising: a) isolating a cell having a monocot plant origin; b) inactivating at least one copy of a genomic ACCase gene; c) providing a monocot-derived plastidic ACCase gene to said cell; d) isolating the cell comprising the monocot-derived plastidic ACCase gene; and optionally; e) inactivating at least additional copy of a genomic ACCase gene and wherein said cell is deficient in ACCase activity provided by the genomic ACCase gene.

In one embodiment, the the cycloxydim-tolerant mutational frequency is greater than 0.03 %.

In one embodiment, the present invention provides a method for screening, wherein cycloxydim-tolerant plant cells or tissues are also tolerant to other ACCase inhibitors.

In one embodiment, the present invention provides a method for screening, wherein the cycloxydim-tolerant plant cells or tissues comprise only one mutation not present in the monocot plastidic ACCase prior to culturing in the presence of the herbicide.

In one embodiment, the present invention provides a method for screening, wherein the cycloxydim-tolerant plant cells or tissues comprise two or more mutations not present in the monocot plastidic ACCase prior to culturing in the presence of the herbicide.

In one embodiment, the present invention provides a method for screening, wherein the cycloxydim is present at a sub-lethal dose.

In one embodiment, the present invention provides a method for screening, wherein the culturing in the presence of cycloxydim is performed in step-wise or gradual increase in cycloxydim concentrations.

In one embodiment, the present invention provides a method for screening, wherein the method comprises culturing of cells on a membrane. In a preferred embodiment, the present invention provides a method for screening comprises culturing of cells on a nylon membrane.

In one embodiment, the present invention provides a method for screening cycloxydim-tolerant plant cells, wherein the culturing of cells is in liquid media or semi-solid media.

In one embodiment, the present invention provides a method for screening, wherein the method further comprises identification of the at least one mutation not present in the exogenous monocot plastidic ACCase prior to culturing in the presence of the cycloxidim.

In one embodiment, the present invention provides a method for screening, wherein said monocot is rice.

In one embodiment, the present invention provides a method for screening, wherein said exogenous monocot plastidic ACCase is from rice.

### BRIEF DESCRIPTION OF THE DRAWINGS

Figure 1 is a bar graph showing relative growth rice calli derived from *Oryza sativa* subsp. *indica* grown in the presence of difference selection levels of herbicide. Figure 1A shows the results obtained with tepraloxydim, Figure 1B shows the results obtained with sethoxydim, and Figure 1C shows the results obtained with cycloxydim.
Figure 2 is a diagram of the selection process used to produce herbicide-tolerant rice plants.
Figure 3 shows photographs of plants taken one week after treatment with herbicide.
Figure 4 shows photographs of plants taken two weeks after treatment with herbicide.
Figure 5 provides the amino acid sequence of acetyl-coenzyme A carboxylase from *Alopecurus myosuroides* (GenBank accession number CAC84161).
Figure 6 provides the mRNA encoding acetyl-coenzyme A carboxylase from *Alopecurus myosuroides* (GenBank accession number AJ310767 region: 157..7119) (SEQ ID NO:4).
Figure 7A provides the genomic nucleotide sequence for *Oryza sativa* Indica & Japonica acetyl-Coenzyme A carboxylase gene (SEQ ID NO:5).
Figure 7B provides the nucleotide sequence encoding *Oryza sativa* Indica & Japonica acetyl-Coenzyme A carboxylase (SEQ ID NO:6).
Figure 7C provides the amino acid sequence of *Oryza sativa* Indica acetyl-Coenzyme A carboxylase (SEQ ID NO:3).
Figure 8A provides the nucleotide sequence encoding *Zea mays* acetyl-Coenzyme A carboxylase (SEQ ID NO: 11).
Figure 8B provides the amino acid sequence of *Zea mays* acetyl-Coenzyme A carboxylase (SEQ ID NO:12).
Figure 9A provides the nucleotide sequence encoding *Zea mays* acetyl-Coenzyme A carboxylase (SEQ ID NO:13).
Figure 9B provides the amino acid sequence of *Zea mays* acetyl-Coenzyme A carboxylase (SEQ ID NO:14).
Figure 10A provides the nucleotide sequence encoding *Triticum aestivum* acetyl-Coenzyme A carboxylase (SEQ ID NO:15).
Figure 10B provides the amino acid sequence of *Triticum aestivum* acetyl-Coenzyme A carboxylase (SEQ ID NO:16).
Figure 11A provides the nucleotide sequence encoding *Setaria italica* acetyl-Coenzyme A carboxylase (SEQ ID NO:17).
Figure 11B provides the amino acid sequence of *Setaria italica* acetyl-Coenzyme A carboxylase (SEQ ID NO:18).
Figure 12A provides the nucleotide sequence encoding *Setaria italica* acetyl-Coenzyme A carboxylase (SEQ ID NO:19).
Figure 12B provides the amino acid sequence of *Setaria italica* acetyl-Coenzyme A carboxylase (SEQ ID NO:20).
Figure 13A provides the nucleotide sequence encoding *Setaria italica* acetyl-Coenzyme A carboxylase (SEQ ID NO:21).
Figure 13B provides the amino acid sequence of *Setaria italica* acetyl-Coenzyme A carboxylase (SEQ ID NO:22).
Figure 14A provides the nucleotide sequence encoding *Alopecurus myosuroides* acetyl-Coenzyme A carboxylase (SEQ ID NO:23).
Figure 14B provides the amino acid sequence of *Alopecurus myosuroides* acetyl-Coenzyme A carboxylase (SEQ ID NO:24).
Figure 15A provides the nucleotide sequence encoding *Aegilops tauschii* acetyl-Coenzyme A carboxylase (SEQ ID NO:25).
Figure 15B provides the amino acid sequence of *Aegilops tauschii* acetyl-Coenzyme A carboxylase (SEQ ID NO:26).
Figure 16 provides a comparison of single and double mutants.
Figure 17 provides a graph showing results for mutant rice versus various ACCase inhibitors.
Figure 18 provides *Alopecurus myosuroides* acetyl-Coenzyme A carboxylase amino acid sequence (GenBank accession no. CAC84161). Amino acids that may be altered in the acetyl-Coenzyme A carboxylase enzymes of the invention are indicated in bold double underline.
Figure 19 provides amino acid sequence of wild-type *Oryza sativa* acetyl-Coenzyme A carboxylases aligned with *Alopecurus myosuroides* acetyl-Coenzyme A carboxylase with some critical residues denoted.

### DETAILED DESCRIPTION OF THE INVENTION

### Definitions

As used herein, "tolerant" or "herbicide-tolerant" indicates a plant or portion thereof capable of growing in the presence of an amount of herbicide that normally causes growth inhibition in a non-tolerant (e.g., a wild-type) plant or portion thereof. Levels of herbicide that normally inhibit growth of a non-tolerant plant are known and readily determined by those skilled in the art. Examples include the amounts recommended by manufacturers for application. The maximum rate is an example of an amount of herbicide that would normally inhibit growth of a non-tolerant plant.

As used herein, "recombinant" refers to an organism having genetic material from different sources.

As used herein, "mutagenized" refers to an organism having an altered genetic material as compared to the genetic material of a corresponding wild-type organism, wherein the alterations in genetic material were induced and/or selected by human action. Examples of human action that can be used to produce a mutagenized organism include, but are not limited to, tissue culture of plant cells (e.g., calli) in sub-lethal concentrations of herbicides (e.g., acetyl-Coenzyme A carboxylase inhibitors such as cycloxydim or sethoxydim), treatment of plant cells with a chemical mutagen and subsequent selection with herbicides (e.g., acetyl-Coenzyme A carboxylase inhibitors such as cycloxydim or sethoxydim); or by treatment of plant cells with x-rays and subsequent selection with herbicides (e.g., acetyl-Coenzyme A carboxylase inhibitors such as cycloxydim or sethoxydim). Any method known in the art may be used to induce mutations. Methods of inducing mutations may induce mutations in random positions in the genetic material or may induce mutations in specific locations in the genetic material (i.e., may be directed mutagenesis techniques).

As used herein, a "genetically modified organism" (GMO) is an organism whose genetic characteristics have been altered by insertion of genetic material from another source organism or progeny thereof that retain the inserted genetic material. The source organism may be of a different type of organism (e.g., a GMO plant may contain bacterial genetic material) or from the same type of organism (e.g., a GMO plant may contain genetic material from another plant). As used herein, recombinant and GMO are considered synonyms and indicate the presence of genetic material from a different source whereas mutagenized indicates altered genetic material from a corresponding wild-type organism but no genetic material from another source organism.

As used herein, "wild-type" or "corresponding wild-type plant" means the typical form of an organism or its genetic material, as it normally occurs, as distinguished from mutagenized and/or recombinant forms.

For the present invention, the terms "herbicide-tolerant" and "herbicide-resistant" are used interchangeably and are intended to have an equivalent meaning and an equivalent scope. Similarly, the terms "herbicide-tolerance" and "herbicide-resistance" are used interchangeably and are intended to have an equivalent meaning and an equivalent scope. Similarly, the terms "tolerant" and "resistant" are used interchangeably and are intended to have an equivalent meaning and an equivalent scope.

As used herein in regard to herbicides useful in various embodiments hereof, terms such as auxinic herbicide, AHAS inhibitor, acetyl-Coenzyme A carboxylase (ACCase) inhibitor, PPO inhibitor, EPSPS inhibitor, imidazolinone, sulfonylurea, and the like, refer to those agronomically acceptable herbicide active ingredients (A.I.) recognized in the art. Similarly, terms such as fungicide, nematicide, pesticide, and the like, refer to other agronomically acceptable active ingredients recognized in the art.

When used in reference to a particular mutant enzyme or polypeptide, terms such as herbicide tolerant (HT) and herbicide tolerance refer to the ability of such enzyme or polypeptide to perform its physiological activity in the presence of an amount of an herbicide A.I. that would normally inactivate or inhibit the activity of the wild-type (non-mutant) version of said enzyme or polypeptide. For example, when used specifically in regard to an AHAS enzyme, or AHASL polypeptide, it refers specifically to the ability to tolerate an AHAS-inhibitor. Classes of AHAS-inhibitors include sulfonylureas, imidazolinones, triazolopyrimidines, sulfonylaminocarbonyltriazolinones, and pyrimidinyloxy[thio]benzoates.

As used herein, "descendant" refers to any generation plant.

As used herein, "progeny" refers to a first generation plant.

### Plants

The present invention provides herbicide-tolerant monocotyledonous plants of the grass family Poaceae. The family Poaceae may be divided into two major clades, the clade containing the subfamilies Bambusoideae, Ehrhartoideae, and Pooideae (the BEP clade) and the clade containing the subfamilies Panicoideae, Arundinoideae, Chloridoideae, Centothecoideae, Micrairoideae, Aristidoideae, and Danthonioideae (the PACCMAD clade). The subfamily Bambusoideae includes tribe *Oryzeae.* The present invention relates to plants of the BEP clade, in particular plants of the subfamilies Bambusoideae and Ehrhartoideae. Plants of the invention are typically tolerant to at least one herbicide that inhibits acetyl-Coenzyme A carboxylase activity as a result of expressing an acetyl-Coenzyme A carboxylase enzyme of the invention as described below. The BET clade includes subfamilies Bambusoideae, Ehrhartoideae, and group Triticodae and no other subfamily Pooideae groups. BET crop plants are plants grown for food or forage that are members of BET subclade, for example barley, corn, etc.

The present invention also provides commerially important herbicide-tolerant monocots, including Sugarcane (Saccharum spp.), as well as Turfgrasses, e.g., Poa pratensis (Bluegrass), Agrostis spp. (Bentgrass), Lolium spp. (Ryegrasses), Festuca spp. (Fescues), Zoysia spp. (Zoysia grass), Cynodon spp. (Bermudagrass), Stenotaphrum secundatum (St. Augustine grass), Paspalum spp. (Bahiagrass), Eremochloa ophiuroides (Centipedegrass), Axonopus spp. (Carpetgrass), Bouteloua dactyloides (Buffalograss), and Bouteloua var. spp. (Grama grass).

In one embodiment, the present invention provides herbicide-tolerant plants of the Bambusoideae subfamily. Such plants are typically tolerant to one or more herbicides that inhibit acetyl-Coenzyme A carboxylase activity. Examples of herbicide-tolerant plants of the subfamily Bambusoideae include, but are not limited to, those of the genera Arundinaria, Bambusa, Chusquea, Guadua, and Shibataea.

In one embodiment, the present invention provides herbicide-tolerant plants of the Ehrhartoideae subfamily. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Erharta*, *Leersia*, *Microlaena*, *Oryza*, and *Zizania.*

In one embodiment, the present invention provides herbicide-tolerant plants of the Pooideae subfamily. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Triticeae*, *Aveneae*, and *Poeae.*

In one embodiment, herbicide-tolerant plants of the invention are rice plants. Two species of rice are most frequently cultivated, *Oryza sativa* and *Oryza glaberrima.* Numerous subspecies of *Oryza sativa* are commercially important including *Oryza sativa* subsp. *indica*, *Oryza sativa* subsp. *japonica*, *Oryza sativa* subsp*. javanica*, *Oryza sativa* subsp. *glutinosa* (glutinous rice), *Oryza sativa* Aromatica group (e.g., basmati), and *Oryza sativa* (Floating rice group). The present invention encompasses herbicide-tolerant plants in all of the aforementioned species and subspecies.

In one embodiment, herbicide-tolerant plants of the invention are wheat plants. Two species of wheat are most frequently cultivated, *Triticum Triticum aestivum, and Triticum turgidum.* Numerous other species are commercially important including, but not limited to, *Triticum timopheevii*, *Triticum monococcum*, *Triticum zhukovskyi* and *Triticum urartu* and hybrids thereof. The present invention encompasses herbicide-tolerant plants in all of the aforementioned species and subspecies. Examples of T. aestivum subspecies included within the present invention are aestivum (common wheat), compactum (club wheat), macha (macha wheat), vavilovi (vavilovi wheat), spelta and sphaecrococcum (shot wheat). Examples of T. turgidum subspecies included within the present invention are turgidum, carthlicum, dicoccon, durum, paleocolchicuna, polonium, turanicum and dicoccoides. Examples of T. monococcum subspecies included within the present invention are monococcum (einkorn) and aegilopoides. In one embodiment of the present invention, the wheat plant is a member of the Triticum aestivum species, and more particularly, the CDC Teal cultivar.

In one embodiment, herbicide-tolerant plants of the invention are barley plants. Two species of barley are most frequently cultivated, *Hordeum vulgare* and *Hordeum arizonicum.* Numerous other species are commercially important including, but not limited, *Hordeum_bogdanii*, *Hordeum_brachyantherum*, *Hordeum brevisubulatum, Hordeum bulbosum, Hordeum comosum, Hordeum depressum, Hordeum intercedens, Hordeum jubatum, Hordeum marinum, Hordeum marinum, Hordeum parodii, Hordeum pusillum, Hordeum_secalinum*, and *Hordeum spontaneum.* The present invention encompasses herbicide-tolerant plants in all of the aforementioned species and subspecies.

In one embodiment, herbicide-tolerant plants of the invention are rye plants. Commercially important species include, but are not limited to,*Secale sylvestre, Secale strictum, Secale cereale, Secale vavilovii, Secale africanum, Secale ciliatoglume, Secale ancestrale,* and *Secale montanum.* The present invention encompasses herbicide-tolerant plants in all of the aforementioned species and subspecies.

In one embodiment, herbicide-tolerant plants of the invention are turf plants. Numerous commercially important species of Turf grass include *Zoysia japonica*, *Agrostris palustris, Poa pratensis, Poa annua*, *Digitaria sanguinalis, Cyperus rotundus, Kyllinga brevifolia, Cyperus amuricus, Erigeron canadensis, Hydrocotyle sibthorpioides, Kummerowia striata*, *Euphorbia humifusa*, and *Viola arvensis.* The present invention encompasses herbicide-tolerant plants in all of the aforementioned species and subspecies.

In addition to being able to tolerate herbicides that inhibit acetyl-Coenzyme A carboxylase activity, plants of the invention may also be able to tolerate herbicides that work on other physiological processes. For example, plants of the invention may be tolerant to acetyl-Coenzyme A carboxylase inhibitors and also tolerant to other herbicides, for example, enzyme inhibitors. Examples of other enzyme inhibitors to which plants of the invention may be tolerant include, but are not limited to, inhibitors of 5-enolpyruvylshikimate-3-phosphate synthase (EPSPS) such as glyphosate, inhibitors of acetohydroxyacid synthase (AHAS) such as imidazolinones, sulfonylureas and sulfonamide herbicides, and inhibitors of glutamine synthase such as glufosinate. In addition to enzyme inhibitors, plants of the invention may also be tolerant of herbicides having other modes of action, for example, auxinic herbicides such as 2,4-D or dicamba, chlorophyll/carotenoid pigment inhibitors such as hydroxyphenylpyruvate dioxygenase (HPPD) inhibitors or phytoene desaturase (PDS) inhibitors, protoporphyrinogen-IX oxidase inhibitors, cell membrane destroyers, photosynthetic inhibitors such as bromoxynil or ioxynil, cell division inhibitors, root inhibitors, shoot inhibitors, and combinations thereof. Thus, plants of the invention tolerant to acetyl-Coenzyme A carboxylase inhibitors can be made resistant to multiple classes of herbicides.

For example, plants of the invention tolerant to acetyl-Coenzyme A carboxylase inhibitors, such as "dims" (*e.g*., cycloxydim, sethoxydim, clethodim, or tepraloxydim), "fops" (*e.g*., clodina.fop, diclofop, fluazifop, haloxyfop, or quizalofop), and "dens" (such as pinoxaden), in some embodiments, may be auxinic-herbicide tolerant, tolerant to EPSPS inhibitors, such as glyphosate; to PPO inhibitors, such as pyrimidinedione, such as saflufenacil, triazolinone, such as sulfentrazone, carfentrazone, flumioxazin, diphenylethers, such as acifluorfen, fomesafen, lactofen, oxyfluorfen, N-phenylphthalamides, such as flumiclorac, CGA-248757, and/or to GS inhibitors, such as glufosinate. In addition to these classes of inhibitors, plants of the invention tolerant to acetyl-Coenzyme A carboxylase inhibitors may also be tolerant to herbicides having other modes of action, for example, chlorophyll/carotenoid pigment inhibitors, cell membrane disruptors, photosynthesis inhibitors, cell division inhibitors, root inhibitors, shoot inhibitors, and combinations thereof. Such tolerance traits may be expressed, *e.g*., as mutant EPSPS proteins, or mutant glutamine synthetase proteins; or as mutant native, inbred, or transgenic aryloxyalkanoate dioxygenase (AAD or DHT), haloarylnitrilase (BXN), 2,2-dichloropropionic acid dehalogenase (DEH), glyphosate-N-acetyltransferase (GAT), glyphosate decarboxylase (GDC), glyphosate oxidoreductase (GOX), glutathione-S-transferase (GST), phosphinothricin acetyltransferase (PAT or bar), or cytochrome P450 (CYP450) proteins having an herbicide-degrading activity. Plants tolerant to acetyl-Coenzyme A carboxylase inhibitors hereof can also be stacked with other traits including, but not limited to, pesticidal traits such as *Bt* Cry and other proteins having pesticidal activity toward coleopteran, lepidopteran, nematode, or other pests; nutrition or nutraceutical traits such as modified oil content or oil profile traits, high protein or high amino acid concentration traits, and other trait types known in the art.

Furthermore, plants are also covered that, in addition to being able to tolerate herbicides that inhibit acetyl-Coenzyme A carboxylase activity, are by the use of recombinant DNA techniques capable to synthesize one or more insecticidal proteins, especially those known from the bacterial genus *Bacillus*, particularly from *Bacillus thuringiensis*, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda).

Furthermore, in one embodiment, plants are also covered that are, e.g., by the use of recombinant DNA techniques and/or by breeding and/or otherwise selected for such traits, able to synthesize one or more proteins to increase the resistance or tolerance of those plants to bacterial, viral or fungal pathogens. The methods for producing such genetically modified plants are generally known to the person skilled in the art. The plants produced as described herein can also be stacked with other traits including, but not limited to, disease resistance, enhanced mineral profile, enhanced vitamin profile, enhanced oil profile (e.g., high oleic acid content), amino acid profile (e.g, high lysine corn), and other trait types known in the art.

Furthermore, in one embodiment, plants are also covered that are, e.g., by the use of recombinant DNA techniques and/or by breeding and/or by other means of selection, able to synthesize one or more proteins to increase the productivity (e.g. bio mass production, grain yield, starch content, oil content or protein content), tolerance to drought, salinity or other growth-limiting environmental factors or tolerance to pests and fungal, bacterial or viral pathogens of those plants.

Furthermore, in one embodiment, plants are also covered that contain, e.g., by the use of recombinant DNA techniques and/or by breeding and/or by other means of selection, a modified amount of substances of content or new substances of content, specifically to improve human or animal nutrition. Furthermore, plants are also covered that contain by the use of recombinant DNA techniques a modified amount of substances of content or new substances of content, specifically to improve raw material production.

Furthermore, in some embodiments, plants of the instant invention are also covered which are, *e.g*. by the use of recombinant DNA techniques and/or by breeding and/or otherwise selected for such traits, altered to contain increased amounts of vitamins and/or minerals, and/or improved profiles of nutraceutical compounds.

In one embodiment, plants of the invention tolerant to acetyl-Coenzyme A carboxylase inhibitors, relative to a wild-type plant, comprise an increased amount of, or an improved profile of, a compound selected from the group consisting of: glucosinolates (*e.g*., glucoraphanin (4-methylsulfinylbutyl-glucosinolate), sulforaphane, 3-indolylmethyl-glucosinolate (glucobrassicin), 1-methoxy-3-indolylmethyl-glucosinolate (neoglucobrassicin)); phenolics (*e.g.,* flavonoids (*e.g.,* quercetin, kaempferol), hydroxycinnamoyl derivatives (*e.g*., 1,2,2'-trisinapoylgentiobiose, 1,2-diferuloylgentiobiose, 1,2'-disinapoyl-2-feruloylgentiobiose, 3-O-caffeoyl-quinic (neochlorogenic acid)); and vitamins and minerals (*e.g*., vitamin C, vitamin E, carotene, folic acid, niacin, riboflavin, thiamine, calcium, iron, magnesium, potassium, selenium, and zinc).

In another embodiment, plants of the invention tolerant to acetyl-Coenzyme A carboxylase inhibitors, relative to a wild-type plant, comprise an increased amount of, or an improved profile of, a compound selected from the group consisting of: progoitrin; isothiocyanates; indoles (products of glucosinolate hydrolysis); glutathione; carotenoids such as beta-carotene, lycopene, and the xanthophyll carotenoids such as lutein and zeaxanthin; phenolics comprising the flavonoids such as the flavonols (*e.g*. quercetin, rutin), the flavans/tannins (such as the procyanidins comprising coumarin, proanthocyanidins, catechins, and anthocyanins); flavones; phytoestrogens such as coumestans, lignans, resveratrol, isoflavones *e.g.*, genistein, daidzein, and glycitein; resorcyclic acid lactones; organosulphur compounds; phytosterols; terpenoids such as carnosol, rosmarinic acid, glycyrrhizin and saponins; chlorophyll; chlorphyllin, sugars, anthocyanins, and vanilla.

In other embodiments, plants of the invention tolerant to acetyl-Coenzyme A carboxylase inhibitors, relative to a wild-type plant, comprise an increased amount of, or an improved profile of, a compound selected from the group consisting of: vincristine, vinblastine, taxanes (e*.g.,* taxol (paclitaxel), baccatin III, 10-desacetylbaccatin III, 10-desacetyl taxol, xylosyl taxol, 7-epitaxol, 7-epibaccatin III, 10-desacetylcephalomannine, 7-epicephalomannine, taxotere, cephalomannine, xylosyl cephalomannine, taxagifine, 8-benxoyloxy taxagifine, 9-acetyloxy taxusin, 9-hydroxy taxusin, taiwanxam, taxane Ia, taxane Ib, taxane Ic, taxane Id, GMP paclitaxel, 9-dihydro 13-acetylbaccatin III, 10-desacetyl-7-epitaxol, tetrahydrocannabinol (THC), cannabidiol (CBD), genistein, diadzein, codeine, morphine, quinine, shikonin, ajmalacine, serpentine, and the like.

The present invention also encompasses progeny of the plants of the invention as well as seeds derived from the herbicide-tolerant plants of the invention and cells derived from the herbicide-tolerant plants of the invention.

In various embodiments, plants hereof can be used to produce plant products. Thus, a method for preparing a descendant seed comprises planting a seed of a capable of producing a plant hereof, growing the resulting plant, and harvesting descendant seed thereof. In some embodiments, such a method can further comprise applying an ACCase-inhibiting herbicide composition to the resulting plant. Similarly, a method for producing a derived product from a plant hereof can comprise processing a plant part thereof to obtain a derived product. In some embodiments, such a method can be used to obtain a derived product that is any of, e.g., fodder, feed, seed meal, oil, or seed-treatment-coated seeds. Seeds, treated seeds, and other plant products obtained by such methods are useful products that can be commercialized.

In various embodiment, the present invention provides production of food products, consumer products, industrial products, and veterinary products from any of the plants described herein.

### Acetyl-Coenzyme A carboxylase Enzymes

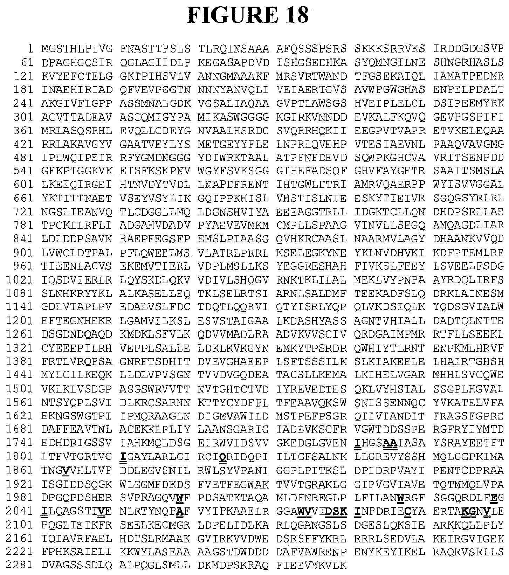

The present invention provides plants expressing acetyl-Coenzyme A carboxylase enzymes with amino acid sequences that differ from the amino acid sequence of the acetyl-Coenzyme A carboxylase enzyme found in the corresponding wild-type plant. For ease of understanding, the amino acid numbering system used herein will be the numbering system used for the acetyl-Coenzyme A carboxylase from *Alopecurus myosuroides* [Huds.] (also referred to as black grass). The mRNA sequence encoding the *A. myosuroides* acetyl-Coenzyme A carboxylase is available at GenBank accession number AJ310767 and the protein sequence is available at GenBank accession no. CAC84161 both of which are specifically incorporated herein by reference. The number of the amino acid referred to will be followed with *(Am)* to indicate the amino acid in the *Alopecurus myosuroides* sequence to which the amino acid corresponds. Figure 18 provides *Alopecurus myosuroides* acetyl-Coenzyme A carboxylase amino acid sequence (GenBank accession no. CAC84161). Amino acids that may be altered in the acetyl-Coenzyme A carboxylase enzymes of the invention are indicated in bold double underline, and Figure 19 depicts the amino acid sequence of wild-type *Oryza sativa* acetyl-Coenzyme A carboxylases aligned with *Alopecurus myosuroides* acetyl-Coenzyme A carboxylase with some critical residues denoted.

In one embodiment, an acetyl-Coenzyme A carboxylase of the invention differs from the corresponding wild-type acetyl-Coenzyme A carboxylase at amino acid position 1,781*(Am).* Wild-type *A. myosuroides* acetyl-Coenzyme A carboxylase has an isoleucine at position 1,781*(Am)* (11781). The 1,781*(Am)* ACCase mutants of the invention will have an amino acid other than isoleucine at this position. Suitable examples of amino acids that may be found at this position in the acetyl-Coenzyme A carboxylase enzymes of the invention include, but are not limited to, leucine (I1781L), valine (I1781V), threonine (I1781T) and alanine(I1781A). In one embodiment, an acetyl-Coenzyme A carboxylase enzyme of the invention will have a leucine at position 1,781*(Am).*

In one embodiment, an acetyl-Coenzyme A carboxylase of the invention differs from the corresponding wild-type acetyl-Coenzyme A carboxylase at amino acid position 1,785*(Am).* Wild-type *A. myosuroides* acetyl-Coenzyme A carboxylase has an alanine at position 1,785*(Am)* (A1785). The 1,785*(Am)* ACCase mutants of the invention will have an amino acid other than alanine at this position. Suitable examples of amino acids that may be found at this position in the acetyl-Coenzyme A carboxylase enzymes of the invention include, but are not limited to, glycine (A1785G). In one embodiment, an acetyl-Coenzyme A carboxylase enzyme of the invention will have a glycine at position 1,785*(Am).*

In one embodiment, an acetyl-Coenzyme A carboxylase of the invention differs from the corresponding wild-type acetyl-Coenzyme A carboxylase at amino acid position 1,786*(Am).* Wild-type *A. myosuroides* acetyl-Coenzyme A carboxylase has an alanine at position 1,786*(Am)* (A1786). The 1,786*(Am)* ACCase mutants of the invention will have an amino acid other than alanine at this position. Suitable examples of amino acids that may be found at this position in the acetyl-Coenzyme A carboxylase enzymes of the invention include, but are not limited to, proline (A1786P). In one embodiment, an acetyl-Coenzyme A carboxylase enzyme of the invention will have a proline at position 1,786*(Am).*

In one embodiment, an acetyl-Coenzyme A carboxylase of the invention differs from the corresponding wild-type acetyl-Coenzyme A carboxylase at amino acid position 1,811*(Am),* Wild-type *A. myosuroides* acetyl-Coenzyme A carboxylase has an isoleucine at position 1,811*(Am)* (I1811). The 1,811*(Am)* ACCase mutants of the invention will have an amino acid other than isoleucine at this position. Suitable examples of amino acids that may be found at this position in the acetyl-Coenzyme A carboxylase enzymes of the invention include, but are not limited to, asparagine (I1811N). In one embodiment, an acetyl-Coenzyme A carboxylase enzyme of the invention will have an asparagine at position 1,811*(Am).*

In one embodiment, an acetyl-Coenzyme A carboxylase of the invention differs from the corresponding wild-type acetyl-Coenzyme A carboxylase at amino acid position 1,824*(Am)*. Wild-type *A. myosuroides* acetyl-Coenzyme A carboxylase has a glutamine at position 1,824*(Am)* (Q1824). The 1,824*(Am)* ACCase mutants of the invention will have an amino acid other than glutamine at this position. Suitable examples of amino acids that may be found at this position in the acetyl-Coenzyme A carboxylase enzymes of the invention include, but are not limited to, proline (Q1824P). In one embodiment, an acetyl-Coenzyme A carboxylase enzyme of the invention will have a proline at position 1,824*(Am)*.

In one embodiment, an acetyl-Coenzyme A carboxylase of the invention differs from the corresponding wild-type acetyl-Coenzyme A carboxylase at amino acid position 1,864*(Am)*. Wild-type *A. myosuroides* acetyl-Coenzyme A carboxylase has a valine at position 1,864*(Am)* (V1864). The 1,864*(Am)* ACCase mutants of the invention will have an amino acid other than valine at this position. Suitable examples of amino acids that may be found at this position in the acetyl-Coenzyme A carboxylase enzymes of the invention include, but are not limited to, phenylalanine (V1864F). In one embodiment, an acetyl-Coenzyme A carboxylase enzyme of the invention will have a phenylalanine at position 1,864*(Am).*

In one embodiment, an acetyl-Coenzyme A carboxylase of the invention differs from the corresponding wild-type acetyl-Coenzyme A carboxylase at amino acid position 1,999*(Am)*. Wild-type *A. myosuroides* acetyl-Coenzyme A carboxylase has a tryptophan at position 1,999*(Am)* (W1999). The 1,999*(Am)* ACCase mutants of the invention will have an amino acid other than tryptophan at this position. Suitable examples of amino acids that may be found at this position in the acetyl-Coenzyme A carboxylase enzymes of the invention include, but are not limited to, cysteine (W1999C) and glycine (W1999G). In one embodiment, an acetyl-Coenzyme A carboxylase enzyme of the invention will have a glycine at position 1,999*(Am)*.

In one embodiment, an acetyl-Coenzyme A carboxylase of the invention differs from the corresponding wild-type acetyl-Coenzyme A carboxylase at amino acid position 2,027*(Am).* Wild-type *A. myosuroides* acetyl-Coenzyme A carboxylase has a tryptophan at position 2,027*(Am)*(W2027). The 2,027*(Am)* ACCase mutants of the invention will have an amino acid other than tryptophan at this position. Suitable examples of amino acids that may be found at this position in the acetyl-Coenzyme A carboxylase enzymes of the invention include, but are not limited to, cysteine (W2027C) and arginine (W2027R). In one embodiment, an acetyl-Coenzyme A carboxylase enzyme of the invention will have a cysteine at position 2,027*(Am).*

In one embodiment, an acetyl-Coenzyme A carboxylase of the invention differs from the corresponding wild-type acetyl-Coenzyme A carboxylase at amino acid position 2,039(Am). Wild-type *A. myosuroides* acetyl-Coenzyme A carboxylase has a glutamic acid at position 2,039*(Am)* (E2039). The 2,039*(Am)* ACCase mutants of the invention will have an amino acid other than glutamic acid at this position. Suitable examples of amino acids that may be found at this position in the acetyl-Coenzyme A carboxylase enzymes of the invention include, but are not limited to, glycine (E2039G). In one embodiment, an acetyl-Coenzyme A carboxylase enzyme of the invention will have an glycine at position 2,039*(Am)*.

In one embodiment, an acetyl-Coenzyme A carboxylase of the invention differs from the corresponding wild-type acetyl-Coenzyme A carboxylase at amino acid position 2,041*(Am).* Wild-type *A. myosuroides* acetyl-Coenzyme A carboxylase has an isoleucine at position 2,041*(Am)* (12041). The 2,041*(Am)* ACCase mutants of the invention will have an amino acid other than isoleucine at this position. Suitable examples of amino acids that may be found at this position in the acetyl-Coenzyme A carboxylase enzymes of the invention include, but are not limited to, asparagine (I2041N), or valine (I2041V). In one embodiment, an acetyl-Coenzyme A carboxylase enzyme of the invention will have an asparagine at position 2,041*(Am).*

In one embodiment, an acetyl-Coenzyme A carboxylase of the invention differs from the corresponding wild-type acetyl-Coenzyme A carboxylase at amino acid position *2,049(Am).* Wild-type *A. myosuroides* acetyl-Coenzyme A carboxylase has an valine at position 2,049*(Am)* (V2049). The 2,049*(Am)* ACCase mutants of the invention willhave an amino acid other than valine at this position. Suitable examples of amino acids that may be found at this position in the acetyl-Coenzyme A carboxylase enzymes of the invention include, but are not limited to, phenylalanine (V2049F), isoleucine (V2049I) and leucine (V2049L). In one embodiment, an acetyl-Coenzyme A carboxylase enzyme of the invention will have an phenylalanine at position 2,049*(Am).*

In one embodiment, an acetyl-Coenzyme A carboxylase of the invention differs from the corresponding wild-type acetyl-Coenzyme A carboxylase at amino acid position 2,059*(Am)*. Wild-type *A. myosuroides* acetyl-Coenzyme A carboxylase has an alanine at position 2,059*(Am)* (A2059). The 2,059*(Am)* ACCase mutants of the invention willhave an amino acid other than an alanine at this position. Suitable examples of amino acids that may be found at this position in the acetyl-Coenzyme A carboxylase enzymes of the invention include, but are not limited to, valine (A2059V). In one embodiment, an acetyl-Coenzyme A carboxylase enzyme of the invention will have an valine at position 2,059*(Am)*.

In one embodiment, an acetyl-Coenzyme A carboxylase of the invention differs from the corresponding wild-type acetyl-Coenzyme A carboxylase at amino acid position *2074(Am).* Wild-type *A. myosuroides* acetyl-Coenzyme A carboxylase has a tryptophan at position 2074*(Am)* (W2074). The 2,074*(Am)* ACCase mutants of the invention will have an amino acid other than tryptophan at this position. Suitable examples of amino acids that may be found at this position in the acetyl-Coenzyme A carboxylase enzymes of the invention include, but are not limited to, leucine (W2074L). In one embodiment, an acetyl-Coenzyme A carboxylase enzyme of the invention will have a leucine at 2074*(Am).*

In one embodiment, an acetyl-Coenzyme A carboxylase of the invention differs from the corresponding wild-type acetyl-Coenzyme A carboxylase at amino acid position 2,075*(Am).* Wild-type *A. myosuroides* acetyl-Coenzyme A carboxylase has a valine at position 2,075*(Am)* (V2075). The 2,075*(Am)* ACCase mutants of the invention will have an amino acid other than valine at this position. Suitable examples of amino acids that may be found at this position in the acetyl-Coenzyme A carboxylase enzymes of the invention include, but are not limited to, methionine (V2075M), leucine (V2075L) and isoleucine (V2075I). In one embodiment, an acetyl-Coenzyme A carboxylase enzyme of the invention will have a leucine at position 2,075*(Am).* In some embodiments, an acetyl-Coenzyme A carboxylase enzyme of the invention will have a valine at position 2075(*Am*) and an additional valine immediately after position 2075(*Am*) and before the valine at position 2076(*Am*), i.e., may have three consecutive valines where the wild-type enzyme has two.

In one embodiment, an acetyl-Coenzyme A carboxylase of the invention differs from the corresponding wild-type acetyl-Coenzyme A carboxylase at amino acid position 2,078*(Am).* Wild-type *A. myosuroides* acetyl-Coenzyme A carboxylase has an aspartate at position 2,078*(Am)* (D2078). The 2,078*(Am)* ACCase mutants of the invention will have an amino acid other than aspartate at this position. Suitable examples of amino acids that may be found at this position in the acetyl-Coenzyme A carboxylase enzymes of the invention include, but are not limited to, lysine (D2,078K), glycine (D2078G), or threonine (D2078T). In one embodiment, an acetyl-Coenzyme A carboxylase enzyme of the invention will have a glycine at position 2,078*(Am).*

In one embodiment, an acetyl-Coenzyme A carboxylase of the invention differs from the corresponding wild-type acetyl-Coenzyme A carboxylase at amino acid position 2,079*(Am).* Wild-type *A. myosuroides* acetyl-Coenzyme A carboxylase has a serine at position 2,079*(Am)* (S2079). The 2,079*(Am)* ACCase mutants of the invention will have an amino acid other than serine at this position. Suitable examples of amino acids that may be found at this position in the acetyl-Coenzyme A carboxylase enzymes of the invention include, but are not limited to, phenylalanine (S2079F). In one embodiment, an acetyl-Coenzyme A carboxylase enzyme of the invention will have a phenylalanine at position 2,079*(Am).*

In one embodiment, an acetyl-Coenzyme A carboxylase of the invention differs from the corresponding wild-type acetyl-Coenzyme A carboxylase at amino acid position 2,080*(Am)*. Wild-type *A. myosuroides* acetyl-Coenzyme A carboxylase has a lysine at position 2,080*(Am)* (K2080). The 2,080*(Am)* ACCase mutants of the invention will have an amino acid other than lysine at this position. Suitable examples of amino acids that may be found at this position in the acetyl-Coenzyme A carboxylase enzymes of the invention include, but are not limited to, glutamic acid (K2080E). In one embodiment, an acetyl-Coenzyme A carboxylase enzyme of the invention will have a glutamic acid at position 2,080*(Am)*. In another embodiment, acetyl-Coenzyme A carboxylase enzymes of the invention will typically have a deletion of this position (Δ2080).

In one embodiment, an acetyl-Coenzyme A carboxylase of the invention differs from the corresponding wild-type acetyl-Coenzyme A carboxylase at amino acid position 2,081*(Am).* Wild-type *A. myosuroides* acetyl-Coenzyme A carboxylase has a isoleucine at position 2,081*(Am)* (12081). The 2,081*(Am)* ACCase mutants of the invention will have an amino acid other than isoleucine at this position. In one embodiment, acetyl-Coenzyme A carboxylase enzymes of the invention will typically have a deletion of this position (Δ2081).

In one embodiment, an acetyl-Coenzyme A carboxylase of the invention differs from the corresponding wild-type acetyl-Coenzyme A carboxylase at amino acid position 2,088(Am). Wild-type *A. myosuroides* acetyl-Coenzyme A carboxylase has a cysteine at position 2,088*(Am)* (C2088). The 2,088*(Am)* ACCase mutants of the invention will have an amino acid other than cysteine at this position. Suitable examples of amino acids that may be found at this position in the acetyl-Coenzyme A carboxylase enzymes of the invention include, but are not limited to, arginine (C2088R), tryptophan (C2088W), phenylalanine (C2088F), glycine (C2088G), histidine (C2088H), lysine (C2088K), serine (C2088S), threonine (C2088T), leucine (C2088L) or valine (C2088V). In one embodiment, an acetyl-Coenzyme A carboxylase enzyme of the invention will have an arginine at position 2,088*(Am).*

In one embodiment, an acetyl-Coenzyme A carboxylase of the invention differs from the corresponding wild-type acetyl-Coenzyme A carboxylase at amino acid position 2,095*(Am)*. Wild-type *A. myosuroides* acetyl-Coenzyme A carboxylase has a lysine at position 2,095*(Am)* (K2095). The 2,095*(Am)* ACCase mutants of the invention will have an amino acid other than lysine at this position. Suitable examples of amino acids that may be found at this position in the acetyl-Coenzyme A carboxylase enzymes of the invention include, but are not limited to, glutamic acid (K2095E). In one embodiment, an acetyl-Coenzyme A carboxylase enzyme of the invention will have a glutamic acid at position 2,095*(Am)*.

In one embodiment, an acetyl-Coenzyme A carboxylase of the invention differs from the corresponding wild-type acetyl-Coenzyme A carboxylase at amino acid position 2,096*(Am)*. Wild-type *A. myosuroides* acetyl-Coenzyme A carboxylase has a glycine at position 2,096*(Am)* (G2096). The 2,096*(Am)* ACCase mutants of the invention will have an amino acid other than glycine at this position. Suitable examples of amino acids that may be found at this position in the acetyl-Coenzyme A carboxylase enzymes of the invention include, but are not limited to, alanine (G2096A), or serine (G2096S). In one embodiment, an acetyl-Coenzyme A carboxylase enzyme of the invention will have an alanine at position 2,096*(Am)*.

In one embodiment, an acetyl-Coenzyme A carboxylase of the invention differs from the corresponding wild-type acetyl-Coenzyme A carboxylase at amino acid position 2,098*(Am)*. Wild-type *A. myosuroides* acetyl-Coenzyme A carboxylase has a valine at position 2,098*(Am)* (V2098). The 2,098*(Am)* ACCase mutants of the invention will have an amino acid other than valine at this position. Suitable examples of amino acids that may be found at this position in the acetyl-Coenzyme A carboxylase enzymes of the invention include, but are not limited to, alanine (V2098A), glycine (V2098G), proline (V2098P), histidine (V2098H), serine (V2098S) or cysteine (V2098C). In one embodiment, an acetyl-Coenzyme A carboxylase enzyme of the invention will have an alanine at position 2,098*(Am)*.

In one embodiment, the present invention emcompasses acetyl-Coenzyme A carboxylase of an herbicide-tolerant plant of the invention which differs from the acetyl-Coenzyme A carboxylase of the corresponding wild-type plant at only one of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am),* or 2,098*(Am)*. In one embodiment the acetyl-Coenzyme A carboxylase of an herbicide-tolerant plant of the invention will differ at only one of the following positions: 2,078*(Am)*, 2,088*(Am)*, or 2,075*(Am).* In a preferred embodiment the acetyl-Coenzyme A carboxylase of an herbicide-tolerant plant of the invention will differ at only one of the following positions:2,039*(Am)*, 2,059*(Am)*, 2,080*(Am)*, or 2,095*(Am)*. In a more preferred embodiment the acetyl-Coenzyme A carboxylase of a herbicide-tolerant plant of the invention will differ at only one of the following positions:1,785*(Am)*, 1,786*(Am)*, 1,811*(Am)*, 1,824*(Am)*, 1,864*(Am)*, 2,041*(Am)*, 2,049*(Am)*, 2,074*(Am)*, 2,079*(Am)*, 2,081*(Am)*, 2,096*(Am)*, or 2,098*(Am)*. In a most preferred embodiment the acetyl-Coenzyme A carboxylase of an herbicide-tolerant plant of the invention will differ at only one of the following positions: 1,781*(Am)*, 1,999*(Am)*, 2,027*(Am)*, 2,041*(Am)*, or *2,096(Am).*

In one embodiment, Acetyl-Coenzyme A carboxylase enzymes of the invention will have only one of the following substitutions: an isoleucine at position 2,075*(Am)*, glycine at position 2,078*(Am)*, or arginine at position 2,088*(Am).* In a preferred embodiment, Acetyl-Coenzyme A carboxylase enzymes of the invention will have only one of the following substitutions: a glycine at position 2,039*(Am)*, valine at position 2,059*(Am)*, methionine at position 2,075*(Am)*, duplication of position 2,075*(Am)* (i.e., an insertion of valine between 2,074*(Am)* and 2,075*(Am)*, or an insertion of valine between position 2,075*(Am)* and 2,076*(Am)*), deletion of amino acid position 2,080*(Am)*, glutamic acid at position 2,080*(Am)*, deletion of position 2,081*(Am)*, or glutamic acid at position 2,095*(Am).* In a more preferred embodiment, Acetyl-Coenzyme A carboxylase enzymes of the invention will have only one of the following substitutions: a glycine at position 1,785*(Am)*, a proline at position 1,786*(Am)*, an asparagine at position 1,811*(Am)*, a leucine at position 2,075*(Am)*, a methionine at position 2,075*(Am)*, a threnonine at position 2,078*(Am)*, a deletion at position 2,080*(Am)*, a deletion at position 2,081*(Am)*, a tryptophan, phenylalanine, glycine, histidine, lysine, leucine, serine, threonine, or valine at position 2,088*(Am)*, a serine at position 2,096*(Am)*, an alanine at position 2,096*(Am)*, an alanine at position 2,098*(Am)*, a glycine at position 2,098*(Am)*, an histidine at position 2,098*(Am)*, a proline at position 2,098*(Am)*, or a serine at position 2,098*(Am)*. In a most preferred embodiment, Acetyl-Coenzyme A carboxylase enzymes of the invention will have only one of the following substitutions: a leucine at position 1,781*(Am)*, a threonine at position 1,781*(Am)*, a valine at position 1,781*(Am)*, an alanine at position 1,781*(Am)*, a glycine at position 1,999*(Am)*, a cysteine or arginine at position 2,027*(Am)*, an arginine at position 2,027*(Am)*, an asparagine at position 2,041*(Am)*, a valine at position 2,041*(Am)*, an alanine at position 2,096*(Am)*, and a serine at position 2,096*(Am)*.

In one embodiment, nucleic acids encoding Acetyl-Coenzyme A carboxylase polypeptide having only one of the following substitutions: isoleucine at position 2,075*(Am)*, glycine at position 2,078*(Am)*, or arginine at position 2,088*(Am)* are used transgenically. In another embodiment, a monocot plant cell is transformed with an expression vector construct comprising the nucleic acid encoding Acetyl-Coenzyme A carboxylase polypeptide having only one of the following substitutions: isoleucine at position 2,075*(Am)*, glycine at position 2,078*(Am)*, or arginine at position 2,088*(Am).*

In one embodiment, the invention provides rice plants comprising nucleic acids encoding Acetyl-Coenzyme A carboxylase polypeptides having a substitution at only one amino acid position as described above.

In one embodiment, the invention provides BEP clade plants comprising nucleic acids encoding Acetyl-Coenzyme A carboxylase polypeptides having a substitution at only one amino acid position as described above.

In one embodiment, the invention provides BET subclade plants comprising nucleic acids encoding Acetyl-Coenzyme A carboxylase polypeptides having a substitution at only one amino acid position as described above.

In one embodiment, the invention provides BET crop plants comprising nucleic acids encoding Acetyl-Coenzyme A carboxylase polypeptides having a substitution at only one amino acid position as described above.

In one embodiment, the invention provides monocot plants comprising nucleic acids encoding Acetyl-Coenzyme A carboxylase polypeptides having a substitution at only one amino acid position as described above.

In one embodiment, the invention provides monocot plants comprising nucleic acids encoding Acetyl-Coenzyme A carboxylase polypeptides having a substitution at amino acid position 1,781*(Am)*, wherein the amino acid at position 1,781*(Am)* differs from that of wild type and is not leucine.

In one embodiment, the invention provides monocot plants comprising nucleic acids encoding Acetyl-Coenzyme A carboxylase polypeptides having a substitution at amino acid position 1,999*(Am)*, wherein the amino acid at position 1,999*(Am)* differs from that of wild type and is not cysteine.

In one embodiment, the invention provides monocot plants comprising nucleic acids encoding Acetyl-Coenzyme A carboxylase polypeptides having a substitution at amino acid position 2,027*(Am)*, wherein the amino acid at position 2,027*(Am)* differs from that of wild type and is not cysteine.

In one embodiment, the invention provides monocot plants comprising nucleic acids encoding Acetyl-Coenzyme A carboxylase polypeptides having a substitution at amino acid position 2,041*(Am)*, wherein the amino acid at position 2,041*(Am)* differs from that of wild type and is not valine or asparagine.

In one embodiment, the invention provides monocot plants comprising nucleic acids encoding Acetyl-Coenzyme A carboxylase polypeptides having a substitution at amino acid position 2,096*(Am)*, wherein the amino acid at position 2,096*(Am)* differs from that of wild type and is not alanine.

The present invention also encompasses acetyl-Coenzyme A carboxylase enzymes with an amino acid sequence that differs in more than one amino acid position from that of the acetyl-Coenzyme A carboxylase enzyme found in the corresponding wild-type plant. For example, an acetyl-Coenzyme A carboxylase of the invention may differ in 2, 3, 4, 5, 6, or 7 positions from that of the acetyl-Coenzyme A carboxylase enzyme found in the corresponding wild-type plant.

In one embodiment, an acetyl-Coenzyme A carboxylase of the invention differs from the corresponding wild-type acetyl-Coenzyme A carboxylase at amino acid position 1,781*(Am)* and at one or more additional amino acid positions. Acetyl-Coenzyme A carboxylase enzymes of the invention will typically have a leucine, a threonine, a valine, or an alanine at position 1,781*(Am).* In addition, enzymes of this embodiment will also comprise one or more of a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine, or an additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine tryptophan, phenylalanine, glycine, histidine, lysine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one embodiment, an acetyl-Coenzyme A carboxylase of the invention will have a leucine, a threonine, a valine, or an alanine at position 1,781*(Am)* and a glycine at position 1,785*(Am).* In one embodiment, an acetyl-Coenzyme A carboxylase of the invention will have a leucine, a threonine, a valine, or an alanine at position 1,781*(Am)* and a proline at position 1,786*(Am).* In one embodiment, an acetyl-Coenzyme A carboxylase of the invention will have a leucine, a threonine, a valine, or an alanine at position 1,781*(Am)* and an asparagine at position 1,811*(Am).* In one embodiment, an acetyl-Coenzyme A carboxylase of the invention will have a leucine, a threonine, a valine, or an alanine at position 1,781(*Am*) and a proline at position 1824(*Am*)*.* In one embodiment, an acetyl-Coenzyme A carboxylase of the invention will have a leucine, a threonine, a valine, or an alanine at position 1,781*(Am)* and a phenylalanine at position 1864(*Am*). In one embodiment, an acetyl-Coenzyme A carboxylase of the invention will have a leucine, a threonine, a valine, or an alanine at position 1,781*(Am)* and a cysteine or glycine at position 1,999*(Am)*. In one embodiment, an acetyl-Coenzyme A carboxylase of the invention will have a leucine, a threonine, a valine, or an alanine at position 1,781*(Am)* and a cysteine or an arginine at position *2,027(Am).* In one embodiment, an acetyl-Coenzyme A carboxylase of the invention will have a leucine, a threonine, a valine, or an alanine at position 1,781(*Am*) and a glycine at position 2039(*Am*). In one embodiment, an acetyl-Coenzyme A carboxylase of the invention will have a leucine, a threonine, a valine, or an alanine at position 1,781*(Am)* and an asparagine at position 2,041*(Am).* In one embodiment, an acetyl-Coenzyme A carboxylase of the invention will have a leucine, a threonine, a valine, or an alanine at position 1,781*(Am)* and a phenylalanine, leucine or isoleucine at position 2,049*(Am).* In one embodiment, an acetyl-Coenzyme A carboxylase of the invention will have a leucine, a threonine, a valine, or an alanine at position 1,781(*Am*) and a valine at position 2059(*Am*). In one embodiment, an acetyl-Coenzyme A carboxylase of the invention will have a leucine, a threonine, a valine, or an alanine at position 1,781*(Am)* and a leucine at position 2,074*(Am).* In one embodiment, an acetyl-Coenzyme A carboxylase of the invention will have a leucine, a threonine, a valine, or an alanine at position 1,781*(Am)* and a leucine, isoleucine methionine, or additional valine at position 2,075*(Am).* In one embodiment, an acetyl-Coenzyme A carboxylase of the invention will have a leucine, a threonine, a valine, or an alanine at position 1,781*(Am)* and a glycine or threonine at position *2,078(Am).* In one embodiment, an acetyl-Coenzyme A carboxylase of the invention will have a leucine, a threonine, a valine, or an alanine at position 1,781(*Am*) and a phenylalanine at position 2079(*Am*)*.* In one embodiment, an acetyl-Coenzyme A carboxylase of the invention will have a leucine, a threonine, a valine, or an alanine at position 1,781(*Am*) and a glutamic acid or a deletion at position 2080(*Am*). In one embodiment, an acetyl-Coenzyme A carboxylase of the invention will have a leucine, a threonine, a valine, or an alanine at position 1,781(*Am*) and a deletion at position 2081(*Am*). In one embodiment, an acetyl-Coenzyme A carboxylase of the invention will have a leucine, a threonine, a valine, or an alanine at position 1,781*(Am)* and an arginine, tryptophan, phenylalanine, glycine, histidine, lysine, serine, threonine, or valine at position 2,088*(Am).* In one embodiment, an acetyl-Coenzyme A carboxylase of the invention will have a leucine, a threonine, a valine, or an alanine at position 1,781*(Am)* and a glutamic acid at position 2,095*(Am)*. In one embodiment, an acetyl-Coenzyme A carboxylase of the invention will have a leucine, a threonine, a valine, or an alanine at position 1,781*(Am)* and an alanine or serine at position *2,096(Am).* In one embodiment, an acetyl-Coenzyme A carboxylase of the invention will have a leucine, a threonine, a valine, or an alanine at position 1,781*(Am)* and an alanine, glycine, proline, histidine, cysteine, or serine at position 2,098*(Am)*. In one embodiment, an acetyl-Coenzyme A carboxylase of the invention will have a leucine, a threonine, a valine, or an alanine at position 1,781*(Am)*, a cysteine or arginine at position 2,027*(Am)*, and an asparagine at position 2,041*(Am).* In one embodiment, an acetyl-Coenzyme A carboxylase of the invention will have a leucine, a threonine, a valine, or an alanine at position 1,781*(Am)*, a cysteine or arginine at position 2,027*(Am)*, an asparagine at position 2,041*(Am)*, and an alanine at position 2,096*(Am).*

In one embodiment, an acetyl-Coenzyme A carboxylase of the invention differs from the corresponding wild-type acetyl-Coenzyme A carboxylase at amino acid position 1,785*(Am)* and at one or more additional amino acid positions. Acetyl-Coenzyme A carboxylase enzymes of the invention will typically have an glycine at position 1,785*(Am).* In addition, enzymes of this embodiment will also comprise one or more of a leucine, threonine, a valine, or alanine at position 1,781*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am),* an alanine or serine at position 2,096*(Am)*, and an alanine, glycine, proline, histidine, cysteine, or serine at position 2,098*(Am)*. In one embodiment, an acetyl-Coenzyme A carboxylase of the invention will have a glycine at position 1,785*(Am)* and a leucine, a threonine, a valine, or an alanine at position 1,781*(Am).* In one embodiment, an acetyl-Coenzyme A carboxylase of the invention will have a glycine at position 1,785*(Am)* and a proline at position 1,786*(Am)*. In one embodiment, an acetyl-Coenzyme A carboxylase of the invention will have a glycine at position 1,785*(Am)* and an asparagine at position 1,811*(Am).* In one embodiment, an acetyl-Coenzyme A carboxylase of the invention will have a glycine at position 1,785*(Am)* and a proline at position 1,824*(Am)*. In one embodiment, an acetyl-Coenzyme A carboxylase of the invention will have a glycine at position 1,785*(Am)* and a phenylalanine at position 1,864*(Am).* In one embodiment, an acetyl-Coenzyme A carboxylase of the invention will have a glycine at position 1,785*(Am)* and a cysteine or glycine at position 1,999*(Am)*. In one embodiment, an acetyl-Coenzyme A carboxylase of the invention will have a glycine at position 1,785*(Am)* and a cysteine or an arginine at position 2,027*(Am).* In one embodiment, an acetyl-Coenzyme A carboxylase of the invention will have a glycine at position 1,785*(Am)* and a glycine at position 2,039*(Am)*. In one embodiment, an acetyl-Coenzyme A carboxylase of the invention will have a glycine at position 1,785*(Am)* and an asparagine at position 2,041*(Am).* In one embodiment, an acetyl-Coenzyme A carboxylase of the invention will have a glycine at position 1,785*(Am)* and a phenylalanine, isoleucine or leucine at position 2,049*(Am).* In one embodiment, an acetyl-Coenzyme A carboxylase of the invention will have a glycine at position 1,785*(Am)* and a valine at position 2,059*(Am)*. In one embodiment, an acetyl-Coenzyme A carboxylase of the invention will have a glycine at position 1,785*(Am)* and a leucine at position 2,074*(Am).* In one embodiment, an acetyl-Coenzyme A carboxylase of the invention will have a glycine at position 1,785*(Am)* and a leucine, isoleucine, methionine or additional valine at position 2,075*(Am).* In one embodiment, an acetyl-Coenzyme A carboxylase of the invention will have a glycine at position 1,785*(Am)* and a glycine or threonine at position 2,078*(Am).* In one embodiment, an acetyl-Coenzyme A carboxylase of the invention will have a glycine at position 1,785*(Am)* and a phenylalanine at position 2,079*(Am).* In one embodiment, an acetyl-Coenzyme A carboxylase of the invention will have a glycine at position 1,785*(Am)* and a glutamic acid or deletion at position 2,080*(Am)*. In one embodiment, an acetyl-Coenzyme A carboxylase of the invention will have a glycine at position 1,785*(Am)* and a deletion at position 2,081*(Am).* In one embodiment, an acetyl-Coenzyme A carboxylase of the invention will have a glycine at position 1,785*(Am)* and an arginine, tryptophan, phenylalanine, glycine, histidine, lysine, leucine, serine, threonine, or valine at position 2,088*(Am).* In one embodiment, an acetyl-Coenzyme A carboxylase of the invention will have a glycine at position 1,785*(Am)* and a glutamic acid at position 2,095*(Am)*. In one embodiment, an acetyl-Coenzyme A carboxylase of the invention will have a glycine at position 1,785*(Am)* and an alanine or serine at position 2,096*(Am)*. In one embodiment, an acetyl-Coenzyme A carboxylase of the invention will have a glycine at position 1,785*(Am)* and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 1,786(Am) and at one or more additional amino acid positions. Acetyl-Coenzyme A carboxylase enzymes of the invention will typically have a proline at position 1,786*(Am).* In addition, enzymes of this embodiment will also comprise one or more of a leucine, threonine, a valine, or alanine at position 1,781*(Am)*, a glycine at position 1,785*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid or deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one embodiment, an acetyl-Coenzyme A carboxylase of the invention will have a proline at position 1786*(Am)* and a leucine, a threonine, a valine, or an alanine at position 1,781*(Am).* In one embodiment, an acetyl-Coenzyme A carboxylase of the invention will have a proline at position 1,786*(Am)* and a glycine at position 1,785*(Am).* In one embodiment, an acetyl-Coenzyme A carboxylase of the invention will have a proline at position 1,786*(Am)* and an asparagine at position 1,811*(Am).* In one embodiment, an acetyl-Coenzyme A carboxylase of the invention will have a proline at position 1,786*(Am)* and a proline at position 1,824*(Am)*. In one embodiment, an acetyl-Coenzyme A carboxylase of the invention will have a proline at position 1,786*(Am)* and phenylalanine at position 1,864*(Am).* In one embodiment, an acetyl-Coenzyme A carboxylase of the invention will have a proline at position 1,786*(Am)* and a cysteine or glycine at position 1,999*(Am)*. In one embodiment, an acetyl-Coenzyme A carboxylase of the invention will have a proline at position 1,786*(Am)* and a cysteine or an arginine at position 2,027*(Am).* In one embodiment, an acetyl-Coenzyme A carboxylase of the invention will have a proline at position 1,786*(Am)* and a glycine at position 2,039*(Am)*. In one embodiment, an acetyl-Coenzyme A carboxylase of the invention will have a proline at position 1,786*(Am)* and an asparagine at position 2,041*(Am).* In one embodiment, an acetyl-Coenzyme A carboxylase of the invention will have a proline at position 1,786*(Am)* and phenylalanine, isoleucine or leucine at position 2,049*(Am)* In one embodiment, an acetyl-Coenzyme A carboxylase of the invention will have a proline at position 1,786*(Am)* and a valine at position 2,059*(Am)*. In one embodiment, an acetyl-Coenzyme A carboxylase of the invention will have a proline at position 1,786*(Am)* and a leucine at position 2,074*(Am)*. In one embodiment, an acetyl-Coenzyme A carboxylase of the invention will have a proline at position 1,786*(Am)* and a leucine, isoleucine, methionine or additional valine at position 2,075*(Am).* In one embodiment, an acetyl-Coenzyme A carboxylase of the invention will have a proline at position 1,786*(Am)* and a glycine or threonine at position 2,078*(Am).* In one embodiment, an acetyl-Coenzyme A carboxylase of the invention will have a proline at position 1,786*(Am)* and a phenylalanine at position 2,079*(Am).* In one embodiment, an acetyl-Coenzyme A carboxylase of the invention will have a proline at position 1,786*(Am)* and a glutamic acid or deletion at position 2,080*(Am)*. In one embodiment, an acetyl-Coenzyme A carboxylase of the invention will have a proline at position 1,786*(Am)* and a deletion at position 2,081*(Am).* In one embodiment, an acetyl-Coenzyme A carboxylase of the invention will have a proline at position 1,786*(Am)* and an arginine, tryptophan, phenylalanine, glycine, histidine, lysine, leucine, serine, threonine, or valine at position 2,088*(Am).* In one embodiment, an acetyl-Coenzyme A carboxylase of the invention will have a proline at position 1,786*(Am)* and a glutamic acid at position 2,095*(Am)*. In one embodiment, an acetyl-Coenzyme A carboxylase of the invention will have a proline at position 1,786*(Am)* and an alanine or serine at position 2,096*(Am)*. In one embodiment, an acetyl-Coenzyme A carboxylase of the invention will have a proline at position 1,786*(Am)* and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 1,811*(Am)* and at one or more additional amino acid positions. Acetyl-Coenzyme A carboxylase enzymes of the invention will typically have an asparagine at position 1,811 *(Am).* In addition, enzymes of this embodiment will also comprise one or more of a leucine, threonine, a valine, or alanine at position 1,781 *(Am)*, a glycine at position 1,785*(Am)*, a proline at position 1,786*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).* In one embodiment, an acetyl-Coenzyme A carboxylase of the invention will have an asparagine at position 1,811*(Am)* and a leucine, a threonine, a valine, or an alanine at position 1,781*(Am).* In one embodiment, an acetyl-Coenzyme A carboxylase of the invention will have an asparagine at position 1,811*(Am)* and a glycine at position 1,785*(Am)*. In one embodiment, an acetyl-Coenzyme A carboxylase of the invention will have an asparagine at position 1,811*(Am)* and a proline at position 1,786*(Am)*. In one embodiment, an acetyl-Coenzyme A carboxylase of the invention will have an asparagine at position 1,811*(Am)* and a proline at position 1,824*(Am)*. In one embodiment, an acetyl-Coenzyme A carboxylase of the invention will have an asparagine at position 1,811*(Am)* and phenylalanine at position 1,864*(Am)*. In one embodiment, an acetyl-Coenzyme A carboxylase of the invention will have an asparagine at position 1,811*(Am)* and a cysteine or glycine at position 1,999*(Am)*. In one embodiment, an acetyl-Coenzyme A carboxylase of the invention will have an asparagine at position 1,811 *(Am)* and a cysteine or an arginine at position 2,027*(Am)*. In one embodiment, an acetyl-Coenzyme A carboxylase of the invention will have an asparagine at position 1,811 *(Am)* and a glycine at position 2,039*(Am).* In one embodiment, an acetyl-Coenzyme A carboxylase of the invention will have an asparagine at position 1,811*(Am)* and an asparagine at position 2,041*(Am).* In one embodiment, an acetyl-Coenzyme A carboxylase of the invention will have an asparagine at position 1,811 *(Am)* and phenylalanine, isoleucine or leucine at position 2,049*(Am)*. In one embodiment, an acetyl-Coenzyme A carboxylase of the invention will have an asparagine at position 1,811*(Am)* and a valine at position 2,059*(Am)*. In one embodiment, an acetyl-Coenzyme A carboxylase of the invention will have an asparagine at position 1,811*(Am)* and a leucine at position 2,074*(Am).* In one embodiment, an acetyl-Coenzyme A carboxylase of the invention will have an asparagine at position 1,811*(Am)* and a leucine, isoleucine, methionine or additional valine at position *2,075(Am).* In one embodiment, an acetyl-Coenzyme A carboxylase of the invention will have an asparagine at position 1,811*(Am)* and a glycine or threonine at position 2,078*(Am)*. In one embodiment, an acetyl-Coenzyme A carboxylase of the invention will have an asparagine at position 1,811 *(Am)* and a phenylalanine at position 2,079*(Am)*. In one embodiment, an acetyl-Coenzyme A carboxylase of the invention will have an asparagine at position 1,811 *(Am)* and a glutamic acid or deletion at position 2,080*(Am)*. In one embodiment, an acetyl-Coenzyme A carboxylase of the invention will have an asparagine at position 1,811 *(Am)* and a deletion at position 2,081*(Am).* In one embodiment, an acetyl-Coenzyme A carboxylase of the invention will have an asparagine at position 1,811*(Am)* and an arginine, tryptophan, phenylalanine, glycine, histidine, lysine, leucine, serine, threonine, or valine at position 2,088*(Am)*. In one embodiment, an acetyl-Coenzyme A carboxylase of the invention will have an asparagine at position 1,811*(Am)* and a glutamic acid at position *2,095(Am).* In one embodiment, an acetyl-Coenzyme A carboxylase of the invention will have an asparagine at position 1,811*(Am)* and an alanine or serine at position 2,096*(Am)*. In one embodiment, an acetyl-Coenzyme A carboxylase of the invention will have an asparagine at position 1,811*(Am)* and an alanine, glycine, proline, histidine, cysteine, or serine at position *2,098(Am).*

In one embodiment, an acetyl-Coenzyme A carboxylase of the invention differs from the corresponding wild-type acetyl-Coenzyme A carboxylase at amino acid position 1,824*(Am)* and at one or more additional amino acid positions. Acetyl-Coenzyme A carboxylase enzymes of the invention will typically have a proline at position 1,824*(Am)*. In addition, enzymes of this embodiment will also comprise one or more of a leucine, threonine, a valine, or alanine at position 1,781*(Am),* a glycine at position 1,785*(Am),* a proline at position 1,786*(Am)*, an asparagine at position 1,811*(Am),* a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am),* a phenylalanine, isoleucine or leucine at position 2,049*(Am)*, a valine at position 2,059*(Am)*, a leucine at position 2,074*(Am),* a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 1,864*(Am)* and at one or more additional amino acid positions. Acetyl-Coenzyme A carboxylase enzymes of the invention will typically have a phenylalanine at position 1,864*(Am)*. In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a cysteine or glycine at position 1,999*(Am)*, a cysteine or arginine at position 2,027*(Am)*, a glycine at position 2,039*(Am)*, an asparagine at position 2,041*(Am),* a phenylalanine, isoleucine or leucine at position 2,049*(Am)*, a valine at position 2,059*(Am)*, a leucine at position 2,074*(Am),* a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 1,999*(Am)* and at one or more additional amino acid positions. Acetyl-Coenzyme A carboxylase enzymes of the invention will typically have a cysteine or glycine at position 1,999*(Am)*. In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one embodiment, an acetyl-Coenzyme A carboxylase of the invention will have a cysteine or glycine at position 1,999*(Am)* and a leucine, a threonine, a valine, or an alanine at position 1,781(*Am*). In one embodiment, an acetyl-Coenzyme A carboxylase of the invention will have a cysteine or glycine at position 1,999*(Am)* and a glycine at position 1,785*(Am)*. In one embodiment, an acetyl-Coenzyme A carboxylase of the invention will have a cysteine or glycine at position 1,999*(Am)* and a proline at position 1,786*(Am)*. In one embodiment, an acetyl-Coenzyme A carboxylase of the invention will have a cysteine or glycine at position 1,999*(Am)* and have an asparagine at position 1,811*(Am)*. In one embodiment, an acetyl-Coenzyme A carboxylase of the invention will have a cysteine or glycine at position 1,999*(Am)* and a proline at position 1,824*(Am)*. In one embodiment, an acetyl-Coenzyme A carboxylase of the invention will have a cysteine or glycine at position 1,999*(Am)* and phenylalanine at position 1,864*(Am)*. In one embodiment, an acetyl-Coenzyme A carboxylase of the invention will have a cysteine or glycine at position 1,999*(Am)* and a cysteine or an arginine at position 2,027*(Am)*. In one embodiment, an acetyl-Coenzyme A carboxylase of the invention will have a cysteine or glycine at position 1,999*(Am)* and a glycine at position 2,039*(Am).* In one embodiment, an acetyl-Coenzyme A carboxylase of the invention will have a cysteine or glycine at position 1,999*(Am)* and an asparagine at position 2,041*(Am).* In one embodiment, an acetyl-Coenzyme A carboxylase of the invention will have a cysteine or glycine at position 1,999*(Am)* and a phenylalanine, isoleucine or leucine at position 2,049*(Am)*. In one embodiment, an acetyl-Coenzyme A carboxylase of the invention will have a cysteine or glycine at position 1,999*(Am)* and a cysteine or a valine at position 2,059*(Am)*. In one embodiment, an acetyl-Coenzyme A carboxylase of the invention will have a cysteine or glycine at position 1,999*(Am)* and a leucine at position 2,074*(Am).* In one embodiment, an acetyl-Coenzyme A carboxylase of the invention will have a cysteine or glycine at position 1,999*(Am)* and a leucine, isoleucine, methionine or additional valine at position 2,075 *(Am).* In one embodiment, an acetyl-Coenzyme A carboxylase of the invention will have a cysteine or glycine at position 1,999*(Am)* and a glycine or threonine at position 2,078*(Am)*. In one embodiment, an acetyl-Coenzyme A carboxylase of the invention will have a cysteine or glycine at position 1,999*(Am)* and a phenylalanine at position 2,079*(Am).* In one embodiment, an acetyl-Coenzyme A carboxylase of the invention will have a cysteine or glycine at position 1,999*(Am)* and a glutamic acid or deletion at position 2,080*(Am)*. In one embodiment, an acetyl-Coenzyme A carboxylase of the invention will have a cysteine or glycine at position 1,999*(Am)* and a deletion at position 2,081*(Am).* In one embodiment, an acetyl-Coenzyme A carboxylase of the invention will have a cysteine or glycine at position 1,999*(Am)* and an arginine, tryptophan, phenylalanine, glycine, histidine, lysine, leucine, serine, threonine, or valine at position 2,088*(Am)*. In one embodiment, an acetyl-Coenzyme A carboxylase of the invention will have a cysteine or glycine at position 1,999*(Am)* and a glutamic acid at position 2,095*(Am)*. In one embodiment, an acetyl-Coenzyme A carboxylase of the invention will have a cysteine or glycine at position 1,999*(Am)* and an alanine or serine at position 2,096*(Am)*. In one embodiment, an acetyl-Coenzyme A carboxylase of the invention will have a cysteine or glycine at position 1,999*(Am)* and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 2,027*(Am)* and at one or more additional amino acid positions. Acetyl-Coenzyme A carboxylase enzymes of the invention will typically have a cysteine or arginine at position 2,027*(Am)*. In addition, enzymes of this embodiment will also comprise one or more of a leucine, threonine, a valine, or alanine at position 1,781*(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a glycine at position 2,039*(Am)*, an asparagine at position 2,041*(Am)*, a phenylalanine, isoleucine or leucine at position 2,049*(Am)*, a valine at position 2,059*(Am),* a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).* In one embodiment, an acetyl-Coenzyme A carboxylase of the invention will have a cysteine or arginine at position 2,027*(Am)* and a leucine, a threonine, a valine, or an alanine at position 1,781*(Am).* In one embodiment, an acetyl-Coenzyme A carboxylase of the invention will have a cysteine or arginine at position 2,027*(Am)* and a glycine at position 1,785*(Am)*. In one embodiment, an acetyl-Coenzyme A carboxylase of the invention will have a cysteine or arginine at position 2,027*(Am)* and a proline at position 1,786*(Am)*. In one embodiment, an acetyl-Coenzyme A carboxylase of the invention will have a cysteine or arginine at position 2,027*(Am)* and have an asparagine at position 1,811*(Am).* In one embodiment, an acetyl-Coenzyme A carboxylase of the invention will have a cysteine or arginine at position 2,027*(Am)* and have a proline at position 1,824*(Am)*. In one embodiment, an acetyl-Coenzyme A carboxylase of the invention will have a cysteine or arginine at position 2,027*(Am)* and have a phenylalanine at position 1,864*(Am).* In one embodiment, an acetyl-Coenzyme A carboxylase of the invention will have a cysteine or arginine at position 2,027*(Am)* and a cysteine or glycine at position 1,999*(Am)*. In one embodiment, an acetyl-Coenzyme A carboxylase of the invention will have a cysteine or arginine at position 2,027*(Am)* and have a glycine at position 2,039*(Am)*. In one embodiment, an acetyl-Coenzyme A carboxylase of the invention will have a cysteine or arginine at position 2,027*(Am)* and an asparagine at position 2,041*(Am).* In one embodiment, an acetyl-Coenzyme A carboxylase of the invention will have a cysteine or arginine at position 2,027*(Am)* and a phenylalanine, isoleucine or leucine at position 2,049*(Am)*. In one embodiment, an acetyl-Coenzyme A carboxylase of the invention will have a cysteine or arginine at position 2,027*(Am)* and have a valine at position 2,059*(Am)*. In one embodiment, an acetyl-Coenzyme A carboxylase of the invention will have a cysteine or arginine at position 2,027*(Am)* and a leucine at position 2,074*(Am).* In one embodiment, an acetyl-Coenzyme A carboxylase of the invention will have a cysteine or arginine at position 2,027*(Am)* and a leucine, isoleucine, methionine or additional valine at position 2,075*(Am).* In one embodiment, an acetyl-Coenzyme A carboxylase of the invention will have a cysteine or arginine at position 2,027*(Am)* and a glycine or threonine at position 2,078*(Am)*. In one embodiment, an acetyl-Coenzyme A carboxylase of the invention will have a cysteine or arginine at position 2,027*(Am)* and a phenylalanine at position 2,079*(Am)*. In one embodiment, an acetyl-Coenzyme A carboxylase of the invention will have a cysteine or arginine at position 2,027*(Am)* and a glutamic acid or deletion at position 2,080*(Am)*. In one embodiment, an acetyl-Coenzyme A carboxylase of the invention will have a cysteine or arginine at position 2,027*(Am)* and a deletion at position 2,081*(Am).* In one embodiment, an acetyl-Coenzyme A carboxylase of the invention will have a cysteine or arginine at position 2,027*(Am)* and an arginine, tryptophan, phenylalanine, glycine, histidine, lysine, leucine, serine, threonine, or valine at position 2,088*(Am)*. In one embodiment, an acetyl-Coenzyme A carboxylase of the invention will have a cysteine or arginine at position 2,027*(Am)* and a glutamic acid at position 2,095*(Am)*. In one embodiment, an acetyl-Coenzyme A carboxylase of the invention will have a cysteine or arginine at position 2,027*(Am)* and an alanine or serine at position 2,096*(Am)*. In one embodiment, an acetyl-Coenzyme A carboxylase of the invention will have a cysteine or arginine at position 2,027*(Am)* and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 2,039*(Am)* and at one or more additional amino acid positions. Acetyl-Coenzyme A carboxylase enzymes of the invention will typically have a glycine at position 2,039*(Am).* In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, an asparagine at position 2,041*(Am),* a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080(*Am*), a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 2,041*(Am)* and at one or more additional amino acid positions. Acetyl-Coenzyme A carboxylase enzymes of the invention will typically have an asparagine at position 2,041*(Am).* In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine or serine at position 2,098*(Am).* In one embodiment, an acetyl-Coenzyme A carboxylase of the invention will have an asparagine at position 2,041*(Am)* and a leucine, a threonine, a valine, or an alanine at position 1,781*(Am)*. In one embodiment, an acetyl-Coenzyme A carboxylase of the invention will have an asparagine at position 2,041*(Am)* and a glycine at position 1,785*(Am).* In one embodiment, an acetyl-Coenzyme A carboxylase of the invention will have an asparagine at position 2,041*(Am)* and a proline at position 1,786*(Am)*. In one embodiment, an acetyl-Coenzyme A carboxylase of the invention will have an asparagine at position 2,041*(Am)* and have an asparagine at position 1,811*(Am)*. In one embodiment, an acetyl-Coenzyme A carboxylase of the invention will have an asparagine at position 2,041*(Am)* and a proline at position 1824*(Am)*. In one embodiment, an acetyl-Coenzyme A carboxylase of the invention will have an asparagine at position 2,041*(Am)* and a phenylalanine at position 1864*(Am)*. In one embodiment, an acetyl-Coenzyme A carboxylase of the invention will have an asparagine at position 2,041*(Am)* and a cysteine or glycine at position 1,999*(Am)*. In one embodiment, an acetyl-Coenzyme A carboxylase of the invention will have an asparagine at position 2,041*(Am)* and a cysteine or arginine at position 2,027*(Am)*. In one embodiment, an acetyl-Coenzyme A carboxylase of the invention will have an asparagine at position 2,041*(Am)* and a glycine at position 2039*(Am)*. In one embodiment, an acetyl-Coenzyme A carboxylase of the invention will have an asparagine at position 2,041*(Am)* and an asparagine at position 2,041*(Am).* In one embodiment, an acetyl-Coenzyme A carboxylase of the invention will have an asparagine at position 2,041*(Am)* and a phenylalanine, isoleucine or leucine at position 2,049*(Am)* In one embodiment, an acetyl-Coenzyme A carboxylase of the invention will have an asparagine at position 2,041*(Am)* and a valine at position 2,059*(Am)..* In one embodiment, an acetyl-Coenzyme A carboxylase of the invention will have an asparagine at position 2,041*(Am)* and a leucine at position 2,074*(Am).* In one embodiment, an acetyl-Coenzyme A carboxylase of the invention will have an asparagine at position 2,041*(Am)* and a leucine, isoleucine, methionine or additional valine at position 2,075*(Am).* In one embodiment, an acetyl-Coenzyme A carboxylase of the invention will have an asparagine at position 2,041 *(Am)* and a glycine or threonine at position 2,078*(Am)*. In one embodiment, an acetyl-Coenzyme A carboxylase of the invention will have an asparagine at position 2,041*(Am)* and a phenylalanine at position 2079*(Am).* In one embodiment, an acetyl-Coenzyme A carboxylase of the invention will have an asparagine at position 2,041*(Am)* and a glutamic acid or a deletion at position 2080*(Am).* In one embodiment, an acetyl-Coenzyme A carboxylase of the invention will have an asparagine at position 2,041*(Am)* and a deletion at position 2081*(Am).* In one embodiment, an acetyl-Coenzyme A carboxylase of the invention will have an isoleucine at position 2,041*(Am)* and an arginine, tryptophan, phenylalanine, glycine, histidine, lysine, leucine, serine, threonine, or valine at position 2,088*(Am)*. In one embodiment, an acetyl-Coenzyme A carboxylase of the invention will have an isoleucine at position 2,041*(Am)* and a glutamic acid at position 2,095*(Am)*. In one embodiment, an acetyl-Coenzyme A carboxylase of the invention will have an isoleucine at position 2,041*(Am)* and an alanine or serine at position 2,096*(Am)*. In one embodiment, an acetyl-Coenzyme A carboxylase of the invention will have an isoleucine at position 2,041*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,049*(Am)* and at one or more additional amino acid positions. Acetyl-Coenzyme A carboxylase enzymes of the invention will typically have a phenylalanine, isoleucine or leucine at position 2,049*(Am)*. In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039(Am), an asparagine at position 2,041 *(Am)*, a valine at position *2,059(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position *2,095(Am)*, an alanine or serine at position 2,096*(Am)*, and an alanine, glycine, proline, histidine, cysteine, or serine at position 2,098*(Am).* In one embodiment, an acetyl-Coenzyme A carboxylase of the invention will have a phenylalanine, isoleucine or leucine at position 2,049*(Am)* and a leucine, a threonine, a valine, or an alanine at position 1,781 *(Am).* In one embodiment, an acetyl-Coenzyme A carboxylase of the invention will have a phenylalanine, isoleucine or leucine at position 2,049*(Am)* and a glycine at position 1,785*(Am)*. In one embodiment, an acetyl-Coenzyme A carboxylase of the invention will have a phenylalanine, isoleucine or leucine at position 2,049*(Am)* and a proline at position 1,786*(Am)*. In one embodiment, an acetyl-Coenzyme A carboxylase of the invention will have a phenylalanine, isoleucine or leucine at position 2,049*(Am)* and have an asparagine at position 1,811*(Am).* In one embodiment, an acetyl-Coenzyme A carboxylase of the invention will have a phenylalanine, isoleucine or leucine at position 2,049*(Am)* and a proline at position 1824*(Am)*. In one embodiment, an acetyl-Coenzyme A carboxylase of the invention will have a phenylalanine, isoleucine or leucine at position 2,049*(Am)* and a phenylalanine at position 1864*(Am)*. In one embodiment, an acetyl-Coenzyme A carboxylase of the invention will have a phenylalanine, isoleucine or leucine at position 2,049*(Am)* and a cysteine or glycine at position 1,999*(Am)*. In one embodiment, an acetyl-Coenzyme A carboxylase of the invention will have a phenylalanine, isoleucine or leucine at position 2,049*(Am)* and a cysteine or an arginine at position 2,027*(Am).* In one embodiment, an acetyl-Coenzyme A carboxylase of the invention will have a phenylalanine, isoleucine or leucine at position 2,049*(Am)* and a glycine at position 2039*(Am)*. In one embodiment, an acetyl-Coenzyme A carboxylase of the invention will have a phenylalanine, isoleucine or leucine at position 2,049*(Am)* and an asparagine at position 2,041*(Am).* In one embodiment, an acetyl-Coenzyme A carboxylase of the invention will have a phenylalanine, isoleucine or leucine at position 2,049*(Am)* and a valine at position 2059*(Am).* In one embodiment, an acetyl-Coenzyme A carboxylase of the invention will have a phenylalanine, isoleucine or leucine at position 2,049*(Am)* and a leucine at position 2,074*(Am).* In one embodiment, an acetyl-Coenzyme A carboxylase of the invention will have a phenylalanine, isoleucine or leucine at position 2,049*(Am)* and a leucine, isoleucine methionine, or additional valine at position 2,075*(Am).* In one embodiment, an acetyl-Coenzyme A carboxylase of the invention will have a phenylalanine, isoleucine or leucine at position 2,049*(Am)* and a glycine or threonine at position 2,078*(Am).* In one embodiment, an acetyl-Coenzyme A carboxylase of the invention will have a phenylalanine, isoleucine or leucine at position 2,049*(Am)* and a phenylalanine at position 2079*(Am).* In one embodiment, an acetyl-Coenzyme A carboxylase of the invention will have a phenylalanine, isoleucine or leucine at position 2,049*(Am)* and a glutamic acid or a deletion at position 2080*(Am)*. In one embodiment, an acetyl-Coenzyme A carboxylase of the invention will have a phenylalanine, isoleucine or leucine at position 2,049*(Am)* and a deletion at position 2081*(Am)*. In one embodiment, an acetyl-Coenzyme A carboxylase of the invention will have a phenylalanine, isoleucine or leucine at position 2,049*(Am)* and an arginine, tryptophan, phenylalanine, glycine, histidine, lysine, serine, threonine, or valine at position 2,088*(Am)*. In one embodiment, an acetyl-Coenzyme A carboxylase of the invention will have a phenylalanine, isoleucine or leucine at position 2,049*(Am)* and a glutamic acid at position 2,095*(Am).* In one embodiment, an acetyl-Coenzyme A carboxylase of the invention will have a phenylalanine, isoleucine or leucine at position 2,049*(Am)* and an alanine or serine at position 2,096*(Am)*. In one embodiment, an acetyl-Coenzyme A carboxylase of the invention will have a phenylalanine, isoleucine or leucine at position 2,049*(Am)* and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 2,059*(Am)* and at one or more additional amino acid positions. Acetyl-Coenzyme A carboxylase enzymes of the invention will typically have a valine at position 2,059*(Am).* In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a leucine at position 2,074*(Am),* a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine or tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,074*(Am)* and at one or more additional amino acid positions. Acetyl-Coenzyme A carboxylase enzymes of the invention will typically have a leucine at position 2,074*(Am).* In addition, enzymes of this embodiment will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position *2,049(Am),* a valine at position 2,059*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one embodiment, an acetyl-Coenzyme A carboxylase of the invention will have a leucine at position 2,074*(Am)* and a leucine, a threonine, a valine, or an alanine at position 1,781*(Am).* In one embodiment, an acetyl-Coenzyme A carboxylase of the invention will have a leucine at position 2,074*(Am)* and a glycine at position 1,785*(Am)*. In one embodiment, an acetyl-Coenzyme A carboxylase of the invention will have a leucine at position 2,074*(Am)* and a proline at position 1,786*(Am)*. In one embodiment, an acetyl-Coenzyme A carboxylase of the invention will have a leucine at position 2,074*(Am)* and have an asparagine at position 1,811*(Am).* In one embodiment, an acetyl-Coenzyme A carboxylase of the invention will have a leucine at position 2,074*(Am)* and a proline at position 1824*(Am)*. In one embodiment, an acetyl-Coenzyme A carboxylase of the invention will have a leucine at position 2,074*(Am)* and a phenylalanine at position 1864*(Am)*. In one embodiment, an acetyl-Coenzyme A carboxylase of the invention will have a leucine at position 2,074*(Am)* and a cysteine or glycine at position 1,999*(Am)*. In one embodiment, an acetyl-Coenzyme A carboxylase of the invention will have a leucine at position 2,074*(Am)* and a cysteine or an arginine at position 2,027*(Am)*. In one embodiment, an acetyl-Coenzyme A carboxylase of the invention will have a leucine at position 2,074*(Am)* and a glycine at position 2039*(Am)*. In one embodiment, an acetyl-Coenzyme A carboxylase of the invention will have a leucine at position 2,074*(Am)* and an asparagine at position 2,041 *(Am).* In one embodiment, an acetyl-Coenzyme A carboxylase of the invention will have a leucine at position 2,074*(Am)* and a phenylalanine, leucine or isoleucine at position 2,049*(Am).* In one embodiment, an acetyl-Coenzyme A carboxylase of the invention will have a leucine at position 2,074*(Am)* and a valine at position 2059*(Am).* In one embodiment, an acetyl-Coenzyme A carboxylase of the invention will have a leucine at position *2,074(Am)* and a leucine, isoleucine methionine, or additional valine at position 2,075*(Am).* In one embodiment, an acetyl-Coenzyme A carboxylase of the invention will have a leucine at position 2,074*(Am)* and a glycine or threonine at position 2,078*(Am)*. In one embodiment, an acetyl-Coenzyme A carboxylase of the invention will have a leucine at position 2,074*(Am)* and a phenylalanine at position *2079(Am).* In one embodiment, an acetyl-Coenzyme A carboxylase of the invention will have a leucine at position 2,074*(Am)* and a glutamic acid or a deletion at position 2080*(Am).* In one embodiment, an acetyl-Coenzyme A carboxylase of the invention will have a leucine at position 2,074*(Am)* and a deletion at position 2081*(Am).* In one embodiment, an acetyl-Coenzyme A carboxylase of the invention will have a leucine at position 2,074*(Am)* and an arginine, tryptophan, phenylalanine, glycine, histidine, lysine, serine, threonine, or valine at position 2,088*(Am)*. In one embodiment, an acetyl-Coenzyme A carboxylase of the invention will have a leucine at position 2,074*(Am)* and a glutamic acid at position 2,095*(Am)*. In one embodiment, an acetyl-Coenzyme A carboxylase of the invention will have a leucine at position 2,074*(Am)* and an alanine or serine at position 2,096*(Am)*. In one embodiment, an acetyl-Coenzyme A carboxylase of the invention will have a leucine at position 2,074*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,075*(Am)* and at one or more additional amino acid positions. Acetyl-Coenzyme A carboxylase enzymes of the invention will typically have a leucine, isoleucine, methionine or additional valine at position 2,075*(Am).* In addition, enzymes of this embodiment will also comprise one or more of a leucine, threonine, or alanine at position 1,781*(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am),* a phenylalanine, isoleucine or leucine at position 2,049*(Am)*, a valine at position 2,059*(Am)*, a leucine at position 2,074*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081 *(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).* In one embodiment, an acetyl-Coenzyme A carboxylase of the invention will have a leucine, isoleucine, methionine or additional valine at position 2,075*(Am)* and a leucine, a threonine, a valine, or an alanine at position 1,781*(Am).* In one embodiment, an acetyl-Coenzyme A carboxylase of the invention will have a leucine, isoleucine, methionine or additional valine at position 2,075*(Am)* and a glycine at position 1,785*(Am)*. In one embodiment, an acetyl-Coenzyme A carboxylase of the invention will have a leucine, isoleucine, methionine or additional valine at position 2,075*(Am)* and a proline at position 1,786*(Am)*. In one embodiment, an acetyl-Coenzyme A carboxylase of the invention will have a leucine, isoleucine, methionine or additional valine at position 2,075*(Am)* and have an asparagine at position 1,811*(Am).* In one embodiment, an acetyl-Coenzyme A carboxylase of the invention will have a leucine, isoleucine, methionine or additional valine at position 2,075*(Am)* and a cysteine or glycine at position 1,999*(Am)*. In one embodiment, an acetyl-Coenzyme A carboxylase of the invention will have a leucine, isoleucine, methionine or additional valine at position 2,075*(Am)* and a cysteine or arginine at position 2,027*(Am)*. In one embodiment, an acetyl-Coenzyme A carboxylase of the invention will have a leucine, isoleucine, methionine or additional valine at position 2,075*(Am)* and an isoleucine at position 2,041*(Am).* In one embodiment, an acetyl-Coenzyme A carboxylase of the invention will have a leucine, isoleucine, methionine or additional valine at position 2,075*(Am) an*d a phenylalanine, isoleucine or leucine at position 2,049*(Am).* In one embodiment, an acetyl-Coenzyme A carboxylase of the invention will have a leucine, isoleucine, methionine or additional valine at position 2,075*(Am)* and a leucine at position 2,074*(Am).* In one embodiment, an acetyl-Coenzyme A carboxylase of the invention will have a leucine, isoleucine, methionine or additional valine at position 2,075*(Am)* and a glycine or threonine at position 2,078*(Am)*. In one embodiment, an acetyl-Coenzyme A carboxylase of the invention will have a leucine, isoleucine, methionine or additional valine at position 2,075*(Am)* and an arginine or tryptophan, phenylalanine, glycine, histidine, lysine, leucine, serine, threonine, or valine at position 2,088*(Am)*. In one embodiment, an acetyl-Coenzyme A carboxylase of the invention will have a leucine, isoleucine, methionine or additional valine at position 2,075*(Am)* and an alanine or serine at position 2,096*(Am)*. In one embodiment, an acetyl-Coenzyme A carboxylase of the invention will have a leucine, isoleucine, methionine or additional valine at position 2,075*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,078*(Am)* and at one or more additional amino acid positions. Acetyl-Coenzyme A carboxylase enzymes of the invention will typically have a glycine or threonine at position 2,078*(Am)*. In addition, enzymes of this embodiment will also comprise one or more of a leucine, threonine, a valine, or alanine at position 1,781*(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine or serine at position 2,098*(Am).* In one embodiment, an acetyl-Coenzyme A carboxylase of the invention will have a glycine or threonine at position 2,078*(Am)* and a leucine, a threonine or an alanine at position 1,781*(Am).* In one embodiment, an acetyl-Coenzyme A carboxylase of the invention will have a glycine or threonine at position 2,078*(Am)* and a glycine at position 1,785*(Am)*. In one embodiment, an acetyl-Coenzyme A carboxylase of the invention will have a glycine or threonine at position 2,078*(Am)* and a proline at position 1,786*(Am)*. In one embodiment, an acetyl-Coenzyme A carboxylase of the invention will have a glycine or threonine at position 2,078*(Am)* and an asparagine at position 1,811*(Am).* In one embodiment, an acetyl-Coenzyme A carboxylase of the invention will have a glycine or threonine at position 2,078*(Am)* and a cysteine or glycine at position 1,999*(Am)*. In one embodiment, an acetyl-Coenzyme A carboxylase of the invention will have a glycine or threonine at position 2,078*(Am)* and a cysteine or arginine at position 2,027*(Am)*. In one embodiment, an acetyl-Coenzyme A carboxylase of the invention will have a glycine or threonine at position 2,078*(Am)* and an isoleucine at position 2,041 *(Am).* In one embodiment, an acetyl-Coenzyme A carboxylase of the invention will have a glycine or threonine at position 2,078*(Am)* and a phenylalanine, isoleucine or leucine at position 2,049*(Am)*. In one embodiment, an acetyl-Coenzyme A carboxylase of the invention will have a glycine or threonine at position 2,078*(Am)* and a leucine at position 2,074*(Am).* In one embodiment, an acetyl-Coenzyme A carboxylase of the invention will have a glycine or threonine at position 2,078*(Am)* and a leucine, isoleucine, methionine or additional valine at position 2,075*(Am).* In one embodiment, an acetyl-Coenzyme A carboxylase of the invention will have a glycine or threonine at position 2,078*(Am)* and an arginine, tryptophan, phenylalanine, glycine, histidine, lysine, leucine, serine, threonine, or valine at position 2,088*(Am)*. In one embodiment, an acetyl-Coenzyme A carboxylase of the invention will have a glycine or threonine at position 2,078*(Am)* and an alanine or serine at position 2,096*(Am)*. In one embodiment, an acetyl-Coenzyme A carboxylase of the invention will have a glycine or threonine at position 2,078*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,079*(Am)* and at one or more additional amino acid positions. Acetyl-Coenzyme A carboxylase enzymes of the invention will typically have a phenylalanine at position 2,079*(Am)*. In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041 *(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,080*(Am)* and at one or more additional amino acid positions. Acetyl-Coenzyme A carboxylase enzymes of the invention will typically have a glutamic acid or a deletion at position 2,080*(Am)*. In addition, enzymes of this embodiment will also comprise one or more of a leucine, threonine, valine, or alanine at position 1,781 *(Am),* a glycine at position 1,785*(Am)*, a proline at position 1,786*(Am)*, an asparagine at position *1,811(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041 *(Am),* a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,081*(Am)* and at one or more additional amino acid positions. Acetyl-Coenzyme A carboxylase enzymes of the invention will typically have a deletion at position 2,081*(Am)*,. In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811 *(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position *2,078(Am),* a phenylalanine at position 2,079*(Am)*, a glutamic acid at position 2,080*(Am)*, a deletion at position 2,080*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,088(Am) and at one or more additional amino acid positions. Acetyl-Coenzyme A carboxylase enzymes of the invention will typically have an arginine, tryptophan, phenylalanine, glycine, histidine, lysine, leucine, serine, threonine, or valine at position 2,088(Am). In addition, enzymes of this embodiment will also comprise one or more of a leucine, threonine, valine, or alanine at position 1,781*(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am),* a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081 *(Am),* a glutamic acid at position 2,095*(Am)*, an alanine or serine at position 2,096*(Am)*, and an alanine, glycine, proline, histidine, cysteine, or serine at position 2,098*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a leucine, a threonine, valine, or an alanine at position 1,781*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a glycine at position 1,785*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and a proline at position 1,786*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and an asparagine at position 1,811*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a cysteine or glycine at position 1,999*(Am)*. In one embodiment, an acetyl-Coenzyme A carboxylase of the invention will have an arginine or tryptophan, phenylalanine, glycine, histidine, lysine, leucine, serine, threonine, or valine at position 2,088*(Am)* and a cysteine or arginine at position 2,027*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and an isoleucine at position 2,041*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a phenylalanine, isoleucine or leucine at position 2,049*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and a leucine at position 2,074*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a leucine, isoleucine, methionine or additional valine at position 2,075*(Am).* In one embodiment, an acetyl-Coenzyme A carboxylase of the invention will have an arginine, tryptophan, phenylalanine, glycine, histidine, lysine, leucine, serine, threonine, or valine at position 2,088*(Am)* and a glycine or threonine at position 2,078*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and an alanine or serine at position 2,096*(Am)*. In one embodiment, an acetyl-Coenzyme A carboxylase of the invention will have an arginine, tryptophan, phenylalanine, glycine, histidine, lysine, leucine, serine, threonine, or valine at position 2,088*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,095*(Am)* and at one or more additional amino acid positions. Acetyl-Coenzyme A carboxylase enzymes of the invention will typically have a glutamic acid at position 2,095*(Am)*. In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824(Am), a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081 *(Am),* an arginine or tryptophan, phenylalanine, glycine, histidine, lysine, leucine, serine, threonine, or valine at position 2,088*(Am)*, an alanine or serine at position 2,096*(Am)*, and an alanine, glycine, proline, histidine, cysteine, or serine at position 2,098*(Am)*.

In one embodiment, an acetyl-Coenzyme A carboxylase of the invention differs from the corresponding wild-type acetyl-Coenzyme A carboxylase at amino acid position 2,096*(Am)* and at one or more additional amino acid positions. Acetyl-Coenzyme A carboxylase enzymes of the invention will typically have an alanine or serine at position 2,096*(Am)*. In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, and an alanine, glycine, proline, histidine, cysteine, or serine at position 2,098*(Am).* In one embodiment, an acetyl-Coenzyme A carboxylase of the invention will have an alanine or serine at position 2,096*(Am)* and a leucine, a threonine or an alanine at position 1,781*(Am).* In one embodiment, an acetyl-Coenzyme A carboxylase of the invention will have an alanine or serine at position 2,096*(Am)* and a glycine at position 1,785*(Am)*. In one embodiment, an acetyl-Coenzyme A carboxylase of the invention will have an alanine or serine at position 2,096*(Am)* and a proline at position 1,786*(Am)*. In one embodiment, an acetyl-Coenzyme A carboxylase of the invention will have an alanine or serine at position 2,096*(Am)* and an asparagine at position 1,811*(Am).* In one embodiment, an acetyl-Coenzyme A carboxylase of the invention will have an alanine or serine at position 2,096*(Am)* and a cysteine or glycine at position 1,999*(Am)*. In one embodiment, an acetyl-Coenzyme A carboxylase of the invention will have an alanine or serine at position 2,096*(Am)* and a cysteine or arginine at position 2,027*(Am)*. In one embodiment, an acetyl-Coenzyme A carboxylase of the invention will have an alanine or serine at position 2,096*(Am)* and an isoleucine at position 2,041*(Am).* In one embodiment, an acetyl-Coenzyme A carboxylase of the invention will have an alanine or serine at position 2,096*(Am)* and a phenylalanine, isoleucine or leucine at position 2,049*(Am)*. In one embodiment, an acetyl-Coenzyme A carboxylase of the invention will have an alanine or serine at position 2,096*(Am)* and a leucine at position 2,074*(Am).* In one embodiment, an acetyl-Coenzyme A carboxylase of the invention will have an alanine or serine at position 2,096*(Am)* and a leucine, isoleucine, methionine or additional valine at position 2,075*(Am).* In one embodiment, an acetyl-Coenzyme A carboxylase of the invention will have an alanine or serine at position 2,096*(Am)* and a glycine or threonine at position 2,078*(Am)*. In one embodiment, an acetyl-Coenzyme A carboxylase of the invention will have an alanine or serine at position 2,096*(Am)* and an an arginine, tryptophan, phenylalanine, glycine, histidine, lysine, leucine, serine, threonine, or valine at position 2,088*(Am)*. In one embodiment, an acetyl-Coenzyme A carboxylase of the invention will have an alanine or serine at position 2,096*(Am)* and an alanine, glycine, proline, histidine, cysteine, or serine at position 2,098*(Am).*

In one embodiment, an acetyl-Coenzyme A carboxylase of the invention differs from the corresponding wild-type acetyl-Coenzyme A carboxylase at amino acid position 2,098*(Am)* and at one or more additional amino acid positions. Acetyl-Coenzyme A carboxylase enzymes of the invention will typically have an alanine, glycine, proline, histidine, cysteine, or serine at position 2,098*(Am).* In addition, enzymes of this embodiment will also comprise one or more of a leucine, threonine, valine, or alanine at position 1,781*(Am)*, a glycine at position 1,785*(Am)*, a proline at position 1,786*(Am)*, an asparagine at position 1,811 *(Am),* a proline at position 1,824*(Am)*, a phenylalanine at position 1,864*(Am)*, a cysteine or glycine at position 1,999*(Am)*, a cysteine or arginine at position 2,027*(Am),* a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, and an alanine or serine at position 2,096*(Am)*. In one embodiment, an acetyl-Coenzyme A carboxylase of the invention will have an alanine, glycine, proline, histidine, cysteine, or serine at position 2,098*(Am)* and a leucine, a threonine, valine, or an alanine at position 1,781 *(Am).* In one embodiment, an acetyl-Coenzyme A carboxylase of the invention will have an alanine, glycine, proline, histidine, cysteine, or serine at position 2,098*(Am)* and a glycine at position 1,785*(Am)*. In one embodiment, an acetyl-Coenzyme A carboxylase of the invention will have an alanine, glycine, proline, histidine, cysteine, or serine at position 2,098*(Am)* and a proline at position 1,786*(Am)*. In one embodiment, an acetyl-Coenzyme A carboxylase of the invention will have an alanine, glycine, proline, histidine, cysteine, or serine at position 2,098*(Am)* and an asparagine at position 1,811*(Am).* In one embodiment, an acetyl-Coenzyme A carboxylase of the invention will have an alanine, glycine, proline, histidine, cysteine, or serine at position 2,098*(Am)* and a cysteine or glycine at position *1,999(Am).* In one embodiment, an acetyl-Coenzyme A carboxylase of the invention will have an alanine, glycine, proline, histidine, cysteine, or serine at position 2,098*(Am)* and a cysteine or arginine at position 2,027*(Am).* In one embodiment, an acetyl-Coenzyme A carboxylase of the invention will have an alanine, glycine, proline, histidine, cysteine, or serine at position 2,098*(Am)* and an isoleucine at position 2,041 *(Am).* In one embodiment, an acetyl-Coenzyme A carboxylase of the invention will have an alanine, glycine, proline, histidine, cysteine, or serine at position 2,098*(Am)* and a phenylalanine, isoleucine or leucine at position 2,049*(Am)*. In one embodiment, an acetyl-Coenzyme A carboxylase of the invention will have an alanine, glycine, proline, histidine, cysteine, or serine at position 2,098*(Am)* and a leucine at position 2,074*(Am).* In one embodiment, an acetyl-Coenzyme A carboxylase of the invention will have an alanine, glycine, proline, histidine, cysteine, or serine at position 2,098*(Am)* and a leucine, isoleucine, methionine or additional valine at position 2,075*(Am).* In one embodiment, an acetyl-Coenzyme A carboxylase of the invention will have an alanine, glycine, proline, histidine, cysteine, or serine at position 2,098*(Am)* and a glycine or threonine at position 2,078*(Am).* In one embodiment, an acetyl-Coenzyme A carboxylase of the invention will have an alanine, glycine, proline, histidine, cysteine, or serine at position 2,098*(Am)* and an arginine or tryptophan, phenylalanine, glycine, histidine, lysine, leucine, serine, threonine, or valine at position 2,088*(Am)*. In one embodiment, an acetyl-Coenzyme A carboxylase of the invention will have an alanine, glycine, proline, histidine, cysteine, or serine at position 2,098*(Am)* and an alanine or serine at position 2,096*(Am)*.

In one embodiment, the invention includes acetyl-Coenzyme A carboxylases having an isoleucine at position 2,075*(Am)* and a glycine at position 1,999*(Am)*; acetyl-Coenzyme A carboxylases having a methionine at position 2,075*(Am)* and a glutamic acid at position 2,080*(Am)*, acetyl-Coenzyme A carboxylases having a methionine at position 2,075*(Am)* and a glutamic acid at position 2,095*(Am)*; acetyl-Coenzyme A carboxylases having a glycine at position 2,078*(Am)* and a valine at position 2,041*(Am)*; acetyl-Coenzyme A carboxylases having a glycine at position 2,078*(Am)* and a glycine at position 2,039*(Am)*; acetyl-Coenzyme A carboxylases having a glycine at position 2,078*(Am)* and an alanine at position 2,049*(Am)*; acetyl-Coenzyme A carboxylases having a glycine at position 2,078*(Am)* and a cysteine at position 2,049*(Am)*; acetyl-Coenzyme A carboxylases having a glycine at position 2,078*(Am)* and a serine at position 2,049*(Am)*; acetyl-Coenzyme A carboxylases having a glycine at position 2,078*(Am)* and a threonine at position 2,049*(Am)*; acetyl-Coenzyme A carboxylases having a glycine at position 2,078*(Am)* and a valine at position 2,059*(Am)*; acetyl-Coenzyme A carboxylases having a glycine at position 2,078*(Am)* and a phenylalanine at position 2,079*(Am)*; acetyl-Coenzyme A carboxylases having a glycine at position 2,078*(Am)* and a proline at position at position 2,079*(Am)*; and acetyl-Coenzyme A carboxylases having a glycine at position 2,078*(Am)* and a glycine at position 2,088*(Am)*.

In a preferred embodiment, the invention includes acetyl-Coenzyme A carboxylases having a leucine at position 1,781*(Am)* and a proline at position 1,824*(Am)*; acetyl-Coenzyme A carboxylases having a leucine at position 1,781*(Am)* and an arginine at position 2027*(Am)*; and acetyl-Coenzyme A carboxylases having a glycine at position 2,078*(Am)* and a proline at position 1,824*(Am)*.

In a more preferred embodiment, the invention includes, acetyl-Coenzyme A carboxylases having a leucine at position 1,781*(Am)* and a phenylalanine at position 2,049*(Am)*; acetyl-Coenzyme A carboxylases having an alanine at position 2,098*(Am)* and a leucine at position 2,049*(Am)*; acetyl-Coenzyme A carboxylases having an alanine at position 2,098*(Am)* and a histidine at position 2088*(Am)*; acetyl-Coenzyme A carboxylases having an alanine at position 2,098*(Am)* and a phenylalanine at position 2,088*(Am)*; acetyl-Coenzyme A carboxylases having an alanine at position 2,098*(Am)* and a lysine at position 2,088*(Am)*; acetyl-Coenzyme A carboxylases having an alanine at position 2,098*(Am)* and a leucine at position 2,088*(Am)*; acetyl-Coenzyme A carboxylases having an alanine at position 2,098*(Am)* and a threonine at position 2,088*(Am)*; acetyl-Coenzyme A carboxylases having a glycine at position 2,098*(Am)* and a glycine at position 2,088*(Am)*; acetyl-Coenzyme A carboxylases having a glycine at position 2,098*(Am)* and a histidine at position 2,088*(Am)*; acetyl-Coenzyme A carboxylases having a glycine at position 2,098*(Am)* and leucine at position 2,088*(Am)*; acetyl-Coenzyme A carboxylases having a glycine at position 2,098*(Am)* and a serine at position 2,088*(Am)*; acetyl-Coenzyme A carboxylases having a glycine at position 2,098*(Am)* and threonine at position 2,088*(Am)*; acetyl-Coenzyme A carboxylases having a glycine at position 2,098*(Am)* and a valine at position 2,088*(Am)*; acetyl-Coenzyme A carboxylases having a cysteine at position 2,098*(Am)* and a tryptophan at position 2088*(Am)*; acetyl-Coenzyme A carboxylases having a serine at position 2,098*(Am)* and a tryptophan at position 2088*(Am)*; and acetyl-Coenzyme A carboxylases having a deletion at position 2,080*(Am)* and a deletion at position 2081*(Am)*.

In a most preferred embodiment, the invention includes acetyl-Coenzyme A carboxylases having a leucine at position 1,781*(Am)* and a asparagine at position 2,041*(Am)*; acetyl-Coenzyme A carboxylases having a leucine at position 1,781*(Am)* and a cysteine at position 2,027*(Am)*; acetyl-Coenzyme A carboxylases having a leucine at position 1,781*(Am)* and a leucine at position 2,075*(Am)*; acetyl-Coenzyme A carboxylases having a leucine at position 1,781*(Am)* and a phenylalanine at position 1,864*(Am)*; acetyl-Coenzyme A carboxylases having a leucine at position 1,781 *(Am)* and an alanine at position 2098*(Am)*; acetyl-Coenzyme A carboxylases having a leucine at position 1,781 *(Am)* and a glycine at position 2,098*(Am)*; acetyl-Coenzyme A carboxylases having a leucine at position 1,781*(Am)* and a duplication 2,075*(Am)*; acetyl-Coenzyme A carboxylases having a glycine at position 1,999*(Am)* and a phenylalanine at position 1,864*(Am)*; acetyl-Coenzyme A carboxylases having a glycine at position 1,999*(Am)* and isoleucine at position 2,049*(Am)*; acetyl-Coenzyme A carboxylases having a glycine at position 1,999*(Am)* and leucine at position 2,075*(Am)*; and acetyl-Coenzyme A carboxylases having a glycine at position 1,999*(Am)* and alanine at position 2,098*(Am)*.

### Nucleic acid molecules:

The present invention also encompasses nucleic acid molecules that encode all or a portion of the acetyl-Coenzyme A carboxylase enzymes described above. Nucleic acid molecules of the invention may comprise a nucleic acid sequence encoding an amino acid sequence comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, isoleucine or leucine; the amino acid at position 2,059*(Am)* is valine; the amino acid at position 2,074*(Am)* is leucine; the amino acid at position 2,075*(Am)* is leucine, isoleucine, methionine or additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081 *(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences. In some embodiments, a nucleic acid molecule of the invention may encode an acetyl-Coenzyme A carboxylase having multiple differences from the wild type acetyl-Coenzyme A carboxylase as described above.

In one embodiment, the present invention emcompasses a nucleic acid molecule encoding an acetyl-Coenzyme A carboxylase which differs from the acetyl-Coenzyme A carboxylase of the corresponding wild-type plant at only one of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, *2,074(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098*(Am).* In one embodiment the acetyl-Coenzyme A carboxylase of an herbicide-tolerant plant of the invention will differ at only one of the following positions: 2,078*(Am)*, 2,088*(Am)*, or 2,075*(Am).* In a preferred embodiment the acetyl-Coenzyme A carboxylase of an herbicide-tolerant plant of the invention will differ at only one of the following positions: 2,039*(Am)*, 2,059*(Am)*, 2,080*(Am)*, or 2,095*(Am)*. In a more preferred embodiment the acetyl-Coenzyme A carboxylase of an herbicide-tolerant plant of the invention will differ at only one of the following positions: 1,785*(Am)*, 1,786*(Am)*, 1,811*(Am)*, 1,824*(Am)*, 1,864*(Am)*, 2,041*(Am)*, 2,049*(Am)*, 2,074*(Am)*, 2,079*(Am)*, 2,081*(Am)*, 2,096*(Am)*, or 2,098*(Am).* In a most preferred embodiment the acetyl-Coenzyme A carboxylase of an herbicide-tolerant plant of the invention will differ at only one of the following positions: 1,781*(Am)*, 1,999*(Am)*, 2,027*(Am)*, 2,041 *(Am),* or 2,096*(Am)*.

In one embodiment, the present invention emcompasses a nucleic acid molecule encoding an acetyl-Coenzyme A carboxylase having only one of the following substitutions: isoleucine at position 2,075*(Am)*, glycine at position 2,078*(Am)*, or arginine at position *2,088(Am).* In a preferred embodiment, the present invention emcompasses a nucleic acid molecule encoding an acetyl-Coenzyme A carboxylase having only one of the following substitutions: glycine at position 2,039*(Am)*, valine at position 2,059*(Am)*, methionine at position 2,075*(Am)*, duplication of position 2,075*(Am)* (i.e., an insertion of valine between 2,074*(Am)* and 2,075*(Am)*, or an insertion of valine between position 2,075*(Am)* and 2,076*(Am)*, deletion of amino acid position 2,088*(Am)*, glutamic acid at position 2,080*(Am)*, deletion of position 2,088*(Am)*, or glutamic acid at position 2,095(Am). In a more preferred embodiment, the present invention emcompasses a nucleic acid molecule encoding an acetyl-Coenzyme A carboxylase having only one of the following substitutions: a glycine at position 1,785*(Am)*, a proline at position 1,786*(Am)*, an asparagine at position 1,811*(Am)*, a leucine at position 2,075*(Am)*, a methionine at position 2,075*(Am)*, a threnonine at position 2,078*(Am)*, a deletion at position 2,080*(Am)*, a deletion at position 2,081*(Am)*, a tryptophan at position 2,088*(Am)*, a serine at position 2,096*(Am)*, an alanine at position 2,096*(Am)*, an alanine at position 2,098*(Am)*, a glycine at position 2,098*(Am)*, an histidine at position 2,098*(Am)*, a proline at position 2,098*(Am)*, or a serine at position 2,098*(Am).* In a most preferred embodiment, the present invention emcompasses a nucleic acid molecule encoding an acetyl-Coenzyme A carboxylase having only one of the following substitutions: a leucine at position 1,781*(Am)*, a threonine at position 1,781*(Am)*, a valine at position 1,781*(Am)*, an alanine at position 1,781*(Am)*, a glycine at position 1,999*(Am)*, a cysteine at position 2,027*(Am)*, an arginine at position 2,027*(Am)*, an asparagine at position 2,041*(Am)*, a valine at position 2,041*(Am)*, an alanine at position 2,096*(Am)*, and a serine at position 2,096*(Am)*.

In one embodiment, a nucleic acid molecule of the invention may encode an acetyl-Coenzyme A carboxylase comprising a leucine, threonine, valine, or an alanine at position 1,781*(Am)* and a cysteine or glycine at position 1,999*(Am)*. In one embodiment, a nucleic acid molecule of the invention may encode an acetyl-Coenzyme A carboxylase comprising a leucine, threonine, valine, or an alanine at position 1,781*(Am)* and a cysteine or arginine at position 2,027*(Am)*. In one embodiment, a nucleic acid molecule of the invention may encode an acetyl-Coenzyme A carboxylase comprising a leucine, threonine, valine, or an alanine at position 1,781*(Am)* and an asparagine at position 2,041 *(Am).* In one embodiment, a nucleic acid molecule of the invention may encode an acetyl-Coenzyme A carboxylase comprising a leucine, threonine, valine, or an alanine at position 1,781*(Am)* and a phenylalanine, isoleucine or leucine at position 2,049*(Am)*. In one embodiment, a nucleic acid molecule of the invention may encode an acetyl-Coenzyme A carboxylase comprising a leucine, threonine, valine, or an alanine at position 1,781*(Am)* and a leucine or isoleucine at position 2,075*(Am).* In one embodiment, a nucleic acid molecule of the invention may encode an acetyl-Coenzyme A carboxylase comprising a leucine, threonine, valine, or an alanine at position 1,781*(Am)* and a glycine at position 2,078*(Am)*. In one embodiment, a nucleic acid molecule of the invention may encode an acetyl-Coenzyme A carboxylase comprising a leucine, threonine, valine, or an alanine at position 1,781*(Am)* and an arginine at position 2,088*(Am)*. In one embodiment, a nucleic acid molecule of the invention may encode an acetyl-Coenzyme A carboxylase comprising a leucine, threonine, valine, or an alanine at position 1,781*(Am)* and an alanine at position 2,096*(Am)*. In one embodiment, a nucleic acid molecule of the invention may encode an acetyl-Coenzyme A carboxylase comprising a leucine, threonine, valine, or an alanine at position 1,781*(Am)* and an alanine at position 2,098*(Am).* In one embodiment, a nucleic acid molecule of the invention may encode an acetyl-Coenzyme A carboxylase comprising a leucine, threonine, valine, or an alanine at position 1,781*(Am)*, a cysteine at position 2,027*(Am)*, and an asparagine at position 2,041*(Am).* In one embodiment, a nucleic acid molecule of the invention may encode an acetyl-Coenzyme A carboxylase comprising a leucine, threonine, valine, or an alanine at position 1,781*(Am)*, a cysteine at position 2,027*(Am)*, an asparagine at position 2,041*(Am)*, and an alanine at position 2,096*(Am)*.

In one embodiment, the invention includes, a nucleic acid molecule encoding an acetyl-Coenzyme A carboxylase having an isoleucine at position 2,075*(Am)* and a glycine at position 1,999*(Am)*; a nucleic acid molecule encoding an acetyl-Coenzyme A carboxylase having a methionine at position 2,075*(Am)* and a glutamic acid at position 2,080*(Am)*, a nucleic acid molecule encoding an acetyl-Coenzyme A carboxylase having a methionine at position 2,075*(Am)* and a glutamic acid at position 2,095*(Am)*; a nucleic acid molecule encoding an acetyl-Coenzyme A carboxylase having a glycine at position *2,078(Am)* and a valine at position 2,041 *(Am);* a nucleic acid molecule encoding an acetyl-Coenzyme A carboxylase having a glycine at position 2,078*(Am)* and a glycine at position 2,039*(Am)*; a nucleic acid molecule encoding an acetyl-Coenzyme A carboxylase having a glycine at position 2,078*(Am)* and an alanine at position 2,049*(Am)*; a nucleic acid molecule encoding an acetyl-Coenzyme A carboxylase having a glycine at position 2,078*(Am)* and a cysteine at position 2,049*(Am)*; a nucleic acid molecule encoding an acetyl-Coenzyme A carboxylase having a glycine at position 2,078*(Am)* and a serine at position 2,049*(Am)*; a nucleic acid molecule encoding an acetyl-Coenzyme A carboxylase having a glycine at position 2,078*(Am)* and a threonine at position 2,049*(Am)*; a nucleic acid molecule encoding an acetyl-Coenzyme A carboxylase having a glycine at position 2,078*(Am)* and a valine at position 2,059*(Am)*; a nucleic acid molecule encoding an acetyl-Coenzyme A carboxylase having a glycine at position 2,078*(Am)* and a phenylalanine at position 2,079*(Am)*; a nucleic acid molecule encoding an acetyl-Coenzyme A carboxylase having a glycine at position 2,078*(Am)* and a proline at position at position 2,079*(Am)*; or a nucleic acid molecule encoding an acetyl-Coenzyme A carboxylase having a glycine at position 2,078*(Am)* and a glycine at position 2,088*(Am)*.

In a preferred embodiment, the invention includes a nucleic acid molecule encoding an acetyl-Coenzyme A carboxylase having a leucine at position 1,781*(Am)* and a proline at position 1,824*(Am)*; a nucleic acid molecule encoding an acetyl-Coenzyme A carboxylase having a leucine at position 1,781*(Am)* and an arginine at position 2027*(Am)*; or a nucleic acid molecule encoding an acetyl-Coenzyme A carboxylase having a glycine at position 2,078*(Am)* and a proline at position 1,824*(Am)*.

In a more preferred embodiment, the invention includes a nucleic acid molecule encoding an acetyl-Coenzyme A carboxylase having a leucine at position 1,781 *(Am)* and a phenylalanine at position 2,049*(Am)*; a nucleic acid molecule encoding an acetyl-Coenzyme A carboxylase having an alanine at position 2,098*(Am)* and a leucine at position 2,049*(Am)*; a nucleic acid molecule encoding an acetyl-Coenzyme A carboxylase having an alanine at position 2,098*(Am)* and a histidine at position 2088*(Am)*; a nucleic acid molecule encoding an acetyl-Coenzyme A carboxylase having an alanine at position 2,098*(Am)* and a phenylalanine at position 2,088*(Am)*; a nucleic acid molecule encoding an acetyl-Coenzyme A carboxylase having an alanine at position 2,098*(Am)* and a lysine at position 2,088*(Am)*; a nucleic acid molecule encoding an acetyl-Coenzyme A carboxylase having an alanine at position 2,098*(Am)* and a leucine at position 2,088*(Am)*; a nucleic acid molecule encoding an acetyl-Coenzyme A carboxylase having an alanine at position 2,098*(Am)* and a threonine at position 2,088*(Am)*; a nucleic acid molecule encoding an acetyl-Coenzyme A carboxylase having a glycine at position 2,098*(Am)* and a glycine at position 2,088*(Am)*; a nucleic acid molecule encoding an acetyl-Coenzyme A carboxylase having a glycine at position 2,098*(Am)* and a histidine at position 2,088*(Am)*; a nucleic acid molecule encoding an acetyl-Coenzyme A carboxylase having a glycine at position 2,098*(Am)* and leucine at position 2,088*(Am)*; a nucleic acid molecule encoding an acetyl-Coenzyme A carboxylase having a glycine at position 2,098*(Am)* and a serine at position 2,088*(Am)*; a nucleic acid molecule encoding an acetyl-Coenzyme A carboxylase having a glycine at position 2,098*(Am)* and threonine at position 2,088*(Am)*; a nucleic acid molecule encoding an acetyl-Coenzyme A carboxylase having a glycine at position 2,098*(Am)* and a valine at position 2,088*(Am)*; a nucleic acid molecule encoding an acetyl-Coenzyme A carboxylase having a cysteine at position 2,098*(Am)* and a tryptophan at position 2088*(Am)*; a nucleic acid molecule encoding an acetyl-Coenzyme A carboxylase having a serine at position 2,098*(Am)* and a tryptophan at position 2088*(Am)*; or a nucleic acid molecule encoding an acetyl-Coenzyme A carboxylase having a deletion at position 2,080*(Am)* and a deletion at position 2081*(Am).*

In a most preferred embodiment, the invention includes, a nucleic acid molecule encoding an acetyl-Coenzyme A carboxylase having a leucine at position 1,781*(Am)* and a asparagine at position 2,041*(Am);* a nucleic acid molecule encoding an acetyl-Coenzyme A carboxylase having a leucine at position 1,781*(Am)* and a cysteine at position 2,027*(Am)*; a nucleic acid molecule encoding an acetyl-Coenzyme A carboxylase having a leucine at position 1,781*(Am)* and a leucine at position 2,075*(Am)*; a nucleic acid molecule encoding an acetyl-Coenzyme A carboxylase having a leucine at position 1,781*(Am)* and a phenylalanine at position 1,864*(Am)*; a nucleic acid molecule encoding an acetyl-Coenzyme A carboxylase having a leucine at position 1,781*(Am)* and an alanine at position 2098*(Am)*; a nucleic acid molecule encoding an acetyl-Coenzyme A carboxylase having a leucine at position 1,781*(Am)* and a glycine at position 2,098*(Am)*; a nucleic acid molecule encoding an acetyl-Coenzyme A carboxylase having a leucine at position 1,781*(Am)* and a duplication 2,075*(Am)*; a nucleic acid molecule encoding an acetyl-Coenzyme A carboxylase having a glycine at position 1,999*(Am)* and a phenylalanine at position 1,864*(Am)*; a nucleic acid molecule encoding an acetyl-Coenzyme A carboxylase having a glycine at position 1,999*(Am)* and isoleucine at position 2,049*(Am)*; a nucleic acid molecule encoding an acetyl-Coenzyme A carboxylase having a glycine at position 1,999*(Am)* and leucine at position 2,075*(Am)*; or a nucleic acid molecule encoding an acetyl-Coenzyme A carboxylase having a glycine at position 1,999*(Am)* and alanine at position 2,098*(Am).*

In one embodiment, the invention provides rice plants comprising nucleic acids encoding Acetyl-Coenzyme A carboxylase polypeptide having one or more substitutions as described above.

In one embodiment, the invention provides BEP clade plants comprising nucleic acids encoding Acetyl-Coenzyme A carboxylase polypeptide having one or more substitutions as described above.

In one embodiment, the invention provides BET subclade plant comprising nucleic acids encoding Acetyl-Coenzyme A carboxylase polypeptide having one or more substitutions as described above.

In one embodiment, the invention provides BET crop plants comprising nucleic acids encoding Acetyl-Coenzyme A carboxylase polypeptide having one or more substitutions as described above.

In one embodiment, the invention provides monocot plants comprising nucleic acids encoding Acetyl-Coenzyme A carboxylase polypeptide having one or more substitutions as described above.

A nucleic acid molecule of the invention may be DNA, derived from genomic DNA or cDNA, or RNA. A nucleic acid molecule of the invention may be naturally occurring or may be synthetic. A nucleic acid molecule of the invention may be isolated, recombinant and/or mutagenized.

In one embodiment, a nucleic acid molecule of the invention encodes an acetyl-Coenzyme A carboxylase enzyme in which the amino acid at position 1,781*(Am)* is leucine or alanine or is complementary to such a nucleic acid molecule. Such nucleic acid molecules include, but are not limited to, genomic DNA that serves as a template for a primary RNA transcription, a plasmid molecule encoding the acetyl-Coenzyme A carboxylase, as well as an mRNA encoding such an acetyl-Coenzyme A carboxylase.

Nucleic acid molecules of the invention may comprise non-coding sequences, which may or may not be transcribed. Non-coding sequences that may be included in the nucleic acid molecules of the invention include, but are not limited to, 5' and 3' UTRs, polyadenylation signals and regulatory sequences that control gene expression (e.g., promoters). Nucleic acid molecules of the invention may also comprise sequences encoding transit peptides, protease cleavage sites, covalent modification sites and the like. In one embodiment, nucleic acid molecules of the invention encode a chloroplast transit peptide sequence in addition to a sequence encoding an acetyl-Coenzyme A carboxylase enzyme.

In another embodiment, nucleic acid molecules of the invention may encode an acetyl-Coenzyme A carboxylase enzyme having at least 50%, 60%, 70%, 75%, 80%, 85%, 90%, 95% or more sequence identity to a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, leucine or isoleucine; the amino acid at position 2,059*(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081 *(Am)* is deleted; the amino acid at position 2,088(Am) is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences.

As used herein, "percent (%) sequence identity" is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program BLAST available at http://blast.ncbi.nlm.nih.gov/Blast.cgi with search parameters set to default values.

The present invention also encompasses nucleic acid molecules that hybridize to nucleic acid molecules encoding acetyl-Coenzyme A carboxylase of the invention as well as nucleic acid molecules that hybridize to the reverse complement of nucleic acid molecules encoding an acetyl-Coenzyme A carboxylase of the invention. In one embodiment, nucleic acid molecules of the invention comprise nucleic acid molecules that hybridize to a nucleic acid molecule encoding one or more of a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, isoleucine or leucine; the amino acid at position *2,059(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081 *(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences, or the reverse complement of such nucleic acid molecules under stringent conditions. The stringency of hybridization can be controlled by temperature, ionic strength, pH, and the presence of denaturing agents such as formamide during hybridization and washing. Stringent conditions that may be used include those defined in Current Protocols in Molecular Biology, Vol. 1, Chap. 2.10, John Wiley & Sons, Publishers (1994) and Sambrook et al., Molecular Cloning, Cold Spring Harbor (1989) which are specifically incorporated herein as they relate to teaching stringent conditions.

Any of the mutants described above in a plasimd with a combinatinon of the gene of interest can be used in transformation.

In one embodiment, the present invention provides expression vectors comprising nucleic acid molecules encoding any of the ACCase mutants described above.

In one embodiment, the present invention provides for the use of mutant ACCase nucleic acids and proteins encoded by such mutant ACCase nucleic acids as described above as selectable markers.

In one embodiment, nucleic acid molecules invention encompasses oligonucleotides that may be used as hybridization probes, sequencing primers, and/or PCR primers. Such oligonucleotides may be used, for example, to determine a codon sequence at a particular position in a nucleic acid molecule encoding an acetyl-Coenzyme A carboxylase, for example, by allele specific PCR. Such oligonucleotides may be from about 15 to about 30, from about 20 to about 30, or from about 20-25 nucleotides in length.

Test for double mutant ACCase genes "DBLM Assay":
(1) In a test population (of, e.g., at least 12 and preferably at least 20) whole rice plants containing 1 or 2 copies of a transgenic ACCase gene encoding an at-least-double-mutant ACCase (i.e. 1 min. and 2 max. chromosomal insertions of the transgenic ACCase gene to be tested),
   wherein the rice plants are T0 ("T-zero") regenerants
   and in parallel with a control population of such plants to be used as untreated check plants;
(2) Application to the test population at 200 L / ha spray volume of a composition comprising Tepraloxydim (AI) and 1% Crop Oil Concentrate (COC), to provide an AI application rate equivalent to 50 g/ha of Tepraloxydim (AI);
(3) Determining a phytotoxicity score for each test and check plant, based on a traditional plant injury rating system (e.g., evaluating visual evidence of herbicide burn, leaf morphology changes, wilt, yellowing, and other morphological characteristics, preferably according to a typical, at least-5-level injury rating scale);
(4) Analyzing the collected data to determine whether at least 75% of the plants in the test population exhibit an average phytotoxicity, i.e. increase in injury relative to check plants, of less than 10%; and
(5) Identifying a positive result so determined as demonstrating that the double-mutant ACCase provides an acceptable AIT.

### Herbicides

The present invention provides plants, e.g., rice plants, that are tolerant of concentrations of herbicide that normally inhibit the growth of wild-type plants. The plants are typically resistant to herbicides that interfere with acetyl-Coenzyme A carboxylase activity. Any herbicide that inhibits acetyl-Coenzyme A carboxylase activity can be used in conjunction with the plants of the invention. Suitable examples include, but are not limited to, cyclohexanedione herbicides, aryloxyphenoxy propionate herbicides, and phenylpyrazole herbicides. In some methods of controlling weeds and/or growing herbicide-tolerant plants, at least one herbicide is selected from the group consisting of sethoxydim, cycloxydim, tepraloxydim, haloxyfop, haloxyfop-P or a derivative of any of these herbicides. Table 1 provides a list of cyclohexanedione herbicides (DIMs, also referred to as: cyclohexene oxime cyclohexanedione oxime; and CHD) that interfere with acetyl-Coenzyme A carboxylase activity and may be used in conjunction with the herbicide-tolerant plants of the invention. One skilled in the art will recognize that other herbicides in this class exist and may be used in conjunction with the herbicide-tolerant plants of the invention. Also included in Table 1 is a list of aryloxyphenoxy propionate herbicides (also referred to as aryloxyphenoxy propanoate; aryloxyphenoxyalkanoate; oxyphenoxy; APP; AOPP; APA; APPA; FOP, note that these are sometime written with the suffix '-oic') that interfere with acetyl-Coenzyme A carboxylase activity and may be used in conjunction with the herbicide-tolerant plants of the invention. One skilled in the art will recognize that other herbicides in this class exist and may be used in conjunction with the herbicide-tolerant plants of the invention.

**Table 1**

| **ACCase Inhibitor** | **Class** | **Company** | **Examples of Synonyms and Trade Names** |
|---|---|---|---|
| alloxydim | DIM | BASF | Fervin, Kusagard, NP-48Na, BAS 9021 H, Carbodimedon, Zizalon |
| butroxydim | DIM | Syngenta | Falcon, ICI-A0500, Butroxydim |
| clethodim | DIM | Valent | Select, Prism, Centurion, RE-45601, Motsa |
| Clodinafop-propargyl | FOP | Syngenta | Discover, Topik, CGA 184 927 |
| clofop | FOP | | Fenofibric Acid, Alopex |
| cloproxydim | FOP | | |
| chlorazifop | FOP | | |
| cycloxydim | DIM | BASF | Focus, Laser, Stratos, BAS 517H |
| cyhalofop-butyl | FOP | Dow | Clincher, XDE 537, DEH 112, Barnstorm |
| diclofop-methyl | FOP | Bayer | Hoegrass, Hoelon, Illoxan, HOE 23408, Dichlorfop, Illoxan |
| fenoxaprop-P-ethyl | FOP | Bayer | Super Whip, Option Super, Exel Super, HOE-46360, Aclaim, Puma S, Fusion |
| fenthiaprop | FOP | | Taifun; Joker |
| fluazifop-P-butyl | FOP | Syngenta | Fusilade, Fusilade 2000, Fusilade DX, ICI-A 0009, ICI-A 0005, SL-236, IH-773B, TF-1169, Fusion |
| haloxyfop-etotyl | FOP | Dow | Gallant, DOWCO 453EE |
| haloxyfop-methyl | FOP | Dow | Verdict, DOWCO 453ME |
| haloxyfop-P-methyl | FOP | Dow | Edge, DE 535 |
| isoxapyrifop | FOP | | |
| Metamifop | FOP | Dongbu | NA |
| pinoxaden | DEN | Syngenta | Axial |
| profoxydim | DIM | BASF | Aura, Tetris, BAS 625H, Clefoxydim |
| propaquizafop | FOP | Syngenta | Agil, Shogun, Ro 17-3664, Correct |
| quizalofop-P-ethyl | FOP | DuPont | Assure, Assure II, DPX-Y6202-3, Targa Super, NC-302, Quizafop |
| quizalofop-P-tefuryl | FOP | Uniroyal | Pantera, UBI C4874 |
| sethoxydim | DIM | BASF | Poast, Poast Plus, NABU, Fervinal, NP-55, Sertin, BAS 562H, Cyethoxydim, Rezult |
| tepraloxydim | DIM | BASF | BAS 620H, Aramo, Caloxydim |
| tralkoxydim | DIM | Syngenta | Achieve, Splendor, ICI-A0604, Tralkoxydime, Tralkoxidym |
| trifop | FOP | | |

In addition to the herbicides listed above, other ACCAse-inhibitors can be used in conjunction with the herbicide-tolerant plants of the invention. For example, ACCase-inhibiting herbicides of the phenylpyrazole class, also known as DENs, can be used. An exemplary DEN is pinoxaden, which is a phenylpyrazoline-type member of this class. Herbicide compositions containing pinoxaden are sold under the brands Axial and Traxos.

The herbicidal compositions hereof comprising one or more acetyl-Coenzyme A carboxylase-inhibiting herbicides, and optionally other agronomic A.I.(s), *e.g.,* one or more sulfonylureas (SUs) selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, or one or more imidazolinones selected from the group of imazamox, imazethapyr, imazapyr, imazapic, combinations thereof, and their agriculturally suitable salts and esters, can be used in any agronomically acceptable format. For example, these can be formulated as ready-to-spray aqueous solutions, powders, suspensions; as concentrated or highly concentrated aqueous, oily or other solutions, suspensions or dispersions; as emulsions, oil dispersions, pastes, dusts, granules, or other broadcastable formats. The herbicide compositions can be applied by any means known in the art, including, for example, spraying, atomizing, dusting, spreading, watering, seed treatment, or co-planting in admixture with the seed. The use forms depend on the intended purpose; in any case, they should ensure the finest possible distribution of the active ingredients according to the invention.

In other embodiments, where the optional A.I. includes an herbicide from a different class to which the plant(s) hereof would normally be susceptible, the plant to be used is selected from among those that further comprise a trait of tolerance to such herbicide. Such further tolerance traits can be provided to the plant by any method known in the art, e.g., including techniques of traditional breeding to obtain a tolerance trait gene by hybridization or introgression, of mutagenesis, and/or of transformation. Such plants can be described as having "stacked" traits.

In addition, any of the above acetyl-Coenzyme A carboxylase-inhibiting herbicides can be combined with one or more herbicides of another class, for example, any of the acetohydroxyacid synthase-inhibiting herbicides, EPSP synthase-inhibiting herbicides, glutamine synthase-inhibiting herbicides, lipid- or pigment-biosynthesis inhibitor herbicides, cell-membrane disruptor herbicides, photosynthesis or respiration inhibitor herbicides, or growth regulator or growth inhibitor herbicides known in the art. Non-limiting examples include those recited in Weed Science Society of America's Herbicide Handbook, 9th Edition edited by S.A. Senseman, copy right 2007. An herbicidal composition herein can contain one or more agricultural active ingredient(s) selected from the agriculturally-acceptable fungicides, strobilurin fungicides, insecticides (including nematicides), miticides, and molluscicides. Non-limiting examples include those recited in 2009 Crop Protection Reference (www.greenbook.net), Vance Publications.

In one embodiment of the invention, any of the above acetyl-Coenzyme A carboxylase-inhibiting herbicides are combined with herbicides which exhibit low damage to rice, whereby the rice tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, azimsulfuron, bensulfuron, chlorimuron, cyclosulfamuron, ethoxysulfuron, flucetosulfuron, halosulfuron, imazosulfuron, metsulfuron, orthosulfamuron, propyrisulfuron, pyrazosulfuron, bispyribac, pyrimisulfan or penoxsulam, the EPSP synthase-inhibiting herbicides glyphosate or sulfosate, the glutamine synthase-inhibiting herbicides glufosinate, glufosinate-P or bialaphos, the lipid biosynthesis inhibitor herbicides benfuresate, molinate or thiobencarb, the photosynthesis inhibitor herbicides bentazon, paraquat, prometryn or propanil, the bleacher herbicides benzobicyclone, clomazone or tefuryltrione, the auxin herbicides 2,4-D, fluroxypyr, MCPA, quinclorac, quinmerac or triclopyr, the microtubule inhibitor herbicide pendimethalin, the VLCFA inhibitor herbicides anilofos, butachlor, fentrazamide, ipfencarbazone, mefenacet, pretilachlor, acetochlor, metolachlor or S-metolachlor or the protoporphyrinogen-IX-oxidase inhibitor herbicides carfentrazone, oxadiazon, oxyfluorfen, pyraclonil or saflufenacil.

In one embodiment of the invention, any of the above acetyl-Coenzyme A carboxylase-inhibiting herbicides are combined with herbicides which exhibit low damage to cereals such as wheat, barley or rye, whereby the cereals tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, amidosulfuron, chlorsulfuron, flucetosulfuron, flupyrsulfuron, iodosulfuron, mesosulfuron, metsulfuron, sulfosulfuron, thifensulfuron, triasulfuron, tribenuron, tritosulfuron, florasulam, pyroxsulam, pyrimisulfan, flucarbazone, propoxycarbazone or thiencarbazone, the EPSP synthase-inhibiting herbicides glyphosate or sulfosate, the glutamine synthase-inhibiting herbicides glufosinate, glufosinate-P or bialaphos, the lipid biosynthesis inhibitor herbicides prosulfocarb, the photosynthesis inhibitor herbicides bentazon, chlorotoluron, isoproturon, ioxynil, bromoxynil, the bleacher herbicides diflufenican, flurtamone, picolinafen or pyrasulfotole, the auxin herbicides aminocyclopyrachlor, aminopyralid, 2,4-D, dicamba, fluroxypyr, MCPA, clopyralid, MCPP, or MCPP-P, the microtubule inhibitor herbicides pendimethalin or trifluralin, the VLCFA inhibitor herbicide flufenacet, or the protoporphyrinogen-IX-oxidase inhibitor herbicides bencarbazone, carfentrazone or saflufenacil, or the herbicide difenzoquat.

In one embodiment of the invention, any of the above acetyl-Coenzyme A carboxylase-inhibiting herbicides are combined with herbicides which exhibit low damage to turf, whereby the turf tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, flazasulfuron, foramsulfuron, halosulfuron, trifloxysulfuron, bispyribac or thiencarbazone, the EPSP synthase-inhibiting herbicides glyphosate or sulfosate, the glutamine synthase-inhibiting herbicides glufosinate, glufosinate-P or bialaphos, the photosynthesis inhibitor herbicides atrazine or bentazon, the bleacher herbicides mesotrione, picolinafen, pyrasulfotole or topramezone, the auxin herbicides aminocyclopyrachlor, aminopyralid, 2,4-D, 2,4-DB, clopyralid, dicamba, dichlorprop, dichlorprop-P, fluroxypyr, MCPA, MCPB, MCPP, MCPP-P, quinclorac, quinmerac or trichlopyr, the microtubule inhibitor herbicide pendimethalin, the VLCFA inhibitor herbicides dimethenamide, dimethenamide-P or ipfencarbazone, the protoporphyrinogen-IX-oxidase inhibitor herbicides saflufenacil or sulfentrazone, or the herbicide indaziflam.

Furthermore, any of the above acetyl-Coenzyme A carboxylase-inhibiting herbicides can be combined with safeners. Safeners are chemical compounds which prevent or reduce damage on useful plants without having a major impact on the herbicidal action of the herbicides towards unwanted plants. They can be applied either before sowings (e. g. on seed treatments, shoots or seedlings) or in the pre-emergence application or post-emergence application of the useful plant. The safeners and the aforementioned herbicides can be applied simultaneously or in succession. Suitable safeners are e. g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates. Examples of saferners are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

In some embodiments, an herbicidal composition hereof can comprise, e.g., a combination of: auxinic herbicide(s), e.g., dicamba; AHAS-inhibitor(s), e.g., imidazolinone(s) and/or sulfonylurea(s); ACCase-inhibitor(s); EPSPS inhibitor(s), e.g., glyphosate; glutamine synthetase inhibitor(s), e.g.., glufosinate; protoporphyrinogen-IX oxidase (PPO) inhibitor(s), e.g., saflufenacil; fungicide(s), e.g., strobilurin fungicide(s) such as pyraclostrobin; and the like. In some embodiments, an herbicidal composition hereof can comprise, e.g., a combination of auxinic herbicide(s), e.g., dicamba; a microtubule inhibitor herbicide, e.g., pendimethalin and strobilurin fungicide(s) such as pyraclostrobin(s). An herbicidal composition will be selected according to the tolerances of a plant hereof, and the plant can be selected from among those having stacked tolerance traits.

The herbicides individually and/or in combination as described in the present invention can be used as pre-mixes or tank mixes. Such herbicides can also be incorporated into an agronomically acceptable compositions.

Those skilled in the art will recognize that some of the above mentioned herbicides and/or safeners are capable of forming geometrical isomers, for example E/Z isomers. It is possible to use both, the pure isomers and mixtures thereof, in the compositions according to the invention. Furthermore, some of the above mentioned herbicides and/or safeners have one or more centers of chirality and, as a consequence, are present as enantiomers or diastereomers. It is possible to use both, the pure enantiomers and diastereomers and their mixtures, in the compositions according to the invention. In particular, some of the aryloxyphenoxy propionate herbicides are chiral, and some of them are commonly used in enantiomerically enriched or enantiopure form, e. g. clodinafop, cyhalofop, fenoxaprop-P, fluazifop-P, haloxyfop-P, metamifop, propaquizafop or quizalofop-P. As a further example, glufosinate may be used in enantiomerically enriched or enantiopure form, also known as glufosinate-P.

Those skilled in the art will recognize that any derivative of the above mentioned herbicides and/or safeners can be used in the practice of the invention, for example agriculturally suitable salts and esters.

The herbicides and/or safeners, or the herbicidal compositions comprising them, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in any case, they should ensure the finest possible distribution of the active ingredients according to the invention.

The herbicidal compositions comprise an herbicidal effective amount of at least one of the acetyl-Coenzyme A carboxylase-inhibiting herbicides and potentially other herbicides and/or safeners and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, optionally colorants and, for seed formulations, adhesives. The person skilled in the art is sufficiently familiar with the recipes for such formulations.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon^{®} SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following: mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Suitable carriers include liquid and solid carriers. Liquid carriers include e.g. non-aqeuos solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, e.g. amines such as N-methylpyrrolidone, and water as well as mixtures thereof. Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF AG), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denaturated proteins, polysaccharides (e.g. methylcellulose), hydrophobically modified starches, polyvinyl alcohol (Mowiol types Clariant), polycarboxylates (BASF AG, Sokalan types), polyalkoxylates, polyvinylamine (BASF AG, Lupamine types), polyethyleneimine (BASF AG, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the herbicidal compositions, either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

### Methods of controlling weeds

Herbicide-tolerant plants of the invention may be used in conjunction with an herbicide to which they are tolerant. Herbicides may be applied to the plants of the invention using any techniques known to those skilled in the art. Herbicides may be applied at any point in the plant cultivation process. For example, herbicides may be applied pre-planting, at planting, pre-emergence, post-emergence or combinations thereof.

Herbicide compositions hereof can be applied, e.g., as foliar treatments, soil treatments, seed treatments, or soil drenches. Application can be made, e.g., by spraying, dusting, broadcasting, or any other mode known useful in the art.

In one embodiment, herbicides may be used to control the growth of weeds that may be found growing in the vicinity of the herbicide-tolerant plants invention. In embodiments of this type, an herbicide may be applied to a plot in which herbicide-tolerant plants of the invention are growing in vicinity to weeds. An herbicide to which the herbicide-tolerant plant of the invention is tolerant may then be applied to the plot at a concentration sufficient to kill or inhibit the growth of the weed. Concentrations of herbicide sufficient to kill or inhibit the growth of weeds are known in the art.

It will be readily apparent to one of ordinary skill in the relevant arts that other suitable modifications and adaptations to the methods and applications described herein are obvious and may be made without departing from the scope of the invention or any embodiment thereof. Having now described the present invention in detail, the same will be more clearly understood by reference to the following examples, which are included herewith for purposes of illustration only and are not intended to be limiting of the invention.

### Use of Tissue Culture for Selection of Herbicide

Herbicide tolerant crops offer farmers additional options for weed management. Currently, there are genetically modified (GMO) solutions available in some crop systems. Additional, mutational techniques have been used to select for altered enzyme, activities or structures that confer herbicide resistance such as the current CLEARFIELD solutions from BASF. In the US, CLEARFIELD Rice is the premier tool for managing red rice in infested areas (USDA-ARS, 2006); however, gene flow between red rice and CLEARFIELD Rice represents a considerable risk for the AHAS tolerance since out-crossing, has been reported at up to 170 F1 hybrids/ha (Shivrain et al, 2007). Stewardship guidelines including, amongst many other aspects, alternation non CLEARFIELD Rice can limit CLEARFIELD Rice market penetration. The generation of cultivated rice with tolerance to a different mode of action (MOA) graminicides would reduce these risks and provide more tools for weed management.

One enzyme that is already a target for many different graminaceous herbicides is acetyl CoA carboxylase (ACCase, EC 6.4.1.2), which catalyzes the first committed step in fatty acid (FA) biosynthesis. Aryloxyphenoxypropionate (APP or FOP) and cyclohexanedione (CHD or DIM) type herbicides are used post-emergence in dicot crops, with the exception of cyhalofop-butyl which is selective in rice to control grass weeds. Furthermore, most of these herbicides have relatively low persistence in soil and provide growers with flexibility for weed control and crop rotation. Mutations in this enzyme are known that confer tolerance to specific sets of FOPS and/or DIMS (Liu et al, 2007; Delye et al, 2003, 2005).

Tissue culture offers an alternative approach in that single clumps of callus represent hundreds or even thousands of cells, each of which can be selected for a novel trait such as herbicide resistance (Jain, 2001). Mutations arising spontaneously in tissue culture or upon some kind of induction can be directly selected in culture and mutated events selected.

The exploitation of somaclonal variation that is inherent to *in vitro* tissue culture techniques has been a successful approach to selectively generate mutations that confer DIM and FOP tolerance in corn (Somers, 1996; Somers et al., 1994; Marshal et al., 1992; Parker et al., 1990) and in seashore paspalum (Heckart et al, 2009). In the case of maize, the efficiencies of producing regenerable events can be calculated. In Somers et al, 1994, sethoxydim resistant maize plants were obtained using tissue culture selection. They utilized 100 g of callus and obtained 2 tolerant lines following stepwise selection at 0.5, 1.0, 2.0, 5.0 and 10 µM sethoxydim. A calculated mutation rate in their protocol would be 2 lines/100 g of callus or 0.02 lines/g.

In the case of seashore paspalum, Heckert directly utilized a high level of sethoxydim and recovered 3 regenerable lines in approx 10,000 callus pieces or, essentially, a 0.03 % rate. While not comparable, these numbers will be later used for comparison with rice tissue culture mutagenesis. In the maize work, calli were constantly culled at each selection stage with only growing callus being transferred; however, in the case of seashore paspalum, all calli were transferred at each subculture.ACCase genes as selectable markers:

Plant transformation involves the use of selectable marker genes to identify the few transformed cells or individuals from the larger group of non-transformed cells or individuals. Selectable marker genes exist, but they are limited in number and availability. Alternative marker genes are required for stacking traits. In addition, the use of a selectable marker gene that confers an agronomic trait (i.e. herbicide resistance) is often desirable. The present invention discloses ACCase genes as selectable markers that can be added to the current limited suite of available selectable marker genes. Any of the mutants described herein can be introduced into a plasmid with a gene of interest and tranformed into the whole plant, plant tissue or plant cell for use as selectable markers. A detailed method is outlined in example 7 below. The selectable markers of the inventions may be utilized to produce events that confer field tolerance to a given group of herbicides and other where cross protection has been shown (i.e., FOP's).

Modem, high throughput plant transformation systems require an effective selectable marker system; however, there is a limited number available that are acceptable in the market. Therefore, selection systems which also convey a commercial trait are always valuable. The system described herein is an effective selection system in/for plant cells which also encode for an herbicide tolerance trait suitable for use in any monocotyledonous crop.

In one embodiment, the present invention provides a method for selecting a tranformed plant comprising introducing a nucleic acid molecule encoding a gene of interest into a plant cell, wherein the nucleic acid molecule further encodes a mutant acetyl-Coenzyme A carboxylase (ACCase) in which the amino acid sequence differs from an amino acid sequence of an ACCase of a corresponding wild-type rice plant at one amino acid position; and contacting the plant cells with an ACCase inhibitor to obtain the transformed plant, wherein said mutant ACCase confers upon the transformed plant increased herbicide tolerance as compared to the corresponding wild-type variety of the plant when expressed therein.

In one embodiment, the present invention provides a method of marker-assisted breeding, the method comprising breeding any plant of the invention with a second plant; and contacting progeny of the breeding step with an ACCase inhibitor to obtain the progeny comprising said mutant ACCase; wherein said mutant ACCase confers upon the progeny plant increased herbicide tolerance as compared to the second plant.

In one embodiment, a single ACCase gene is linked to a single gene of interest. The ACCase gene may be linked upstream or downstream of the gene of interest.

In one embodiment, the present invention provides for the use of ACCase nucleic acid and protein as described above in diagnostic assays. The diagnostic uses for selectable markers described herein can be employed to identify ACCase gene. Diagnostic methods can include PCR methodologies, proteins assays, labeled probes, and any other standard diagnostic methods known in the art.

### EXAMPLES

### EXAMPLE 1

### Tissue culture conditions

An *in vitro* tissue culture mutagenesis assay has been developed to isolate and characterize plant tissue (e.g., rice tissue) that is tolerant to acetyl-Coenzyme A carboxylase inhibiting herbicides, e.g., tepraloxydim, cycloxydim, and sethoxydim. The assay utilizes the somaclonal variation that is found in *in vitro* tissue culture. Spontaneous mutations derived from somaclonal variation can be enhanced by chemical mutagenesis and subsequent selection in a stepwise manner, on increasing concentrations of herbicide.

The present invention provides tissue culture conditions for encouraging growth of friable, embryogenic rice callus that is regenerable. Calli were initiated from 4 different rice cultivars encompassing both Japonica (Taipei 309, Nipponbare, Koshihikari) and Indica (Indica 1) varieties. Dehusked seed were surface sterilized in 70% ethanol for approximately 1 min followed by 20% commercial Clorox bleach for 20 minutes. Seeds were rinsed with sterile water and plated on callus induction media. Various callus induction media were tested. The ingredient lists for the media tested are presented in Table 2.

**Table 2**

| **Ingredient** | **Supplier** | **R001M** | **R025M** | **R026M** | **R327M** | **R008M** | **MS711R** |
|---|---|---|---|---|---|---|---|
| B5 Vitamins | Sigma | | | | | 1.0 X | |
| MS salts | Sigma | | | 1.0 X | 1.0 X | 1.0 X | 1.0 X |
| MS Vitamins | Sigma | | | 1.0 X | 1.0 X | | |
| N6 salts | Phytotech | 4.0 g/L | 4.0g/L | | | | |
| N6 vitamins | Phytotech | 1.0 X | 1.0 X | | | | |
| L-Proline | Sigma | 2.9 g/L | 0.5 g/L | | | | 1.2 g/L |
| Casamino Acids | BD | 0.3 g/L | 0.3 g/L | 2 g/L | | | |
| Casein Hydrolysate | Sigma | | | | | | 1.0 g/L |
| L-Asp Monohydrate | Phytotech | | | | | | 150 mg/L |
| Nicotinic Acid | Sigma | | | | | | 0.5 mg/L |
| Pyridoxine HCl | Sigma | | | | | | 0.5 mg/L |
| Thiamine HCl | Sigma | | | | | | 1.0 mg/L |
| Myo-inositol | Sigma | | | | | | 100 mg/L |
| MES | Sigma | 500 mg/L | 500 mg/L | 500 mg/L | 500 mg/L | 500 mg/L | 500 mg/L |
| Maltose | VWR | 30 g/L | 30 g/L | 30 g/L | 30 g/L | | |
| Sorbitol | Duchefa | | | 30 g/L | | | |
| Sucrose | VWR | | | | | 10 g/L | 30 g/L |
| NAA | Duchefa | | | | | 50 µg/L | |
| 2,4-D | Sigma | 2.0 mg/L | | | | | 1.0 mg/L |
| MgCl₂·6H₂O | VWR | | | | | 750 mg/L | |
| →pH | | 5.8 | 5.8 | 5.8 | 5.8 | 5.8 | 5.7 |
| Gelrite | Duchefa | 4.0 g/L | | | | 2.5 g/L | |
| Agarose Type1 | Sigma | | 7.0 g/L | 10 g/L | 10 g/L | | |
| →Autoclave | | 15 min | 15 min | 15 min | 15 min | 15 min | 20 min |
| Kinetin | Sigma | | 2.0 mg/L | 2.0 mg/L | | | |
| NAA | Duchefa | | 1.0 mg/L | 1.0 mg/L | | | |
| ABA | Sigma | | 5.0 mg/L | | | | |
| Cefotaxime | Duchefa | | 0.1 g/L | 0.1 g/L | 0,1 g/L | | |
| Vancomycin | Duchefa | | 0.1 g/L | 0.1 g/L | 0.1 g/L | | |
| G418 Disulfate | Sigma | | 20 mg/L | 20 mg/L | 20 mg/L | | |

R001M callus induction media was selected after testing numerous variations. Cultures were kept in the dark at 30°C. Embryogenic callus was subcultured to fresh media after 10-14 days.

### EXAMPLE 2

### Selection of herbicide-tolerant calli

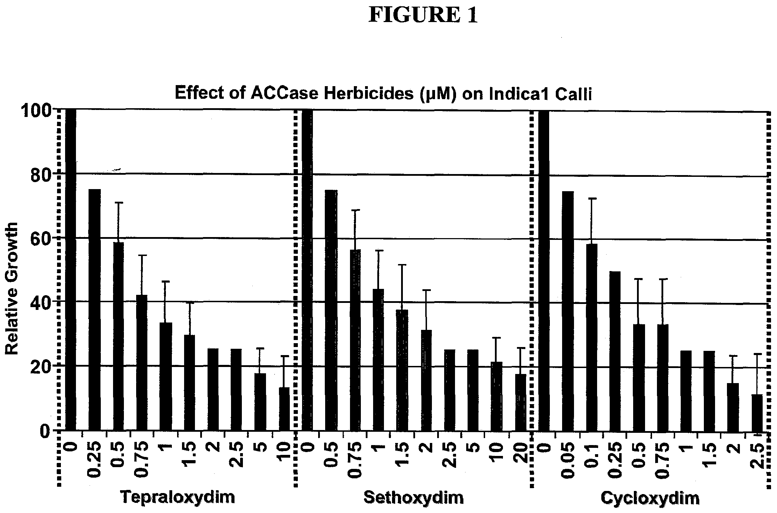

Once tissue culture conditions were determined, further establishment of selection conditions were established through the analysis of tissue survival in kill curves with cycloxydim, tepraloxydim, sethoxydim (Figure 1) or haloxyfop (not shown). Careful consideration of accumulation of the herbicide in the tissue, as well as its persistence and stability in the cells and the culture media was performed. Through these experiments, a sub-lethal dose has been established for the initial selection of mutated material.

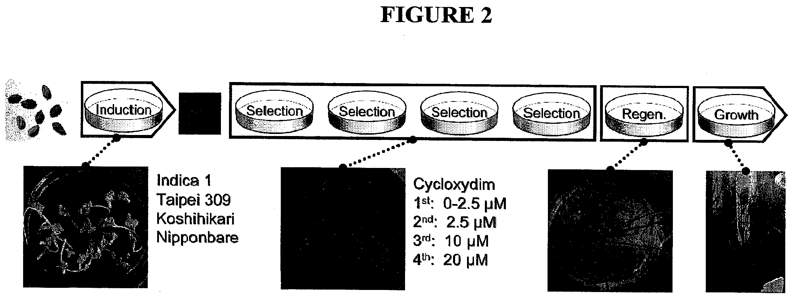

After the establishment of the starting dose of sethoxydim, cycloxydim, tepraloxydim, and haloxyfop in selection media, the tissues were selected in a step-wise fashion by increasing the concentration of the ACCase inhibitor with each transfer until cells are recovered that grew vigorously in the presence of toxic doses (see Figure 2). The resulting calli were further subcultured every 3-4 weeks to R001M with selective agent. Over 26,000 calli were subjected to selection for 4-5 subcultures until the selective pressure was above toxic levels as determined by kill curves and observations of continued culture. Toxic levels were determined to be 50 µM sethoxydim, 20 µM cycloxydim, 2.5 µM tepraloxydim (Figure 1) and 10 µM haloxyfop (not shown).

Alternatively, liquid cultures initiated from calli in MS711R (Table 2) with slow shaking and weekly subcultures. Once liquid cultures were established, selection agent was added directly to the flask at each subculture. Following 2-4 rounds of liquid selection, cultures were transferred to filters on solid R001M media for further growth.

### EXAMPLE 3

### Regeneration of plants

Tolerant tissue was regenerated and characterized molecularly for ACCase gene sequence mutations and/or biochemically for altered ACCase activity in the presence of the selective agent.

Following herbicide selection, calli were regenerated using a media regime of R025M for 10 - 14 days, R026M for ca. 2 weeks, R327M until well formed shoots were developed, and R008S until shoots were well rooted for transfer to the greenhouse (Table 2). Regeneration was carried out in the light. No selection agent was included during regeneration.

Once strong roots were established, M0 regenerants were transplant to the greenhouse in 4" square pots in a mixture of sand, NC Sandhills loamy soil, and Redi-earth (2:4:6) supplemented with gypsum. Transplants were maintained under a clear plastic cup until they were adapted to greenhouse conditions (ca. 1 week). The greenhouse was set to a day/night cycle of 27°C/21°C (80°F/70°F) with 600W high pressure sodium lights supplementing light to maintain a 14 hour day length. Plants were watered 2-3 times a day depending in the weather and fertilized daily. Rice plants selected for seed increase were transplanted into one gallon pots. As plants approached maturity and prepared to bolt, the pots were placed in small flood flats to better maintain water and nutrient delivery. Plants were monitored for insects and plant health and managed under standard Integrated Pest Management practices.

### EXAMPLE 4

### Sequence analysis

Leaf tissue was collected from clonal plants separated for transplanting and analyzed as individuals. Genomic DNA was extracted using a Wizard® 96 Magnetic DNA Plant System kit (Promega, US Patent Nos. 6,027,945 & 6,368,800) as directed by the manufacturer. Isolated DNA was PCR amplified using one forward and one reverse primer.
Forward Primers:
   OsACCpU5142: 5'-GCAAATGATATTACGTTCAGAGCTG-3' (SEQ ID NO:7)
   OsACCpU5205: 5'-GTTACCAACCTAGCCTGTGAGAAG-3' (SEQ ID NO: 8)
Reverse Primers:
   OsACCpL7100: 5'-GATTTCTTCAACAAGTTGAGCTCTTC-3' (SEQ ID NO: 9)
   OsACCpL7054: 5'-AGTAACATGGAAAGACCCTGTGGC-3' (SEQ ID NO: 10)

PCR amplification was performed using Hotstar Taq DNA Polymerase (Qiagen) using touchdown thermocycling program as follows: 96°C for 15 min, followed by 35 cycles (96°C, 30 sec; 58°C - 0.2 °C per cycle, 30 sec; 72°C, 3 min and 30 see), 10 min at 72°C.

PCR products were verified for concentration and fragment size via agarose gel electrophoresis. Dephosphorylated PCR products were analyzed by direct sequence using the PCR primers (DNA Landmarks). Chromatogram trace files (.scf) were analyzed for mutation relative to Os05g0295300 using Vector NTI Advance 10™ (Invitrogen). Based on sequence information, two mutations were identified in several individuals. I1,781(*Am)*L and D2,078(*Am)*G were present in the heterozygous state. Sequence analysis was performed on the representative chromatograms and corresponding AlignX alignment with default settings and edited to call secondary peaks.

Samples inconsistent with an ACCase mutation were spray tested for tolerance and discarded as escapes. Surprisingly, most of the recovered lines were heterozygous for the I1,781(*Am)*L mutation and resistant events were generated in all tested genotypes using cycloxydim or sethoxydim: Indica1 (≥18 lines), Taipei 309 (≥14 lines), Nipponbare (≥3 lines), and Koshihikare (≥6 lines). One line was heterozygous for a D2,078(*Am*)G mutation. The D2,078(*Am*)G heterozygote line appeared stunted with narrow leaves, while the I1,781(*Am*)L heterozygotes varied in appearance, but most looked normal relative to their parental genotype. Several escapes were recovered and confirmed by sequencing and spray testing; however, sequencing results of the herbicide sensitive region of ACCase revealed that most tolerant mutants were heterozygous for an I1,781(*Am*)L, A to T mutation (See Table 3). One line, OsARWI010, was heterozygous for a D2,078(*Am*)G, A to G mutation. To date, all recovered plants lacking an ACCase mutation have been sensitive to herbicide application in the greenhouse.

**Table 3: Genotype of Rice Lines Recovered via Tissue Culture Selection**

| **Line** | **Parental Genotype** | **Rice Type** | **Mutation Identified** | **ATCC® Patent Deposit Designation** |
|---|---|---|---|---|
| OsARWI1 | Indica 1 | *indica* | I1781(*Am*)L | PTA-10568 |
| OsARWI3 | Indica 1 | *indica* | I1781(*Am*)L | PTA-10569 |
| OsARWI8 | Indica 1 | *indica* | I1781(*Am*)L | PTA-10570 |
| OsARWI10 | Indica 1 | *indica* | D2078(*Am*)G | NA, sterile |
| OsARWI15 | Indica 1 | *indica* | I1781(*Am*)L | NA |
| OsHPHI2 | Indica 1 | *indica* | I1781(*Am*)L | PTA-10267 |
| OsHPHI3 | Indica 1 | *indica* | I1781(*Am*)L | NA |
| OsHPHI4 | Indica 1 | *indica* | I1781(*Am*)L | NA |
| OsHPHK1 | Koshihikari | *japonica* | I1781(*Am)*L | NA |
| OsHPHK2 | Koshihikari | *japonica* | I1781(*Am*)L | NA |
| OsHPHK3 | Koshihikari | *japonica* | I1781(*Am)*L | NA |
| OsHPHK4 | Koshihikari | *japonica* | I1781(*Am*)L | NA |
| OsHPHK6 | Koshihikari | *japonica* | I1781(*Am*)L | NA |
| OsHPHN1 | Nipponbare | *japonica* | I1781(*Am*)L | PTA-10571 |
| OsHPHT1 | Taipei 309 | *japonica* | I1781(*Am*)L | NA |
| OsHPHT4 | Taipei 309 | *japonica* | I1781(*Am*)L | NA |
| OsHPHT6 | Taipei 309 | *japonica* | I1781(*Am*)L | NA |

### EXAMPLE 5

### Demonstration of herbicide-tolerance

Selected mutants and escapes were transferred to small pots. Wild-type cultivars and 3 biovars of red rice were germinated from seed to serve as controls.

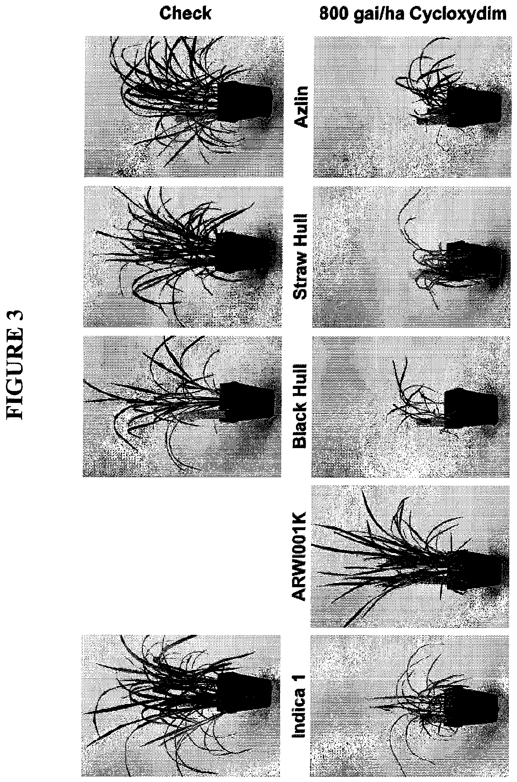

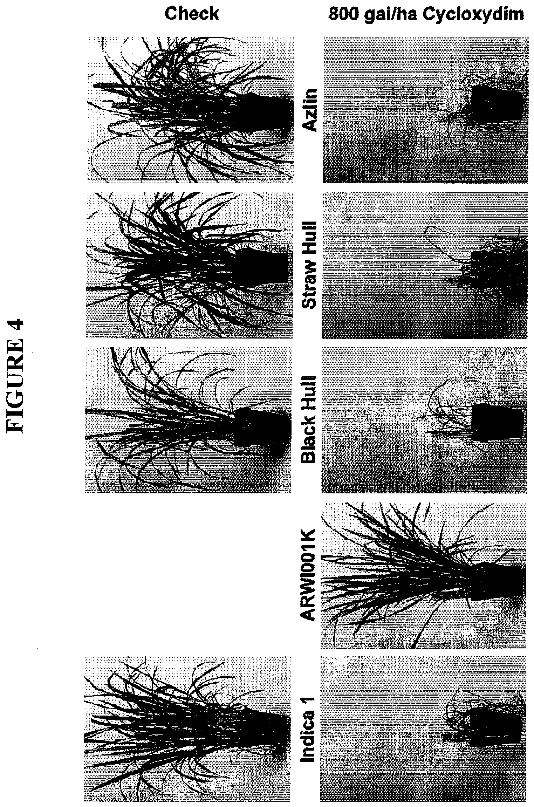

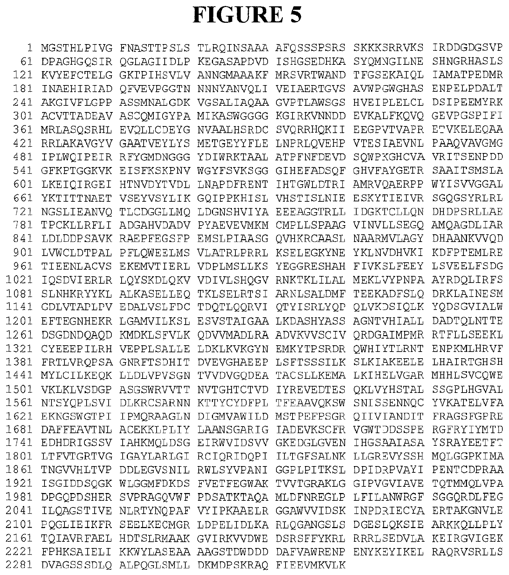

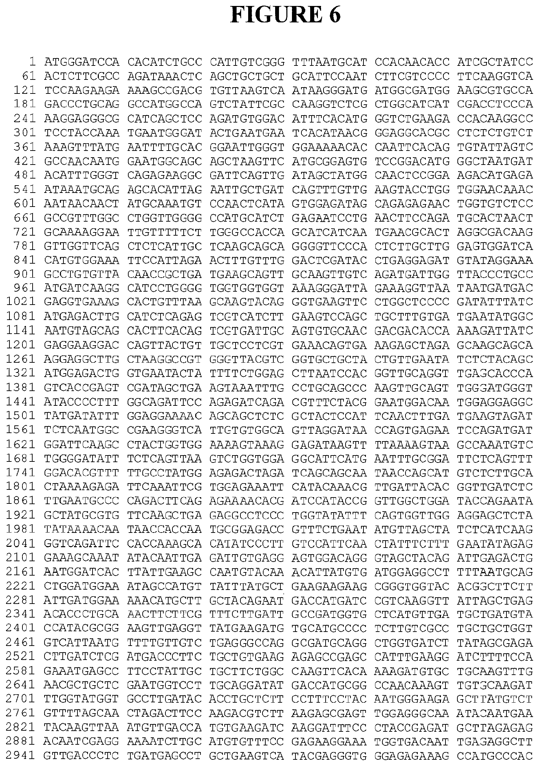

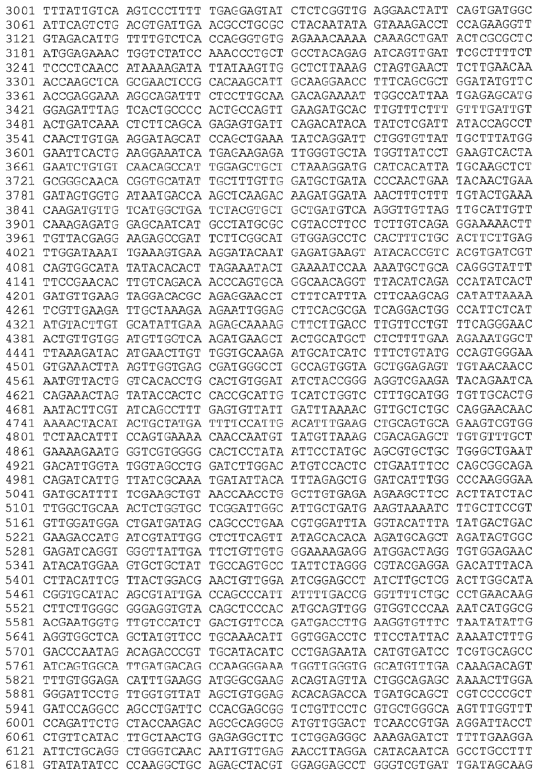

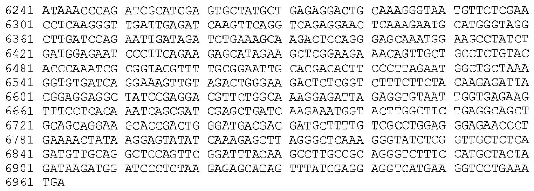

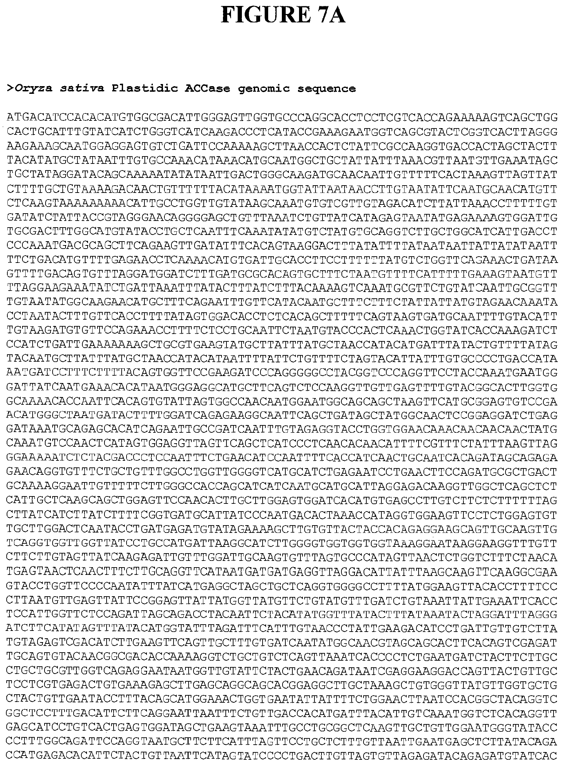

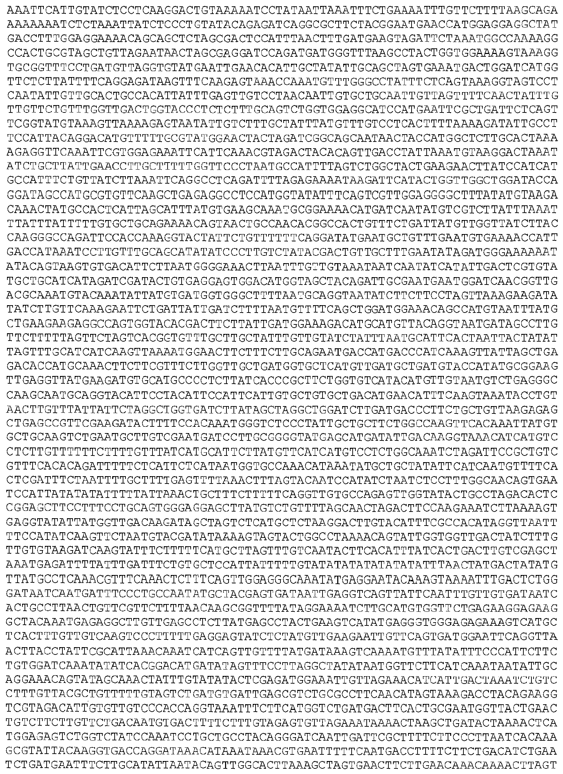

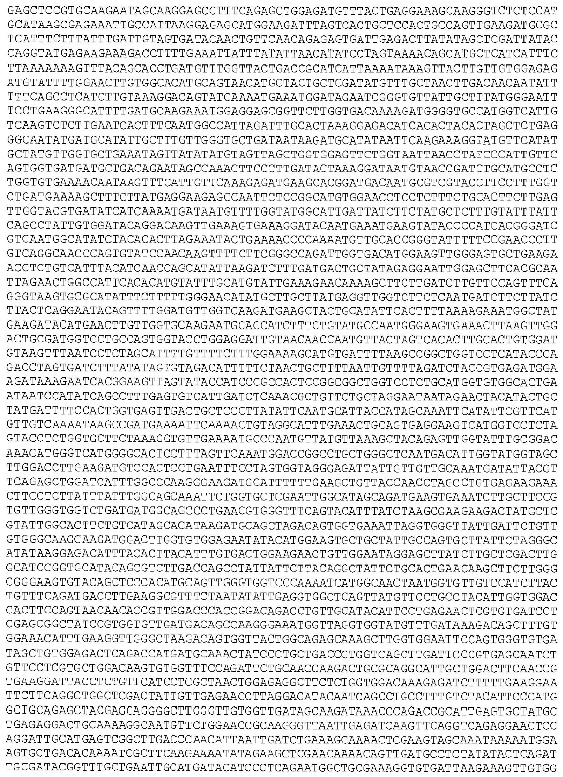

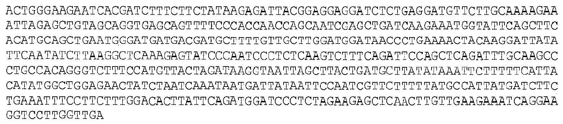

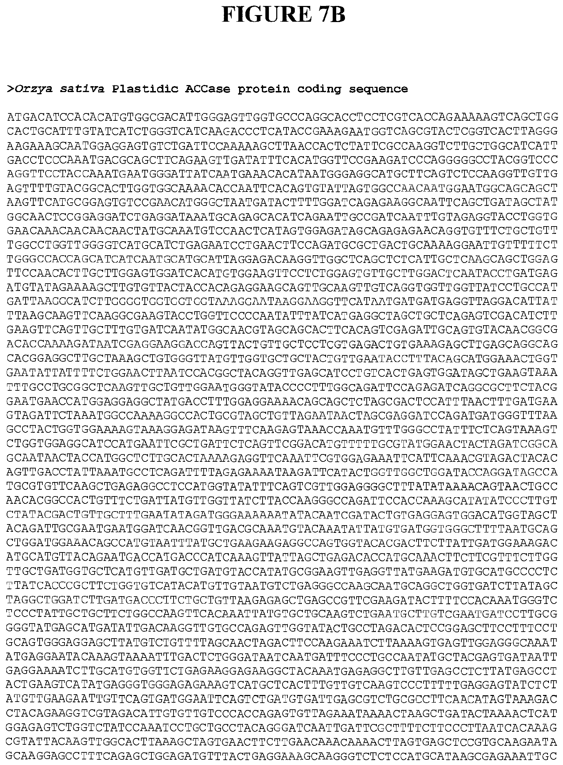

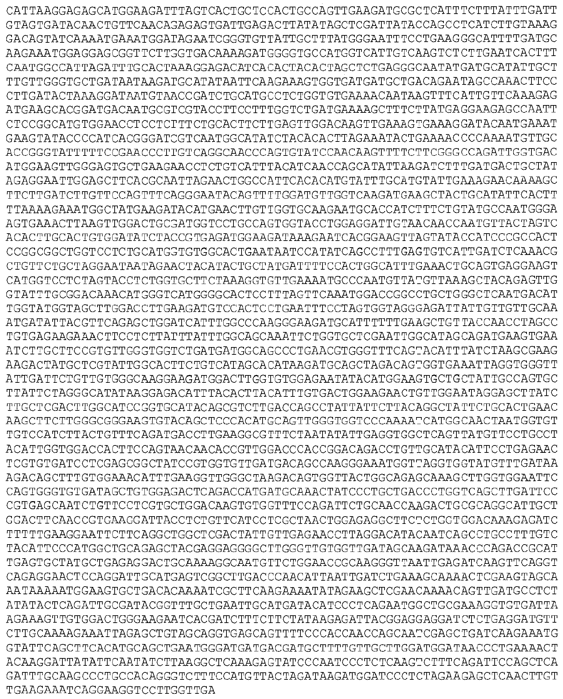

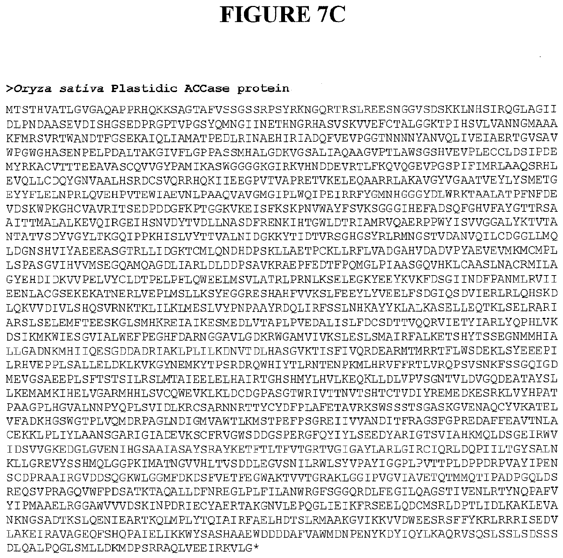

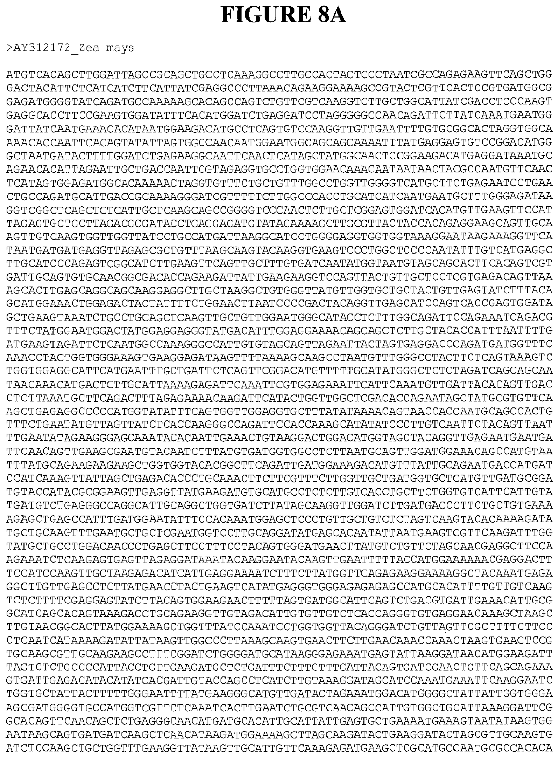

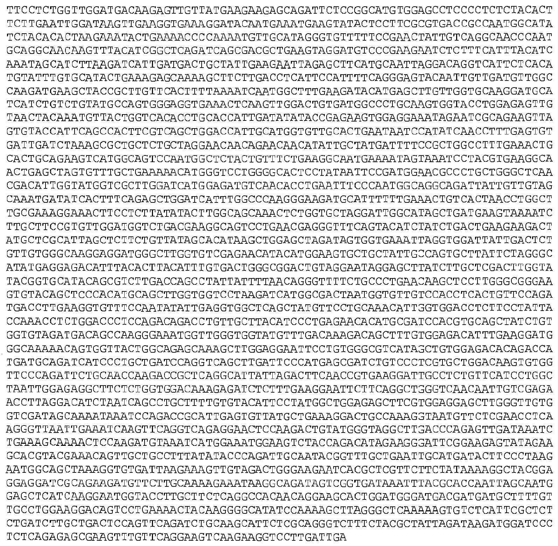

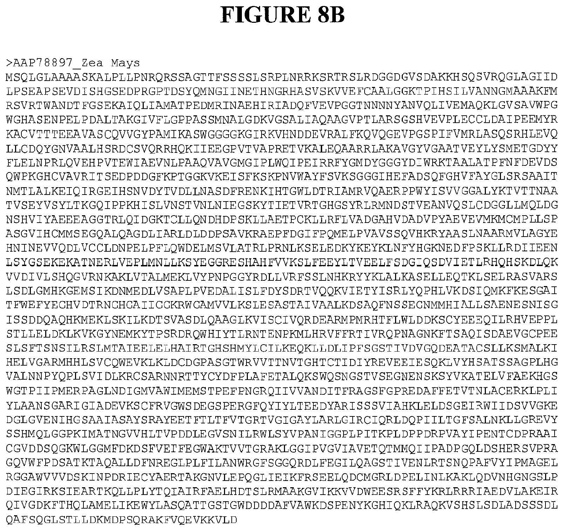

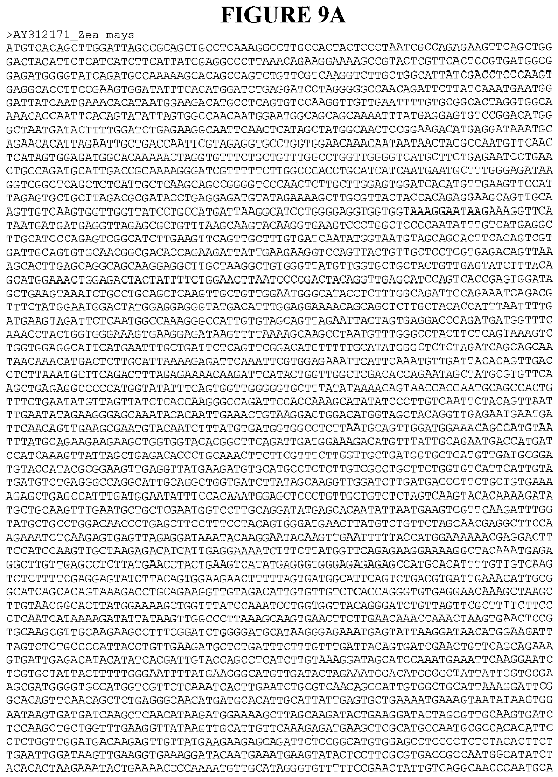

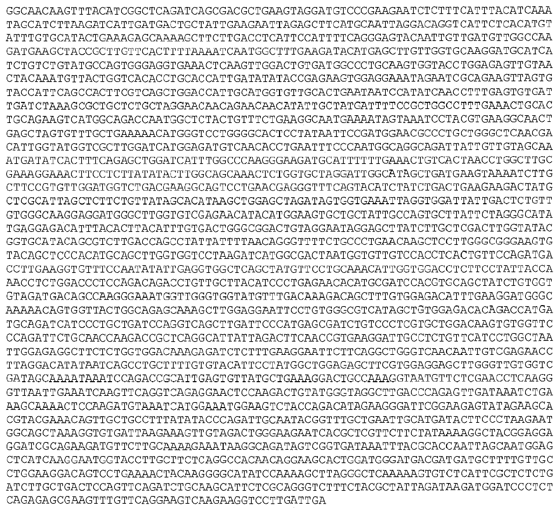

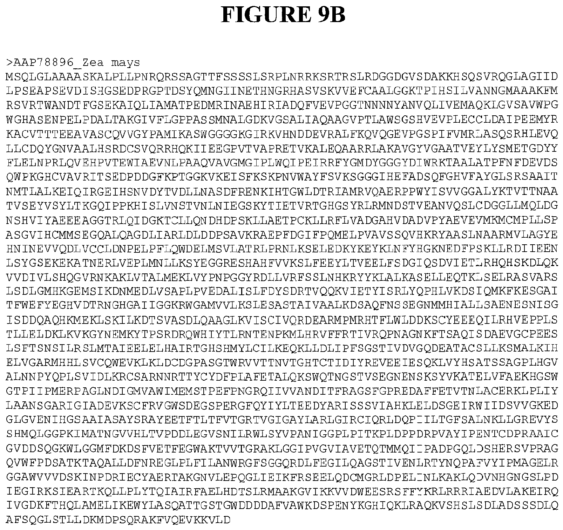

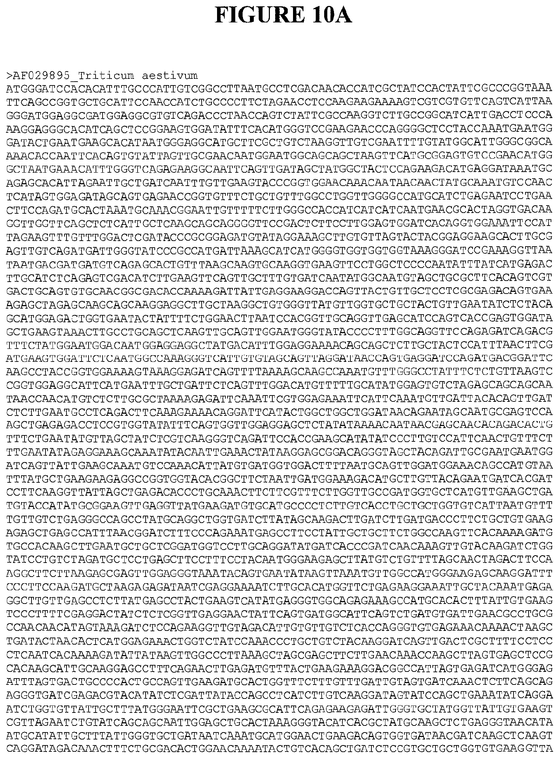

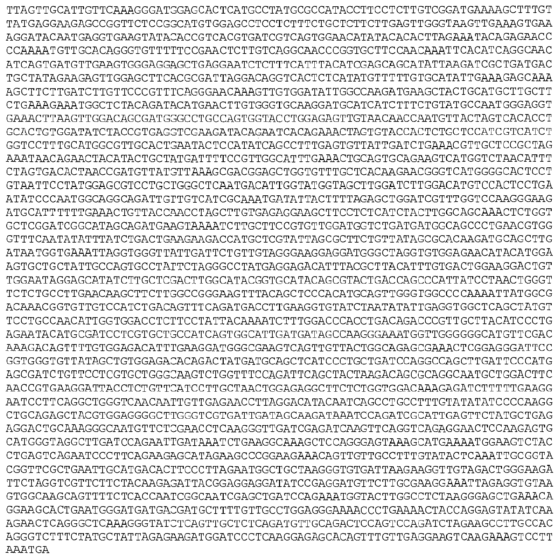

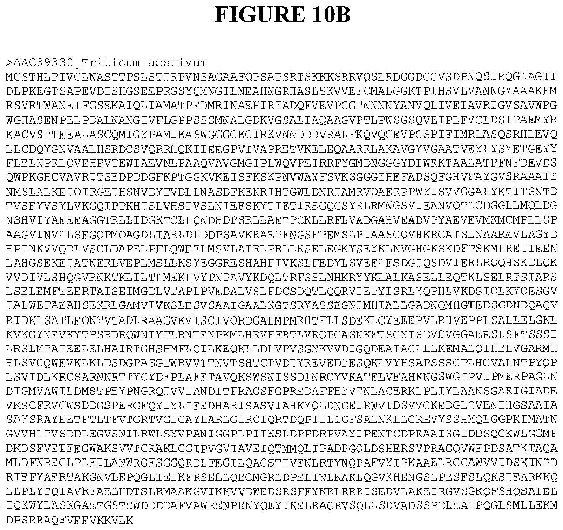

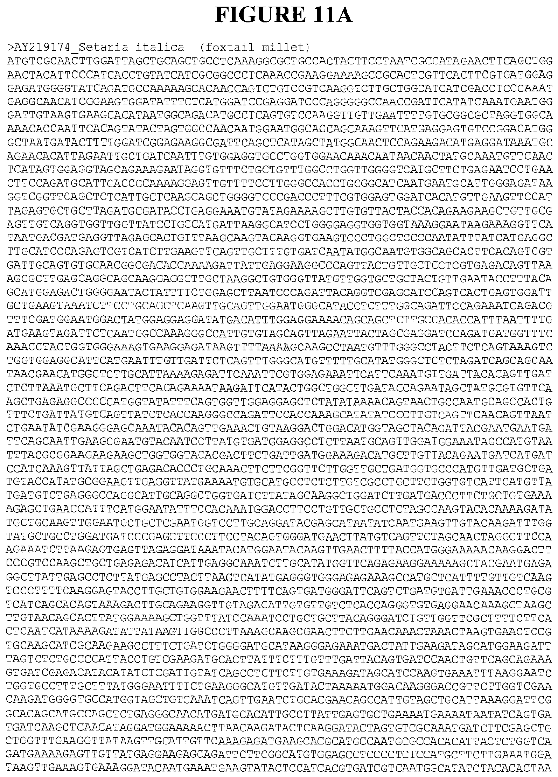

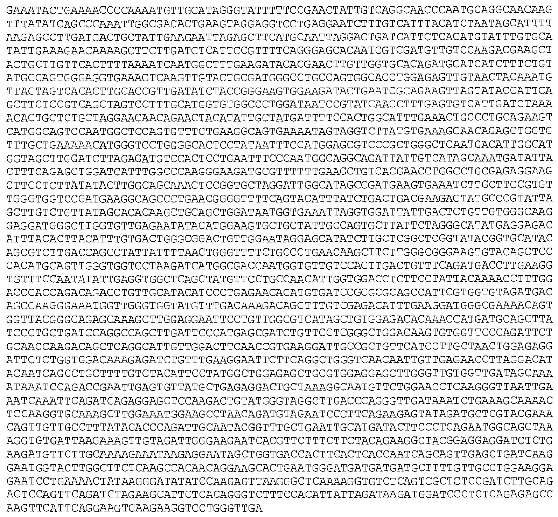

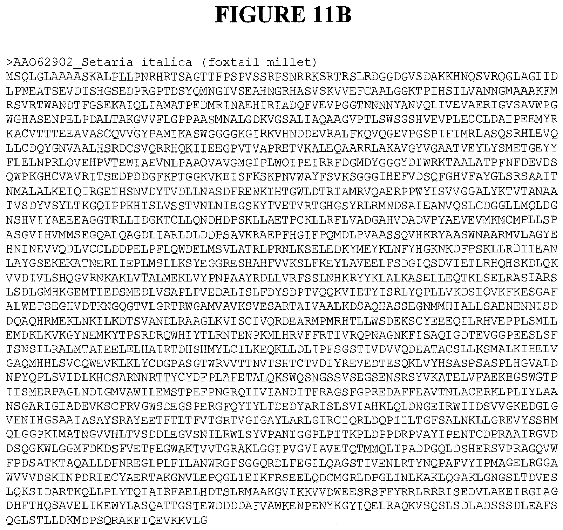

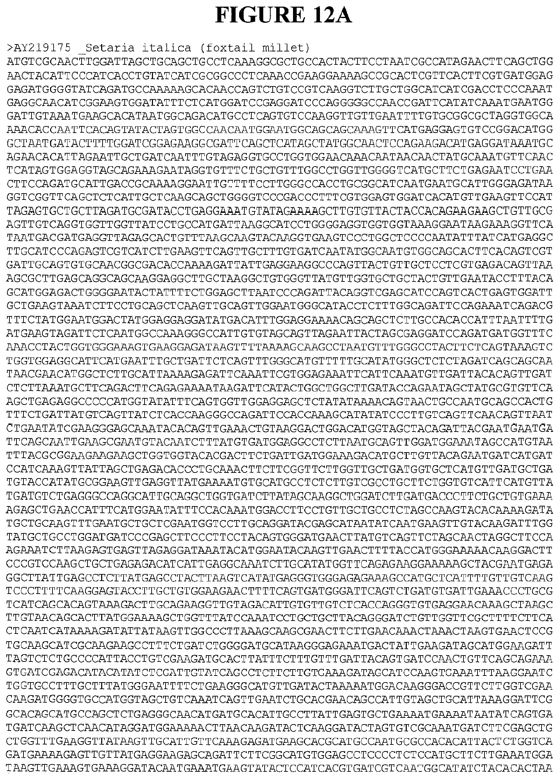

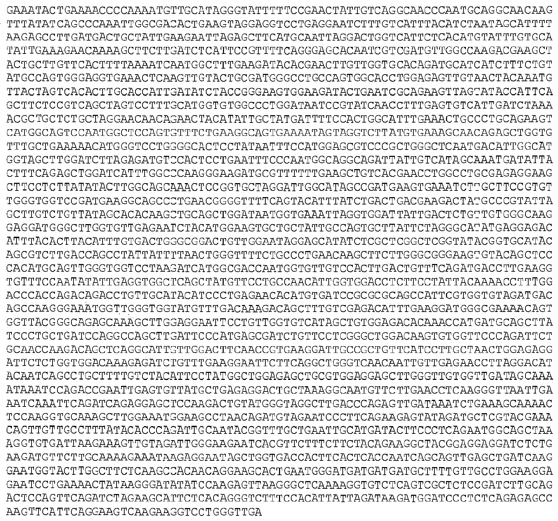

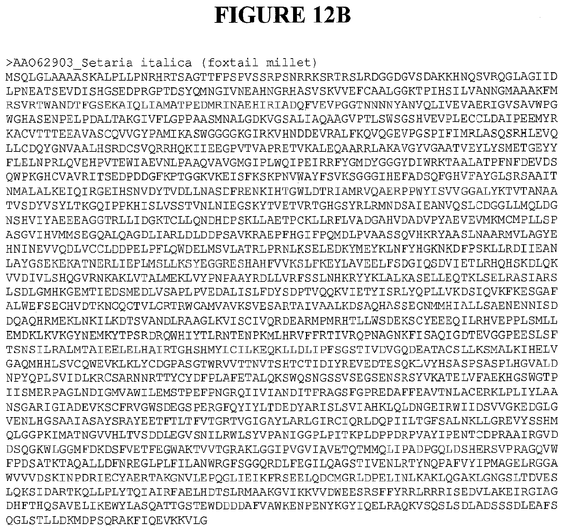

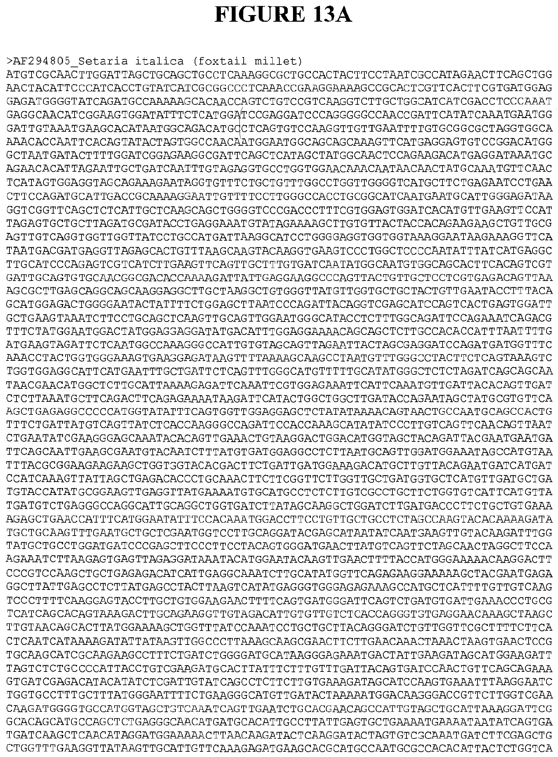

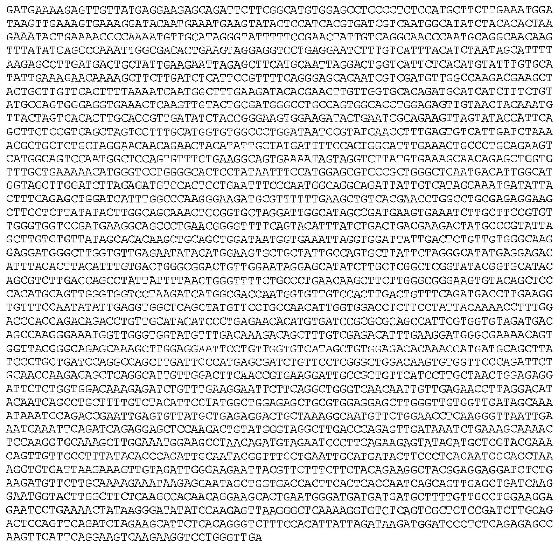

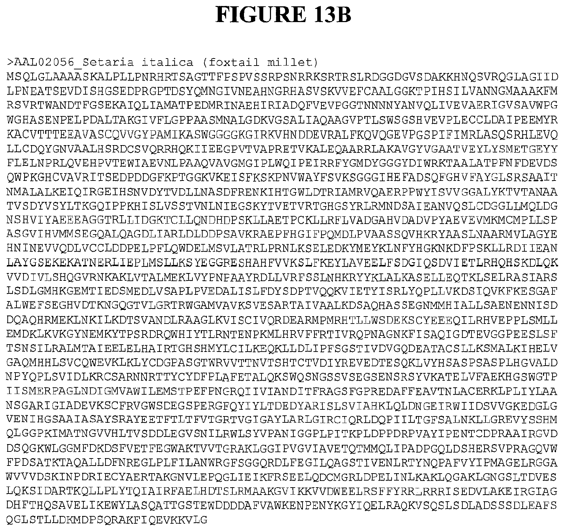

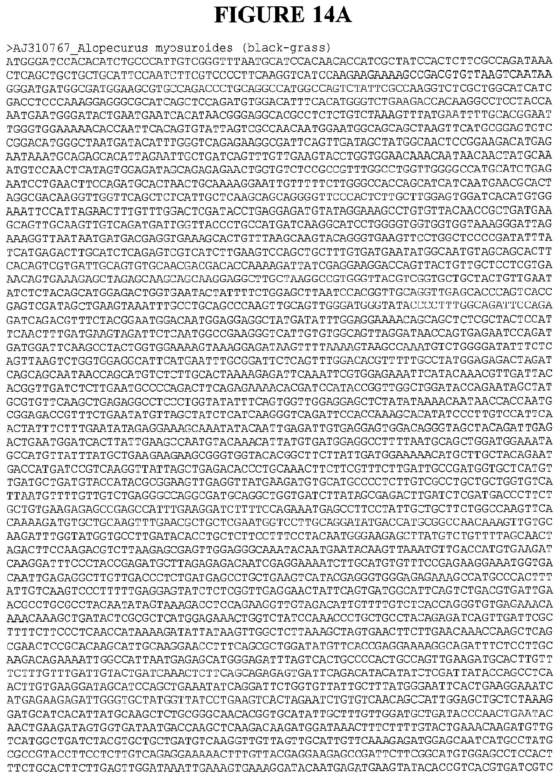

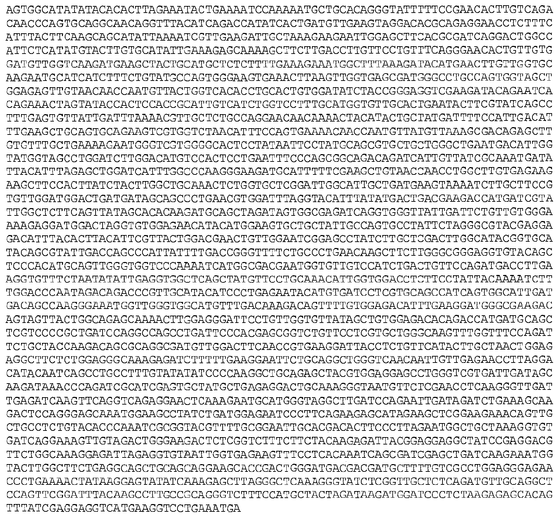

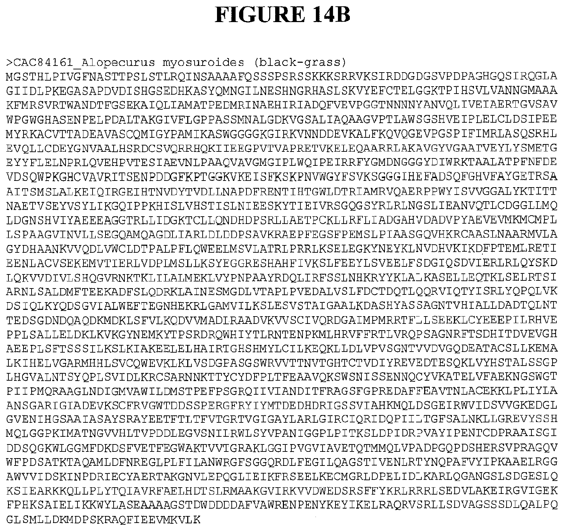

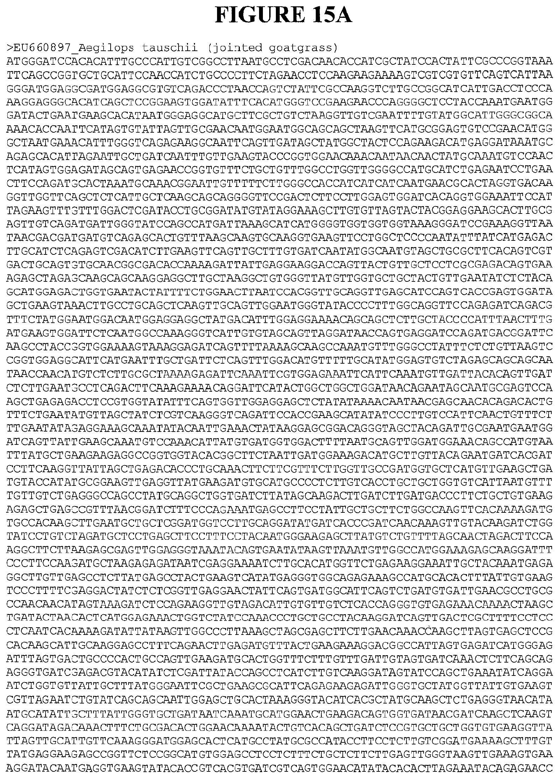

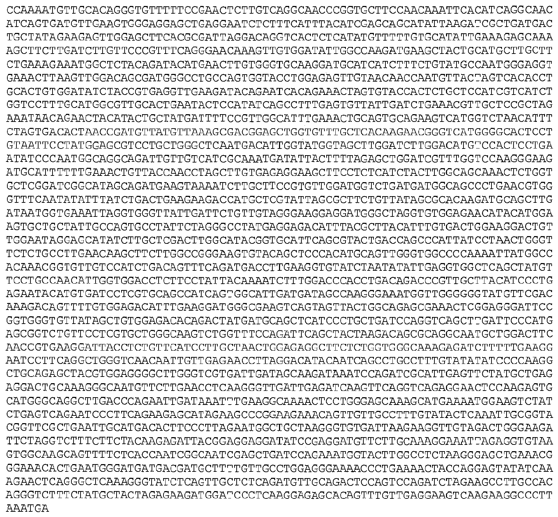

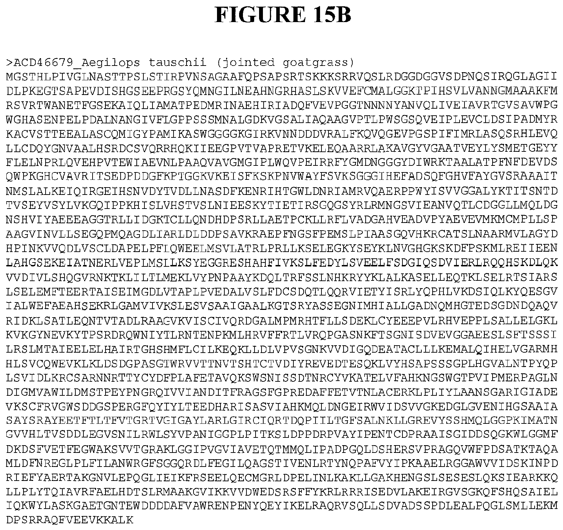

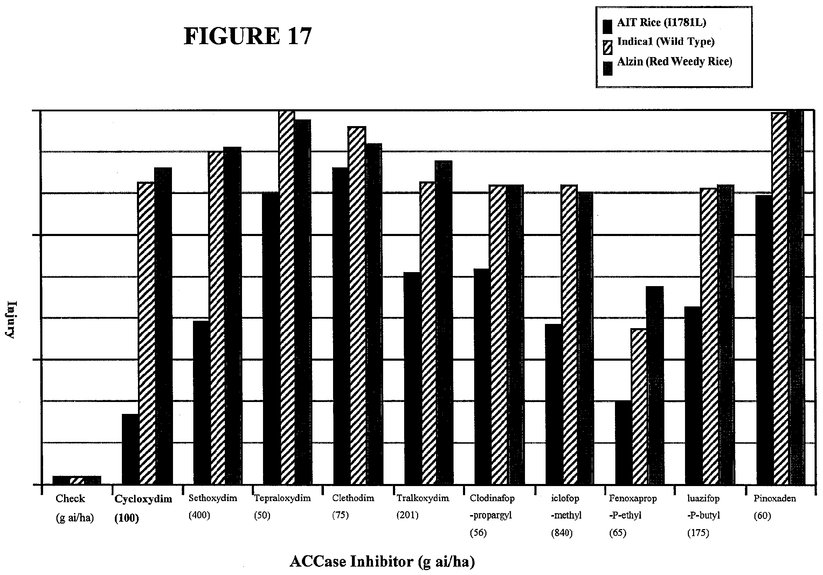

After ca. 3 weeks post-transplant, M0 regenerants were sprayed using a track sprayer with 400-1600 g ai/ha cycloxydim (BAS 517H) supplemented with 0.1% methylated seed oil. After the plants had adapted to greenhouse conditions, a subset were sprayed with 800 g ai/ha cycloxydim. Once sprayed, plants were kept on drought conditions for 24 hours before being watered and fertilized again. Sprayed plants were photographed and rated for herbicide injury at 1 (Figure 3) and 2 weeks after treatment (Figure 4). No injury was observed on plants containing the 11,781(*Am*)L heterozygous mutation while control plants and tissue culture escapes (regenerated plants negative for the sequenced mutations) were heavily damaged after treatment (Figures 3 & 4). Figures 5-15 provide nucleic acid and/or amino acid sequences of acetyl-Coenzyme A carboxylase enzymes from various plants. Figure 17 provides a graph showing results for mutant rice versus various ACCase inhibitors.

### EXAMPLE 6

### Herbicide selection using tissue Culture

Media was selected for use and kill curves developed as specified above. For selection, different techniques were utilized. Either a step wise selection was applied, or an immediate lethal level of herbicide was applied. In either case, all of the calli were transferred for each new round of selection. Selection was 4-5 cycles of culture with 3-5 weeks for each cycle. Cali were placed onto nylon membranes to: facilitate transfer (200 micron pore sheets, Biodesign, Saco, Maine). Membranes were cut to fit 100x20 mm Petri dishes and were autoclaved prior to use 25-35 calli (average weight/calli being 22mg) were utilized in every plate. In addition, one set of calli were subjected to selection in liquid culture media with weekly subcultures followed by further selection on semi-solid media.

Mutant lines were selected using cycloxydim or sethoxydim in 4 different rice genotypes. Efficiencies of obtaining mutants was high either based on a percentage of calli that gave rise to a regenerable, mutant line or the number of lines as determined by the gram of tissue utilized. Overall, the mutation frequency compared to seashore paspalum is 5 fold and compared to maize is 2 fold. In some cases, this difference is much higher (>10 fold) as shown in Table 4 below.

**Table 4**

| Genotype | # Calli | Selection | Mutants | Rate | Weight (g) | # / gm callus |
|---|---|---|---|---|---|---|
| Indica 1 | 1865 | Cycloxidim | 3 | 0.161% | 41.04 | 0.07 |
| Indica 1 | 2640 | Sethoxydim | 3 | 0.114% | 58.08 | 0.05 |
| Koshi | 1800 | Cycloxidim | 6 | 0.333% | 39.6 | 0.15 |
| NB | 3400 | Cycloxidim | 1 | 0.029% | 74.8 | 0.01 |
| NB | 725 | Sethoxydim | 0 | 0.000% | 15.95 | 0.00 |
| T309 | 1800 | Cycloxidim | 8 | 0.444% | 36.9 | 0.20 |
| T309 | 1015 | Sethoxydim | 0 | 0.000% | 22.33 | 0.00 |
| Total | 13245 | | 21 | 0.159% | 291.39 | 0.07 |

If the data is analyzed using the criteria of selection, it is possible to see that cylcoxydim selection contributes to a higher rate of mutants isolated than sethoxydim, as shown in Table 5.

**Table 5**

| Genotype | # Calli | Selection | Mutants | Rate | Weight (g) | # / gm callus |
|---|---|---|---|---|---|---|
| Indica 1 | 1865 | Cycloxidim | 3 | 0.161% | 41.03 | 0.07 |
| Koshi | 1800 | Cycloxidim | 6 | 0.333% | 39.6 | 0.15 |
| NB | 3400 | Cycloxidim | 1 | 0.029% | 74.8 | 0.01 |
| T309 | 1800 | Cycloxidim | 8 | 0.444% | 39.6 | 0.20 |
| Total | 8865 | | 18 | 0.203% | 195.03 | 0.09 |
| | | | | | | |
| Indica 1 | 2640 | Sethoxydim | 3 | 0.114% | 58.08 | 0.05 |
| NB | 725 | Sethoxydim | 0 | 0.000% | 15.95 | 0.00 |
| T309 | 1015 | Sethoxydim | 0 | 0.000% | 22.33 | 0.00 |
| Total | 4380 | | 3 | 0.068% | 96.36 | 0.03 |

Using this analysis, the rate for cycloxydim is almost 10 fold higher than either of the previous reports using sethoxydim selection, whereas rates using sethoxydim selection are similar to those previously reported. Further, 68% of the lines were confirmed as mutants when selection was on cycloxydim compared to 21% of the lines when selection was on sethoxydim. Increases seem to come from using cycloxydim instead of sethoxydim as a selection agent. Further, the use of membranes made transfer of callus significantly easier than moving each piece individually during subcultures. Over 20 mutants were obtained. Fertility appears to be high with the exception of one mutant that has a mutation known to cause a fitness penalty (D2,078(*Am*)G).

### EXAMPLE 7

### Use of mutant ACCase genes as selectable markers in plant transformation

### Methods:

Indica1 and Nipponbare rice callus transformation was carried out essentially as described in Hiei and Komari (2008) with the exception of media substitutions as specified (see attached media table for details). Callus was induced on R001M media for 4-8 weeks prior to use in transformation. Agrobacterium utilized was LBA4404(pSB1) (Ishida et al. 1996) transformed with RLM185 (L. Mankin, unpublished: contains DsRed and a mutant AHAS for selection), ACC gene containing I1781(*Am*)L, ACC gene containing I1781(*Am*)L and W2027C, ACC gene containing I1781(*Am*)L and I2041(*Am*)N, or ACC gene containing I1781(*Am*)A or wild type which also contains a mutant AHAS gene for selection. Agrobacterium grown for 1-3 days on solid media was suspended in M-LS-002 medium and the OD₆₆₀ adjusted to approximately 0.1. Callus was immersed in the Agrobacterium solution for approximately 30 minutes. Liquid was removed, and then callus was moved to filter paper for co-culture on semi-solid rice cc media. Co-culture was for 3 days in the dark at 24°C. Filters containing rice callus were directly transferred to R001M media containing Timentin for 1-2 weeks for recovery and cultured in the dark at 30°C. Callus was subdivided onto fresh R001M media with Timentin and supplemented with 100µM Imazethapyr, 10µM Cycloxydim or 2.5µM Tepraloxydim. After 3-4 weeks, callus was transferred to fresh selection media. Following another 3-4 weeks, growing callus was transferred to fresh media and allowed to grow prior to Taqman analysis. Taqman analysis was for the Nos terminator and was conducted to provide for a molecular confirmation of the transgenic nature of the selected calli. Growth of transgenic calli was measured with various selection agents by subculturing calli on media containing either 10µM Cycloxydim or Haloxyfop, 2.5µM Tepraloxydim or 100µM Imazethapry. Calli size was measured from scanned images following initial subculture and then after approximately 1 month of growth.

Transformation of maize immature embryos was carried out essentially as described by Lai et al (submitted). Briefly, immature embryos were co-cultured with the same *Agrobacterium* strains utilized for rice transformation suspended in M-LS-002 medium to an OD₆₆₀ of 1.0. Co-culture was on Maize CC medium for 3 days in the dark at 22°C. Embryos were removed from co-culture and transferred to M-MS-101 medium for 4-7 days at 27°C. Responding embryos were transferred to M-LS-202 medium for Imazethapyr selection or M-LS-213 media supplemented with either 1µM Cycloxydim or 0.75µM Tepraloxydim. Embryos were cultured for 2 weeks and growing callus was transferred to a second round of selection using the same media as previous except that Cycloxydim selection was increased to 5µM. Selected calli were transferred to M-LS-504 or M-LS-513 media supplemented with either 5µM Cycloxydim or 0.75µM of Tepraloxydim for and moved to the light (16hr/8hr day/night) for regeneration. Shoots appeared between 2-3 weeks and were transferred to plantcon boxes containing either M-LS-618 or M-LS-613 supplemented with either 5µM Cycloxydim or 0.75µM of Tepraloxydim for further shoot development and rooting. Leaf samples were submitted for Taqman analysis. Positive plants were transferred to soil for growth and seed generation. In the second set of experiments, conditions were identical except that Tepraloxydim selection was decreased to 0.5µM during regeneration and shoot and root formation. In the third set of experiments, Haloxyfop was also tested as a selection agent. In these experiments, 1µM was used throughout for selection

### Results and Discussion:

Transgenic calli were obtained from Indical rice transformation experiments using ACC gene containing I1781(*Am*)L and W2027(*Am*)C, and ACC gene containing I1781(*Am*)L and I2041(*Am*)N. One callus was obtained from ACC gene containing I1781(*Am*)L and W2027(*Am*)C following Tepraloxydim selection and 3 calli were obtained from ACC gene containing I1781(*Am*)L and I2041(*Am*)N. One callus was obtained from ACC gene containing I1781(*Am)*L and I2041(*Am*)N using Cycloxydim selection. Nos Taqman showed that all of these calli were transgenic. Calli were screened for growth under various selection agents including Imazethapry (Pursuit - P) for the mutant AHAS selectable marker.

As can be observed in Table 6, the double mutant constructs allowed for growth on both Cycloxydim and Tepraloxydim in addition to Haloxyfop. The levels utilized in these growth experiments are inhibitory for wild type material.

**Table 6: Growth of transgenic Indica1 callus on various selection media. Growth was measured as a % change in size following 1 month of culture on the selection media.**

| **Selection µM** | | | | |
|---|---|---|---|---|
| **Construct** | **H10** | **C10** | **T2.5** | **P100** |
| **I1781(*Am*)L, W2027(*Am*)C** | 1669% | 867% | 1416% | 739% |
| **I1781(*Am*)L, I2041(*Am*)N** | 1613% | 884% | 1360% | 634% |

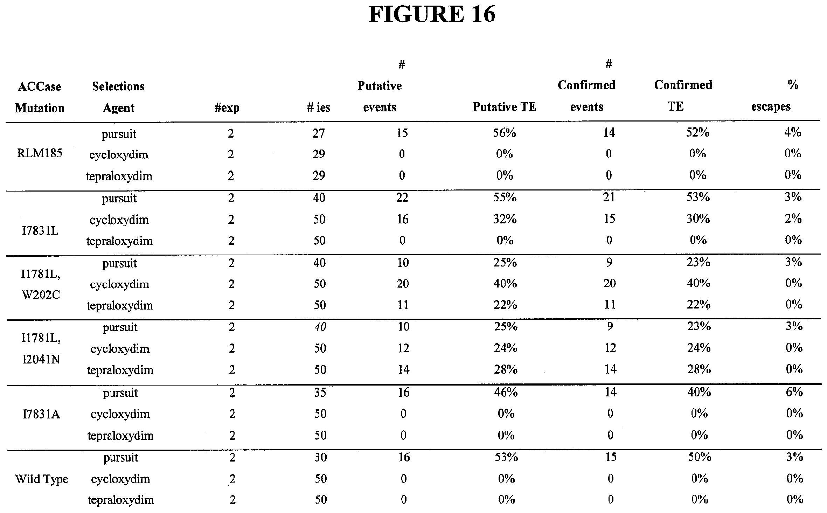

Results from the first set of maize experiments reveal that both the single of the double mutant can be used to select for Cycloxydim resistance or both Cylcoxydim or Tepraloxydim resistance at a relatively high efficiency (Figure 16).

Efficiencies between selection agents was relatively comparable in these experiments with maybe a slight decrease in the overall efficiency with the single mutant on Cycloxydim compared to Pursuit selection. However, the double mutant may have a slight increased efficiency. The escape rate - the percentage of non-confirmed putative events - was lower for Cycloxydim or Tepraloxydim. Further, under the conditions described, it was possible to differentiate between the single and double mutants using Tepraloxydim selection.

Similar results have been obtained in the second set of experiments (not shown). In the third set of experiments, Haloxyfop is also an efficient selectable marker for use in transformation with either the single or the double mutant (not shown).

The single mutant is useful for high efficiency transformation using Cycloxydim or Haloxyfop selection. It should also be useful for other related compounds such as Sethoxydim. The double mutant is useful for these selection agents with the addition that Tepraloxydim can be used. The single and the double mutant can be used in a two stage transformation in that the single mutant can be differentiated from the double with Tepraloxydim selection. In combination with other current BASF selection markers, these give two more options for high efficiency transformations of monocots and maize in particular.

Herbicide tolerance phenotypes as described herein have also been exhibited by ACCase-inhibitor tolerant rice plants hereof, in the field under 600 g/ha cycloxydim treatment (data not shown).

While the foregoing invention has been described in some detail for purposes of clarity and understanding, it will be appreciated by one skilled in the art from a reading of this disclosure that various changes in form and detail can be made without departing from the true scope of the invention and appended claims. All patents and publications cited herein are entirely incorporated herein by reference. The present invention further relates to the following embodiments E1 to E102:
E1. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at amino acid position 1,781(*Am*), wherein said ACCase confers upon the plant increased herbicide tolerance as compared to the corresponding wild-type variety of the plant when expressed therein.
E2. The plant of E1, wherein the amino acid at position at 1,781(*Am*) is leucine.
E3. The plant of E1, wherein the amino acid position at 1,781(*Am*) is alanine.
E4. The plant of E1, wherein the amino acid at position at 1,781(*Am*) is valine.
E5. The plant of E1, wherein the amino acid at position at 1,781(*Am*) is threonine.
E6. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at amino acid position 1,999(*Am*), wherein said ACCase confers upon the plant increased herbicide tolerance as compared to a wild-type variety of the plant when expressed therein.
E7. The plant of E6, wherein the amino acid at position at 1,999(*Am*) is glycine.
E8. The plant of E6, wherein the amino acid at position at 1,999(*Am*) is cysteine.
E9. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at amino acid position 2,027(*Am*), wherein said ACCase confers upon the plant increased herbicide tolerance as compared to a wild-type variety of the plant when expressed therein.
E10. The plant of E9, wherein the amino acid at position at 2,027(*Am*) is cysteine.
E11. The plant of E9, wherein the amino acid at position at 2,027(*Am*) is arginine.
E12. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at amino acid position 2,041(*Am*), wherein said ACCase confers upon the plant increased herbicide tolerance as compared to a wild-type variety of the plant when expressed therein.
E13. The plant of E12, wherein the amino acid at position at 2,041(*Am*) is asparagine.
E14. The plant of E 12, wherein the amino acid at position 2,041(*Am*) is valine.
E15. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at amino acid position 2,096(*Am*), wherein said ACCase confers upon the plant increased herbicide tolerance as compared to a wild-type variety of the plant when expressed therein.
E16. The plant of E15, wherein the amino acid at position at 2,096(*Am*) is alanine.
E17. The plant of E15, wherein the amino acid at position at 2,096(*Am*) is serine.
E18. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at only one of the following positions: 1,785(*Am*); 1,786(*Am*); 1,811(*Am*); 2,049(*Am*); 2,074(*Am*); 2,075(*Am*), 2,078(*Am*); deletion at 2,080(*Am*); 2,088(*Am)*; and 2,098(*Am*), wherein said ACCase confers upon the plant increased herbicide tolerance as compared to a wild-type variety of the plant when expressed therein.
E19. The plant of E18, wherein the plant expresses a mutagenized or recombinant acetyl-Coenzyme A carboxylase (ACCase) in which the amino acid sequence comprises only one substitution selected from the group consisting of isoleucine, leucine, or phenylalanine at 2,049(*Am*); leucine at position 2,074(*Am*); leucine, isoleucine, or methionine at position 2,075(*Am*), or duplication of position 2,075(*Am*); threonine, glycine or lysine at position 2,078(*Am*); arginine, tryptophan, phenylalanine, glycine, histidine, lysine, leucine, serine, threonine, or valine at 2,088(*Am*); and alanine, histidine, proline, serine, or glycine at 2,098(*Am*).
E20. The plant of E18, wherein the plant expresses a mutagenized or recombinant acetyl-Coenzyme A carboxylase (ACCase) in which the amino acid sequence comprises only one substitution selected from the group consisting of glycine at position 1,785(*Am*); proline at position 1,786(*Am*); and asparagine at position 1,811(*Am*).
E21. A rice plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice plant at only one of the following positions: 2,039(*Am*); 2,059(*Am*); 2,080(*Am*); and 2,095(*Am*), wherein said ACCase confers upon the plant increased herbicide tolerance as compared to the corresponding wild-type variety of the plant when expressed therein.
E22. The plant of E21, wherein the plant expresses a mutagenized or recombinant acetyl-Coenzyme A carboxylase (ACCase) in which the amino acid sequence comprises only one substitution selected from the group consisting of glycine at position 2,039(*Am*); valine at position 2,059(*Am*); glutamic acid at position 2,080(*Am*); and glutamic acid at position 2,095(*Am*).
E23. A rice plant expressing a mutagenized or recombinant plastidic acetyl-Coenzyme A carboxylase (ACCase), wherein a mutation at only one amino acid position in the plastidic ACCase confers upon the plant increased herbicide tolerance as compared to a corresponding wild-type variety of the rice plant when expressed therein.
E24. The plant of E23, wherein the amino acid position is selected from the group consisting of 1,785(*Am*), 1,786(*Am*), 1,811(*Am*), 1,824(*Am*), 1,864(*Am*), 2,039(*Am*), 2,049(*Am*), 2,059(*Am*), 2,074(*Am*), 2,075(*Am*), 2,078(*Am*), 2,079(*Am*), 2,080(*Am*), 2,081(*Am*), 2,088(*Am*), 2,095(*Am*), and 2,098(*Am*).
E25. The plant of E23, wherein the mutant ACCase is not transgenic.
E26. The plant of any one of E1 to E22, wherein said plants are not transgenic
E27. A plant according to any one E1 to E25, wherein said ACCase is encoded by a genomic nucleic acid, and comprises as its amino acid sequence a modified SEQ ID NO:2, wherein the modified sequences comprise said modification.
E28. A method for controlling growth of weeds within the vicinity of a plant of any one of E1 to E25, comprising applying to the weeds and plants an amount of an acetyl- Coenzyme A carboxylase-inhibiting herbicide that inhibits growth of a wild type plant.
E29. The method according to E28, wherein at least one herbicide is selected from the group consisting of alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, trifop, and pinoxaden or agronomically acceptable salts or esters of any of these herbicides at levels of herbicide that would normally inhibit the growth of a wild type plant.
E30. A method for controlling growth of weeds, comprising:
   a. crossing a plant of any one of E1 to E25 with other plant germplasm, and harvesting the resulting hybrid seed;
   b. planting the hybrid seed; and
   c. applying one or more acetyl-Coenzyme A carboxylase-inhibiting herbicides to the hybrid plant and to the weeds in vicinity to the hybrid plant at levels of herbicide that would normally inhibit the growth of a wild type plant.
E31. A plant cell of the plant of any one of E1 to E25.
E32. A plant part of the plant of any one of E1 to E25.
E33. A seed produced by the plant of any one of E1 to E25.
E34. A method of producing a hybrid plant, comprising breeding the plant of any one E1 to E25 with a second plant, wherein the hybrid plant exhibits increased herbicide tolerance as compared to the second plant.
E35. A food product prepared from the plant of any one of E1 to E25.
E36. A consumer product prepared from the plant of any one of E1 to E25.
E37. An industrial product prepared from the plant of any one of E1 to E25.
E38. A veterinary product prepared from the plant of any one of E1 to E25.
E39. As isolated, recombinant, or mutagenized nucleic acid molecule encoding the ACCase as described in any one E1 to E25.
E40. Use of nucleic acid molecule according to E39 as a selectable marker.
E41. A method of treating the plant of any one of E1 to E25, comprising contacting said plant with an agronomically acceptable composition.
E42. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at amino acid position 1,781(*Am*), wherein said ACCase confers upon the plant increased herbicide tolerance as compared to the corresponding wild-type variety of the plant when expressed therein.
E43. The plant of E42, wherein the amino acid at position at 1,781(*Am*) is leucine.
E44. The plant of E42, wherein the amino acid position at 1,781(*Am*) is alanine.
E45. The plant of E42, wherein the amino acid at position at 1,781(*Am*) is valine.
E46. The plant of E42, wherein the amino acid at position at 1,781(*Am*) is threonine.
E47. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at amino acid position 1,999(*Am*), wherein said ACCase confers upon the plant increased herbicide tolerance as compared to a wild-type variety of the plant when expressed therein.
E48. The plant of E47, wherein the amino acid at position at 1,999(*Am*) is glycine.
E49. The plant of E47, wherein the amino acid at position at 1,999(*Am*) is cysteine.
E50. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at amino acid position 2,027(*Am*), wherein said ACCase confers upon the plant increased herbicide tolerance as compared to a wild-type variety of the plant when expressed therein.
E51. The plant of E50, wherein the amino acid at position at 2,027(*Am*) is cysteine.
E52. The plant of E50, wherein the amino acid at position at 2,027(*Am*) is arginine.
E53. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at amino acid position 2,041(*Am*), wherein said ACCase confers upon the plant increased herbicide tolerance as compared to a wild-type variety of the plant when expressed therein.
E54. The plant of E53, wherein the amino acid at position at 2,041(*Am*) is asparagine.
E55. The plant of E53, wherein the amino acid at position 2,041(*Am*) is valine.
E56. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at amino acid position 2,096(*Am*), wherein said ACCase confers upon the plant increased herbicide tolerance as compared to a wild-type variety of the plant when expressed therein.
E57. The plant of E56, wherein the amino acid at position at 2,096(*Am*) is alanine.
E58. The plant of E56, wherein the amino acid at position at 2,096(*Am*) is serine.
E59. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type BEP clade plant at only one of the following positions: 1,785(*Am*); 1,786(*Am*); 1,811(*Am*); 2,049(*Am*); 2,074(*Am*); 2,075(*Am*), 2,078(*Am*); 2,080(*Am*); 2,088(*Am*); and 2,098(*Am*), wherein said ACCase confers upon the plant increased herbicide tolerance as compared to a wild-type variety of the plant when expressed therein.
E60. The plant of E59, wherein the plant expresses a mutagenized or recombinant acetyl-Coenzyme A carboxylase (ACCase) in which the amino acid sequence comprises only one substitution selected from the group consisting of isoleucine, leucine, or phenylalanine at 2,049(*Am*); leucine at position 2,074(*Am*); leucine, isoleucine, or methionine at position 2,075(*Am*), or duplication of position 2,075(*Am*); threonine, glycine or lysine at position 2,078(*Am*); arginine, tryptophan, phenylalanine, glycine, histidine, lysine, leucine, serine, threonine, or valine at 2,088(*Am*); and alanine, histidine, proline, serine, or glycine at 2,098(*Am*).
E61. The plant of E59, wherein the plant expresses a mutagenized or recombinant acetyl-Coenzyme A carboxylase (ACCase) in which the amino acid sequence comprises only one substitution selected from the group consisting of glycine at position 1,785(*Am*) proline at position 1,786(*Am*); and asparagine at position 1,811(*Am*).
E62. A BEP clade plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type rice BEP clade at only one of the following positions: 2,039(*Am*); 2,059(*Am*); 2,080(*Am*); and 2,095(*Am*).
E63. The plant of E62, wherein the plant expresses a mutagenized or recombinant acetyl-Coenzyme A carboxylase (ACCase) in which the amino acid sequence comprises only one substitution selected from the group consisting of glycine at position 2,039(*Am*) valine at position 2,059(*Am*); glutamic acid at position 2,080(*Am*); and glutamic acid at position 2,095(*Am*).
E64. The plant of any one of E42 to E63, wherein said plants are not transgenic.
E65. The plant of any one of E42 to E63, wherein the BEP clade plant is a BET subclade plant.
E66. The plant of E65, wherein the BET subclade plant is a BET crop plant.
E67. A method for controlling growth of weeds within the vicinity of a plant of any one of E42 to E63, comprising applying to the weeds and monocot plants an amount of an acetyl-Coenzyme A carboxylase-inhibiting herbicide that inhibits growth of a wild type plant.
E68. The method according to E67, wherein at least one herbicide is selected from the group consisting of alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, trifop, and pinoxaden or agronomically acceptable salts or esters of any of these herbicides at levels of herbicide that would normally inhibit the growth of a wild type plant.
E69. A method for controlling growth of weeds, comprising:
   a. crossing a plant of any one of E42 to E63 with other plant germplasm, and harvesting the resulting hybrid seed;
   b. planting the hybrid seed; and
   c. applying one or more acetyl-Coenzyme A carboxylase-inhibiting herbicides to the hybrid plant and to the weeds in vicinity to the hybrid plant at levels of herbicide that would normally inhibit the growth of a wild type plant.
E70. A plant cell of the plant of any one of E42 to E63.
E71. A plant part of the plant of any one of E42 to E63.
E72. A seed produced by the rice plant of any one of E42 to E63.
E73. A method of producing a hybrid plant, comprising breeding the plant of any one E42 to E63 with a second plant, wherein the hybrid plant exhibits increased herbicide tolerance as compared to the second plant.
E74. A food product prepared from the plant of any one of E42 to E63.
E75. A consumer product prepared from the plant of any one of E42 to E63.
E76. An industrial product prepared from the plant of any one of E42 to E63.
E77. A veternary product prepared from the plant of any one of E42 to E63.
E78. As isolated, recombinant, or mutagenized nucleic acid molecule encoding the ACCase as described in any one E42 to E63.
E79. Use of nucleic acid molecule according to E78 as a selectable marker.
E80. A method of treating the plant of any one of E42 to E63, comprising contacting said plant with an agronomically acceptable composition.
E81. A monocot plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type monocot plant at only one amino acid position selected from the group consisting of:
   a. amino acid at position 1,781(*Am*), wherein the amino acid at position 1,781(*Am*) is not leucine, and not a wild type amino acid;
   b. amino acid at position 1,999(*Am*), wherein the amino acid at position 1,999(*Am*) is not cysteine, and not a wild type amino acid;
   c. amino acid at position 2,027(*Am*), wherein the amino acid at position 2,027(*Am*) is not cysteine, and not a wild type amino acid;
   d. amino acid at position 2,041(*Am*), wherein the amino acid at position 2,041(*Am*) is not valine or asparagine, and not a wild type amino acid; and
   e. amino acid at position 2,096(*Am*), wherein the amino acid at position 2,096(*Am*) is not alanine, and not a wild type amino acid;
   wherein said ACCase confers upon the plant increased herbicide tolerance as compared to a wild-type variety of the plant when expressed therein.
E82. The plant of E81, wherein:
   the difference at 1,781(*Am*) is a substitution with alanine, valine, threonine; the difference at 1,999(*Am*) is a substitution with glycine; the difference at 2,027(*Am*) is arginine; and the difference at 2,096(*Am*) is a substitution with serine.
E83. A monocot plant that expresses a mutagenized or recombinant acetyl-Coenzyme A carboxylase (ACCase) in which the amino acid sequence differs from an amino acid sequence of an acetyl-Coenzyme A carboxylase of a corresponding wild-type monocot plant at only one amino acid position:
   a. selected from the group consisting of 1,785(*Am*); 1,786(*Am*); 1,811(*Am*); 2,049(*Am*); 2,074(*Am*); 2,075(*Am*), 2,078(*Am*); 2,081(*Am*); 2,088(*Am*); and 2,098(*Am*); or
   b. selected from the group consisting of 2,039(*Am*); 2,059(*Am*); 2,080(*Am*); and 2,095(*Am*).
E84. The plant of E83, wherein:
   a. the difference at position 1,785(*Am*) is a substitution with glycine; the difference at position 1,786(*Am*) is a substitution with proline; the difference at position 1,811(*Am*) is a substitution with asparagine, the difference at position 2,049(*Am*) is a substitution with phenylalanine, isoleucine, or leucine; wherein the difference at position 2,074(*Am*) is a substitution with leucine; wherein the difference at position 2,075(*Am*) is methionine, leucine, isoleucine, or a duplication of 2,075(*Am*); wherein the difference at position 2,078(*Am*) is lysine, glycine, or threonine; wherein the substitution at position 2,098(*Am*) is alanine, glycine, proline, histidine, serine or cysteine; or
   b. the difference at position 2,039(*Am*) is a substitution with glycine; the difference at position 2,059(*Am*) is a substitution with valine; the difference at position 2,080(*Am*) is a substitution with glutamic acid, or a deletion of 2,080(*Am*); and the difference at position 2,095(*Am*) is a substitution with glutamic acid.
E85. A method for controlling growth of weeds within the vicinity of a plant of any one of E81 to E84, comprising applying to the weeds and monocot plants an amount of an acetyl-Coenzyme A carboxylase-inhibiting herbicide that inhibits growth of a wild type plant.
E86. The method according to E85, wherein at least one herbicide is selected from the group consisting of alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, trifop, and pinoxaden or agronomically acceptable salts or esters of any of these herbicides at levels of herbicide that would normally inhibit the growth of a wild type plant.
E87. A method for controlling growth of weeds, comprising:
   a. crossing a plant of any one of E81 to E84 with other plant germplasm, and harvesting the resulting hybrid seed;
   b. planting the hybrid seed; and
   c. applying one or more acetyl-Coenzyme A carboxylase-inhibiting herbicides to the hybrid plant and to the weeds in vicinity to the hybrid plant at levels of herbicide that would normally inhibit the growth of a wild type plant.
E88. A plant cell of the plant of any one of E81 to E84.
E89. A plant part of the plant of any one of E81 to E84.
E90. A seed produced by the plant of any one of E81 to E84.
E91. A method of producing a hybrid plant, comprising breeding the plant of any one E81 to E84 with a second plant, wherein the hybrid plant exhibits increased herbicide tolerance as compared to the second plant.
E92. A food product prepared from the plant of any one of E81 to E84.
E93. A consumer product prepared from the plant of any one of E81 to E84.
E94. An industrial product prepared from the plant of any one of E81 to E84.
E95. A veterinary product prepared from the plant of any one of E81 to E84.
E96. An isolated, recombinant, or mutagenized nucleic acid molecule encoding the ACCase as described in any one of E81 to E84.
E97. Use of the nucleic acid according to E96 as a selectable marker.
E98. A method of treating the rice plant of any one of E81 to E84, contacting said plant with an agronomically acceptable composition.
E99. A method for selecting a transformed plant cell, the method comprising:
   a. introducing a nucleic acid molecule encoding a gene of interest into a plant cell, wherein the nucleic acid molecule further encodes a mutant acetyl-Coenzyme A carboxylase (ACCase) in which the amino acid sequence differs from an amino acid sequence of an ACCase of a corresponding wild-type plant at one amino acid position; and
   b. contacting the plant cell with an ACCase inhibitor to identify the transformed plant cell, wherein said mutant ACCase exhibits increased herbicide tolerance to said ACCase inhibitor as compared to the corresponding wild-type ACCase.
E100. A method of breeding, the method comprising:
   a. breeding a plant comprising the cell of E99 with a second plant to obtain a progeny plant; and
   b. determining whether said progeny plant expresses said mutant ACCase; wherein said mutant ACCase confers upon the progeny plant increased herbicide tolerance as compared to the second plant.
E101. The method of any one of E99 and E100, wherein the mutant ACCase comprises a substitution at only one amino acid position selected from the group consisting of: 1,781(*Am*), 1,785(*Am*), 1,786(*Am*), 1,811(*Am*), 1,824(*Am*), 1,864(*Am*), 1,999(*Am*), 2,027(*Am*), 2,039(*Am*), 2,041(*Am*), 2,049(*Am*), 2,059(*Am*), 2,074(*Am*), 2,075(*Am*), 2,078(*Am*), 2,079(*Am*), 2,080(*Am*), 2,0881(*Am*), 2,088(*Am*), 2,095(*Am*), 2,096(*Am*), and 2,098(*Am*).
E102. A rice plant wherein:
   a. growth of said plant is tolerant to acetyl-Coenzyme A carboxylase-inhibiting herbicides at levels of herbicide that would normally inhibit the growth of a rice plant;
   b. said plant is a plant of any one of lines OsHPHI2, OsARWI1, OsARWI3,
      OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively; or is a mutant, recombinant, or genetically engineered derivative of a plant of any one of lines OsHPHI2, OsARWI1, OsARWI3, OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively; or is a plant which is the progeny of any of these plants; and
   c. said plant has the herbicide tolerance characteristics of a plant of any one of lines OsHPHI2, OsARWI1, OsARWI3, OsARWI8, or OsHPHN1, a representative sample of seed of each line having been deposited with American Type Culture Collection (ATCC) under Patent Deposit Designation Number PTA-10267, PTA- 10568, PTA-10569, PTA-10570, or PTA-10571, respectively.

## Claims

1. A rice plant comprising a mutagenized rice plastidic acetyl-Coenzyme A carboxylase (ACCase) nucleic acid having a sequence obtained by an induced, random mutagenesis method and encoding a rice plastidic ACCase having, as a result of said mutagenesis, an glycine-to-serine substitution at the amino acid position corresponding to position 2,096 of the *Alopecurus myosuroides (Am)* plastidic ACCase, wherein the rice plastidic ACCase is a protein comprising a modified version of one or both of SEQ ID NOs 2 and 3.

2. The rice plant of claim 1, wherein said plastidic ACCase further comprises: an amino acid substitution at position 1,781; 1,785; 1,786; 1,811; 1,864; 2,027; 2,039; 2,041; 2,049; 2,074; 2,075; 2,078; 2,080; 2,088; 2,095; or 2,098 corresponding to the position of the *Alopecurus myosuroides (Am)* plastidic ACCase; a duplication of valine at position 2,075 *(Am);* or a deletion of the amino acid at position 2,080 (*Am*).

3. The rice plant of claim 1, further comprising:
a. a glycine substitution at position 1,785 (*Am*);
b. a proline substitution at position 1,786 (*Am*);
c. an asparagine substitution at position 1,811 (*Am*);
d. a glycine substitution at position 2,039 (*Am*);
e. an isoleucine, leucine, or phenylalanine substitution at position 2,049 (*Am*);
f. a leucine substitution at position 2,074 (*Am*);
g. a leucine, isoleucine, or methionine substitution at position 2,075 (*Am*);
h. a duplication of the valine at position 2,075 (*Am*);
i. a threonine, or glycine substitution at position 2,078 (*Am*);
j. a glutamic acid substitution at position 2,080 (*Am*);
k. an arginine, tryptophan, phenylalanine, glycine, histidine, lysine, leucine, serine, threonine, or valine substitution at position 2,088 (*Am*);
l. a glutamic acid substitution at position 2,095 (*Am*); or
m. an alanine, histidine, proline, serine, or glycine substitution at position 2,098 (*Am*)*.*

4. The rice plant of claim 1, wherein said plant is a plant of any one of lines: OsHPHI2, OsARWI1, OsARWI3, OsARWI8, or OsHPHN1, a representative sample of seed of each line having been with American Type Culture Collection (ATCC) under Patent Deposit Designation Number PTA-10267, PTA-10568, PTA-10569, PTA-10570, or PTA-10571, respectively; or is a hybrid, descendant, or genetically engineered derivative of a plant of any one of lines OsHPHI2, OsARWI1, OsARWI3, OsARWI8, or OsHPHN1.

5. The rice plant of claim 1, wherein said plant is tolerant to at least one ACCase-inhibiting herbicide selected from: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, and agronomically acceptable salts or esters thereof.

6. A rice seed or rice cell comprising a mutagenized acetyl-Coenzyme A carboxylase (ACCase) nucleic acid having a sequence obtained by an induced, random mutagenesis method and encoding a rice plastidic ACCase having, as a result of said mutagenesis, an glycine-to-serine substitution at an amino acid position corresponding to position 2,096 of the *Alopecurus myosuroides (Am)* plastidic ACCase, wherein the rice plastidic ACCase is a protein comprising a modified version of one or both of SEQ ID NOs 2 and 3.

7. The rice seed or rice cell of claim 6, wherein said plastidic ACCase further comprises:
an amino acid substitution at position 1,781; 1,785; 1,786; 1,811; 1,864; 2,027; 2,039; 2,041; 2,049; 2,074; 2,075; 2,078; 2,080; 2,088; 2,095; or 2,098 corresponding to the position of the *Alopecurus myosuroides (Am)* plastidic ACCase; a duplication of valine at position 2,075 *(Am);* or a deletion of the amino acid at position 2,080 (*Am*).

8. The rice seed or rice cell of claim 6, further comprising:
a. a glycine substitution at position 1,785 (*Am*);
b. a proline substitution at position 1,786 (*Am*);
c. an asparagine substitution at position 1,811 (*Am*);
d. a glycine substitution at position 2,039 (*Am*);
e. an isoleucine, leucine, or phenylalanine substitution at position 2,049 (*Am*);
f. a leucine substitution at position 2,074 (*Am*);
g. a leucine, isoleucine, or methionine substitution at position 2,075 (*Am*);
h. a duplication of the valine at position 2,075 (*Am*);
i. a threonine, or glycine substitution at position 2,078 (*Am*);
j. a glutamic acid substitution at position 2,080 (*Am*);
k. an arginine, tryptophan, phenylalanine, glycine, histidine, lysine, leucine, serine, threonine, or valine substitution at position 2,088 (*Am*);
l. a glutamic acid substitution at position 2,095 (*Am*); or
m. an alanine, histidine, proline, serine, or glycine substitution at position 2,098 (*Am*)*.*

9. The rice seed or rice cell of claim 6, wherein said plant is a plant of any one of lines:
OsHPHI2, OsARWI1, OsARWI3, OsARWI8, or OsHPHN1, a representative sample of seed of each line having been with American Type Culture Collection (ATCC) under Patent Deposit Designation Number PTA-10267, PTA-10568, PTA-10569, PTA-10570, or PTA-10571, respectively; or is a hybrid, descendant, or genetically engineered derivative of a plant of any one of lines OsHPHI2, OsARWI1, OsARWI3, OsARWI8, or OsHPHN1.

10. The rice seed or rice cell of claim 6, wherein said plant is tolerant to at least one ACCase-inhibiting herbicide selected from: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, and agronomically acceptable salts or esters thereof.

11. A rice plastidic ACCase as described in any one of claims 1-10, wherein said rice plastidic ACCase is non-transgenic.

12. The plant or seed of any one of claims 1-10, wherein said plant or seed is non-transgenic.

13. A method for growing a herbicide-tolerant rice plant, said method comprising:
a) growing the plant of any one of claims 1-5 or 12, or growing a plant from the rice seed of anyone of claims 6-10 or 12 by allowing the seed to sprout; and
b) applying to the plant and the vicinity thereof an acetyl-Coenzyme A carboxylase-inhibiting herbicide in an amount that inhibits growth of a corresponding wild type plant.

14. The method of claim 13, wherein said ACCase-inhibiting herbicide comprises at least one of: alloxydim, butroxydim, clethodim, cloproxydim, cycloxydim, sethoxydim, tepraloxydim, tralkoxydim, chlorazifop, clodinafop, clofop, diclofop, fenoxaprop, fenoxaprop-P, fenthiaprop, fluazifop, fluazifop-P, haloxyfop, haloxyfop-P, isoxapyrifop, propaquizafop, quizalofop, quizalofop-P, quizalofop-P-ethyl, quizalofop-P-tefuryl, trifop, pinoxaden, or an agronomically acceptable salt or ester thereof.

15. Use of a plant of any one of claims 1-5 or a seed of any one of claims 6-10 for producing a food product, a consumer product, an industrial product or a veterinary product.

16. An isolated nucleic acid comprising a nucleic acid sequence as described in any one of claims 1-12.

17. A method for identifying a plant of any one of claims 1-5 or 12 or a seed of any one of claims 6-10 or 12, comprising:
a) providing biological material from a plant or a seed;
b) performing PCR or hybridization testing of the ACCase genes in said biological material to determine if the biological material comprises an ACCase nucleic acid as described in any one of claims 1-12; and
c) identifying, based on the results of step b), that the plant of step a) comprises the nucleic acid as described in any one of claims 1-12.
